(19) Europäisches Patentamt
European Patent Office
Office européen des brevets

(11) EP 3 947 378 B1

(12) # EUROPEAN PATENT SPECIFICATION

(45) Date of publication and mention of the grant of the patent:
**13.11.2024 Bulletin 2024/46**

(21) Application number: **20715295.0**

(22) Date of filing: **24.03.2020**

(51) International Patent Classification (IPC):
***C07D 471/04*** *(2006.01)* ***A61P 31/18*** *(2006.01)*
***A61K 31/662*** *(2006.01)*

(52) Cooperative Patent Classification (CPC):
**C07D 471/04; A61P 31/18**

(86) International application number:
**PCT/EP2020/058064**

(87) International publication number:
**WO 2020/200900 (08.10.2020 Gazette 2020/41)**

(54) **NOVEL COMPOUNDS AND PHARMACEUTICAL COMPOSITIONS THEREOF FOR THE TREATMENT OF INFLAMMATORY DISORDERS**

NEUARTIGE VERBINDUNGEN UND PHARMAZEUTISCHE ZUSAMMENSETZUNGEN DAVON ZUR BEHANDLUNG VON ENTZÜNDUNGSERKRANKUNGEN

NOUVEAUX COMPOSÉS ET COMPOSITIONS PHARMACEUTIQUES DE CEUX-CI POUR LE TRAITEMENT DE TROUBLES INFLAMMATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **29.03.2019 GB 201904373**

(43) Date of publication of application:
**09.02.2022 Bulletin 2022/06**

(73) Proprietor: **Galapagos N.V.**
**2800 Mechelen (BE)**

(72) Inventors:
- **MAMMOLITI, Oscar**
  **2800 Mechelen (BE)**
- **JARY, Hélène, Marie**
  **93230 Romainville (FR)**
- **ORSULIC, Mislav**
  **10000 Zagreb (HR)**
- **VRBAN, Denana**
  **10000 Zagreb (HR)**
- **KOMAC, Marijana**
  **10000 Zagreb (HR)**
- **BRYS, Reginald, Christophe, Xavier**
  **2800 Mechelen (BE)**
- **AKKARI, Rhalid**
  **93230 Romainville (FR)**

(74) Representative: **Henry, Neil**
**Galapagos NV**
**Generaal De Wittelaan, L11 A3**
**2800 Mechelen (BE)**

(56) References cited:
**WO-A1-2012/129258 WO-A1-2017/009798**
**WO-A1-2018/083085**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 947 378 B1

## Description

### FIELD OF THE INVENTION

The present invention relates to compounds that may be useful in the prophylaxis and/or treatment of inflammatory diseases, autoimmune diseases, pain, fibrosis and/or proliferative diseases. In particular, the compounds of the invention may inhibit Interleukin-1 Receptor Associated Kinases (IRAKs), a family of kinases that are involved in inflammatory diseases, autoimmune diseases, pain, fibrosis and/or proliferative diseases, and more particularly IRAK-4. The present invention also provides methods for the production of the compounds of the invention, pharmaceutical compositions comprising the compounds of the invention, methods for the prophylaxis and/or treatment of inflammatory diseases, autoimmune diseases, pain, fibrosis and/or proliferative diseases by administering the compounds of the invention.

### BACKGROUND OF THE INVENTION

Kinases are involved in many essential processes of cell physiology, for example protein phosphorylation. In particular, protein and lipid kinases are involved in the activation, growth, differentiation, and survival of cells. Protein kinases can be divided between those preferentially phosphorylating tyrosine residues, and those preferentially phosphorylating serine and/or threonine residues.

Over the years, kinases have grown to become very important targets for the development of anti-inflammatory drugs (Cohen, 2009). In particular, IRAK kinases, and more particularly IRAK-4 have been identified as playing a role in inflammation and autoimmune diseases (Ringwood and Li, 2008, Wang et al., 2009).

IRAKs are expressed in many cell types and mediate signals from various cell receptors including interleukin-1 (IL-1) and toll-like receptors (TLRs). In the IRAK family, 4 members have been identified namely IRAK 1-4 (Wang et al., 2009), and IRAK-4, the newest member of the family represents an attractive therapeutic target (Li et al., 2002). Indeed, IRAK-4 is believed to be the key protein kinase activated early downstream of the IL-1 receptor and TLRs (except TLR3), initiating signaling via rapid activation of IRAK-1 and IRAK-2, leading to innate immune responses. Also, other interleukins, such as IL-18 and IL-33, are dependent on IRAK-4 for signaling. As such, diseases for which these cytokines are involved in the pathogenic process (*e.g.*, fibrosis (Li et al., 2014; McHedlidze et al., 2013; Rankin et al., 2010) and atopic dermatitis (Salimi et al., 2013)) are potential target diseases for treatment by IRAK-4 inhibitors.

In mice expressing an inactive IRAK-4 mutant instead of wild type, complete resistance to septic shock triggered by several TLR agonists as well as impaired response to IL-1 is observed. Furthermore, mice expressing an inactive IRAK-4 mutant instead of wild type are partially protected in several models of auto-immune diseases, such as rheumatoid arthritis (Koziczak-Holbro et al., 2009) and multiple sclerosis (Staschke et al., 2009). Interestingly, the serum of rheumatoid arthritis and systemic lupus erythematosus patients has been shown to activate plasmacytoid dendritic cells in an IRAK-4 dependent manner (Chiang et al., 2011). Finally, recurring pyogenic bacterial infection has been observed in children suffering from genetic defects leading to IRAK-4 inactivity. As these pyogenic infections are not observed in adults carrying inactivating IRAK-4 mutations, the IRAK-4 signaling system appears to be redundant for certain aspects of adult innate immunity.

The dysregulation of signaling components of the innate immune system is also increasingly being recognized as an important factor in cancer initiation and progression (Rhyasen and Starczynowski, 2015). Indeed, there is evidence that IL-1 plays a direct role in tumor cell growth, angiogenesis, invasion, drug resistance, and metastasis (Carmi et al., 2013; Vidal-Vanaclocha et al., 2000). Additionally, TLRs are involved in a multitude of protumor responses, depending on the tumor cell context. As essential mediators of IL-1 receptor and TLRs signaling, IRAK family kinases represent promising cancer drug targets. In addition, several cancer types have been shown to be dependent on activated forms of MYD88, an adaptor molecule downstream of the TLR and IL-1R, which activates IRAK-4. Activating MYD88 mutations have been identified in *e.g.,* diffuse large B-cell lymphomas (DLBCL) (Ngo et al., 2011), and in Waldenstrom macroglobulinemia (Treon et al., 2012). Another report supports the role of IRAK-4 in the field of oncology, T-cell acute lymphoblastic leukemia (T-ALL) in particular (Li et al., 2015). The pharmacological inhibition of IRAK-4 has been shown to enhance the sensitivity of T-ALL to chemotherapeutic agents.

IL-33 has been shown to play a role in the development of fibrotic and allergic diseases, asthma and atopic dermatitis in particular (Nabe, 2014). As this cytokine signals through an IRAK-4 dependent pathway (Kroeger et al., 2009), these diseases might also represent a target for IRAK-4 inhibitors.

Finally, several auto-inflammatory diseases have been shown to be dependent on IL-1 activity and, as a consequence, IL-1 blocking biologicals show some benefit to these patients. Gout, juvenile idiopathic arthritis, Muckle-Wells disease, familial Mediterranean fever, Behçet's disease, adult onset Still's disease are examples of such auto-inflammatory diseases (Dinarello et al., 2012).

The inhibition of cytokine signaling with small molecules may help in reducing disease outcome in immune-inflammatory diseases (Sundberg et al., 2014). In particular, cytokines may play a role in the defense of organisms

against pathogens and infections. However, when developing new therapies for immune-inflammatory diseases, it is crucial on one hand to select a target involved in a pathway that can be inhibited without compromising the adaptive and/or innate immune responses since the simultaneous inhibition of multiple cytokine response pathways may excessively weaken the immune system. However, drug selectivity towards kinases is difficult to achieve (Bain et al., 2003; Fabian et al., 2005), but is highly desirable in order to avoid off-target associated side effects, particularly in the context of chronic treatments (Broekman et al., 2011; Dy and Adjei, 2013; Force and Kolaja, 2011).

In particular, it was recently shown that concomitant use of an IL-1 blocking agent (Anakinra) and a TNF$\alpha$ blocker (Etanercept) resulted in increased risk of neutropenia and infection. (Genovese et al., 2004, 2003). This finding highlights that selectivity is a crucial element when developing new medicines, and therefore, it would be desirable to develop compounds that are able to selectively modulate a signaling pathway without affecting others, in particular compounds able to selectively modulate IL-1 response, without affecting TNF$\alpha$ signaling pathways.

The current therapies are not satisfactory and therefore there remains a need to identify further compounds with reduced off-target related side effects that may be of use in the prophylaxis and/or treatment of inflammatory diseases, autoimmune diseases and/or proliferative diseases.

WO2012/129258 discloses amizopyrazole compounds that are described as being inhibitors of IRAKs. WO2018/083085 discloses pyrazolo[1,5a]pyrimidine compounds that are described as being useful for inhibition of IRAKs. WO2017/009798 discloses indazole and azaindazole compounds that are described as being useful for inhibition of IRAKs.

## SUMMARY OF THE INVENTION

The present invention relates to compounds that may be useful in the prophylaxis and/or treatment of inflammatory diseases, autoimmune diseases, pain, fibrosis and/or proliferative diseases. In particular, the compounds of the invention may inhibit Interleukin-1 Receptor Associated Kinases (IRAKs), a family of kinases that are involved in inflammatory diseases, autoimmune diseases, pain, fibrosis and/or proliferative diseases, and more particularly IRAK-4. The present invention also provides methods for the production of the compounds of the invention, pharmaceutical compositions comprising the compounds of the invention, methods for the prophylaxis and/or treatment of inflammatory diseases, autoimmune diseases, pain, fibrosis and/or proliferative diseases by administering the compound of the invention.

Accordingly, in a first aspect of the invention, the compounds of the invention are provided having a Formula I:

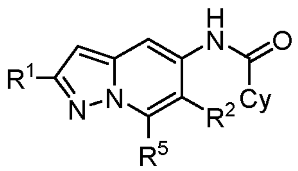

I

wherein

$R^1$ is

a) $C_{2-6}$ alkyl substituted with one or more independently selected -OH, -CN, $C_{1-4}$ alkoxy, halo, or $-S(=O)_2-C_{1-4}$ alkyl, or
b) 6 membered heterocycloalkyl comprising one or two independently selected S, N, or O atoms, which heterocycloalkyl is unsubstituted or substituted with one or more independently selected oxo, halo, or $C_{1-4}$ alkyl, which alkyl is unsubstituted or substituted with one or more halo;

$R^2$ is

a) $C_{1-4}$ alkoxy which alkoxy is unsubstituted or substituted with one or more independently selected halo or -OH,
b) $-O-C_{3-4}$ cycloalkyl, which cycloalkyl is unsubstituted or substituted with one or more independently selected halo or -OH, or
c) $-C(=O)NR^{3a}R^{3b}$;

Cy is 6 membered heteroaryl, comprising 1 or 2 N atoms, substituted with one or two independently selected $R^4$ substituents;
Each $R^{3a}$ and $R^{3b}$ is independently selected from

a) H,
b) $C_{1-4}$ alkyl, which alkyl is unsubstituted or substituted with one or more independently selected halo, -OH, -CN, $C_{1-4}$ alkoxy, or $C_{3-7}$ cycloalkyl, which cycloalkyl is unsubstituted or substituted with one or more independently selected halo,
c) $C_{3-6}$ cycloalkyl which cycloalkyl is unsubstituted or substituted with one or more independently selected oxo, -OH, -CN, $C_{1-4}$ alkyl, $C_{1-4}$ alkoxy, or halo, or
d) 4-6 membered heterocycloalkyl comprising one or two independently selected N, S, or O atoms, which heterocycloalkyl is unsubstituted or substituted with one or more independently selected oxo, -OH, -CN, $C_{1-4}$ alkyl, $C_{1-4}$ alkoxy, or halo;

$R^{3a}$ and $R^{3b}$ together with N atom to which they are attached may form a 4-6 membered monocyclic heterocycloalkyl;
Each $R^4$ is independently

a) oxo,
b) -OH,
c) -CN,
d) halo,
e) $C_{1-4}$ alkyl unsubstituted or substituted with one or more independently selected halo, -OH, or -CN,
f) $C_{1-4}$ alkoxy unsubstituted or substituted with one or more independently selected halo, -OH, or - CN, or
g) $C_{3-7}$ cycloalkyl unsubstituted or substituted with one or more independently selected halo, -OH or -CN; and

$R^5$ is selected from H, halo, $-CH_3$ or $-CF_3$.

In one aspect, the compounds of the invention are provided for use in the prophylaxis and/or treatment of inflammatory diseases, autoimmune diseases, pain, fibrosis and/or proliferative diseases. In a particular aspect, the compounds of the invention may inhibit the IRAK kinase family members, and more particularly IRAK-4.

In a further aspect, the compounds of the invention may exhibit good metabolic stability, and good half-life, which may result in lower dosage regimen. In a particular aspect, the compounds of the invention show good stability in hepatocytes, which may result in low hepatic clearance.

In yet another aspect, the compounds of the invention may show good solubility, in particular thermodynamic solubility, which may result in improved manufacturability.

In yet a further aspect, the compounds of the invention may show selectivity towards IRAK-4, which may result in improved safety and lower off-target related side effects.

In a further aspect, the present invention provides pharmaceutical compositions comprising a compound of the invention, and a pharmaceutical carrier, excipient or diluent. In a particular aspect, the pharmaceutical composition may additionally comprise further therapeutically active ingredients suitable for use in combination with the compounds of the invention. In a more particular aspect, the further therapeutically active ingredient is an agent for the prophylaxis and/or treatment of inflammatory diseases, autoimmune diseases, pain, fibrosis and/or proliferative diseases.

Moreover, the compounds of the invention, useful in the pharmaceutical compositions and treatment methods disclosed herein, are pharmaceutically acceptable as prepared and used.

In a further aspect of the invention, this invention provides a method of treating a mammal, in particular humans, afflicted with a condition selected from among those listed herein, and particularly inflammatory diseases, autoimmune diseases, pain, fibrosis and/or proliferative diseases, which method comprises administering an effective amount of the pharmaceutical composition or compounds of the invention as described herein.

The present invention also provides pharmaceutical compositions comprising a compound of the invention, and a suitable pharmaceutical carrier, excipient or diluent for use in medicine. In a particular aspect, the pharmaceutical composition is for use in the prophylaxis and/or treatment of inflammatory diseases, autoimmune diseases, pain, fibrosis and/or proliferative diseases .

In additional aspects, this invention provides methods for synthesizing the compounds of the invention, with representative synthetic protocols and pathways disclosed later on herein.

Other objects and advantages will become apparent to those skilled in the art from a consideration of the ensuing detailed description.

## DETAILED DESCRIPTION OF THE INVENTION

### Definitions

The following terms are intended to have the meanings presented therewith below and are useful in understanding the description and intended scope of the present invention.

When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein.

The articles 'a' and 'an' may be used herein to refer to one or to more than one (*i.e.* at least one) of the grammatical objects of the article. By way of example 'an analogue' means one analogue or more than one analogue.

'Alkyl' means straight or branched aliphatic hydrocarbon having the specified number of carbon atoms. Particular alkyl groups have 1 to 6 carbon atoms or 1 to 4 carbon atoms. Branched means that one or more alkyl groups such as methyl, ethyl or propyl is attached to a linear alkyl chain. Particular alkyl groups are methyl ($-CH_3$), ethyl ($-CH_2-CH_3$), n-propyl ($-CH_2-CH_2-CH_3$), isopropyl ($-CH(CH_3)_2$), n-butyl ($-CH_2-CH_2-CH_2-CH_3$), tert-butyl ($-CH_2-C(CH_3)_3$), sec-butyl ($-CH(CH_3)-CH_2-CH_3$), n-pentyl ($-CH_2-CH_2-CH_2-CH_2-CH_3$), n-hexyl ($-CH_2-CH_2-CH_2-CH_2-CH_2-CH_3$), and 1,2-dimethylbutyl ($-CHCH_3)-C(CH_3)H-CH_2-CH_3$). Particular alkyl groups have between 1 and 4 carbon atoms.

'Alkenyl' refers to monovalent olefinically (unsaturated) hydrocarbon groups with the number of carbon atoms specified. Particular alkenyl has 2 to 8 carbon atoms, and more particularly, from 2 to 6 carbon atoms, which can be straight-chained or branched and having at least 1 and particularly from 1 to 2 sites of olefinic unsaturation. Particular alkenyl groups include ethenyl ($-CH=CH_2$), n-propenyl ($-CH_2CH=CH_2$), isopropenyl ($-C(CH_3)=CH_2$) and the like.

'Alkylene' refers to divalent alkene radical groups having the number of carbon atoms specified, in particular having 1 to 6 carbon atoms and more particularly 1 to 4 carbon atoms which can be straight-chained or branched. This term is exemplified by groups such as methylene ($-CH_2-$), ethylene ($-CH_2-CH_2-$), or $-CH(CH_3)-$ and the like.

'Alkynylene' refers to divalent alkyne radical groups having the number of carbon atoms and the number of triple bonds specified, in particular 2 to 6 carbon atoms and more particularly 2 to 4 carbon atoms which can be straight-chained or branched. This term is exemplified by groups such as $-C{\equiv}C-$, $-CH_2-C{\equiv}C-$, and $-C(CH_3)H-C{\equiv}CH-$.

'Alkoxy' refers to the group O-alkyl, where the alkyl group has the number of carbon atoms specified. In particular the term refers to the group $-O-C_{1-6}$ alkyl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, i.e. with between 1 and 6 carbon atoms. Further particular alkoxy groups have between 1 and 4 carbon atoms.

'Amino' refers to the radical $-NH_2$.

'Aryl' refers to a monovalent aromatic hydrocarbon group derived by the removal of one hydrogen atom from a single carbon atom of a parent aromatic ring system. In particular aryl refers to an aromatic ring structure, monocyclic or fused polycyclic, with the number of ring atoms specified. Specifically, the term includes groups that include from 6 to 10 ring members. Particular aryl groups include phenyl, and naphthyl.

'Cycloalkyl'refers to a non-aromatic hydrocarbyl ring structure, monocyclic, fused polycyclic, bridged polycyclic, or spirocyclic, with the number of ring atoms specified. A cycloalkyl may have from 3 to 12 carbon atoms, in particular from 3 to 10, and more particularly from 3 to 7 carbon atoms. Such cycloalkyl groups include, by way of example, single ring structures such as cyclopropyl, cyclobutyl, cyclopentyl, cyclohexyl, and cycloheptyl.

'Cyano' refers to the radical -CN.

'Halo' or 'halogen' refers to fluoro (F), chloro (Cl), bromo (Br) and iodo (I). Particular halo groups are either fluoro or chloro.

'Hetero' when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, e.g. heteroalkyl, cycloalkyl, *e.g.* heterocycloalkyl, aryl, *e.g.* heteroaryl, and the like having from 1 to 4, and particularly from 1 to 3 heteroatoms, more typically 1 or 2 heteroatoms, for example a single heteroatom.

'Heteroaryl' means an aromatic ring structure, monocyclic or fused polycyclic, that includes one or more heteroatoms independently selected from O, N and S and the number of ring atoms specified. In particular, the aromatic ring structure may have from 5 to 9 ring members. The heteroaryl group can be, for example, a five membered or six membered monocyclic ring or a fused bicyclic structure formed from fused five and six membered rings or two fused six membered rings or, by way of a further example, two fused five membered rings. Each ring may contain up to four heteroatoms typically selected from nitrogen, sulphur and oxygen. Typically the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of five membered monocyclic heteroaryl groups include but are not limited to pyrrolyl, furanyl, thiophenyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered monocyclic heteroaryl groups include but are not limited to pyridinyl, pyrazinyl, pyridazinyl, pyrimidinyl and triazinyl.

Particular examples of bicyclic heteroaryl groups containing a five membered ring fused to another five-membered ring include but are not limited to imidazothiazolyl and imidazoimidazolyl.

Particular examples of bicyclic heteroaryl groups containing a six membered ring fused to a five membered ring include but are not limited to benzofuranyl, benzothiophenyl, benzoimidazolyl, benzoxazolyl, isobenzoxazolyl, benzisoxazolyl, benzothiazolyl, benzoisothiazolyl, isobenzofuranyl, indolyl, isoindolyl, indolizinyl, purinyl (*e.g.* adenine, guanine), indazolyl, pyrazolopyrimidinyl, triazolopyrimidinyl, and pyrazolopyridinyl groups.

Particular examples of bicyclic heteroaryl groups containing two fused six membered rings include but are not limited to quinolinyl, isoquinolinyl, pyridopyridinyl, quinoxalinyl, quinazolinyl, cinnolinyl, phthalazinyl, naphthyridinyl, and pteridinyl groups. Particular heteroaryl groups are those derived from thiophenyl, pyrrolyl, benzothiophenyl, benzofuranyl, indolyl, pyridinyl, quinolinyl, imidazolyl, oxazolyl and pyrazinyl.

Examples of representative heteroaryls include the following:

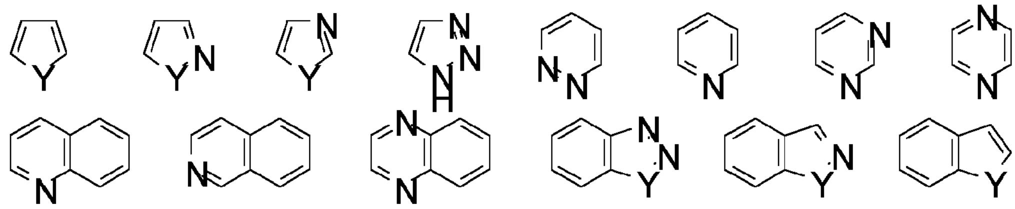

wherein each Y is selected from >C=O, NH, O and S.

'Heterocycloalkyl' means a non-aromatic fully saturated ring structure, monocyclic, fused polycyclic, spirocyclic, or bridged polycyclic, that includes one or more heteroatoms independently selected from O, N and S and the number of ring atoms specified. The heterocycloalkyl ring structure may have from 4 to 12 ring members, in particular from 4 to 10 ring members and more particularly from 4 to 7 ring members. Each ring may contain up to four heteroatoms typically selected from nitrogen, sulphur and oxygen. Typically the heterocycloalkyl ring will contain up to 4 heteroatoms, more typically up to 3 heteroatoms, more usually up to 2, for example a single heteroatom. Examples of heterocyclic rings include, but are not limited to azetidinyl, oxetanyl, thietanyl, pyrrolidinyl (*e.g.* 1-pyrrolidinyl, 2-pyrrolidinyl and 3-pyrrolidinyl), tetrahydrofuranyl (*e.g.* 1-tetrahydrofuranyl, 2-tetrahydrofuranyl and 3-tetrahydrofuranyl), tetrahydrothiophenyl (*e.g.* 1-tetrahydrothiophenyl, 2-tetrahydrothiophenyl and 3-tetrahydrothiophenyl), piperidinyl (*e.g.* 1-piperidinyl, 2-piperidinyl, 3-piperidinyl and 4-piperidinyl), tetrahydropyranyl (*e.g.* 4-tetrahydropyranyl), tetrahydrothiopyranyl (*e.g.* 4-tetrahydrothiopyranyl), morpholinyl, thiomorpholinyl, dioxanyl, or piperazinyl.

As used herein, the term 'heterocycloalkenyl' means a 'heterocycloalkyl', which comprises at least one double bond. Particular examples of heterocycloalkenyl groups are shown in the following illustrative examples:

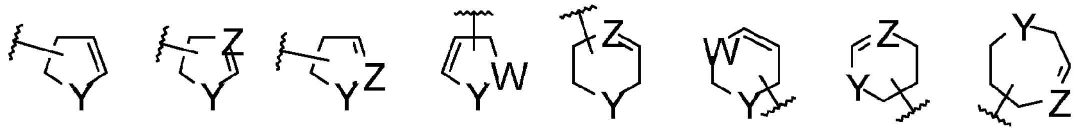

wherein each W is selected from $CH_2$, NH, O and S; each Y is selected from NH, O, C(=O), $SO_2$, and S; and each Z is selected from N or CH.

Particular examples of monocyclic rings are shown in the following illustrative examples:

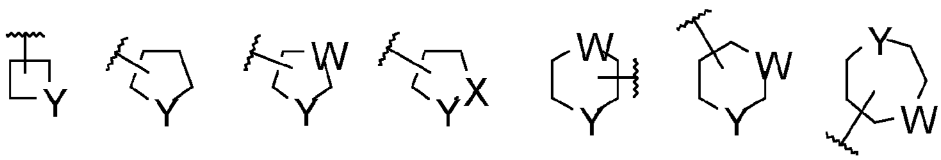

wherein each W and Y is independently selected from $-CH_2-$, -NH-, -O- and -S-.

Particular examples of fused bicyclic rings are shown in the following illustrative examples:

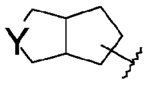 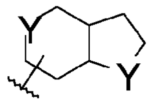 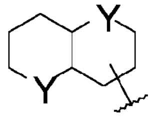 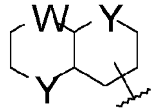

wherein each W and Y is independently selected from $-CH_2-$, -NH-, -O- and -S-.

Particular examples of bridged bicyclic rings are shown in the following illustrative examples:

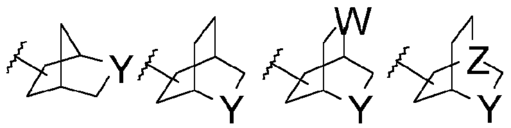

wherein each W and Y is independently selected from $-CH_2-$, -NH-, -O- and -S- and each Z is selected from N or CH.

Particular examples of spirocyclic rings are shown in the following illustrative examples:

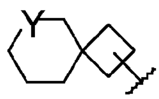 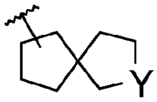 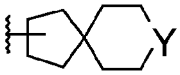

wherein each Y is selected from $-CH_2-$, -NH-, -O- and -S-.

'Hydroxyl' refers to the radical -OH.

'Oxo' refers to the radical =O.

'Substituted' refers to a group in which one or more hydrogen atoms are each independently replaced with the same or different substituent(s).

'Sulfo' or 'sulfonic acid' refers to a radical such as $-SO_3H$.

'Thiol' refers to the group -SH.

As used herein, term 'substituted with one or more' refers to one to four substituents. In one embodiment it refers to one to three substituents. In further embodiments it refers to one or two substituents. In a yet further embodiment it refers to one substituent.

'Thioalkoxy' refers to the group -S-alkyl where the alkyl group has the number of carbon atoms specified. In particular the term refers to the group $-S-C_{1-6}$ alkyl. Particular thioalkoxy groups are thiomethoxy, thioethoxy, n-thiopropoxy, isothiopropoxy, n-thiobutoxy, tert-thiobutoxy, sec-thiobutoxy, n-thiopentoxy, n-thiohexoxy, and 1,2-dimethylthiobutoxy. Particular thioalkoxy groups are lower thioalkoxy, *i.e.* with between 1 and 6 carbon atoms. Further particular alkoxy groups have between 1 and 4 carbon atoms.

One having ordinary skill in the art of organic synthesis will recognize that the maximum number of heteroatoms in a stable, chemically feasible heterocyclic ring, whether it is aromatic or non-aromatic, is determined by the size of the ring, the degree of unsaturation and the valence of the heteroatoms. In general, a heterocyclic ring may have one to four heteroatoms so long as the heteroaromatic ring is chemically feasible and stable.

'Pharmaceutically acceptable' means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

'Pharmaceutically acceptable salt' refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.* an alkali metal ion, an alkaline earth ion, or an aluminum ion; or coordinates with an organic base such as ethanolamine, diethanolamine, triethanolamine, N-methylglucamine and the like. Salts further include, by way of example

only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term 'pharmaceutically acceptable cation' refers to an acceptable cationic counter-ion of an acidic functional group. Such cations are exemplified by sodium, potassium, calcium, magnesium, ammonium, tetraalkylammonium cations, and the like.

'Pharmaceutically acceptable vehicle' refers to a diluent, adjuvant, excipient or carrier with which a compound of the invention is administered.

'Prodrugs' refers to compounds, including derivatives of the compounds of the invention, which have cleavable groups and become by solvolysis or under physiological conditions the compounds of the invention which are pharmaceutically active *in vivo.* Such examples include, but are not limited to, choline ester derivatives and the like, N-alkylmorpholine esters and the like.

'Solvate' refers to forms of the compound that are associated with a solvent, usually by a solvolysis reaction. This physical association includes hydrogen bonding. Conventional solvents include water, EtOH, acetic acid and the like. The compounds of the invention may be prepared *e.g.* in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. 'Solvate' encompasses both solution-phase and isolable solvates. Representative solvates include hydrates, ethanolates and methanolates.

'Subject' includes humans. The terms 'human', 'patient' and 'subject' are used interchangeably herein.

'Effective amount' means the amount of a compound of the invention that, when administered to a subject for treating a disease, is sufficient to effect such treatment for the disease. The "effective amount" can vary depending on the compound, the disease and its severity, and the age, weight, etc., of the subject to be treated.

'Preventing' or 'prevention' refers to a reduction in risk of acquiring or developing a disease or disorder (*i.e.* causing at least one of the clinical symptoms of the disease not to develop in a subject that may be exposed to a disease-causing agent, or predisposed to the disease in advance of disease onset.

The term 'prophylaxis' is related to 'prevention', and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

'Treating' or 'treatment' of any disease or disorder refers, in one embodiment, to ameliorating the disease or disorder (*i.e.* arresting the disease or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease or disorder, either physically, (*e.g.* stabilization of a discernible symptom), physiologically, (*e.g.* stabilization of a physical parameter), or both. In a further embodiment, "treating" or "treatment" relates to slowing the progression of the disease.

As used herein the term 'allergic disease(s)' refers to the group of conditions characterized by a hypersensitivity disorder of the immune system including, allergic airway disease (*e.g.*, asthma, rhinitis), atopic dermatitis, sinusitis, eczema and hives, as well as food allergies or allergies to insect venom.

As used herein the term 'asthma' as used herein refers to any disorder of the lungs characterized by variations in pulmonary gas flow associated with airway constriction of whatever cause (intrinsic, extrinsic, or both; allergic or non-allergic). The term asthma may be used with one or more adjectives to indicate the cause.

As used herein the term 'inflammatory disease(s)' refers to the group of conditions including rheumatoid arthritis, osteoarthritis, juvenile idiopathic arthritis, psoriasis, psoriatic arthritis, ankylosing spondylitis, allergic airway disease (*e.g.*, asthma, rhinitis), chronic obstructive pulmonary disease (COPD), inflammatory bowel diseases (IBD, *e.g.,* Crohn's disease, ulcerative colitis), irritable bowel syndrome, endotoxin-driven disease states (*e.g.*, complications after bypass surgery or chronic endotoxin states contributing to *e.g.,* chronic cardiac failure), adult-onset Still's disease, Muckle-Wells syndrome, familial cold auto inflammatory syndrome (FCAS), Behçet's disease, Cryopyrin-associated periodic syndrome (CAPS), familial Mediterranean fever (FMF), gout, neonatal onset multisystem inflammatory disease (NOMID), Schnitzler syndrome, and related diseases involving cartilage, such as that of the joints. Particularly the term refers to rheumatoid arthritis, juvenile idiopathic arthritis, psoriasis, osteoarthritis, allergic airway disease (*e.g.*, asthma), chronic obstructive pulmonary disease (COPD) and inflammatory bowel diseases. More particularly the term refers to rheumatoid arthritis, juvenile idiopathic arthritis, psoriasis, chronic obstructive pulmonary disease (COPD) and inflammatory bowel diseases.

As used herein the term 'autoimmune disease(s)' refers to the group of diseases including obstructive airways disease, including conditions such as COPD, asthma (*e.g.*, intrinsic asthma, extrinsic asthma, dust asthma, infantile asthma) particularly chronic or inveterate asthma (for example late asthma and airway hyperresponsiveness), bronchitis, including bronchial asthma, systemic lupus erythematosus (SLE), cutaneous lupus erythematosus, lupus nephritis, dermatomyositis, Sjögren's syndrome, multiple sclerosis, psoriasis, dry eye disease, type I diabetes mellitus and complications associated therewith, atopic eczema (atopic dermatitis), hidradenitis suppurativa (HS), thyroiditis (Hashimoto's and autoimmune thyroiditis), contact dermatitis and further eczematous dermatitis, inflammatory bowel disease (e.g., Crohn's disease and ulcerative colitis), atherosclerosis and amyotrophic lateral sclerosis. Particularly the term refers to COPD, asthma, systemic lupus erythematosus, type I diabetes mellitus, atopic dermatitis and inflammatory bowel disease.

As used herein, the term 'pain' refers to diseases or disorders characterized by unpleasant feeling often caused by intense or damaging stimuli, and include but is not limited to nociceptive pain (for example visceral pain, and/or somatic pain), inflammatory pain (associated with tissue damage and inflammatory cell infiltration) and neuropathic or dysfunctional pain (caused by damage to or abnormal function of the nervous system), and/or pain associated or caused by the conditions mentioned herein. Pain can be acute or chronic. In a particular, the term refers to inflammatory and/or neuropathic pain.

As used herein, the term 'fibrosis' refers to systemic sclerosis, idiopathic pulmonary fibrosis and other forms of lung fibrosis and interstitial lung diseases, alcoholic steatohepatitis, non-alcoholic steatohepatitis, renal fibrosis, and fibrosis of the colon as a consequence of inflammatory bowel diseases. In a particular, the term refers to sclerodermatous chronic graft versus host disease.

As used herein the term 'proliferative disease(s)' refers to conditions such as cancer (*e.g.*, uterine leiomyosarcoma or prostate cancer), myeloproliferative disorders (*e.g.*, polycythemia vera, essential thrombocytosis and myelofibrosis), leukemia (*e.g.*, acute myeloid leukemia, acute and chronic lymphoblastic leukemia), multiple myeloma, psoriasis, restenosis, scleroderma or fibrosis. In particular the term refers to cancer, leukemia, multiple myeloma and psoriasis.

As used herein, the term 'cancer' refers to a malignant or benign growth of cells in skin or in body organs, for example but without limitation, breast, prostate, lung, kidney, pancreas, stomach or bowel. A cancer tends to infiltrate into adjacent tissue and spread (metastasize) to distant organs, for example to bone, liver, lung or the brain. As used herein the term cancer includes both metastatic tumor cell types (such as but not limited to, melanoma, lymphoma, leukemia, fibrosarcoma, rhabdomyosarcoma, and mastocytoma) and types of tissue carcinoma (such as but not limited to, colorectal cancer, prostate cancer, small cell lung cancer and non-small cell lung cancer, breast cancer, pancreatic cancer, bladder cancer, renal cancer, gastric cancer, glioblastoma, primary liver cancer, ovarian cancer, prostate cancer and uterine leiomyosarcoma). In particular, the term 'cancer' refers to acute lymphoblastic leukemia, acute myeloid leukemia, adrenocortical carcinoma, anal cancer, appendix cancer, astrocytomas, atypical teratoid/rhabdoid tumor, basal cell carcinoma, bile duct cancer, bladder cancer, bone cancer (osteosarcoma and malignant fibrous histiocytoma), brain stem glioma, brain tumors, brain and spinal cord tumors, breast cancer, bronchial tumors, Burkitt lymphoma, cervical cancer, chronic lymphocytic leukemia, chronic myelogenous leukemia, colon cancer, colorectal cancer, craniopharyngioma, cutaneous T-cell lymphoma, embryonal tumors, endometrial cancer, ependymoblastoma, ependymoma, esophageal cancer, Ewing sarcoma family of tumors, eye cancer, retinoblastoma, gallbladder cancer, gastric (stomach) cancer, gastrointestinal carcinoid tumor, gastrointestinal stromal tumor (GIST), gastrointestinal stromal cell tumor, germ cell tumor, glioma, hairy cell leukemia, head and neck cancer, hepatocellular (liver) cancer, Hodgkin lymphoma, hypopharyngeal cancer, intraocular melanoma, islet cell tumors (endocrine pancreas), Kaposi sarcoma, kidney cancer, Langerhans cell histiocytosis, laryngeal cancer, leukemia, acute lymphoblastic leukemia, acute myeloid leukemia, chronic lymphocytic leukemia, chronic myelogenous leukemia, hairy cell leukemia, liver cancer, non-small cell lung cancer, small cell lung cancer, Burkitt lymphoma, cutaneous T-cell lymphoma, Hodgkin lymphoma, non-Hodgkin lymphoma, lymphoma, medulloblastoma, medulloepithelioma, melanoma, mesothelioma, mouth cancer, chronic myelogenous leukemia, myeloid leukemia, multiple myeloma, nasopharyngeal cancer, neuroblastoma, non-Hodgkin lymphoma, non-small cell lung cancer, oral cancer, oropharyngeal cancer, osteosarcoma, malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, primary central nervous system lymphoma, prostate cancer, rectal cancer, renal cell (kidney) cancer, retinoblastoma, rhabdomyosarcoma, salivary gland cancer, sarcoma, Ewing sarcoma family of tumors, Kaposi sarcoma, Sézary syndrome, skin cancer, small cell Lung cancer, small intestine cancer, soft tissue sarcoma, squamous cell carcinoma, stomach (gastric) cancer, supratentorial primitive neuroectodermal tumors, T -cell lymphoma, testicular cancer, throat cancer, thymoma and thymic carcinoma, thyroid cancer, urethral cancer, uterine cancer, uterine sarcoma, vaginal cancer, vulvar cancer, Waldenstrom macroglobulinemia, and Wilm's tumor.

As used herein the term 'leukemia' refers to neoplastic diseases of the blood and blood forming organs. Such diseases can cause bone marrow and immune system dysfunction, which renders the host highly susceptible to infection and bleeding. In particular the term leukemia refers to acute myeloid leukemia (AML), and acute lymphoblastic leukemia (ALL) and chronic lymphoblastic leukemia (CLL).

'Compound(s) of the invention', and equivalent expressions, are meant to embrace compounds of the Formula(e) as herein described, which expression includes the pharmaceutically acceptable salts, and the solvates, *e.g.* hydrates, and the solvates of the pharmaceutically acceptable salts where the context so permits. Similarly, reference to intermediates, whether or not they themselves are claimed, is meant to embrace their salts, and solvates, where the context so permits.

When ranges are referred to herein, for example but without limitation, Ci-s alkyl, the citation of a range should be considered a representation of each member of said range.

Other derivatives of the compounds of this invention have activity in both their acid and acid derivative forms, but in the acid sensitive form often offers advantages of solubility, tissue compatibility, or delayed release in the mammalian organism (Bundgard, H, 1985). Prodrugs include acid derivatives well know to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particularly useful prodrugs. In some cases it is desirable to prepare double ester type prodrugs such as (acyloxy)alkyl esters or ((alkoxycarbonyl)oxy)alkylesters. Particular such prodrugs are the $C_1$-$_8$ alkyl, $C_2$-$_8$ alkenyl, $C_6$-$_{10}$ optionally substituted aryl, and ($C_6$-$_{10}$ aryl)-($C_{1-4}$ alkyl) esters of the compounds of the invention.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exists as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or> 99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium ($^2H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{37}Cl$), fluorine-18 ($^{18}F$), iodine-123 ($^{121}I$), iodine-125 ($^{125}I$) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^3H$, and carbon-14, i.e. $^{14}C$, are particularly useful for this purpose in view of their ease of incorporation and ready means of detection. Unnatural variant isotopic forms which incorporate deuterium i.e $^2H$ or D may afford certain therapeutic advantages resulting from greater metabolic stability, for example, increased in vivo half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

It is also to be understood that compounds that have the same molecular formula but differ in the nature or sequence of bonding of their atoms or the arrangement of their atoms in space are termed 'isomers'. Isomers that differ in the arrangement of their atoms in space are termed 'stereoisomers'.

Stereoisomers that are not mirror images of one another are termed 'diastereomers' and those that are non-superimposable mirror images of each other are termed 'enantiomers'. When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e. as (+) or (-)-isomers respectively). A chiral compound can exist as either individual enantiomer or as a mixture thereof. A mixture containing equal proportions of the enantiomers is called a 'racemic mixture'.

'Tautomers' refer to compounds that are interchangeable forms of a particular compound structure, and that vary in the displacement of hydrogen atoms and electrons. Thus, two structures may be in equilibrium through the movement of $\pi$ electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base.

Tautomeric forms may be relevant to the attainment of the optimal chemical reactivity and biological activity of a compound of interest.

The compounds of the invention may possess one or more asymmetric centers; such compounds can therefore be produced as individual (R)- or (S)-stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.
It will be appreciated that compounds of the invention may be metabolized to yield biologically active metabolites.

## THE INVENTION

The present invention relates to compounds that may be useful in the prophylaxis and/or treatment of inflammatory diseases, autoimmune diseases, pain, fibrosis and/or proliferative diseases. In particular, the compounds of the invention may inhibit Interleukin-1 Receptor Associated Kinases (IRAKs), a family of kinases that are involved in inflammatory diseases, autoimmune diseases, pain, fibrosis and/or proliferative diseases, and more particularly IRAK-4. The present invention also provides methods for the production of the compound of the invention, pharmaceutical compositions comprising the compound of the invention, methods for the prophylaxis and/or treatment of inflammatory diseases, autoimmune diseases, pain, fibrosis and/or proliferative diseases by administering the compound of the invention.
Accordingly, in a first aspect of the invention, the compounds of the invention are provided having a Formula I:

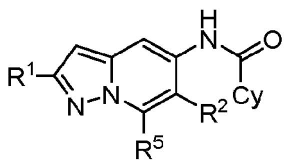

I

wherein

$R^1$ is

a) $C_{2-6}$ alkyl substituted with one or more independently selected -OH, -CN, $C_{1-4}$ alkoxy, halo, or $-S(=O)_2-C_{1-4}$ alkyl, or
b) 6 membered heterocycloalkyl comprising one or two independently selected S, N, or O atoms, which heterocycloalkyl is unsubstituted or substituted with one or more independently selected oxo, halo, or $C_{1-4}$ alkyl, which alkyl is unsubstituted or substituted with one or more halo;

$R^2$ is

a) $C_{1-4}$ alkoxy which alkoxy is unsubstituted or substituted with one or more independently selected halo or -OH,
b) $-O-C_{3-4}$ cycloalkyl, which cycloalkyl is unsubstituted or substituted with one or more independently selected halo or -OH, or
c) $-C(=O)NR^{3a}R^{3b}$;

Cy is 6 membered heteroaryl, comprising 1 or 2 N atoms, substituted with one or two independently selected $R^4$ substituents;
Each $R^{3a}$ and $R^{3b}$ is independently selected from

a) H,
b) $C_{1-4}$ alkyl, which alkyl is unsubstituted or substituted with one or more independently selected halo, -OH, -CN, $C_{1-4}$ alkoxy, or $C_{3-7}$ cycloalkyl, which cycloalkyl is unsubstituted or substituted with one or more independently selected halo,
c) $C_{3-6}$ cycloalkyl which cycloalkyl is unsubstituted or substituted with one or more independently selected oxo, -OH, -CN, $C_{1-4}$ alkyl, $C_{1-4}$ alkoxy, or halo, or
d) 4-6 membered heterocycloalkyl comprising one or two independently selected N, S, or O atoms, which heterocycloalkyl is unsubstituted or substituted with one or more independently selected oxo, -OH, -CN, $C_{1-4}$ alkyl, $C_{1-4}$ alkoxy, or halo;

$R^{3a}$ and $R^{3b}$ together with N atom to which they are attached may form a 4-6 membered monocyclic heterocycloalkyl;
Each $R^4$ is independently

a) oxo,
b) -OH,
c) -CN,
d) halo,
e) $C_{1-4}$ alkyl unsubstituted or substituted with one or more independently selected halo, -OH, or -CN,
f) $C_{1-4}$ alkoxy unsubstituted or substituted with one or more independently selected halo, -OH, or - CN, or
g) $C_{3-7}$ cycloalkyl unsubstituted or substituted with one or more independently selected halo, -OH or -CN; and

$R^5$ is selected from H, halo, $-CH_3$ or $-CF_3$.

In one embodiment, the compound of the invention is according to Formula I, wherein $R^5$ is H, F, $-CH_3$, or $-CF_3$.
In one embodiment, the compound of the invention is according to Formula I, wherein $R^5$ is H.
In one embodiment, the compound of the invention is according to Formula I, wherein $R^1$ is $C_{2-6}$ alkyl substituted with one or more independently selected -OH, -CN, $C_{1-4}$ alkoxy, halo, or $-S(=O)_2-C_{1-4}$ alkyl. In a particular embodiment, $R^1$ is $C_{2-6}$ alkyl substituted with one, two or three independently selected -OH, -CN, $C_{1-4}$ alkoxy, halo, or $-S(=O)_2-C_{1-4}$ alkyl. In a particular embodiment, $R^1$ is $C_{2-6}$ alkyl substituted with one or more independently selected -OH, -CN, $-OCH_3$, F, Cl, or $-S(=O)_2CH_3$. In a more particular embodiment, $R^1$ is $C_{2-6}$ alkyl substituted with one -OH or $-S(=O)_2CH_3$. In another more particular embodiment, $R^1$ is $-CH_2-CH_3$, $-CH_2-CH_2-CH_2-CH_3$, $-CH_2-CH_2-CH(CH_3)_2$, each of which is substituted with one -OH or $-S(=O)_2CH_3$. In a most particular embodiment, $R^1$ is $-CH_2-CH_2-C(CH_3)_2-OH$.
In one embodiment, the compound of the invention is according to Formula I, wherein $R^1$ is 6 membered heterocycloalkyl comprising one or two independently selected S, N, or O atoms. In one embodiment, $R^1$ is tetrahydropyranyl, dioxanyl, morpholinyl, piperidiyl, piperazinyl, thiomorpholinyl, or 1,4-oxathianyl. In a more particular embodiment, $R^1$ is dioxanyl.
In one embodiment, the compound of the invention is according to Formula I, wherein $R^1$ is 6 membered heterocycloalkyl comprising one or two independently selected S, N, or O atoms, which heterocycloalkyl is substituted with one or more independently selected oxo, halo, or $C_{1-4}$ alkyl, which alkyl is unsubstituted or substituted with one or more halo. In a particular embodiment, $R^1$ is 6 membered heterocycloalkyl comprising one, or two independently selected S, N, or O atoms, which heterocycloalkyl is substituted with one, two or three independently selected oxo, halo, or $C_{1-4}$ alkyl, which alkyl is unsubstituted or substituted with one or more halo. In another particular embodiment, $R^1$ is 6 membered heterocycloalkyl comprising one, or two independently selected S, N, or O atoms, which heterocycloalkyl is substituted with one, two or three independently selected oxo, F, Cl, $-CH_3$, $-CH_2-CH_3$, or $-CF_3$. In a more particular embodiment, $R^1$ is tetrahydropyranyl, dioxanyl, morpholinyl, piperidiyl, piperazinyl, thiomorpholinyl, or 1,4-oxathianyl, each of which is substituted with one, two or three independently selected oxo, halo, or $C_{1-4}$ alkyl, which alkyl is unsubstituted or substituted with one or more halo. In another more particular embodiment, $R^1$ is tetrahydropyranyl, dioxanyl, morpholinyl, piperidiyl, piperazinyl, thiomorpholinyl, or 1,4-oxathianyl.each of which is substituted with one, two or three independently selected oxo, F, Cl, $-CH_3$, $-CH_2-CH_3$, or $-CF_3$.
In one embodiment, the compound of the invention is according to Formula IIa:

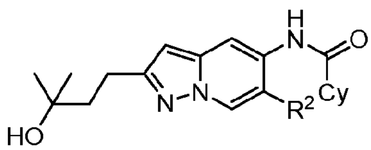

IIa

wherein $R^2$ and Cy are as previously defined.
In one embodiment, the compound of the invention is according to Formula IIb

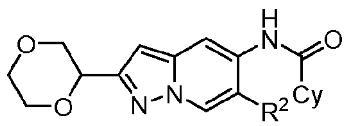

IIb

Wherein $R^2$ and Cy are as previously defined.
In one embodiment, the compound of the invention is according to Formula I, IIa, or IIb, wherein $R^2$ is $C_{1-4}$

alkoxy which alkoxy is unsubstituted or substituted with one or more independently selected halo or -OH. In a particular embodiment, $R^2$ is $-OCH_3$, or $-OCH_2CH_3$, each of which is unsubstituted or substituted with one or more independently selected halo or -OH. In a more particular embodiment, $R^2$ is $-OCH_3$, $-OCH_2CH_3$, or $-OCF_3$. In a most particular embodiment, $R^2$ is $-OCH_3$.

In one embodiment, the compound of the invention is according to Formula I, IIa, or IIb, wherein $R^2$ is $-O-C_{3-4}$ cycloalkyl, which cycloalkyl is unsubstituted or substituted with one or more independently selected halo or -OH. In a particular embodiment, $R^2$ is -O-cyclopropyl, or -O-cyclobutyl, each of which is unsubstituted or substituted with one or more independently selected halo or -OH. In a more particular embodiment, $R^2$ is -O-cyclopropyl.

In one embodiment, the compound of the invention is according to Formula I, IIa, or IIb, wherein $R^2$ is $-C(=O)NR^{3a}R^{3b}$ and each $R^{3a}$ and $R^{3b}$ is as described previously. In a particular embodiment, $R^{3a}$ is H and $R^{3b}$ is as described previously. In another particular embodiment, $R^{3a}$ is as described previously and $R^{3b}$ is H. in a more particular embodiment, $R^{3a}$ and $R^{3b}$ are H.

In one embodiment, the compound of the invention is according to Formula I, IIa, or IIb, wherein $R^2$ is $-C(=O)NR^{3a}R^{3b}$, $R^{3b}$ is as described previously, and $R^{3a}$ is $C_{1-4}$ alkyl. In a particular embodiment, $R^{3a}$ is $-CH_3$, $-CH_2-CH_3$, or $-CH(CH_3)_2$. In a more particular embodiment, $R^{3a}$ is $-CH_3$.

In one embodiment, the compound of the invention is according to Formula I, IIa, or IIb, wherein $R^2$ is $-C(=O)NR^{3a}R^{3b}$, $R^{3b}$ is as described previously, and $R^{3a}$ is $C_{1-4}$ alkyl, which alkyl is substituted with one or more independently selected halo, -OH, -CN, $C_{1-4}$ alkoxy, or $C_{3-7}$ cycloalkyl, which cycloalkyl is unsubstituted or substituted with one or more independently selected halo. In a particular embodiment, $R^{3a}$ is $-CH_3$, $-CH_2-CH_3$, or $-CH(CH_3)_2$, each of which is substituted with one or more independently selected halo, -OH, -CN, $C_{1-4}$ alkoxy, or $C_{3-7}$ cycloalkyl, which cycloalkyl is unsubstituted or substituted with one or more independently selected halo. In a more particular embodiment, $R^{3a}$ is $C_{1-4}$ alkyl, which alkyl is substituted with one or more independently selected halo, -OH, -CN, $-OCH_3$, cyclopropyl, or cyclobutyl.

In one embodiment, the compound of the invention is according to Formula I, IIa, or IIb, wherein $R^2$ is $-C(=O)NR^{3a}R^{3b}$, $R^{3a}$ is as described previously, and $R^{3b}$ is $C_{1-4}$ alkyl. In a particular embodiment, $R^{3b}$ is $-CH_3$, $-CH_2-CH_3$, or $-CH(CH_3)_2$. In a more particular embodiment, $R^{3b}$ is $-CH_3$.

In one embodiment, the compound of the invention is according to Formula I, IIa, or IIb, wherein $R^2$ is $-C(=O)NR^{3a}R^{3b}$, $R^{3a}$ is as described previously, and $R^{3b}$ is $C_{1-4}$ alkyl, which alkyl is substituted with one or more independently selected halo, -OH, -CN, $C_{1-4}$ alkoxy, or $C_{3-7}$ cycloalkyl, which cycloalkyl is unsubstituted or substituted with one or more independently selected halo. In a particular embodiment, $R^{3b}$ is $-CH_3$, $-CH_2-CH_3$, or $-CH(CH_3)_2$, each of which is substituted with one or more independently selected halo, -OH, -CN, $C_{1-4}$ alkoxy, or $C_{3-7}$ cycloalkyl, which cycloalkyl is unsubstituted or substituted with one or more independently selected halo. In a more particular embodiment, $R^{3b}$ is $C_{1-4}$ alkyl, which alkyl is substituted with one or more independently selected halo, -OH, -CN, $-OCH_3$, cyclopropyl, or cyclobutyl.

In one embodiment, the compound of the invention is according to Formula I, IIa, or IIb, wherein $R^2$ is $-C(=O)NR^{3a}R^{3b}$, $R^{3b}$ is as described previously, and $R^{3a}$ is $C_{3-6}$ cycloalkyl. In a particular embodiment, $R^{3a}$ is cyclopropyl, cyclobutyl, or cyclopentyl. In a more particular embodiment, $R^{3a}$ is cyclopropyl.

In one embodiment, the compound of the invention is according to Formula I, IIa, or IIb, wherein $R^2$ is $-C(=O)NR^{3a}R^{3b}$, $R^{3b}$ is as described previously, and $R^{3a}$ is $C_{3-6}$ cycloalkyl, which cycloalkyl is substituted with one or more independently selected oxo, -OH, -CN, $C_{1-4}$ alkyl, $C_{1-4}$ alkoxy, or halo. In a particular embodiment, $R^{3a}$ is $C_{3-6}$ cycloalkyl, which cycloalkyl is substituted with one or more independently selected oxo, -OH, -CN, $-CH_3$, $-CH_2-CH_3$, $-OCH_3$, $-OCH_2CH_3$, F or Cl. In a more particular embodiment, $R^{3a}$ is cyclopropyl, cyclobutyl, or cyclopentyl, each of which is substituted with one or more independently selected oxo, -OH, -CN, $C_{1-4}$ alkyl, $C_{1-4}$ alkoxy, or halo. In another more particular embodiment, $R^{3a}$ is cyclopropyl, cyclobutyl, or cyclopentyl, each of which is substituted with one or more independently selected oxo, -OH, -CN, $-CH_3$, $-CH_2-CH_3$, $-OCH_3$, $-OCH_2CH_3$, F or Cl.

In one embodiment, the compound of the invention is according to Formula I, IIa, or IIb, wherein $R^2$ is $-C(=O)NR^{3a}R^{3b}$, $R^{3a}$ is as described previously, and $R^{3b}$ is $C_{3-6}$ cycloalkyl. In a particular embodiment, $R^{3b}$ is cyclopropyl, cyclobutyl, or cyclopentyl. In a most particular embodiment, $R^{3b}$ is cyclopropyl.

In one embodiment, the compound of the invention is according to Formula I, IIa, or IIb, wherein $R^2$ is $-C(=O)NR^{3a}R^{3b}$, $R^{3a}$ is as described previously, and $R^{3b}$ is $C_{3-6}$ cycloalkyl, which cycloalkyl is substituted with one or more independently selected oxo, -OH, -CN, $C_{1-4}$ alkyl, $C_{1-4}$ alkoxy, or halo. In a particular embodiment, $R^{3b}$ is $C_{3-6}$ cycloalkyl, which cycloalkyl is substituted with one or more independently selected oxo, -OH, -CN, $-CH_3$, $-CH_2-CH_3$, $-OCH_3$, $-OCH_2CH_3$, F or Cl. In a more particular embodiment, $R^{3b}$ is cyclopropyl, cyclobutyl, or cyclopentyl, each of which is substituted with one or more independently selected oxo, -OH, -CN, $C_{1-4}$ alkyl, $C_{1-4}$ alkoxy, or halo. In another more particular embodiment, $R^{3b}$ is cyclopropyl, cyclobutyl, or cyclopentyl, each of which is substituted with one or more independently selected oxo, -OH, -CN, $-CH_3$, $-CH_2-CH_3$, $-OCH_3$, $-OCH_2CH_3$, F or Cl.

In one embodiment, the compound of the invention is according to Formula I, IIa, or IIb, wherein $R^2$ is $-C(=O)NR^{3a}R^{3b}$, $R^{3b}$ is as described previously, and $R^{3a}$ is 4-6 membered heterocycloalkyl comprising one or two

independently selected N, S, or O atoms. In a particular embodiment, $R^{3a}$ is azetidinyl, oxetanyl, pyrrolidinyl, tetrahydrofuranyl, morpholinyl, piperidinyl, piperazinyl, or thiomorpholinyl. In a most particular embodiment, $R^{3a}$ is azetidinyl or oxiranyl.

In one embodiment, the compound of the invention is according to Formula I, IIa, or IIb, wherein $R^2$ is $-C(=O)NR^{3a}R^{3b}$, $R^{3b}$ is as described previously, and $R^{3a}$ is 4-6 membered heterocycloalkyl comprising one or two independently selected N, S, or O atoms, which heterocycloalkyl is substituted with one or more independently selected oxo, -OH, -CN, $C_{1-4}$ alkyl, $C_{1-4}$ alkoxy, or halo. In another particular embodiment, $R^{3a}$ is 4-6 membered heterocycloalkyl comprising one or two independently selected N, S, or O atoms, which heterocycloalkyl is substituted with one or more independently selected oxo, -OH, -CN, $-CH_3$, $-CH_2-CH_3$, $-OCH_3$, $-OCH_2CH_3$, F or Cl. In a more particular embodiment, $R^{3a}$ is azetidinyl, oxetanyl, pyrrolidinyl, tetrahydrofuranyl, morpholinyl, piperidinyl, piperazinyl, or thiomorpholinyl, each of which is substituted with one or more independently selected oxo, -OH, -CN, $C_{1-4}$ alkyl, $C_{1-4}$ alkoxy, or halo. In another more particular embodiment, $R^{3a}$ is azetidinyl, oxetanyl, pyrrolidinyl, tetrahydrofuranyl, morpholinyl, piperidinyl, piperazinyl, or thiomorpholinyl, each of which is substituted with one or more independently selected oxo, -OH, -CN, $-CH_3$, $-CH_2-CH_3$, $-OCH_3$, $-OCH_2CH_3$, F or Cl.

In one embodiment, the compound of the invention is according to Formula I, IIa, or IIb, wherein $R^2$ is $-C(=O)NR^{3a}R^{3b}$, $R^{3a}$ is as described previously, and $R^{3b}$ is 4-6 membered heterocycloalkyl comprising one or two independently selected N, S, or O atoms. In a particular embodiment, $R^{3b}$ is azetidinyl, oxetanyl, pyrrolidinyl, tetrahydrofuranyl, morpholinyl, piperidinyl, piperazinyl, or thiomorpholinyl. In a most particular embodiment, $R^{3b}$ is azetidinyl or oxiranyl.

In one embodiment, the compound of the invention is according to Formula I, IIa, or IIb, wherein $R^2$ is $-C(=O)NR^{3a}R^{3b}$, $R^{3a}$ is as described previously, and $R^{3b}$ is 4-6 membered heterocycloalkyl comprising one or two independently selected N, S, or O atoms, which heterocycloalkyl is substituted with one or more independently selected oxo, -OH, -CN, $C_{1-4}$ alkyl, $C_{1-4}$ alkoxy, or halo. In another particular embodiment, $R^{3b}$ is 4-6 membered heterocycloalkyl comprising one or two independently selected N, S, or O atoms, which heterocycloalkyl is substituted with one or more independently selected oxo, -OH, -CN, $-CH_3$, $-CH_2-CH_3$, $-OCH_3$, $-OCH_2CH_3$, F or Cl. In a more particular embodiment, $R^{3b}$ is azetidinyl, oxetanyl, pyrrolidinyl, tetrahydrofuranyl, morpholinyl, piperidinyl, piperazinyl, or thiomorpholinyl, each of which is substituted with one or more independently selected oxo, -OH, -CN, $C_{1-4}$ alkyl, $C_{1-4}$ alkoxy, or halo. In another more particular embodiment, $R^{3b}$ is azetidinyl, oxetanyl, pyrrolidinyl, tetrahydrofuranyl, morpholinyl, piperidinyl, piperazinyl, or thiomorpholinyl, each of which is substituted with one or more independently selected oxo, -OH, -CN, $-CH_3$, $-CH_2-CH_3$, $-OCH_3$, $-OCH_2CH_3$, F or Cl.

In one embodiment, the compound of the invention is according to Formula I, IIa, or IIb, wherein $R^2$ is $-C(=O)NH_2$, $-C(=O)N(CH_3)_2$, or $-C(=O)NHCH_3$. In a most particular embodiment, $R^2$is $-C(=O)NH_2$.

In one embodiment, the compound of the invention is according to Formula I, IIa, or IIb, wherein $R^2$ is $-C(=O)NR^{3a}R^{3b}$, wherein $R^{3a}$ and $R^{3b}$ together with N atom to which they are attached may form a 4-6 membered monocyclic heterocycloalkyl. In a particular embodiment, $R^2$ is

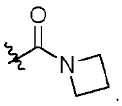

In one embodiment, the compound of the invention is according to Formula IIIa, IIIb or IIIc

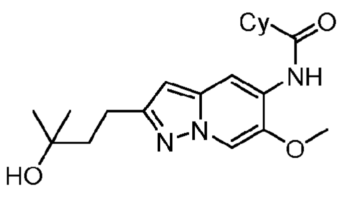
IIIa

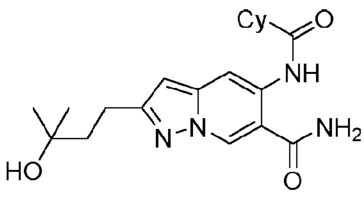
IIIb

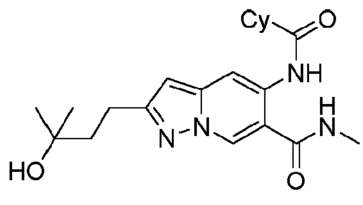
IIIc

wherein Cy is as previously defined.

In one embodiment, the compound of the invention is according to Formula IVa, IVb or IVc

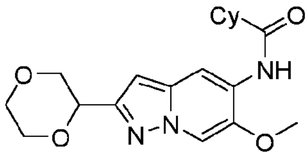
IVa

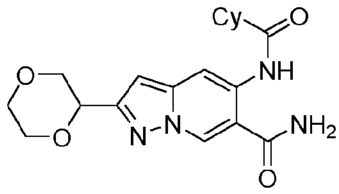
IVb

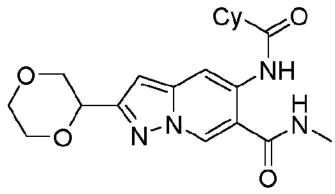
IVc

Wherein Cy is as previously defined.

In one embodiment, the compound of the invention is according to any one of Formulae 1-IVc, wherein Cy is 6 membered heteroaryl, comprising 1 or 2 N atoms, substituted with one or two independently selected $R^4$ substituents. In a particular embodiment, Cy is pyridinyl, or pyrazinyl, each of which is substituted with one or two independently selected $R^4$ substituents. In a more particular embodiment, Cy is pyridinyl substituted with one or two independently selected $R^4$ substituents.

In one embodiment, the compound of the invention is according to any one of Formulae I-IVc, wherein Cy is as previously defined, wherein $R^4$ is oxo.

In one embodiment, the compound of the invention is according to any one of Formulae 1-IVc, wherein Cy is as previously defined, wherein $R^4$ is -OH.

In one embodiment, the compound of the invention is according to any one of Formulae 1-IVc, wherein Cy is as previously defined, wherein $R^4$ is -CN.

In one embodiment, the compound of the invention is according to any one of Formulae 1-IVc, wherein Cy is as previously defined, wherein $R^4$ is halo. In a particular embodiment, $R^4$ is F or Cl.

In one embodiment, the compound of the invention is according to any one of Formulae 1-IVc, wherein Cy is as previously defined, wherein $R^4$ is $C_{1-4}$ alkyl unsubstituted or substituted with one or more independently selected halo, -OH, or -CN. In a particular embodiment, $R^4$ is $-CH_3$, $-CH_2-CH_3$, or $-CH(CH_3)_2$, each of which is unsubstituted or substituted with one or more independently selected halo, -OH, or -CN.

In one embodiment, the compound of the invention is according to any one of Formulae I-IVc, wherein Cy is as previously defined, wherein $R^4$ is $C_{1-4}$ alkoxy unsubstituted or substituted with one or more independently selected halo, -OH, or -CN. In a particular embodiment, $R^4$ is $-OCH_3$, $-OCH_2-CH_3$, or $-OCH(CH_3)_2$, each of which is unsubstituted or substituted with one or more independently selected halo, -OH, or -CN. In a more particular embodiment, $R^4$ is $-OCH_3$.

In one embodiment, the compound of the invention is according to any one of Formulae I-IVc, wherein Cy is as previously defined, wherein $R^4$ is $C_{3-7}$ cycloalkyl unsubstituted or substituted with one or more independently selected halo, -OH or -CN.

In one embodiment, the compound of the invention is according to any one of Formulae I-IVc, wherein Cy is as previously defined, wherein each $R^4$ group is independently selected from oxo, -CN, -OH, F, Cl, $-CH_3$, $-CH_2-CH_3$, $-CH(CH_3)_2$, $-CF_3$, $-CHF_3$, $-CH_2CF_3$, $-CH_2CN$, $-CH_2OH$, $-CH_2CH_2-CN$, $-O-CH_2-CH_3$, cyclopropyl, cyclobutyl, cyclopropyl substituted with one or two independently selected F, or -CN, cyclobutyl substituted with one or two independently selected F, -OH, or -CN.

In one embodiment, the compound of the invention is according to any one of Formulae I-IVc, more particularly Formula IIIa, wherein Cy is:

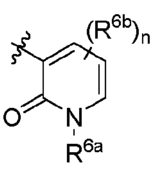
$Cy_1$

wherein

$R^{6a}$ is

a) $C_{1-4}$ alkyl unsubstituted or substituted with one or more independently selected halo, -OH, or -CN, or
b) $C_{3-7}$ cycloalkyl unsubstituted or substituted with one or more independently selected halo, -OH or -CN;

$R^{6b}$ is

a) -OH,
b) -CN,
c) halo,
d) $C_{1-4}$ alkyl unsubstituted or substituted with one or more independently selected halo, -OH, or -CN,
e) $C_{1-4}$ alkoxy unsubstituted or substituted with one or more independently selected halo, -OH, or -CN, or
f) $C_{3-7}$ cycloalkyl unsubstituted or substituted with one or more independently selected halo, -OH or -CN; and

the subscript n is 0, 1, or 2.

In one embodiment, the compound of the invention is according to any one of Formulae I-IVc, more particularly Formula IIIa, wherein Cy is $Cy_1$, wherein $R^{6a}$ and the subscript n are as previously defined, and $R^{6b}$ is -CN, -OH, F, Cl, $-CH_3$, $-CH_2-CH_3$, $-CH(CH_3)_2$, $-CF_3$, $-CHF_3$, $-CH_2CF_3$, $-CH_2CN$, $-CH_2OH$, $-CH_2CH_2-CN$, $-OCH_3$, $-OCH_2-CH_3$, cyclopropyl, cyclobutyl, cyclopropyl substituted with one or two independently selected F, or -CN, or cyclobutyl substituted with one or two independently selected F, -OH, or -CN. In a particular embodiment, $R^{6b}$ is F, Cl, $-CH_3$, $-CF_3$, or $-CHF_2$, or $-OCH_3$. In a more particular embodiment, $R^{6b}$ is F.

In one embodiment, the compound of the invention is according to any one of Formulae I-IVc, more particularly Formula IIIa, wherein Cy is $Cy_1$, wherein $R^{6a}$ is as previously defined, the subscript n is 1, and $R^{6b}$ is -CN, -OH, F, Cl, $-CH_3$, $-CH_2-CH_3$, $-CH(CH_3)_2$, $-CF_3$, $-CHF_3$, $-CH_2CF_3$, $-CH_2CN$, $-CH_2OH$, $-CH_2CH_2-CN$, $-OCH_3$, $-OCH_2-CH_3$, cyclopropyl, cyclobutyl, cyclopropyl substituted with one or two independently selected F, or -CN, or cyclobutyl substituted with one or two independently selected F, -OH, or -CN. In a particular embodiment, $R^{6b}$ is F, Cl, $-CH_3$, $-CF_3$, or $-CHF_2$, or $-OCH_3$. In a more particular embodiment, $R^{6b}$ is F.

In one embodiment, the compound of the invention is according to any one of Formulae I-IVc, more particularly Formula IIIa, wherein Cy is $Cy_1$, wherein $R^{6b}$ and the subscript n are as previously defined, and $R^{6a}$ is $-CH_3$, $-CH_2-CH_3$, $-CH(CH_3)_2$, $-CF_3$, $-CHF_3$, $-CH_2CF_3$, $-CH_2CN$, $-CH_2OH$, $-CH_2CH_2-CN$, cyclopropyl, cyclobutyl, cyclopropyl substituted with one or two independently selected F, or -CN, or cyclobutyl substituted with one or two independently selected F, -OH, or -CN. In a particular embodiment, $R^{6a}$ is $-CH_3$, $-CF_3$, $-CHF_2$, cyclopropyl, or cyclobutyl. In a more particular embodiment, $R^{6a}$ is $-CH_3$, or cyclopropyl. In a most particular embodiment, $R^{6a}$ is cyclopropyl.

In one embodiment, the compound of the invention is according to any one of Formulae I-IVc, more particularly Formula IIIa, wherein Cy is $Cy_1$, wherein $R^{6b}$ is as previously defined, the subscript n is 1 and $R^{6a}$ is $-CH_3$, $-CH_2-CH_3$, $-CH(CH_3)_2$, $-CF_3$, $-CHF_3$, $-CH_2CF_3$, $-CH_2CN$, $-CH_2OH$, $-CH_2CH_2-CN$, cyclopropyl, cyclobutyl, cyclopropyl substituted with one or two independently selected F, or -CN, or cyclobutyl substituted with one or two independently selected F, -OH, or -CN. In a particular embodiment, $R^{6a}$ is $-CH_3$, $-CF_3$, $-CHF_2$, cyclopropyl, or cyclobutyl. In a more particular embodiment, $R^{6a}$ is $-CH_3$, or cyclopropyl. In a most particular embodiment, $R^{6a}$ is cyclopropyl.

In one embodiment, the compound of the invention is according to any one of Formulae I-IVc, more particularly Formula IIIa, wherein Cy is $Cy_1$, wherein $R^{6a}$ is as previously defined, and the subscript n is 0. In a particular embodiment, $R^{6a}$ is $-CH_3$, $-CH_2-CH_3$, $-CH(CH_3)_2$, $-CF_3$, $-CHF_3$, $-CH_2CF_3$, $-CH_2CN$, -CHzOH, $-CH_2CH_2-CN$, cyclopropyl, cyclobutyl, cyclopropyl substituted with one or two independently selected F, or -CN, or cyclobutyl substituted with one or two independently selected F, -OH, or -CN. In a particular embodiment, $R^{6a}$ is $-CH_3$, $-CF_3$, $-CHF_2$, cyclopropyl, or cyclobutyl. In a more particular embodiment, $R^{6a}$ is $-CH_3$, or cyclopropyl. In a most particular embodiment, $R^{6a}$ is cyclopropyl.

In one embodiment, the compound of the invention according to Formula I is selected from: 1-cyclopropyl-N-[2-(3-hydroxy-3-methylbutyl)-6-methoxypyrazolo[1,5-a]pyridin-5-yl]-2-oxopyridine-3-carboxamide,

N-[2-(3-hydroxy-3-methylbutyl)-6-methoxypyrazolo[1,5-a]pyridin-5-yl]-1-methyl-2-oxopyridine-3-carboxamide,
N-[2-(3-hydroxy-3-methylbutyl)-6-methoxypyrazolo[1,5-a]pyridin-5-yl]-6-(trifluoromethyl)pyridine-2-carboxamide,
2-(3-hydroxy-3-methylbutyl)-5-[[6-(trifluoromethyl)pyridine-2-carbonyl]amino]pyrazolo[1,5-a]pyridine-6-carboxamide, and
2-(3-hydroxy-3-methylbutyl)-N-methyl-5-[[6-(trifluoromethyl)pyridine-2-carbonyl]amino]pyrazolo[1,5-a]pyridine-6-carboxamide.

In a further embodiment, the compound of the invention according to Formula I is selected from: 1-cyclopropyl-N-[6-methoxy-2-(2-methylsulfonylethyl)pyrazolo[1,5-a]pyridin-5-yl]-2-oxo-pyridine-3-carboxamide,

1-(difluoromethyl)-N-[2-(3-hydroxy-3-methyl-butyl)-6-methoxy-pyrazolo[1,5-a]pyridin-5-yl]-2-oxo-pyridine-3 -carboxamide,
2-(1,1-dioxothian-3-yl)-N-methyl-5-[[6-(trifluoromethyl)pyridine-2-carbonyl]amino]pyrazolo[1,5-a]pyridine-6-carboxamide,
1-(difluoromethyl)-N-[6-ethoxy-2-(3-hydroxy-3-methyl-butyl)pyrazolo[1,5-a]pyridin-5-yl]-2-oxo-pyridine-3 -carboxamide,

2-(1-methyl-4-piperidyl)-5-[[6-(triffuoromethyl)pyridine-2-carbonyl]amino]pyrazolo[1,5-a]pyridine-6-carboxamide formic acid salt,
2-tetrahydropyran-4-yl-5-[[6-(trifluoromethyl)pyridine-2-carbonyl]amino]pyrazolo[1,5-a]pyridine-6-carboxamide,
N-methyl-2-(1-methyl-4-piperidyl)-5-[[6-(trifluoromethyl)pyridine-2-carbonyl]amino]pyrazolo[1,5-a]pyridine-6-carboxamide,
1-cyclopropyl-N-[2-(3-hydroxy-3-methyl-butyl)-6-methoxy-7-methyl-pyrazolo[1,5-a]pyridm-5-yl]-2-oxo-pyridine-3 -carboxamide,
N-methyl-2-tetrahydropyran-4-yl-5-[[6-(trifluoromethyl)pyridine-2-carbonyl]amino]pyrazolo[1,5-a]pyridine-6-carboxamide,
5-[[6-(difluoromethyl)pyridine-2-carbonyl]amino]-N-methyl-2-tetrahydropyran-4-yl-pyrazolo[1,5-a]pyridine-6-carboxamide,
5-[[6-(difluoromethyl)pyridine-2-carbonyl]amino]-2-tetrahydropyran-4-yl-pyrazolo[1,5-a]pyridine-6-carboxamide,
1-cyclopropyl-N-[6-ethoxy-2-(3-hydroxy-3-methyl-butyl)pyrazolo[1,5-a]pyridin-5-yl]-2-oxo-pyridine-3-carboxamide,
N-[6-ethoxy-2-(3-hydroxy-3-methyl-butyl)pyrazolo[1,5-a]pyridin-5-yl]-1-methyl-2-oxo-pyridine-3-carboxamide,
5-[[6-(difluoromethyl)pyridine-2-carbonyl]amino]-2-(3-hydroxy-3-methyl-butyl)-N-methyl-pyrazolo[1,5-a]pyridine-6-carboxamide,
5-[[6-(difluoromethyl)pyridine-2-carbonyl]amino]-2-(3-hydroxy-3-methyl-butyl)pyrazolo[1,5-a]pyridine-6-carboxamide,
1-cyclobutyl-N-[2-(3-hydroxy-3-methyl-butyl)-6-methoxy-pyrazolo[1,5-a]pyridin-5-yl]-2-oxo-pyridine-3-carboxamide,
1-cyclopropyl-N-[2-(2-fluoro-3-hydroxy-3-methyl-butyl)-6-methoxy-pyrazolo[1,5-a]pyridin-5-yl]-2-oxo-pyridine-3 -carboxamide,
1-cyclopropyl-N-[6-methoxy-2-[4,4,4-trideuterio-3-hydroxy-3-(trideuteriomethyl)butyl]pyrazolo[1,5-a]pyridin-5-yl]-2-oxo-pyridine-3-carboxamide,
1-cyclopropyl-N-[2-(3 -hydroxy-3 -methylbutyl)-6-(trideuteriomethoxy)pyrazolo[1,5-a] pyridin-5 -yl]-2-oxopyridine-3 -carboxamide,
1-cyclopropyl-N-[2-(2-fluoro-3-hydroxy-3-methyl-butyl)-6-methoxy-pyrazolo[1,5-a]pyridin-5-yl]-2-oxo-pyridine-3-carboxamide Enantiomer A, and
1-cyclopropyl-N-[2-(2-fluoro-3-hydroxy-3-methyl-butyl)-6-methoxy-pyrazolo[1,5-a]pyridin-5-yl]-2-oxo-pyridine-3-carboxamide Enantiomer B.

In one embodiment, the compounds of the invention is 1-cyclopropyl-N-[2-(3-hydroxy-3-methylbutyl)-6-methoxypyrazolo[1,5-a]pyridin-5-yl]-2-oxopyridine-3-carboxamide.

In another embodiment, the compound of the invention is not 1-cyclopropyl-N-[2-(3-hydroxy-3-methylbutyl)-6-methoxypyrazolo[1,5-a]pyridin-5-yl]-2-oxopyridine-3-carboxamide.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. $^{2}H$ or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the illustrative example as examples.

In one aspect a compound of the invention according to any one of the embodiments herein described is present as the free base.

In one aspect a compound of the invention according to any one of the embodiments herein described is a pharmaceutically acceptable salt.

In one aspect a compound of the invention according to any one of the embodiments herein described is a solvate of the compound.

In one aspect a compound of the invention according to any one of the embodiments herein described is a solvate of a pharmaceutically acceptable salt of a compound.

While specified groups for each embodiment have generally been listed above separately, a compound of the

invention includes one in which several or each embodiment in the above Formula, as well as other formulae presented herein, is selected from one or more of particular members or groups designated respectively, for each variable. Therefore, this invention is intended to include all combinations of such embodiments within its scope.

While specified groups for each embodiment have generally been listed above separately, a compound of the invention may be one for which one or more variables (for example, R groups) is selected from one or more embodiments according to any of the Formula(e) listed above. Therefore, the present invention is intended to include all combinations of variables from any of the disclosed embodiments within its scope.

Alternatively, the exclusion of one or more of the specified variables from a group or an embodiment, or combinations thereof is also contemplated by the present invention.

In certain aspects, the present invention provides prodrugs and derivatives of the compounds according to the formulae above. Prodrugs are derivatives of the compounds of the invention, which have metabolically cleavable groups and become by solvolysis or under physiological conditions the compounds of the invention, which are pharmaceutically active, *in vivo.* Such examples include, but are not limited to, choline ester derivatives and the like, N-alkylmorpholine esters and the like.

Other derivatives of the compounds of this invention have activity in both their acid and acid derivative forms, but the acid sensitive form often offers advantages of solubility, tissue compatibility, or delayed release in the mammalian organism.(Bundgaard, 1985) Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are preferred prodrugs. In some cases it is desirable to prepare double ester type prodrugs such as (acyloxy)alkyl esters or ((alkoxycarbonyl)oxy)alkylesters. Particularly useful are the $C_1$ to $C_8$ alkyl, $C_2$-$C_8$ alkenyl, aryl, $C_7$-$C_{12}$ substituted aryl, and $C_7$-$C_{12}$ arylalkyl esters of the compounds of the invention.

Embodiments according to the invention are provided as set out below.

Embodiment 1 provides a compound according to Formula I:

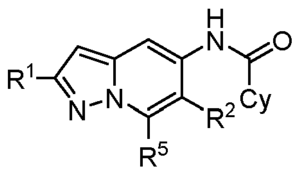

I

wherein

$R^1$ is

a) $C_{2-6}$ alkyl substituted with one or more independently selected -OH, -CN, $C_{1-4}$ alkoxy, halo, or -S(=O)$_2$-$C_{1-4}$ alkyl, or
b) 6 membered heterocycloalkyl comprising one or two independently selected S, N, or O atoms, which heterocycloalkyl is unsubstituted or substituted with one or more independently selected oxo, halo, or $C_{1-4}$ alkyl, which alkyl is unsubstituted or substituted with one or more halo;
b) 6 membered heterocycloalkyl comprising one or two independently selected S, N, or O atoms, which heterocycloalkyl is unsubstituted or substituted with one or more independently selected oxo, halo, or $C_{1-4}$ alkyl, which alkyl is unsubstituted or substituted with one or more halo;

$R^2$ is

a) $C_{1-4}$ alkoxy which alkoxy is unsubstituted or substituted with one or more independently selected halo or -OH,
b) -O-$C_{3-4}$ cycloalkyl, which cycloalkyl is unsubstituted or substituted with one or more independently selected halo or -OH, or
c) -C(=O)NR$^{3a}$R$^{3b}$;

Cy is 6 membered heteroaryl, comprising 1 or 2 N atoms, substituted with one or two independently selected $R^4$ substituents;
Each $R^{3a}$ and $R^{3b}$ is independently selected from

a) H,
b) $C_{1-4}$ alkyl, which alkyl is unsubstituted or substituted with one or more independently selected halo, -OH, -CN, $C_{1-4}$ alkoxy, or $C_{3-7}$ cycloalkyl, which cycloalkyl is unsubstituted or substituted with one or more independently selected halo,
c) $C_{3-6}$ cycloalkyl which cycloalkyl is unsubstituted or substituted with one or more independently selected oxo, -OH, -CN, $C_{1-4}$ alkyl, $C_{1-4}$ alkoxy, or halo, or
d) 4-6 membered heterocycloalkyl comprising one or two independently selected N, S, or O atoms, which heterocycloalkyl is unsubstituted or substituted with one or more independently selected oxo, -OH, -CN, $C_{1-4}$ alkyl, $C_{1-4}$ alkoxy, or halo;

$R^{3a}$ and $R^{3b}$ together with N atom to which they are attached may form a 4-6 membered monocyclic heterocycloalkyl;
Each $R^4$ is independently

a) oxo,
b) -OH,
c) -CN,
d) halo,
e) $C_{1-4}$ alkyl unsubstituted or substituted with one or more independently selected halo, -OH, or -CN,
f) $C_{1-4}$ alkoxy unsubstituted or substituted with one or more independently selected halo, -OH, or - CN, or
g) $C_{3-7}$ cycloalkyl unsubstituted or substituted with one or more independently selected halo, -OH or -CN; and

$R^5$ is selected from H, halo, $-CH_3$ or $-CF_3$;
or a pharmaceutically acceptable salt thereof, or a solvate or the salt of the solvate thereof.

## PHARMACEUTICAL COMPOSITIONS

When employed as a pharmaceutical, a compound of the invention is typically administered in the form of a pharmaceutical composition. Such compositions can be prepared in a manner well known in the pharmaceutical art and comprise at least one active compound of the invention according to Formula I. Generally, a compound of the invention is administered in a pharmaceutically effective amount. The amount of compound of the invention actually administered will typically be determined by a physician, in the light of the relevant circumstances, including the condition to be treated, the chosen route of administration, the actual compound of the invention administered, the age, weight, and response of the individual patient, the severity of the patient's symptoms, and the like.

The pharmaceutical compositions of this invention can be administered by a variety of routes including oral, rectal, transdermal, subcutaneous, intra-articular, intravenous, intramuscular, and intranasal. Depending on the intended route of delivery, a compound of the invention is preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term 'unit dosage forms' refers to physically discrete units suitable as unitary dosages for human subjects and other mammals, each unit containing a predetermined quantity of active material calculated to produce the desired therapeutic effect, in association with a suitable pharmaceutical excipient, vehicle or carrier. Typical unit dosage forms include prefilled, premeasured ampules or syringes of the liquid compositions or pills, tablets, capsules or the like in the case of solid compositions. In such compositions, the compound of the invention according to Formula I is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or non-aqueous vehicle with buffers, suspending and dispensing agents, colorants, flavors and the like. Solid forms may include, for example, any of the following ingredients, or compound of the inventions of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint or orange flavoring.

Injectable compositions are typically based upon injectable sterile saline or phosphate-buffered saline or other injectable carriers known in the art. As before, the active compound of the invention according to Formula I in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about

20% by weight, preferably from about 0.1 to about 10% by weight, and more preferably from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included within the scope of this invention.

A compound of the invention can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.(1985)

A compound of the invention can also be administered in sustained release forms or from sustained release drug delivery systems. A description of representative sustained release materials can be found in Remington's Pharmaceutical Sciences.(1985)

The following formulation examples illustrate representative pharmaceutical compositions that may be prepared in accordance with this invention. The present invention, however, is not limited to the following pharmaceutical compositions.

**Formulation 1 - Tablets**

A compound of the invention according to Formula I may be admixed as a dry powder with a dry gelatin binder in an approximate 1:2 weight ratio. A minor amount of magnesium stearate may be added as a lubricant. The mixture may be formed into 240-270 mg tablets (80-90 mg of active compound of the invention according to Formula I per tablet) in a tablet press.

**Formulation 2 - Capsules**

A compound of the invention according to Formula I may be admixed as a dry powder with a starch diluent in an approximate 1:1 weight ratio. The mixture may be filled into 250 mg capsules (125 mg of active compound of the invention according to Formula I per capsule).

**Formulation 3 - Liquid**

A compound of the invention according to Formula I (125 mg), may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color may be diluted with water and added with stirring. Sufficient water may then be added with stirring. Further sufficient water may be then added to produce a total volume of 5 mL.

**Formulation 4 - Tablets**

A compound of the invention according to Formula I may be admixed as a dry powder with a dry gelatin binder in an approximate 1:2 weight ratio. A minor amount of magnesium stearate may be added as a lubricant. The mixture may be formed into 450-900 mg tablets (150-300 mg of active compound of the invention according to Formula I) in a tablet press.

**Formulation 5 - Injection**

A compound of the invention according to Formula I may be dissolved or suspended in a buffered sterile saline injectable aqueous medium to a concentration of approximately 5 mg/mL.

**Formulation 6 - Topical**

Stearyl alcohol (250 g) and a white petrolatum (250 g) may be melted at about 75°C and then a mixture of A compound of the invention according to Formula I (50 g) methylparaben (0.25 g), propylparaben (0.15 g), sodium lauryl sulfate (10 g), and propylene glycol (120 g) dissolved in water (about 370 g) may be added and the resulting mixture

may be stirred until it congeals.

## METHODS OF TREATMENT

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of inflammatory diseases, autoimmune diseases, pain, fibrosis and/or proliferative diseases.

In another embodiment, the present invention provides compounds of the invention, or pharmaceutical compositions comprising a compound of the invention for use in the manufacture of a medicament for use in the prophylaxis and/or treatment of inflammatory diseases, autoimmune diseases, pain, fibrosis and/or proliferative diseases.

In additional method of treatment aspects, this disclosure provides methods of prophylaxis and/or treatment of a mammal afflicted with inflammatory diseases, autoimmune diseases, pain, fibrosis and/or proliferative diseases, which methods comprise the administration of an effective amount of a compound of the invention or one or more of the pharmaceutical compositions herein described for the treatment or prophylaxis of said condition.

In one embodiment, the present invention provides pharmaceutical compositions comprising a compound of the invention, and another therapeutic agent. In a particular embodiment, the other therapeutic agent is an agent for the prophylaxis and/or treatment of inflammatory diseases, autoimmune diseases, pain, fibrosis and/or proliferative diseases.

In one embodiment, the present invention provides compounds of the invention or pharmaceutical compositions comprising a compound of the invention, for use in the prophylaxis and/or treatment of inflammatory diseases. In a particular embodiment, the inflammatory disease is selected from rheumatoid arthritis, osteoarthritis, juvenile idiopathic arthritis, psoriasis, psoriatic arthritis, ankylosing spondylitis, allergic airway diseases (*e.g.*, asthma, rhinitis), chronic obstructive pulmonary disease (COPD), inflammatory bowel diseases (*e.g.*, Crohn's disease, ulcerative colitis), endotoxin-driven disease states (*e.g.*, complications after bypass surgery or chronic endotoxin states contributing to *e.g.,* chronic cardiac failure), and related diseases involving cartilage, such as that of the joints. More particularly, the inflammatory disease is rheumatoid arthritis, psoriasis or juvenile idiopathic arthritis.

In another embodiment, the present invention provides compounds of the invention, or pharmaceutical compositions comprising a compound of the invention for use in the manufacture of a medicament for use in the prophylaxis and/or treatment of inflammatory diseases. In a particular embodiment, the inflammatory disease is selected from rheumatoid arthritis, osteoarthritis, juvenile idiopathic arthritis, psoriasis, psoriatic arthritis, ankylosing spondylitis, allergic airway diseases (*e.g.*, asthma, rhinitis), chronic obstructive pulmonary disease (COPD), inflammatory bowel diseases (*e.g.*, Crohn's disease, ulcerative colitis), endotoxin-driven disease states (*e.g.*, complications after bypass surgery or chronic endotoxin states contributing to *e.g.*, chronic cardiac failure), and related diseases involving cartilage, such as that of the joints. More particularly, the inflammatory disease is rheumatoid arthritis, psoriasis or juvenile idiopathic arthritis.

In additional method of treatment aspects, this disclosure provides methods of prophylaxis and/or treatment of a mammal afflicted with inflammatory diseases, which methods comprise the administration of an effective amount of a compound of the invention or one or more of the pharmaceutical compositions herein described for the treatment or prophylaxis of said condition. In a particular embodiment, the inflammatory disease is selected from rheumatoid arthritis, osteoarthritis, juvenile idiopathic arthritis, psoriasis, psoriatic arthritis, ankylosing spondylitis, allergic airway diseases (*e.g.*, asthma, rhinitis), chronic obstructive pulmonary disease (COPD), inflammatory bowel diseases (*e.g.*, Crohn's disease, ulcerative colitis), endotoxin-driven disease states (*e.g.*, complications after bypass surgery or chronic endotoxin states contributing to *e.g.*, chronic cardiac failure), and related diseases involving cartilage, such as that of the joints. More particularly, the inflammatory disease is rheumatoid arthritis, psoriasis or juvenile idiopathic arthritis.

In one embodiment, the present invention provides pharmaceutical compositions comprising a compound of the invention, and another therapeutic agent. In a particular embodiment, the other therapeutic agent is an inflammatory diseases treatment agent.. In a particular embodiment, the inflammatory disease is selected from rheumatoid arthritis, osteoarthritis, juvenile idiopathic arthritis, psoriasis, psoriatic arthritis, ankylosing spondylitis, allergic airway diseases (*e.g.*, asthma, rhinitis), chronic obstructive pulmonary disease (COPD), inflammatory bowel diseases (*e.g.*, Crohn's disease, ulcerative colitis), endotoxin-driven disease states (*e.g.*, complications after bypass surgery or chronic endotoxin states contributing to *e.g.,* chronic cardiac failure), and related diseases involving cartilage, such as that of the joints. More particularly, the inflammatory disease is rheumatoid arthritis, psoriasis or juvenile idiopathic arthritis.

In one embodiment, the present invention provides compounds of the invention or pharmaceutical compositions comprising a compound of the invention, for use in the prophylaxis and/or treatment of autoimmune diseases. In a particular embodiment, the autoimmune disease is selected from obstructive airways disease, including conditions such as COPD, asthma (*e.g.*, intrinsic asthma, extrinsic asthma, dust asthma, infantile asthma) particularly chronic or inveterate asthma (for example late asthma and airway hyperresponsiveness), bronchitis, including bronchial asthma, systemic lupus erythematosus (SLE), cutaneous lupus erythematosus, lupus nephritis, dermatomyositis, Sjögren's syndrome, multiple sclerosis, psoriasis, dry eye disease, type I diabetes mellitus and complications associated therewith, atopic

eczema (atopic dermatitis), thyroiditis (Hashimoto's and autoimmune thyroiditis), contact dermatitis and further eczematous dermatitis, inflammatory bowel disease (*e.g.*, Crohn's disease and ulcerative colitis), atherosclerosis and amyotrophic lateral sclerosis. More particularly, the autoimmune disease is systemic lupus erythematosus.

In another embodiment, the present invention provides compounds of the invention, or pharmaceutical compositions comprising a compound of the invention for use in the manufacture of a medicament for use in the prophylaxis and/or treatment of autoimmune diseases. In a particular embodiment, the autoimmune disease is selected from obstructive airways disease, including conditions such as COPD, asthma (*e.g.*, intrinsic asthma, extrinsic asthma, dust asthma, infantile asthma) particularly chronic or inveterate asthma (for example late asthma and airway hyperresponsiveness), bronchitis, including bronchial asthma, systemic lupus erythematosus (SLE), cutaneous lupus erythematosus, lupus nephritis, dermatomyositis, Sjögren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.*, Crohn's disease and ulcerative colitis), atherosclerosis and amyotrophic lateral sclerosis. More particularly, the autoimmune disease is systemic lupus erythematosus.

In additional method of treatment aspects, this disclosure provides methods of prophylaxis and/or treatment of a mammal afflicted with autoimmune diseases, which methods comprise the administration of an effective amount of a compound of the invention or one or more of the pharmaceutical compositions herein described for the treatment or prophylaxis of said condition. In a particular embodiment, the autoimmune disease is selected from obstructive airways disease, including conditions such as COPD, asthma (*e.g.*, intrinsic asthma, extrinsic asthma, dust asthma, infantile asthma) particularly chronic or inveterate asthma (for example late asthma and airway hyperresponsiveness), bronchitis, including bronchial asthma, systemic lupus erythematosus (SLE), cutaneous lupus erythematosus, lupus nephritis, dermatomyositis, Sjögren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.*, Crohn's disease and ulcerative colitis), atherosclerosis and amyotrophic lateral sclerosis. More particularly, the autoimmune disease is systemic lupus erythematosus.

In one embodiment, the present invention provides pharmaceutical compositions comprising a compound of the invention, and another therapeutic agent. In a particular embodiment, the other therapeutic agent is a autoimmune disease treatment agent. In a particular embodiment, the autoimmune disease is selected from obstructive airways disease, including conditions such as COPD, asthma (*e.g.*, intrinsic asthma, extrinsic asthma, dust asthma, infantile asthma) particularly chronic or inveterate asthma (for example late asthma and airway hyperresponsiveness), bronchitis, including bronchial asthma, systemic lupus erythematosus (SLE), cutaneous lupus erythematosus, lupus nephritis, dermatomyositis, Sjögren's syndrome, multiple sclerosis, psoriasis, dry eye disease, type I diabetes mellitus and complications associated therewith, atopic eczema (atopic dermatitis), thyroiditis (Hashimoto's and autoimmune thyroiditis), contact dermatitis and further eczematous dermatitis, inflammatory bowel disease (*e.g.*, Crohn's disease and ulcerative colitis), atherosclerosis and amyotrophic lateral sclerosis. More particularly, the autoimmune disease is systemic lupus erythematosus.

In one embodiment, the present invention provides compounds of the invention or pharmaceutical compositions comprising a compound of the invention, for use in the prophylaxis and/or treatment of pain. In a particular embodiment, the pain is selected from nociceptive pain (for example visceral pain, and/or somatic pain), inflammatory pain (associated with tissue damage and inflammatory cell infiltration) and neuropathic or dysfunctional pain (caused by damage to or abnormal function of the nervous system), and/or pain associated or caused by the conditions mentioned herein. More particularly, the pain is inflammatory and/or neuropathic pain.

In another embodiment, the present invention provides compounds of the invention, or pharmaceutical compositions comprising a compound of the invention for use in the manufacture of a medicament for use in the prophylaxis and/or treatment of pain. In a particular embodiment, the pain is selected from nociceptive pain (for example visceral pain, and/or somatic pain), inflammatory pain (associated with tissue damage and inflammatory cell infiltration) and neuropathic or dysfunctional pain (caused by damage to or abnormal function of the nervous system), and/or pain associated or caused by the conditions mentioned herein. More particularly, the pain is inflammatory and/or neuropathic pain.

In additional method of treatment aspects, this disclosure provides methods of prophylaxis and/or treatment of a mammal afflicted with pain, which methods comprise the administration of an effective amount of a compound of the invention or one or more of the pharmaceutical compositions herein described for the treatment or prophylaxis of said condition. In a particular embodiment, the pain is selected from nociceptive pain (for example visceral pain, and/or somatic pain), inflammatory pain (associated with tissue damage and inflammatory cell infiltration) and neuropathic or dysfunctional pain (caused by damage to or abnormal function of the nervous system), and/or pain associated or caused by the conditions mentioned herein. More particularly, the pain is inflammatory and/or neuropathic pain.

In one embodiment, the present invention provides pharmaceutical compositions comprising a compound of

the invention, and another therapeutic agent. In a particular embodiment, the other therapeutic agent is a pain treatment agent. In a particular embodiment, the pain is selected from nociceptive pain (for example visceral pain, and/or somatic pain), inflammatory pain (associated with tissue damage and inflammatory cell infiltration) and neuropathic or dysfunctional pain (caused by damage to or abnormal function of the nervous system), and/or pain associated or caused by the conditions mentioned herein. More particularly, the pain is inflammatory and/or neuropathic pain.

In one embodiment, the present invention provides compounds of the invention or pharmaceutical compositions comprising a compound of the invention, for use in the prophylaxis and/or treatment of fibrosis. In a particular embodiment, the fibrosis is selected from systemic sclerosis, idiopathic pulmonary fibrosis and other forms of lung fibrosis and interstitial lung diseases, alcoholic steatohepatitis, non-alcoholic steatohepatitis, renal fibrosis, and fibrosis of the colon as a consequence of inflammatory bowel diseases. More particularly, the fibrosis is sclerodermatous chronic graft versus host disease.

In another embodiment, the present invention provides compounds of the invention, or pharmaceutical compositions comprising a compound of the invention for use in the manufacture of a medicament for use in the prophylaxis and/or treatment of fibrosis. In a particular embodiment, the fibrosis is selected from systemic sclerosis, idiopathic pulmonary fibrosis and other forms of lung fibrosis and interstitial lung diseases, alcoholic steatohepatitis, non-alcoholic steatohepatitis, renal fibrosis, and fibrosis of the colon as a consequence of inflammatory bowel diseases. More particularly, the fibrosis is sclerodermatous chronic graft versus host disease.

In additional method of treatment aspects, this disclosure provides methods of prophylaxis and/or treatment of a mammal afflicted with fibrosis, which methods comprise the administration of an effective amount of a compound of the invention or one or more of the pharmaceutical compositions herein described for the treatment or prophylaxis of said condition. In a particular embodiment, the fibrosis is selected from systemic sclerosis, idiopathic pulmonary fibrosis and other forms of lung fibrosis and interstitial lung diseases, alcoholic steatohepatitis, non-alcoholic steatohepatitis, renal fibrosis, and fibrosis of the colon as a consequence of inflammatory bowel diseases. More particularly, the fibrosis is sclerodermatous chronic graft versus host disease.

In one embodiment, the present invention provides pharmaceutical compositions comprising a compound of the invention, and another therapeutic agent. In a particular embodiment, the other therapeutic agent is a fibrosis treatment agent. In a particular embodiment, the fibrosis is selected from systemic sclerosis, idiopathic pulmonary fibrosis and other forms of lung fibrosis and interstitial lung diseases, alcoholic steatohepatitis, non-alcoholic steatohepatitis, renal fibrosis, and fibrosis of the colon as a consequence of inflammatory bowel diseases. More particularly, the fibrosis is sclerodermatous chronic graft versus host disease.

In one embodiment, the present invention provides compounds of the invention or pharmaceutical compositions comprising a compound of the invention, for use in the prophylaxis and/or treatment of proliferative diseases. In a particular embodiment, the proliferative disease is selected from cancer (*e.g.*, uterine leiomyosarcoma or prostate cancer), myeloproliferative disorders (*e.g.*, polycythemia vera, essential thrombocytosis and myelofibrosis), leukemia (*e.g.*, acute myeloid leukemia, acute and chronic lymphoblastic leukemia), multiple myeloma, psoriasis, restenosis, scleroderma or fibrosis. In a particular embodiment, the proliferative disease is sclerodermatous chronic graft-versus-host disease (cGvHD).

In another embodiment, the present invention provides compounds of the invention, or pharmaceutical compositions comprising a compound of the invention for use in the manufacture of a medicament for use in the prophylaxis and/or treatment of proliferative diseases. In a particular embodiment, the proliferative disease is selected from cancer (*e.g.*, uterine leiomyosarcoma or prostate cancer), myeloproliferative disorders (*e.g.*, polycythemia vera, essential thrombocytosis and myelofibrosis), leukemia (*e.g.*, acute myeloid leukemia, acute and chronic lymphoblastic leukemia), multiple myeloma, psoriasis, restenosis, scleroderma or fibrosis. In a particular embodiment, the proliferative disease is sclerodermatous chronic graft-versus-host disease (cGvHD).

In additional method of treatment aspects, this disclosure provides methods of prophylaxis and/or treatment of a mammal afflicted with a proliferative disease, which methods comprise the administration of an effective amount of a compound of the invention or one or more of the pharmaceutical compositions herein described for the treatment or prophylaxis of said condition. In a particular embodiment, the proliferative disease is selected from cancer (*e.g.*, uterine leiomyosarcoma or prostate cancer), myeloproliferative disorders (*e.g.*, polycythemia vera, essential thrombocytosis and myelofibrosis), leukemia (*e.g.*, acute myeloid leukemia, acute and chronic lymphoblastic leukemia), multiple myeloma, psoriasis, restenosis, scleroderma or fibrosis. In a particular embodiment, the proliferative disease is sclerodermatous chronic graft-versus-host disease (cGvHD).

In one embodiment, the present invention provides pharmaceutical compositions comprising a compound of the invention, and another therapeutic agent. In a particular embodiment, the other therapeutic agent is a proliferative disease treatment agent. In a particular embodiment, the proliferative disease is selected from cancer (*e.g.*, uterine leiomyosarcoma or prostate cancer), myeloproliferative disorders (*e.g.*, polycythemia vera, essential thrombocytosis and myelofibrosis), leukemia (*e.g.*, acute myeloid leukemia, acute and chronic lymphoblastic leukemia), multiple myeloma, psoriasis, restenosis, scleroderma or fibrosis. In a particular embodiment, the proliferative disease is sclerodermatous chronic graft-versus-host disease (cGvHD).

Injection dose levels range from about 0.1 mg/kg/h to at least 10 mg/kg/h, all for from about 1 to about 120 h and especially 24 to 96 h. A preloading bolus of from about 0.1 mg/kg to about 10 mg/kg or more may also be administered to achieve adequate steady state levels. The maximum total dose is not expected to exceed about 1 g/day for a 40 to 80 kg human patient.

For the prophylaxis and/or treatment of long-term conditions, such as degenerative conditions, the regimen for treatment usually stretches over many months or years so oral dosing is preferred for patient convenience and tolerance. With oral dosing, one to four (1-4) regular doses daily, especially one to three (1-3) regular doses daily, typically one to two (1-2) regular doses daily, and most typically one (1) regular dose daily are representative regimens. Alternatively for long lasting effect drugs, with oral dosing, once every other week, once weekly, and once a day are representative regimens. In particular, dosage regimen can be every 1-14 days, more particularly 1-10 days, even more particularly 1-7 days, and most particularly 1-3 days.

Using these dosing patterns, each dose provides from about 1 to about 1000 mg of a compound of the invention, with particular doses each providing from about 10 to about 500 mg and especially about 30 to about 250 mg.

Transdermal doses are generally selected to provide similar or lower blood levels than are achieved using injection doses.

When used to prevent the onset of a condition, a compound of the invention will be administered to a patient at risk for developing the condition, typically on the advice and under the supervision of a physician, at the dosage levels described above. Patients at risk for developing a particular condition generally include those that have a family history of the condition, or those who have been identified by genetic testing or screening to be particularly susceptible to developing the condition.

A compound of the invention can be administered as the sole active agent or it can be administered in combination with other therapeutic agents, including other compound of the inventions that demonstrate the same or a similar therapeutic activity and that are determined to be safe and efficacious for such combined administration. In a specific embodiment, co-administration of two (or more) agents allows for significantly lower doses of each to be used, thereby reducing the side effects seen.

In one embodiment, a compound of the invention or a pharmaceutical composition comprising a compound of the invention is administered as a medicament. In a specific embodiment, said pharmaceutical composition additionally comprises a further active ingredient.

In one embodiment, a compound of the invention is co-administered with another therapeutic agent for the treatment and/or prophylaxis of a disease involving inflammation, particular agents include, but are not limited to, immunoregulatory agents *e.g.* azathioprine, corticosteroids (*e.g.* prednisolone or dexamethasone), cyclophosphamide, cyclosporin A, tacrolimus, mycophenolate, mofetil, muromonab-CD3 (OKT3, *e.g.* Orthocolone®), ATG, aspirin, acetaminophen, ibuprofen, naproxen, and piroxicam.

In one embodiment, a compound of the invention is co-administered with another therapeutic agent for the treatment and/or prophylaxis of arthritis (*e.g.* rheumatoid arthritis), particular agents include but are not limited to analgesics, non-steroidal anti-inflammatory drugs (NSAIDS), steroids, synthetic DMARDS (for example but without limitation methotrexate, leflunomide, sulfasalazine, auranofin, sodium aurothiomalate, penicillamine, chloroquine, hydroxychloroquine, azathioprine, tofacitinib, baricitinib, fostamatinib, and cyclosporin), and biological DMARDS (for example but without limitation infliximab, etanercept, adalimumab, rituximab, and abatacept).

In one embodiment, a compound of the invention is co-administered with another therapeutic agent for the treatment and/or prophylaxis of proliferative disorders, particular agents include but are not limited to: methotrexate, leukovorin, adriamycin, prednisone, bleomycin, cyclophosphamide, 5-fluorouracil, paclitaxel, docetaxel, vincristine, vinblastine, vinorelbine, doxorubicin, tamoxifen, toremifene, megestrol acetate, anastrozole, goserelin, anti-HER2 monoclonal antibody (*e.g.* Herceptin™), capecitabine, raloxifene hydrochloride, EGFR inhibitors (*e.g.* Iressa®, Tarceva™, Erbitux™), VEGF inhibitors (*e.g.* Avastin™), proteasome inhibitors *(e.g.* Velcade™), Glivec® and hsp90 inhibitors (*e.g.* 17-AAG). Additionally, the compound of the invention according to Formula I may be administered in combination with other therapies including, but not limited to, radiotherapy or surgery. In a specific embodiment the proliferative disorder is selected from cancer, myeloproliferative disease or leukaemia.

In one embodiment, a compound of the invention is co-administered with another therapeutic agent for the treatment and/or prophylaxis of autoimmune diseases, particular agents include but are not limited to: glucocorticoids, cytostatic agents (*e.g.* purine analogs), alkylating agents, (*e.g* nitrogen mustards (cyclophosphamide), nitrosoureas, platinum compound of the inventions, and others), antimetabolites (*e.g.* methotrexate, azathioprine and mercaptopurine), cytotoxic antibiotics (*e.g.* dactinomycin anthracyclines, mitomycin C, bleomycin, and mithramycin), antibodies (*e.g.* anti-CD20, anti-CD25 or anti-CD3 (OTK3) monoclonal antibodies, Atgam® and Thymoglobuline®), cyclosporin, tacrolimus, rapamycin (sirolimus), interferons (*e.g.* IFN-β), TNF binding proteins (*e.g.* infliximab, etanercept, or adalimumab), mycophenolate, fingolimod and myriocin..

In one embodiment, a compound of the invention is co-administered with another therapeutic agent for the

treatment and/or prophylaxis of transplant rejection, particular agents include but are not limited to: calcineurin inhibitors (*e.g.* cyclosporin or tacrolimus (FK506)), mTOR inhibitors (*e.g.* sirolimus, everolimus), anti-proliferatives (*e.g.* azathioprine, mycophenolic acid), corticosteroids (*e.g.* prednisolone, hydrocortisone), antibodies (*e.g.* monoclonal anti-IL-2Rα receptor antibodies, basiliximab, daclizumab), polyclonal anti-T-cell antibodies (*e.g.* anti-thymocyte globulin (ATG), anti-lymphocyte globulin (ALG)).

In one embodiment, a compound of the invention is co-administered with another therapeutic agent for the treatment and/or prophylaxis of asthma and/or rhinitis and/or COPD, particular agents include but are not limited to: beta2-adrenoceptor agonists (*e.g.* salbutamol, levalbuterol, terbutaline and bitolterol), epinephrine (inhaled or tablets), anticholinergics (*e.g.* ipratropium bromide), glucocorticoids (oral or inhaled). Long-acting β2-agonists (*e.g.* salmeterol, formoterol, bambuterol, and sustained-release oral albuterol), combinations of inhaled steroids and long-acting bronchodilators (*e.g.* fluticasone/salmeterol, budesonide/formoterol), leukotriene antagonists and synthesis inhibitors (*e.g.* montelukast, zafirlukast and zileuton), inhibitors of mediator release (*e.g.* cromoglycate and ketotifen), biological regulators of IgE response (*e.g.* omalizumab), antihistamines (*e.g.* ceterizine, cinnarizine, fexofenadine) and vasoconstrictors (*e.g.* oxymethazoline, xylomethazoline, nafazoline and tramazoline).

Additionally, a compound of the invention may be administered in combination with emergency therapies for asthma and/or COPD, such therapies include oxygen or heliox administration, nebulized salbutamol or terbutaline (optionally combined with an anticholinergic (*e.g.* ipratropium), systemic steroids (oral or intravenous, *e.g.* prednisone, prednisolone, methylprednisolone, dexamethasone, or hydrocortisone), intravenous salbutamol, non-specific beta-agonists, injected or inhaled (*e.g.* epinephrine, isoetharine, isoproterenol, metaproterenol), anticholinergics (IV or nebulized, *e.g.* glycopyrrolate, atropine, ipratropium), methylxanthines (theophylline, aminophylline, bamiphylline), inhalation anesthetics that have a bronchodilatory effect (*e.g.* isoflurane, halothane, enflurane), ketamine and intravenous magnesium sulfate.

In one embodiment, a compound of the invention is co-administered with another therapeutic agent for the treatment and/or prophylaxis of inflammatory bowel disease (IBD), particular agents include but are not limited to: glucocorticoids (*e.g.* prednisone, budesonide) synthetic disease modifying, immunomodulatory agents (*e.g.* methotrexate, leflunomide, sulfasalazine, mesalazine, azathioprine, 6-mercaptopurine and cyclosporin) and biological disease modifying, immunomodulatory agents (infliximab, adalimumab, rituximab, and abatacept).

In one embodiment, a compound of the invention is co-administered with another therapeutic agent for the treatment and/or prophylaxis of SLE, particular agents include but are not limited to: human monoclonal antibodies (belimumab (Benlysta)), Disease-modifying antirheumatic drugs (DMARDs) such as antimalarials (*e.g.* plaquenil, hydroxychloroquine), immunosuppressants (*e.g.* methotrexate and azathioprine), cyclophosphamide and mycophenolic acid, immunosuppressive drugs and analgesics, such as nonsteroidal anti-inflammatory drugs, opiates (*e.g.* dextropropoxyphene and co-codamol), opioids (*e.g.* hydrocodone, oxycodone, MS Contin, or methadone) and the fentanyl duragesic transdermal patch.

In one embodiment, a compound of the invention is co-administered with another therapeutic agent for the treatment and/or prophylaxis of psoriasis, particular agents include but are not limited to: topical treatments such as bath solutions, moisturizers, medicated creams and ointments containing coal tar, dithranol (anthralin), corticosteroids like desoximetasone (Topicort™), fluocinonide, vitamin D3 analogues (for example, calcipotriol), argan oil and retinoids (etretinate, acitretin, tazarotene), systemic treatments such as methotrexate, cyclosporine, retinoids, tioguanine, hydroxyurea, sulfasalazine, mycophenolate mofetil, azathioprine, tacrolimus, fumaric acid esters or biologics such as Amevive™, Enbrel™, Humira™, Remicade™, Raptiva™ and ustekinumab (a IL-12 and IL-23 blocker). Additionally, a compound of the invention may be administered in combination with other therapies including, but not limited to phototherapy, or photochemotherapy (*e.g.* psoralen and ultraviolet A phototherapy (PUVA)).

In one embodiment, a compound of the invention is co-administered with another therapeutic agent for the treatment and/or prophylaxis of allergic reaction, particular agents include but are not limited to: antihistamines (*e.g.* cetirizine, diphenhydramine, fexofenadine, levocetirizine), glucocorticoids (*e.g.* prednisone, betamethasone, beclomethasone, dexamethasone), epinephrine, theophylline or anti-leukotrienes (*e.g.* montelukast or zafirlukast), anti-cholinergics and decongestants.

By co-administration is included any means of delivering two or more therapeutic agents to the patient as part of the same treatment regime, as will be apparent to the skilled person. Whilst the two or more agents may be administered simultaneously in a single formulation, *i.e.* as a single pharmaceutical composition, this is not essential. The agents may be administered in different formulations and at different times.

## CHEMICAL SYNTHETIC PROCEDURES

### General

The compound of the invention can be prepared from readily available starting materials using the following

general methods and procedures. It will be appreciated that where typical or preferred process conditions (*i.e.* reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art (Greene, T W; Wuts, P G M;, 1991).

The following methods are presented with details as to the preparation of a compound of the invention as defined hereinabove and the comparative examples. A compound of the invention may be prepared from known or commercially available starting materials and reagents by one skilled in the art of organic synthesis.

All reagents were of commercial grade and were used as received without further purification, unless otherwise stated. Commercially available anhydrous solvents were used for reactions conducted under inert atmosphere. Reagent grade solvents were used in all other cases, unless otherwise specified. Column chromatography was performed on silica gel 60 (35-70 μm). Thin layer chromatography was carried out using pre-coated silica gel F-254 plates (thickness 0.25 mm). $^1H$ NMR spectra were recorded for example on a Bruker DPX 400 NMR spectrometer (400 MHz) or a Bruker Advance 300 NMR spectrometer (300 MHz). Chemical shifts (δ) for $^1H$ NMR spectra are reported in parts per million (ppm) relative to tetramethylsilane (δ 0.00) or the appropriate residual solvent peak, i.e. $CHCl_3$ (δ 7.27), as internal reference. Multiplicities are given as singlet (s), doublet (d), triplet (t), quartet (q), quintuplet (quin), multiplet (m) and broad (br). Electrospray MS spectra were obtained on a Waters platform LC/MS spectrometer or with Waters Acquity H-Class UPLC coupled to a Waters Mass detector 3100 spectrometer. Columns used: Waters Acquity UPLC BEH C18 1.7 μm, 2.1mm ID x 50mm L, Waters Acquity UPLC BEH C18 1.7 μm, 2.1mm ID x 30 mm L, or Waters Xterra MS 5μm C18, 100 x 4.6mm. The methods are using either MeCN/$H_2O$ gradients ($H_2O$ contains either 0.1% TFA or 0.1% $NH_3$) or MeOH /$H_2O$ gradients ($H_2O$ contains 0.05% TFA). Microwave heating was performed with a Biotage Initiator.

**Table I. List of abbreviations used in the experimental section:**

| Abbreviation | Definition |
|---|---|
| AcOH | acetic acid |
| aq. | aqueous |
| atm | atmosphere |
| BINAP | (+/-)-2,2'-bis(diphenylphosphino)-1,1'-binaphthyl |
| Boc | *tert*-butyloxy-carbonyl |
| br | broad signal |
| BSA | bovine serum albumin |
| calc | calculated |
| cpd | compound |
| d | Doublet |
| δ | chemical shift |
| DCM | dichloromethane |
| DIPEA | diisopropylethylamine |
| DMAP | dimethylaminopyridine |
| DMF | dimethylformamide |
| DMSO | dimethylsulfoxide |
| DTT | dithiothreitol |
| EDCI | *N*-(3-dimethylaminopropyl)-*N'*-ethylcarbodiimide hydrochloride |
| EDTA | ethylenediaminetetraacetic acid |
| eq. | equivalent |
| ES- | electrospray negative |
| ES+ | electrospray positive |
| $Et_2O$ | diethyl ether |
| EtOAc | ethyl acetate |
| EtOH | ethanol |
| g | gram |
| h | hour |

| Abbreviation | Definition |
|---|---|
| HATU | 1-[Bis(dimethylamino) methylene]-1H-1,2,3-triazolo[4,5-b]pyridinium 3-oxid hexafluorophosphate |
| HPLC | high performance liquid chromatography |
| Hz | hertz |
| Int | intermediate |
| iPrOH | isopropanol |
| LiOMe | lithium methoxide |
| LiOtBu | lithium *tert*-butoxide |
| m | multiplet |
| MeCN | acetonitrile |
| MeOH | methanol |
| mg | milligram |
| MgOAc | magnesium acetate |
| MHz | megahertz |
| min | minute |
| mL | millilitre |
| mmol | millimole |
| mol | mole |
| MS | mass spectrometry |
| MW | molecular weight |
| N | normality |

| Abbreviation | Definition |
|---|---|
| $NaBH(OAc)_3$ | sodium triacetoxyborohydride |
| NaOtBu | sodium *tert*-butylate |
| NBS | *N*-bromosuccinimide |
| ND | Not determined |
| NMR | nuclear magnetic resonance |
| Obs | observed |
| PBS | phosphate-buffered saline |
| PBST | phosphate-buffered saline with Tween 20 |
| $Pd(OAc)_2$ | palladium diacetate |
| Pd/C | palladium on carbon |
| ppm | part-per-million |
| q | quartet |
| quin | quintet |
| r.t. | room temperature |
| s | singlet |
| sat. | saturated |
| SEM | standard error of the mean |
| t | triplet |
| TEA | triethylamine |
| TFA | trifluoroacetic acid |
| THF | tetrahydrofurane |
| UPLC | ultra-performance liquid chromatography |

## SYNTHETIC PREPARATION OF THE COMPOUNDS OF THE INVENTION

### Example 1. Preparation of intermediates towards illustrative compounds of the invention

#### *1.1. Intermediate 1: ethyl pent-4-ynoate*

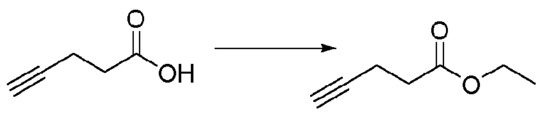

10 mL of concentrated sulfuric acid were added to a solution of pent-4-ynoic acid (10.0 g, 102.0 mmol) in EtOH

(110 mL). The reaction was stirred at r.t. overnight. The mixture was diluted with $H_2O$ (250 mL) and 5% NaOH in $H_2O$ (25 mL) and extracted with EtzO (3 x 200 mL). The organic layer was dried (filtered through hydrophobic frit) and concentrated to afford the desired product.

***1.2. Intermediate 2: 2-methylhex-5-yn-2-ol***

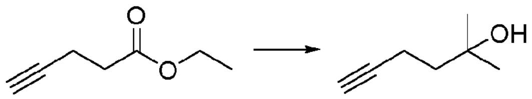

A solution of ethyl pent-4-ynoate (5.00 g, 39.6 mmol, 1.0 eq.) in dry EtzO (24.0 mL) was added dropwise at -78 °C to a mixture of 3 M methylmagnesium bromide in EtzO (27.7 mL, 83.2 mmol, 2.1 eq.) diluted in dry EtzO (50.0 mL). The reaction mixture was stirred at -78 °C for 1 h and then it was allowed to warm to r.t. over a period of 30 min. The mixture was quenched with $NH_4Cl$ (saturated solution, 80 mL) and water (20 mL) and extracted with EtzO (3 x 50 mL). The organic layer was dried and concentrated under reduced pressure. The residue was purified by flash column chromatography (SiOz, 100:0 to 70:30 cyclohexane/EtOAc) to afford the desired product.

***1.3. Intermediate 3: tert-butyl-(1,1-dimethylpent-4-ynoxy)-dimethyl-silane***

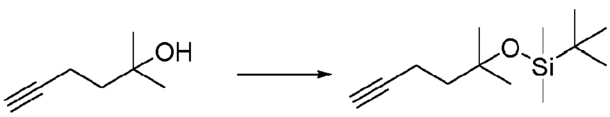

[tert-butyl(dimethyl)silyl] trifluoromethanesulfonate (5.6 g, 21.2 mmol, 1.25 eq.) was added to a solution of 2-methylhex-5-yn-2-ol (1.9 g, 16.9 mmol, 1.0 eq.) and pyridine (3.41 mL, 42.3 mmol, 2.5 eq.) in DCM (40 mL). The reaction mixture was stirred at r.t. for 2 h. The mixture was treated with saturated aq. $NaHCO_3$. The mixture was extracted with DCM. The organic layer was dried ($Na_2SO_4$), filtered and concentrated. The residue was purified by flash column chromatography (SiOz, cyclohexane) to afford the desired product.

***1.4. Intermediate 4: ethyl 6-[tert-butyl(dimethyl)silyl]oxy-6-methyl-hept-2-ynoate***

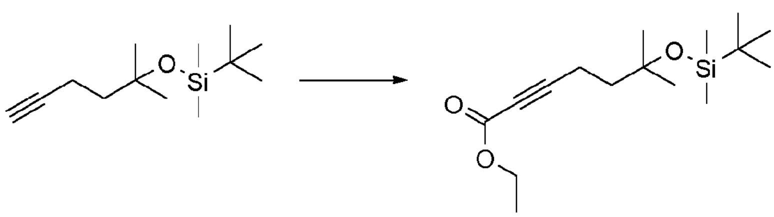

2.5 M *n*-butyllithium in hexanes (7.0 mL, 17.4 mmol, 1.2 eq.) was added to a solution of tert-butyl-(1,1-dimethylpent-4-ynoxy)-dimethyl-silane (3.3 g, 14.5 mmol, 1.0 eq.) in dry THF (150 mL) at -78 °C. The mixture was stirred for 1 h at at -78 °C and ethyl chloroformate (2.37 g, 21.8 mmol, 1.5 eq.) was added. The resulting mixture was stirred at -78 °C for 1 h and then was allowed to warm to r.t. The mixture was quenched (saturated $NH_4Cl$ aq. solution, 150 mL) and extracted (EtzO). The organic layer was dried and concentrated. The residue was purified by flash column chromatography ($SiO_2$, 100:0 to 80:20 cyclohexane/EtOAc) to afford the desired product.

***1.5. Intermediate 5: tert-butyl N-(3-methoxy-4-pyridyl)carbamate***

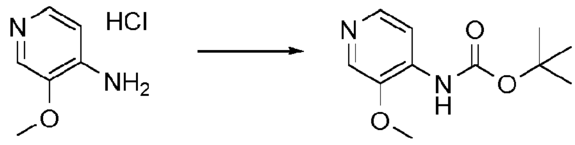

DIPEA (4.6 mL, 26.2 mmol, 2.1 eq.) and di-tert-butyl dicarbonate (3.0 g, 13.7 mmol, 1.1 eq.) were added to a solution of 3-methoxypyridin-4-amine HCl salt (2.0 g, 12.5 mmol, 1.0 eq.) in THF (15.5 mL). The reaction mixture was stirred at r.t. overnight. The mixture was treated with saturated aq. $NH_4Cl$ and extracted (EtOAc). The organic layer was washed (brine) dried ($Na_2SO_4$) and concentrated to afford the desired product.

***1.6. Intermediate 6: ethyl 5-(tert-butoxycarbonylamino)-2-[3-[tert-butyl(dimethyl)silyl]oxy-3-methyl-butyl]-6-methoxy-pyrazolo[1,5-a]pyridine-3-carboxylate***

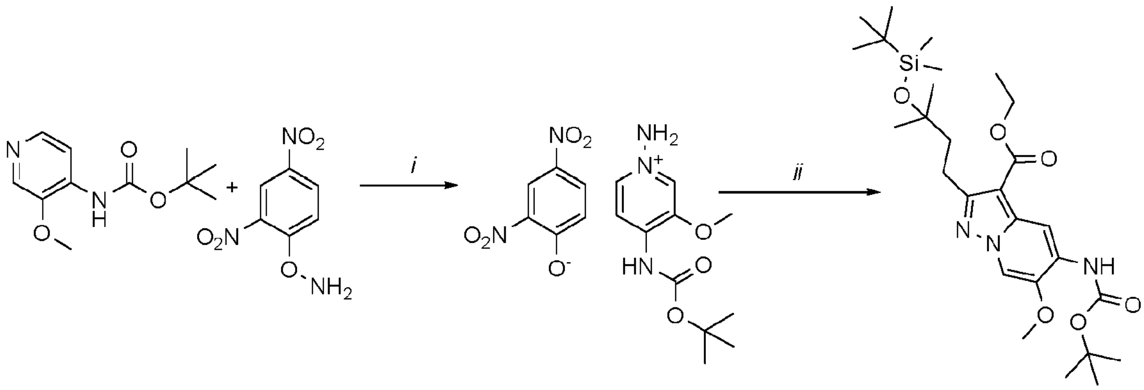


***1.6.1. Step i (tert-butyl N-(1-amino-3-methoxy-pyridin-1-ium-4-yl)carbamate 2,4-dinitrophenolate salt** :*

A mixture of N-(3-methoxy-4-pyridyl)carbamate (3.46 g, 15.4 mmol, 1.0 eq.) and O-(2,4-dinitrophenyl)hydroxylamine (6.14 g, 30.9 mmol, 2.0 eq.) in MeCN (27 mL) was stirred at 50 °C overnight. The mixture was concentrated to afford the desired product.

***1.6.2. Step ii, ethyl 5-(tert-butoxycarbonylamino)-2-[3-[tert-butyl(dimethyl)silyl]oxy-3-methyl-butyl]-6-methoxy-pyrazolo[1,5-a]pyridine-3-carboxylate:***

A mixture containing 2.0 g of tert-butyl N-(1-amino-3-methoxy-pyridin-1-ium-4-yl)carbamate 2,4-dinitrophenolate salt obtained from *Step i* and $K_2CO_3$ (1.96 g, 14.2 mmol) in DMF (8 mL) was stirred at r.t. for 1 h. Ethyl 6-[tert-butyl(dimethyl)silyl]oxy-6-methylhept-2-ynoate (1.41 g, 4.7 mmol) dissolved in a minimal amount of DMF was added to the reaction mixture which was 1et to stir at r.t. for approximately 20 h. A further 0.28 g of ethyl 6-[tert-butyl(dimethyl)silyl]oxy-6-methylhept-2-ynoate (0.9 mmol) were added and the mixture was stirred at r.t. for a further 20 h. The mixture was diluted with $H_2O$. The resulting mixture was extracted with EtOAc. The organic layer was washed ($H_2O$, brine), dried ($Na_2SO_4$) and concentrated. The residue was purified by flash column chromatography (SiOz, 75:25 n-hexane/EtOAc) to afford the desired product.

***1.7. Intermediate 7: 2-[3-[tert-butyl(dimethyl)silyl]oxy-3-methyl-butyl]-6-methoxy-pyrazolo[1,5-a]pyridin-5-amine***

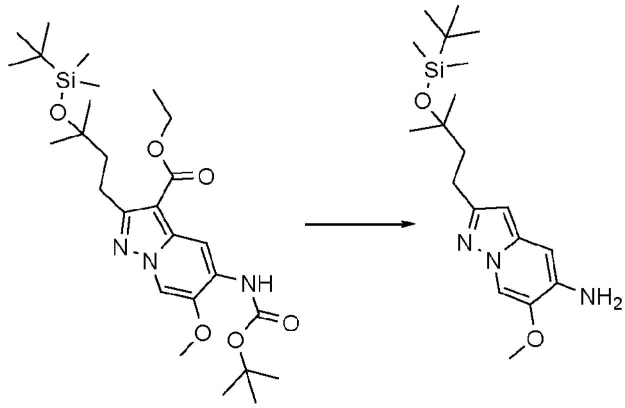

A mixture of ethyl 5-(tert-butoxycarbonylamino)-2-[3-[tert-butyl(dimethyl)silyl]oxy-3-methylbutyl]-6-methoxy-pyrazolo[1,5-a]pyridine-3-carboxylate (427 mg, 0.8 mmol, 1.0 eq.) and LiOH (382 mg, 15.9 mmol, 20.0 eq.) in 9:1 MeOH/$H_2O$ was stirred at 100 °C overnight. The reaction mixture was partitioned between EtOAc and HzO. The two phases were separated and the aq. layer was extracted with EtOAc. The combined organic layers were dried ($Na_2SO_4$) and concentrated. The residue was taken up in toluene (8.5 mL) and refluxed for 70 min. The mixture was concentrated and the residue was purified by flash column chromatography (50:50 n-hexane/EtOAc) to afford the desired product.

***1.8. Intermediate 8: 4-(5-amino-6-methoxy-pyrazolo[1,5-a]pyridin-2-yl)-2-methyl-butan-2-ol***

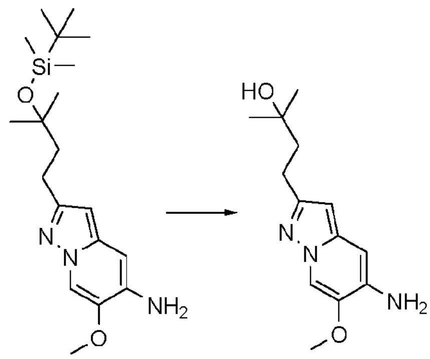

A mixture of 2-[3-[tert-butyl(dimethyl)silyl]oxy-3-methyl-butyl]-6-methoxy-pyrazolo[1,5-a]pyridin-5-amine (409 mg, 1.125 mmol, 1.0 eq.) and TFA (1.7 mL, 22.5 mmol, 20.0 eq.) in DCM (40 mL) was stirred at r.t. for 16 h. Toluene (20 mL) was added and the mixture was concentrated. The residue was taken up in MeOH (1 mL) and the mixture was loaded on a column containing a cation exchange sorbent (SCX). The column was washed with MeOH and the product was recovered by flushing the column with 7 N $NH_3$ in MeOH. The mixture so obtained was concentrated and the residue was purified by flash column chromatography (SiOz, 100:0 to 95:5 DCM/MeOH) to afford the desired product.

***1.9. Intermediate 9: ethyl 6-chloro-4-[(4-methoxyphenyl)methylamino]pyridine-3-carboxylate***

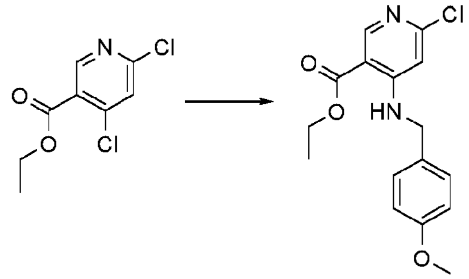

A mixture of ethyl 4,6-dichloropyridine-3-carboxylate (5.0 g, 22.7 mmol, 1.0 eq.), (4-methoxyphenyl)methanamine (3.12 g, 22.7 mmol, 1.0 eq.) and TEA (6.34 mL, 45.4 mmol, 2.0 eq.) in DMSO (45 mL) was stirred at r.t. for 48 h. The mixture was diluted with EtOAc. The resulting mixture was washed ($H_2O$, brine), dried ($Na_2SO_4$) and concentrated. The residue was purified by flash column chromatography (SiOz, 100:0 to 70:30 cyclohexane/EtOAc) to afford the desired product.

### *1.10. Intermediate 10: ethyl 4-amino-6-chloro-pyridine-3-carboxylate*

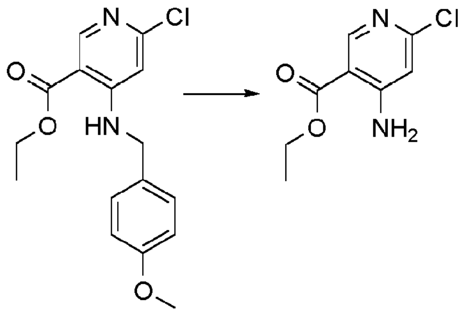

A mixture of 4-(5-amino-6-methoxy-pyrazolo[1,5-a]pyridin-2-yl)-2-methyl-butan-2-ol (5.6 g, 17.4 mmol, 1.0 eq.) in TFA (40 mL) was stirred at 50 °C for 72 h. The reaction mixture was allowed to cool and 1 M NaOH was added until pH was approximately 9. The resulting mixture was extracted (EtOAc). The organic layer was dried ($Na_2SO_4$) and concentrated. The residue was purified by flash column chromatography (SiOz, 100:0 to 70:30 cyclohexane/EtOAc) to afford the desired product.

### *1.11. Intermediate 11: ethyl 4-(tert-butoxycarbonylamino)-6-chloro-pyridine-3-carboxylate*

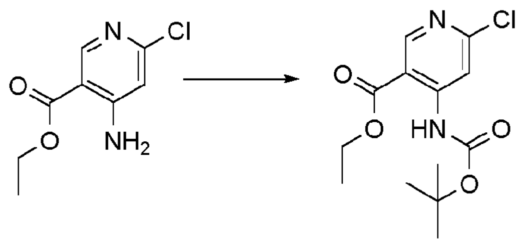

DMAP (0.213 g, 1.74 mmol, 0.1 eq.) and tert-butoxycarbonyl tert-butyl carbonate (4.19 g, 19.2 mmol, 1.1 eq.) were added to a mixture of ethyl 4-amino-6-chloro-pyridine-3-carboxylate (3.50 g, 17.4 mmol, 1.0 eq.) and TEA (9.73 mL, 69.8 mmol, 4.0 eq.) at 0 °C. The mixture was stirred for 4.5 h at r.t. The reaction was quenched with ice and diluted with $H_2O$. The resulting mixture was extracted (EtOAc). The two phases were separated and the organic layer was washed (brine), dried ($Na_2SO_4$) and concentrated. The residue was purified by flash column chromatography ($SiO_2$, 100:0 to 90:10 cyclohexane/EtOAc) to afford the desired product.

***1.12. Intermediate 12: ethyl 4-(tert-butoxycarbonylamino)-6-[5-[tert-butyl(dimethyl)silyl]oxy-5-methyl-hex-1-ynyl]pyridine-3-carboxylate***

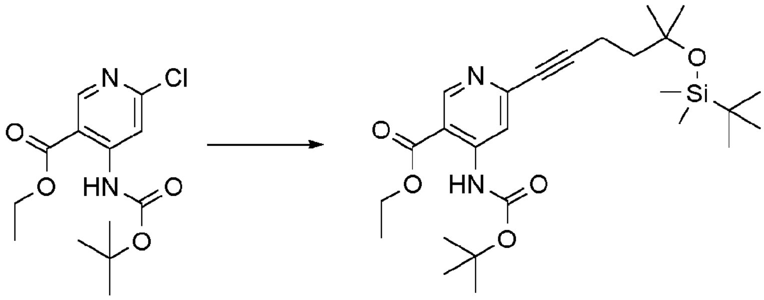

A mixture of ethyl 4-(tert-butoxycarbonylamino)-6-chloro-pyridine-3-carboxylate (1.0 g, 3.33 mmol, 1.0 eq.) and tertbutyl-(1,1-dimethylpent-4-ynoxy)-dimethyl-silane (1.13 g, 5.0 mmol, 1.5 eq.) in dry DMF (5.0 mL) was added to a mixture of $PdCl_2(PPh_3)_2$ (0.117 g, 0.166 mmol, 0.05 eq.), CuI (0.063 g, 0.33 mmol, 0.1 eq.) and TEA (6.49 mL, 46.6 mmol, 14.0 eq.) in dry DMF (25 mL) under inert atmosphere. The mixture was stirred at 90 °C overnight. The reaction mixture was stirred at 100 °C for a further 2 h. The reaction mixture was quenched with saturated aq. $NH_4Cl$. The resulting mixture was extracted (EtOAc) and the two phases were separated. The organic layer was washed (brine), dried ($Na_2SO_4$) and concentrated. The residue was purified by flash column chromatography (SiOz, 100:0 to 90: 10 cyclohexane/EtOAc) to afford the desired product.

***1.13. Intermediate 13: ethyl 5-(tert-butoxycarbonylamino)-2-[3-[tert-butyl(dimethyl)silyl]oxy-3-methyl-butyl]pyrazolo[1,5-a]pyridine-6-carboxylate***

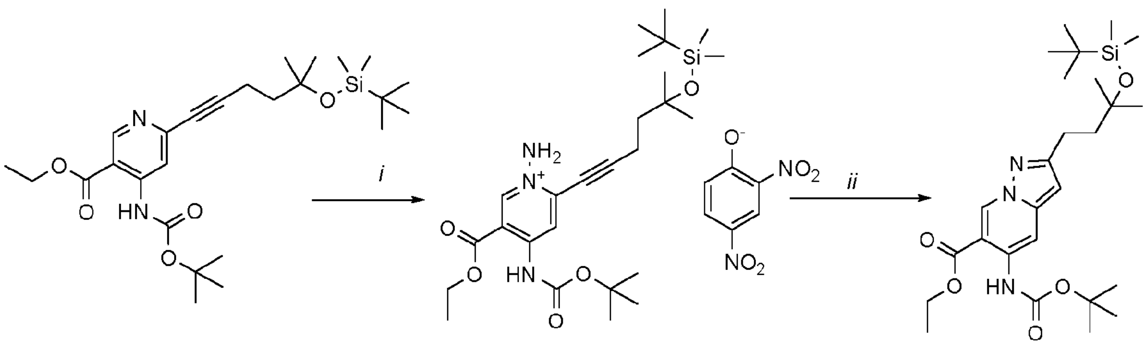


***1.13.1. Step i (ethyl 1-amino-4-(tert-butoxycarbonylamino)-6-(5-[tert-butyl(dimethyl)silyl]oxy-5-methyl-hex-1-ynyl]pyridin-1-ium-3-carboxylate 2,4-dinitrophenolate** salt:*

A mixture of ethyl 4-(tert-butoxycarbonylamino)-6-[5-[tert-butyl(dimethyl)silyl]oxy-5-methyl-hex-1-ynyl]pyndine-3-carboxylate (2.23 g, 4.54 mmol, 1.0 eq.) and O-(2,4-dinitrophenyl)hydroxylamine (1.81 g, 9.09 mmol, 2.0 eq.) in 1:1 THF/$H_2O$ (20 mL) was stirred at 50 °C overnight. The mixture was concentrated to afford the desired product.

***1.13.2. Step ii ethyl 5-(tert-butoxycarbonylamino)-2-[3-[tert-butyl(dimethyl)silyl]oxy-3-methyl-butyl]pyrazolo[1,5-a]pyridine-6-carboxylate :***

A mixture of ethyl l-amino-4-(tert-butoxycarbonylamino)-6-[5-[tert-butyl(dimethyl)silyl]oxy-5-methyl-hex-1-ynyl]pyridin-1-ium-3-carboxylate 2,4-dinitrophenolate salt obtained from Step I in DMF (30 mL) was stirred in DMF for 48 h. The mixture was diluted with aq. $NaHCO_3$ and extracted with EtOAc. The two phases were separated. The organic layer was dried ($Na_2SO_4$) and concentrated. The residue was purified by flash column chromatography (SiOz, 100:0 to 90: 10 cyclohexane/EtOAc) to afford the desired product.

***1.14. Intermediate 14: ethyl 5-amino-2-(3-hydroxy-3-methyl-butyl)pyrazolo[1,5-a]pyridine-6-carboxylate***

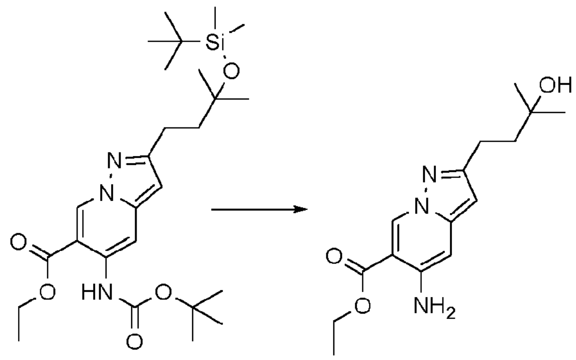

TFA (1.36 mL, 18.0 mmol, 20.0 eq.) was added to a solution of ethyl 5-(tert-butoxycarbonylamino)-2-[3-[tert-butyl(dimethyl)silyl]oxy-3-methyl-butyl]pyrazolo[1,5-a]pyridine-6-carboxylate (449 mg, 0.89 mmol, 1.0 eq.) in DCM (20 mL) at 0 °C. The reaction was stirred at r.t. overnight. The mixture was concentrated and the residue was loaded on a column containing a cation exchange sorbent (SCX). The column was washed with MeOH and the product was recovered by flushing the column with 7 N $NH_3$ in MeOH. The mixture so obtained was concentrated and the residue was purified by flash column chromatography (SiOz, 100:0 to 0:100 DCM/temary mixture constituted by 90:4:1 DCM/MeOH/$NH_4OH$) to afford the desired product.

***1.15. Intermediate 15: ethyl 2-(3-hydroxy-3-methyl-butyl)-5-[[6-(trifluoromethyl)pyridine-2-carbonyl]amino]pyrazolo[1,5-a]pyridine-6-carboxylate***

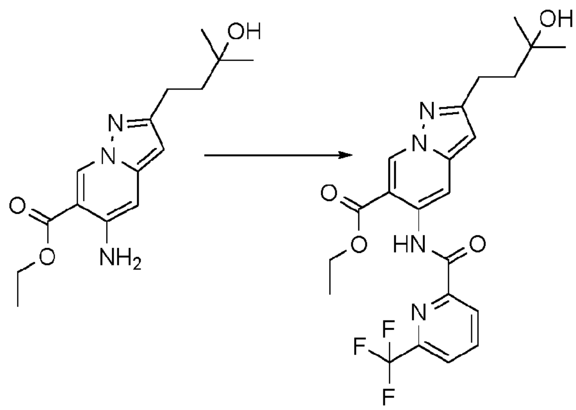

A mixture of ethyl 5-amino-2-(3-hydroxy-3-methyl-butyl)pyrazolo[1,5-a]pyridine-6-carboxylate (148.0 mg, 0.51 mmol, 1.0 eq.), DIPEA (0.18 mL, 1.02 mmol, 2.0 eq.), 6-(trifluoromethyl)pyridine-2-carboxylic acid (116 mg, 0.61 mmol, 1.2 eq.) and HATU, CAS#148893-10-1 (232 mg, 0.61 mmol, 1.2 eq.) in DCM (8 mL) was stirred at r.t. for 4 h. A further 0.3 eq. of 6-(trifluoromethyl)pyridine-2-carboxylic acid and a further 0.3 eq. of HATU, CAS# 148893-10-1 were added and the mixture was stirred at r.t. for a further 4 h. The mixture was diluted (DCM), washed (saturated aq. $NH_4Cl$, saturated aq. $NaHCO_3$, brine), dried ($Na_2SO_4$) and concentrated. The residue was purified by flash column chromatography (SiOz, 100:0 to 20:80 DCM/temary mixture constituted by 90:4:1 DCM/MeOH/$NH_4OH$) to afford the desired product.

***1.16. Intermediate 16: 2-(3-hydroxy-3-methyl-butyl)-5-[[6-(trifluoromethyl)pyridine-2-carbonyl] amino]pyrazolo[1,5-a]pyridine-6-carboxylic acid***

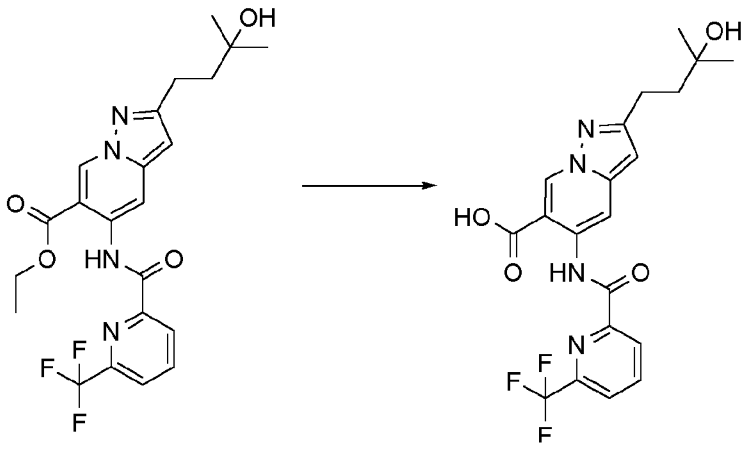

A mixture of ethyl 2-(3-hydroxy-3-methyl-butyl)-5-[[6-(trifluoromethyl)pyridine-2-carbonyl] amino]pyrazolo[1,5-a]pyridine-6-carboxylate (184 mg, 0.396 mmol, 1.0 eq.) and LiOH (29.0 mg, 1.2 mmol, 3.0 eq.) in 4:1 THF/$H_2O$ was stirred at r.t. for 5 h. THF was removed under reduced pressure and the aq. mixture was acidified to pH < 5 by dropwise addition of 1 M HCl. A solid was formed, filtered off and washed with $H_2O$ to yield the desired product.

***1.17. Intermediate 17: tert-butyl N-(2-bromo-5-methoxy-4-pyridyl)carbamate***

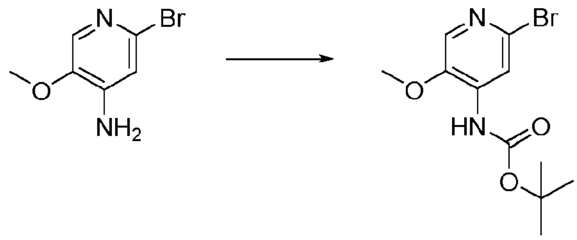

2-bromo-5-methoxy-pyridin-4-amine (175.0 g, provided by Angene, batch G02-16436-2) was dissolved in EtOAc (2 L) and water (2 L) was added. The layers were separated. The aq. layer was extracted with EtOAc (2 x 500 mL). Combined organic layers were dried over $MgSO_4$ and the mixture was evaporated to dryness. A solution containing of the residue so obtained (155 0 g, 762 mmol, 1.0 eq.) and TEA (425 mL, 3.05 mol, 4.0 eq.) in DCM (1 L) was added to a mixture containing di-tert-butyl dicarbonate (216.0 g, 990.0 mmol, 1.3 eq.) and DMAP (9.3 g, 76.0 mmol, 0.1 eq.) in DCM (2.5 L) at 0 °C, in a 5 L reactor under inert atmosphere. The addition was made dropwise while keeping the temperature below 2 °C (~ 30-35 min). After addition, the reaction mixture was warmed to 22 °C and left to stir overnight. The mixture was transferred to a 20 L reactor and quenched by addition of sat. aq. $NaHCO_3$ (5 L). The layers were separated. The aq. layer was extracted with DCM (3 × 1 L). Combined organic layers were evaporated to dryness. The residue was loaded on a pad of silica gel (20 cm thick, 19 cm in diameter) and eluted with a gradient 0-30% EtOAc/cyclohexane over 25 L. Fractions were collected, combined and concentrated to afford the desired product.

***1.18. General method B: N-amination of pyridine derivatives followed by cyclization to pyrazolo[1,5-a]pyridines***

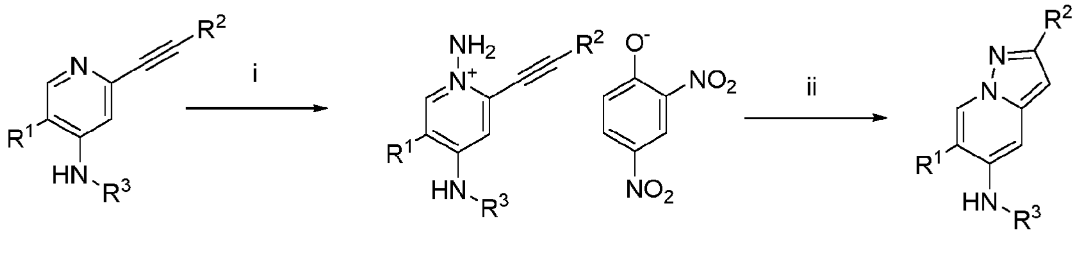

***1.18.1. Step i:***

O-(2,4-dinitrophenyl)hydroxylamine (2.0 eq.) is added portionwise to mixture of the pyridine derivative (1 eq.) in 1: 1 THF/$F_2O$ (approximately 0.2 M). The mixture is stirred at 45 to 50 °C for 16 h. The mixture is concentrated.

***1.18.2. Step ii:***

The residue is taken up in DMF (0.2 to 0.4 M) and the mixture is stirred at 90 °C for 16 h. The mixture is cooled to r.t. and quenched with a basic aq. solution (sat. $NaHCO_3$ or sat. $Na_2CO_3$). The mixture is extracted with an EtOAc. The layers are separated and the organic mixture may undergo further washing. The organic layer is dried and concentrated. The residue is purified by flash column chromatography to afford the expected product.

*Illustrative example of method B, synthesis of intermediate 19: tert-butyl N-[2-[3-[tert-butyl(dimethyl)silyl]oxy-3-methyl-butyl]-6-methoxy-pyrazolo[1,5-a]pyridin-5-yl]carbamate*

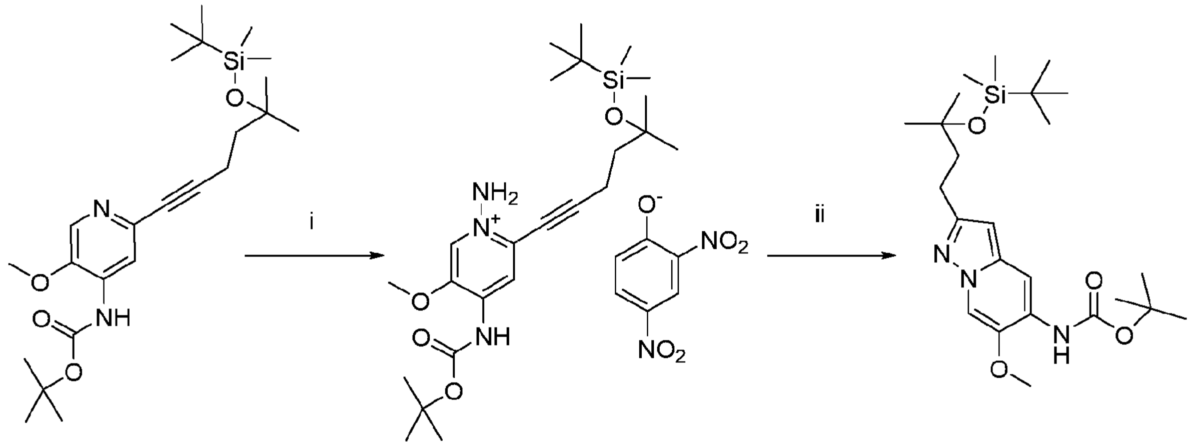


***1.18.3. Step i:***

O-(2,4-dinitrophenyl)hydroxylamine (14.0 g, 70.3 mmol, 2.0 eq.) was added portion-wise to a mixture of N-[2-[5-[tert-butyl(dimethyl)silyl]oxy-5-methyl-hex-1-ynyl]-5-methoxy-4-pyridyl]carbamate (16.0 g, 34.6 mmol, 1.0 eq.) in 1:1 THF/$H_2O$ (240 mL). The resulting mixture was stirred at 45 °C overnight and concentrated.

***1.18.4. Step ii:***

The residue from *Step i* was taken up in DMF (80 mL) and the resulting mixture was stirred at 90 °C overnight. The mixture was cooled to r.t. and quenched with sat. aq. $Na_2CO_3$. The resulting mixture was extracted (EtOAc). The layers were separated and the organic mixture was washed (HzO and brine), dried ($MgSO_4$) and concentrated. The residue was purified by flash column chromatography (SiOz, 100:0 to 80:20 n-heptane/EtOAc) to afford the desired product.

***1.19. Intermediate 21: tert-butyl N-[6-methoxy-2-(2-methylsulfonylethyl)pyrazolo[1,5-a]pyridin-5-yl]carbamate***

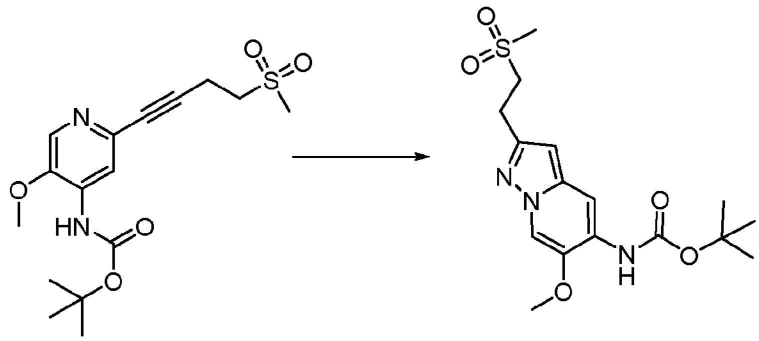

O-(2,4-dinitrophenyl)hydroxylamine (2.75 g, 13.8 mmol, 2.0 eq.) was added portion-wise to a mixture of tert-butyl N-[5-methoxy-2-(4-methylsulfonylbut-1-ynyl)-4-pyridyl]carbamate (2.44 g, 6.88 mmol, 1.0 eq.) in 4:1 THF/$H_2O$ (250 mL). The resulting mixture was stirred at 45 °C for 20 h and diluted with EtOAc. The resulting mixture was washed (HzO, sat. $NaHCO_3$ and brine), dried ($Na_2SO_4$) and concentrated. The residue was purified by flash column chromatography ($SiO_2$, 7:2 to 2:8 n-heptane/EtOAc) to afford the desired product.

***1.20. General method C1: Sonogashira cross coupling between 2-halopyridine derivatives and alkyne derivatives***

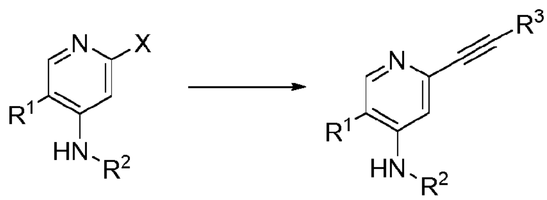

A mixture containing the pyridyl halide (1.0 eq.), the alkyne derivative (2.0 eq.) and TEA (14.0 eq.) in DMF (0.2 to 0.5 M) is flushed with an inert gas. CuI (0.2 eq.) and $PdCl_2(PPh_3)_2$ (0.1 eq.) are added and the resulting mixture is flushed with inert gas. The reaction is stirred at 95 to 100 °C for 2 to 16 h. The mixture undergoes an aq. work up and a flash column chromatography to afford the desired product.

*Illustrative example of method C1, synthesis of intermediate 18: N-[2-[5-[tert-butyl(dimethyl)silyl]oxy-5-methyl-hex-1-ynyl]-5-methoxy-4pyridylJcarbamate*

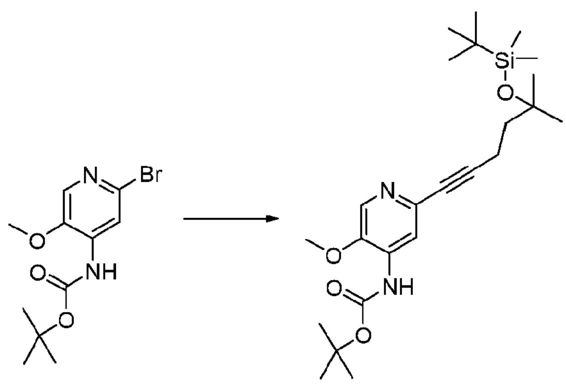

A mixture containing tert-butyl N-(2-bromo-5-methoxy-4-pyridyl)carbamate (57.8 g, 190.5 mmol, 1.0 eq.), tertbutyl-(1,1-dimethylpent-4-ynoxy)-dimethyl-silane (107.8 g, 381 mmol, 2.0 eq.) and TEA (370 mL, 2.65 mol, 14.0 eq.) in DMF (1 L) was flushed with $N_2$. CuI (7.7 g, 38.0 mmol, 0.2 eq.) and $PdCl_2(PPh_3)_2$ (13.4 g, 19.1 mmol, 0.1 eq.) were added and the resulting mixture was flushed with $N_2$. The reaction was stirred at 95 °C for 2 h. After cooling, the mixture was filtered over a Celite pad. The filtrate was diluted with EtOAc. The resulting mixture was washed ($H_2O$ and brine),

dried ($Na_2SO_4$) and concentrated. The residue was purified by flash column chromatography ($SiO_2$, 100:0 to 70:30 n-heptane/EtOAc) to afford the desired product.

***1.21. General method C2: Sonogashira cross coupling between 2-halopyridine derivatives and alkyne derivatives***

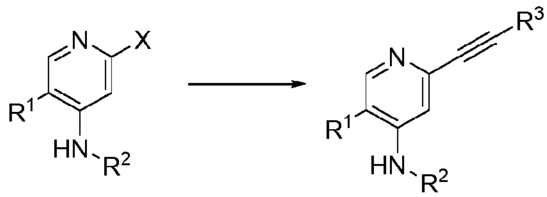

A mixture containing CuI (0.2 eq.), $PdCl_2(PPh_3)_2$ (0.1 eq.) and TEA (14.0 eq.) in DMF (0.2 to 0.5 M) is stirred under inert atmosphere. The pyridyl halide (1.0 eq.) and the alkyne derivative (1.2 to 2 eq.) are added and the resulting mixture is stirred at r.t. for 16 h. The mixture undergoes an aq. work up and a flash column chromatography to afford the desired product.

*Illustrative example of method C2, synthesis of intermediate 41: ethyl 4-(tert-butoxycarbonylamino)-6-(2-tetrahydropyran-4-ylethynyl)pyridine-3-carboxylate*

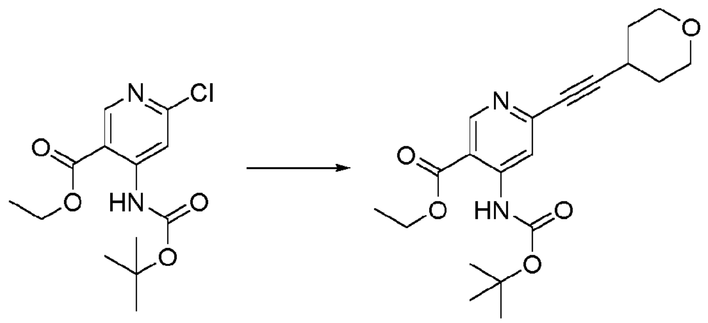

A mixture of $PdCl_2(PPh_3)_2$ (0.583 g, 0.831 mmol, 0.1 eq.), CuI (0.317 g, 1.66 mmol, 0.2 eq.) and TEA (16.2 mL, 116.0 mmol, 14.0 eq.) in dry DMF (75.0 mL) was stirred under Ar atmosphere. Ethyl 4-(tert-butoxycarbonylamino)-6-chloro-pyridine-3-carboxylate (2.50 g, 8.31 mmol, 1.0 eq.) and 4-ethynyltetrahydro-2H-pyran (1.10 g, 9.98 mmol, 1.2 eq.) were added and the mixture was stirred at r.t. for approximately 16 h. The reaction was quenched by addition of saturated $NH_4Cl$. The resulting mixture was extracted with EtOAc. The organic mixture was washed (brine), dried ($Na_2SO_4$) and concentrated. The residue was purified by flash column chromatography (SiOz, cyclohexane/EtOAc 100:0 to 70:30) to afford the desired product.

***1.22. General method D: N-Boc protection of aromatic amines***

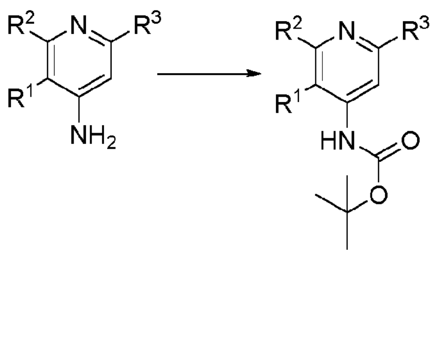

A mixture containing the aromatic amine (1.0 eq.), di-tert-butyl dicarbonate (1.1 eq.) and DIPEA (1.2 eq.) in THF (0.5-1.5 M) is stirred at r.t. for 16 to 48 h. The mixture undergoes an aq. work up to afford the desired product. The product may be further purified by flash column chromatography.

*Illustrative example of method D, synthesis of intermediate 30: tert-butyl N-(3-ethoxy-4-pyridyl)carbamate*

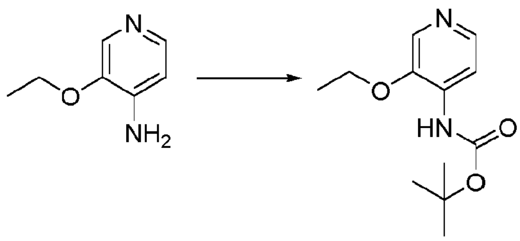

A mixture containing 3-ethoxypyridin-4-amine (5.0 g, 36.2 mmol, 1.0 eq.), di-tert-butyl dicarbonate (8.69 g, 39.8 mmol, 1.1 eq.) and DIPEA (7.6 mL, 43.4 mmol, 1.2 eq.) in THF (30 mL) was stirred at r.t. for 17 h. The mixture was quenched with saturated $NH_4Cl$. The layers were separated and the aq. layer was extracted with EtOAc. The organic layers were combined and concentrated. The residue was taken up in DCM (50 mL) and a saturated solution of $NaHCO_3$ (100 mL) was added. The resulting mixture was stirred for 10 min. The two layers were separated and the organic layer was dried (filtered through phase separator) and concentrated to afford the desired product.

***1.23. General method E: N-amination of pyridine derivatives followed by formation to pyrazolo[1,5-a]pyridines by reaction with alkyne derivatives***

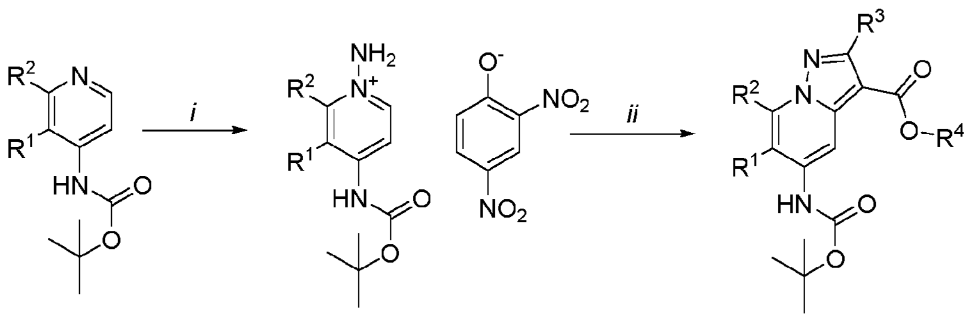


***1.23.1. Step i:***

O-(2,4-dinitrophenyl)hydroxylamine (2.0 eq.) is added to a mixture of the pyridine derivative (1 eq) in MeCN (approximately 0.5 M). The mixture is stirred at 50 °C for 16 h. The mixture is concentrated.

***1.23.2. Step ii:***

The residue obtained in the previous step is taken up in DMF (approximately 0.5 M). $K_2CO_3$ (3.0 eq.) is added and the mixture is stirred at r.t. for 1 h. The alkyne derivative (1.05 eq.) is added and the mixture is stirred for 20 to 24 h at r.t. Additional alkyne may be added and the mixture may be stirred for an additional 24 h. The mixture undergoes an aq. work up and a flash column chromatography to afford the desired product.

*Illustrative example of method E, synthesis of intermediate 29: ethyl 5-(tert-butoxycarbonylamino)-2-[3-[tert-butyl(dimethyl)silyl]oxy-3-methyl-butyl]-6-ethoxy-pyrazolo[1,5-a]pyridine-3-carboxylate*

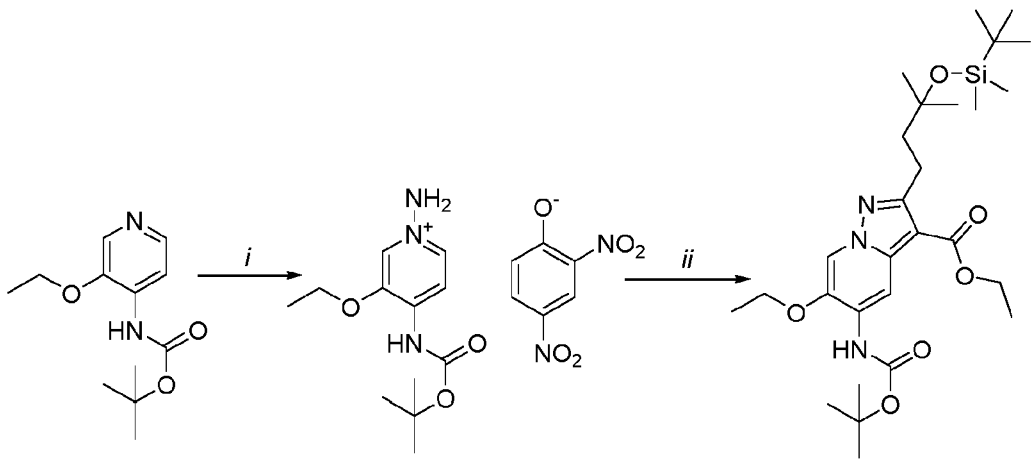


*1.23.3. Step i:*

O-(2,4-dinitrophenyl)hydroxylamine (16.2 g, 81.3 mmol, 2.0 eq.) was added to a mixture of tert-butyl N-(3-ethoxy-4-pyridyl)carbamate (9.69 g, 40.7 mmol, 1.0 eq.) in MeCN (70 mL). The mixture was stirred at 50 °C overnight. The mixture was concentrated.

*1.23.4. Step ii:*

The residue obtained from *Step i* was taken up in DMF (60 mL). $K_2CO_3$ (16.7 g, 121.0 mmol, 3.0 eq.) was added and the mixture was stirred at r.t. for 1 h. Ethyl 6-[tert-butyl(dimethyl)silyl]oxy-6-methyl-hept-2-ynoate (12.7 g, 42.4 mmol, 1.05 eq.) dissolved in 0.5 mL of DMF was added and the mixture was stirred for 24 h at r.t. An additional amount of ethyl 6-[tert-butyl(dimethyl)silyl]oxy-6-methyl-hept-2-ynoate (2.7 g, 0.22 eq.) was added and the mixture was stirred at r.t. overnight. The mixture was partitioned between $H_2O$ (500 mL) and EtOAc (100 mL). The two layers were separated and the aq. layer was extracted with DCM (3 x 100 mL). The organic phases were combined, washed ($H_2O$, brine), dried (filtration through phase separator) and concentrated. The residue was purified by flash column chromatography ($SiO_2$, 100:0 to 70:30 cyclohexane/EtOAc) to afford the desired product.

***1.24. General method F: hydrolysis, Boc deprotection and decarboxylation of pyrazolo[1,5-a]pyridine derivatives***

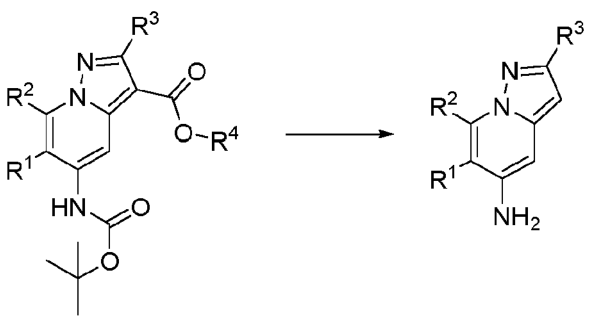

A mixture of the pyrazolo[1,5-a]pyridine derivatives (1.0 eq.) and LiOH (20.0 eq.) in 9:1 MeOH/$H_2O$ (approximately 0.1 M) is stirred at 100 °C for 16 h. The reaction mixture is partitioned between EtOAc and $H_2O$. The two phases are separated and the aq. layer is extracted with EtOAc. The combined organic layers are dried and concentrated. The residue is take up in toluene (approximately 0.1 M) and stirred at 115 °C for 2 to 4 h. The mixture is concentrated and the residue is purified by flash column chromatography to afford the desired product.

*Illustrative example of method F, synthesis of intermediate 43: 2-[3-[tert-butyl(dimethyl)silyl]oxy-3-methyl-butyl]-6-methoxy-7-methyl-pyrazolo[1,5-a]pyridin-5-amine*

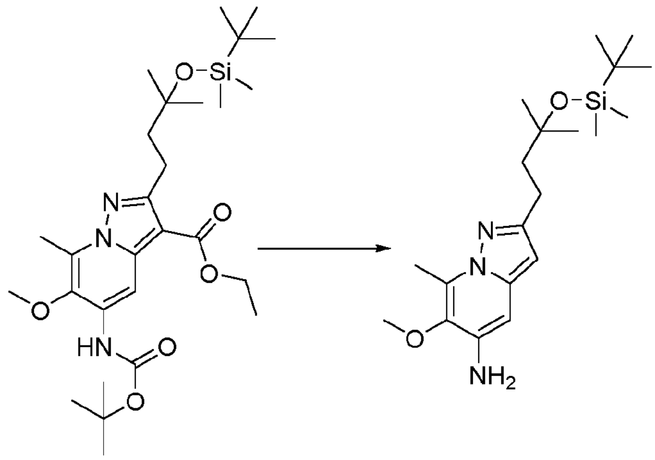

A ethyl 5-(tert-butoxycarbonylamino)-2-[3-[tert-butyl(dimethyl)silyl]oxy-3-methyl-butyl]-6-methoxy-7-methyl-pyrazolo[1,5-a]pyridine-3-carboxylate (3.7 g, 6.7 mmol, 1.0 eq.) and LiOH (3.22 g, 135 mmol, 20.0 eq.) in MeOH (60 mL)/$H_2O$ (7 mL) was stirred at 100 °C overnight. The reaction mixture was partitioned between EtOAc and $H_2O$. The two phases were separated and the aq. layer was extracted with EtOAc. The combined organic layers were dried (filtered through phase separator) and concentrated. The residue was take up in toluene (60 mL) and stirred at 115 °C for 2 h. The mixture was concentrated and the residue was purified by flash column chromatography ($SiO_2$, 100:0 to 75:25 cyclohexane/EtOAc) to afford the desired product.

### *1.25. General method G: removal of TBS protecting group by using TBAF*

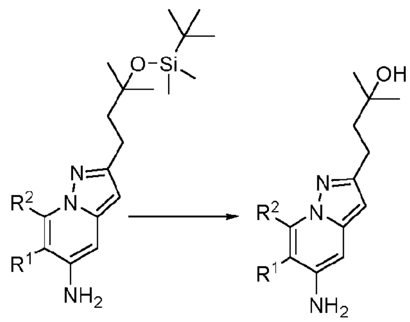

Tetra-n-butyl ammonium fluoride (1.0 M solution in THF, 20.0 eq.) is added to a solution of the tert-butyl(dimethyl)silyl protected alcohol (1.0 eq.) in THF (approximately 0.05 M). The mixture is stirred at 80 °C for 24 h. The mixture is concentrated and the residue is partitioned between DCM and saturated $NH_4Cl$. The two layers are separated and the organic layer is washed (saturated $NH_4Cl$), dried and concentrated. The residue is purified by flash column chromatography to afford the desired product.

*Illustrative example of method G, synthesis of intermediate 27: 4-(5-amino-6-ethoxy-pyrazolo[1,5-a]pyridin-2-yl)-2-methyl-butan-2-ol*

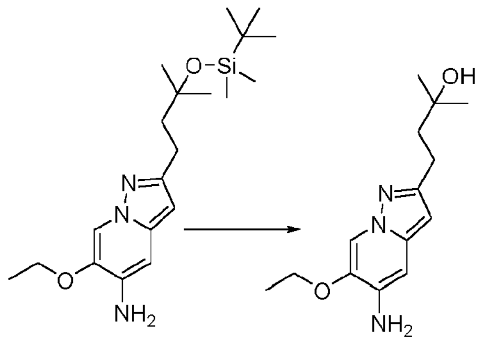

Tetra-n-butyl ammonium fluoride (100 mL of 1.0 M solution in THF, 100 mmol, 20.0 eq.) was added to a solution of 2-[3-[tert-butyl(dimethyl)silyl]oxy-3-methyl-butyl]-6-ethoxy-pyrazolo[1,5-a]pyridin-5-amine (1.88 g, 5.0 mmol, 1.0 eq.) in THF (85 mL). The mixture was stirred at 80 °C for 24 h. The mixture was concentrated and the residue was partitioned between DCM (50 mL) and saturated $NH_4Cl$ (30 mL). The two layers were separated and the organic layer was washed (saturated $NH_4Cl$, 2 x 30 mL), dried (filtered through phase separator) and concentrated. The residue was purified by flash column chromatography ($SiO_2$, 100:0 to 0: 100 cyclohexane/EtOAc) to afford the desired product.

***1.26. General method H: simultaneous removal of TBS and Boc protecting groups***

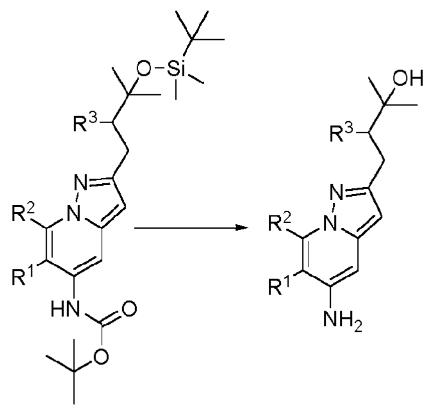

A solution of the Boc and TBS protected starting material (1.0 eq.) in 1:1:1 THF/MeOH/ 2 M aq. HCl (0.1 to 0.2 M, approximately 3 to 7 eq. of HCl) is stirred for 2 to 3 h at 80 to 90 °C. In case of uncomplete conversion, additional 2 M aq. HCl is added (5 eq.) and the mixture is stirred for an additional 2 h at 80 °C. After reaction completion, the mixture is cooled and neutralized with NaOH. The mixture is extracted with EtOAc. The organic layer is washed, dried and concentrated. The residue is purified by flash column chromatography or by recrystallization to obtain the desired product. Alternatively, after reaction completion, the mixture is concentrated. The residue may be directly purified by flash column chromatography to obtain the desired product or it may undergo an aq. work up involving a basic aq. solution. In the latter case, the organic layer is dried and concentrated. The residue is purified by flash column chromatography or by recrystallization to obtain the desired product.

*Illustrative example of method H, alternative synthesis of intermediate 8: 4-(5-amino-6-methoxy-pyrazolo[1,5-a]pyridin-2-yl)-2-methyl-butan-2-ol*

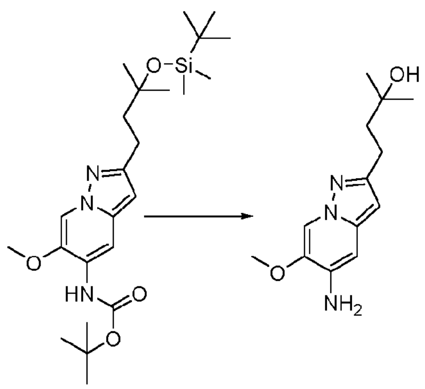

A solution of tert-butyl N-[2-[3-[tert-butyl(dimethyl)silyl]oxy-3-methyl-butyl]-6-methoxy-pyrazolo[1,5-a]pyridin-5-yl]carbamate (13.7 g, 29.5 mmol, 1.0 eq.) in 1:1:1 THF/MeOH/ 2 Maq. HCl (204 mL, 4.7 eq. of HCl) was stirred at 85 °C for 1.5 h. The mixture was cooled to 0 °C, neutralized with a 2 N NaOH solution until pH 7-8 (70 mL) and extracted twice with EtOAc (approximately 600 mL and then 100 mL). The organic layer was washed ($H_2O$, brine), dried ($MgSO_4$) and concentrated. The residue was dissolved in hot MeCN (approximately 250 mL at 95 to 100 °C). The mixture was cooled to r.t. and then to 0 °C. The precipitate was filtered off and further washed with MeCN to afford the desired product.

*Illustrative example of method H, synthesis of intermediate 55: 4-(5-amino-6-methoxy-pyrazolo[1,5-a]pyridin-2-yl)-3-fluoro-2-methyl-butan-2-ol*

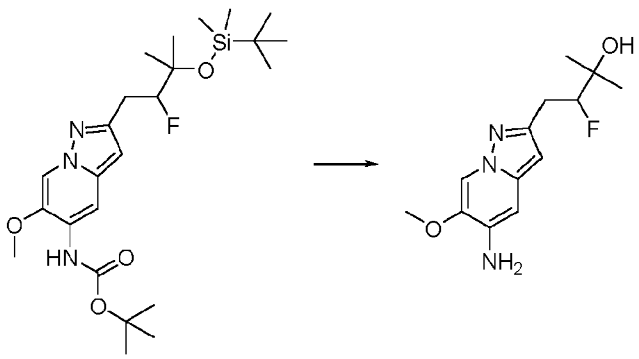

HCl in deionized water (2 M) (2 mL, 12 mmol, 10.4 eq.) was added to a solution of tert-butyl N-[2-[3-[tert-butyl(dimethyl)silyl]oxy-2-fluoro-3-methyl-butyl]-6-methoxy-pyrazolo[1,5-a]pyridin-5-yl]carbamate (556 mg, 1.1 mmol, 1.0 eq.) in a mixture of THF/MeOH (1: 1, 4 mL). The reaction mixture was stirred at 80 °C for 3 h. HCl in deionized water (2 M) (3 mL, 18 mmol, 15.6 eq.) was again added and the stirring at 80 °C was continued for another 2 h. The volatiles were evaporated under reduced pressure and the crude sample was purified by flash column chromatography eluting with DCM/MeOH from 96:4 to 90: 10 to give the desired compound.

***1.27. General method I: removal of Boc protecting group to obtain pyrazolo[1,5-a]pyridin-5-amine derivatives***

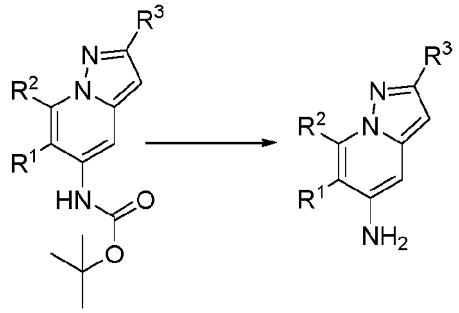

A solution of the Boc protected derivative (1.0 eq.) in 10:1 DCM/TFA (0.05 to 0.06 M, approximately 20.0 eq.) is stirred for 3 to 16 h at r.t. In case of uncomplete conversion, additional TFA is added (2.0 eq.) and the mixture is stirred further for 1 h at r.t. The mixture is diluted (DCM) and washed with a basic aq. solution. The two phases are separated and the organic layer is washed, dried and concentrated to afford the desired product. Alternatively the reaction mixture is adsorbed on an ISOLUTE® SCX column which is washed with MeOH and eluted with $NH_3$/MeOH. The eluted mixture is concentrated to afford the desired product.

*Illustrative example of method I, synthesis of intermediate 20: 6-methoxy-2-(2-methylsulfonylethyl)pyrazolo[1,5-a]pyridin-5-amine*

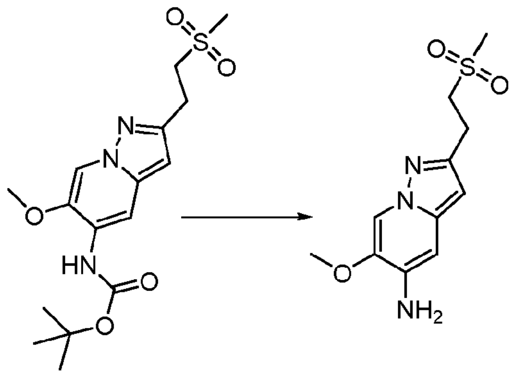

A solution of tert-butyl N-[6-methoxy-2-(2-methylsulfonylethyl)pyrazolo[1,5-a]pyridin-5-yl]carbamate (1.49 g, 4.03 mmol, 1.0 eq.) in 10:1 DCM/TFA (66.0 mL, 20.0 eq.) was stirred for 3 h at r.t. 0.5 mL of TFA (2.0 eq.) were added and the mixture was stirred further for 1 h at r.t. The mixture was diluted (DCM) and carefully washed with saturated $NaHCO_3$. The two phases were separated and the organic layer was washed (brine), dried ($Na_2SO_4$) and concentrated to afford the desired product.

***1.28. Intermediate 34: ethyl 5-amino-2-(4-piperidyl)pyrazolo[1,5-a]pyridine-6-carboxylate***

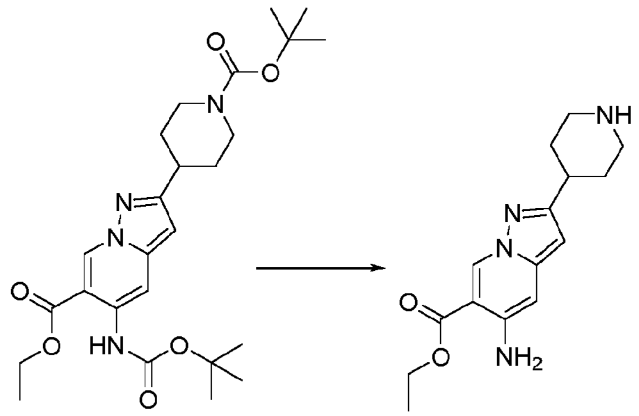

A solution of ethyl 5-(tert-butoxycarbonylamino)-2-(1-tert-butoxycarbonyl-4-piperidyl) pyrazolo[1,5-a]pyridine-6-carboxylate (1.35 g, 2.76 mmol, 1.0 eq.) in DCM (60 mL)/ TFA (4.2 mL, 20.0 eq.) was stirred for 16 h at r.t. 1 mL of TFA (5.0 eq.) was added and the mixture was stirred for 3 additional h at r.t. The reaction mixture was adsorbed on an ISOLUTE® SCX column which was washed with MeOH and eluted with 7 N $NH_3$/MeOH. The eluted mixture was concentrated to afford the desired product.

*1.29. Intermediate 33: ethyl 5-amino-2-(1-methyl-4-piperidyl)pyrazolo[1,5-a]pyridine-6-carboxylate*

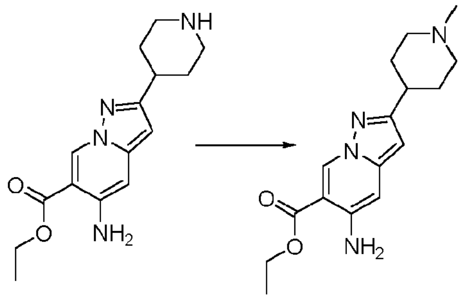

Formaldehyde (36% in $H_2O$, 0.43 mL, 5.6 mmol, 4.5 eq.) was added to a mixture of ethyl 5-amino-2-(4-piperidyl)pyrazolo[1,5-a]pyridine-6-carboxylate (360 mg, 1.25 mmol, 1.0 eq.) in THF (40 mL). Sodium triacetoxyborohydride (1.5 g, 7.24 mmol, 5.8 eq.) was added. The mixture was stirred at r.t. overnight. The mixture was diluted with saturated $NaHCO_3$. The resulting mixture was extracted with EtOAc. The two layers were separated and the organic layer was washed (brine), dried ($Na_2SO_4$) and concentrated. The residue was purified by flash column chromatography ($SiO_2$, 100:0 to 0:100 DCM/[90:4:1 DCM/MeOH/$NH_4OH$]) to afford the desired product.

***1.30. General method J: synthesis of amides from pyrazolo[1,5-a]pyridin-5-amine derivatives***

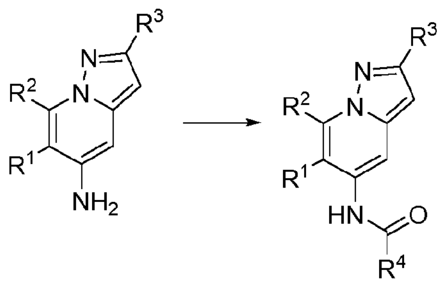

The carboxylic acid (1.2 eq.) and HATU, CAS#148893-10-1 (1.2 eq.) are added to a mixture of the amine (1.0

eq.) and DIPEA (2.0 eq.) in DMF. The resulting mixture is stirred at r.t. for 5 to 16 h. The reaction mixture is added dropwilse to a cooled basic solution ($H_2O$ and saturated aq. $NaHCO_3$) and the desired product is isolated by precipitation.

*Illustrative example of method J, synthesis of intermediate 46: ethyl 5-[[6-(difluoromethyl)pyridine-2-carbonyl]amino]-2-tetrahydropyran-4-yl-pyrazolo[1,5-a]pyridine-6-carboxylate*

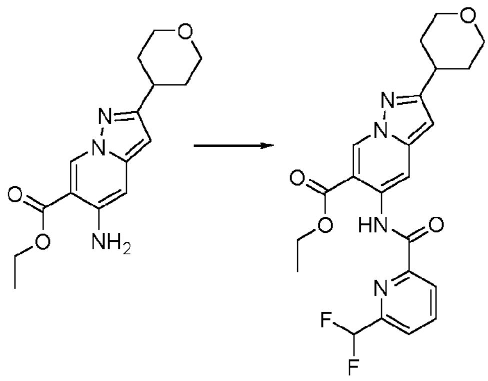

6-(difluoromethyl)pyridine-2-carboxylic acid (145 mg, 0.838 mmol, 1.2 eq.) and HATU, CAS#148893-10-1 (319 mg, 0.838 mmol, 1.2 eq.) were added to a mixture of ethyl 5-amino-2-tetrahydropyran-4-yl-pyrazolo[1,5-a]pyridine-6-carboxylate (202 mg, 0.698 mmol, 1.0 eq.) and DIPEA (0.243 mL, 1.40, 2.0 eq.) in DMF (11 mL) and the resulting mixture was stirred at r.t. for 16 h. The reaction mixture was added dropwise to a cooled basic solution (500 mL of $H_2O$ and 90 mL of saturated aq. $NaHCO_3$) and the desired product was isolated by precipitation, filtration and drying of the solid.

***1.31. Intermediate 48: ethyl 5-[[6-(difluoromethyl)pyridine-2-carbonyl]amino]-2-(3-hydroxy-3-methyl-butyl)pyrazolo[1,5-a]pyridine-6-carboxylate***

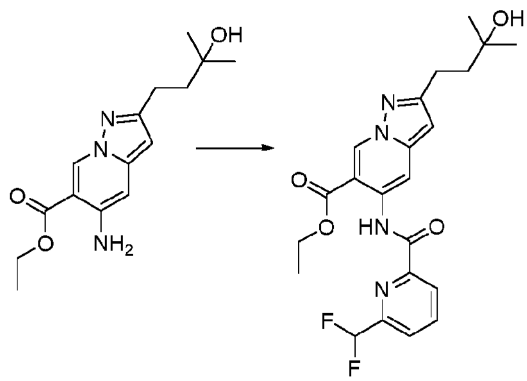

6-(difluoromethyl)pyridine-2-carboxylic acid (135 mg, 0.783 mmol, 1.2 eq.) and HATU, CAS#148893-10-1 (298 mg, 0.783 mmol, 1.2 eq.) were added to a mixture of ethyl 5-amino-2-(3-hydroxy-3-methyl-butyl)pyrazolo[1,5-a]pyridine-6-carboxylate (190 mg, 0.652 mmol, 1.0 eq.) and DIPEA (0.227 mL, 1.30 mmol, 2.0 eq.) in DCM (10 mL). The resulting mixture was stirred at r.t. for 16 h. 6-(difluoromethyl)pyridine-2-carboxylic acid (23 mg, 0.13 mmol, 0.2 eq.) and HATU, CAS#148893-10-1 (34 mg, 0.13 mmol, 0.2 eq.) were added and the mixture was stirred further for 4 h. The mixture was diluted (DCM), washed (saturated $NH_4Cl$, saturated $NaHCO_3$ and brine), dried ($Na_2SO_4$) and concentrated. The residue was purified by flash column chromatography ($SiO_2$, 100:0 to 0:100 DCM/[90:4:1 DCM/MeOH/$NH_4OH$]) to afford the desired product.

***1.32. General method K: ester hydrolysis of pyrazolo[1,5-a]pyridine-6-carboxylate esters derivatives***

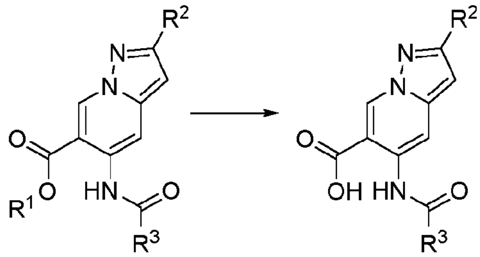

LiOH (3.0 eq.) is added to a mixture of the ester derivative (1.0 eq.) in 4:1 THF/$H_2O$ The reaction mixture is stirred at r.t. for 4 to 24 h. THF is removed under reduced pressure and the mixture is acidified to pH ≤ 5 with 1 M HCl. In case of the formation of a precipitate, the desired product is filtered off, washed with $H_2O$ and dried on air. Alternatively the acidified mixture is extracted with an organic solvent. The organic mixture is washed, dried and concentrated to afford the desired product.

*Illustrative example of method K, synthesis of intermediate 49: 5-[[6-(difluoromethyl)pyridine-2-carbonyl]amino]-2-(3-hydroxy-3-methyl-butyl)pyrazolo[1,5-a]pyridine-6-carboxylic acid*

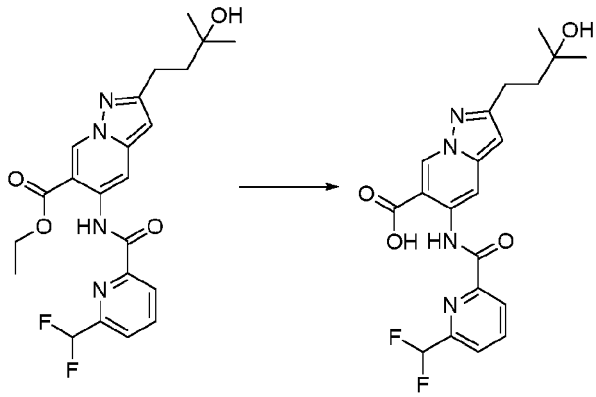

LiOH (22.0 mg, 0.92 mmol, 3.0 eq.) was added to a mixture of ethyl 5-[[6-(difluoromethyl) pyridine-2-carbonyl]amino]-2-(3-hydroxy-3-methyl-butyl)pyrazolo[1,5-a]pyridine-6-carboxylate (137 mg, 0.31 mmol, 1.0 eq.) in 4:1 4:1 THF/$H_2O$ (10 mL). The mixture was stirred at r.t. for 4 h. THF was removed under reduced pressure and the mixture was acidified to pH ≤ 5 with 1 M HCl. The mixture was extracted with EtOAc (3x). The combined organic layers were washed (brine), dried ($Na_2SO_4$) and concentrated to afford the desired product.

***1.33. Intermediate 32: ethyl 2-(1-methyl-4-piperidyl)-5-[[6-(trifluoromethyl)pyridine-2-carbonyl] amino]pyrazolo[1,5-a]pyridine-6-carboxylate***

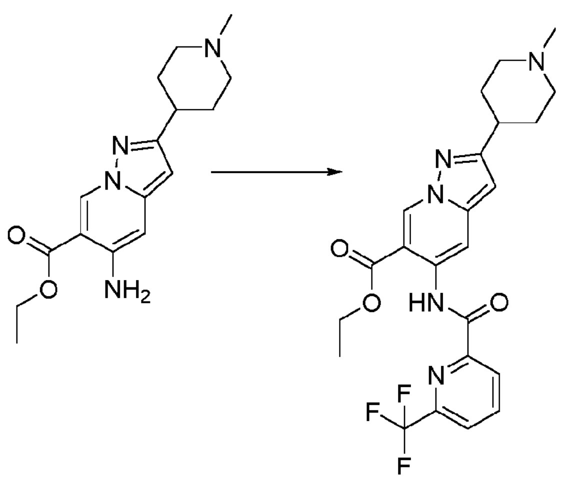

6-(trifluoromethyl)pyridine-2-carboxylic acid (93 mg, 0.488 mmol, 1.2 eq.) and HATU, CAS#148893-10-1 (186 mg, 0.488 mmol, 1.2 eq.) were added to a mixture of ethyl 5-amino-2-(1-methyl-4-piperidyl)pyrazolo[1,5-a]pyridine-6-carboxylate (123 mg, 0.407 mmol, 1.0 eq.) and DIPEA (0.142 mL, 0.84 mmol, 2.1 eq.) in DMF (7 mL). The resulting mixture was stirred at r.t. for 19 h. Saturated aq. $NaHCO_3$ (30 mL) was added and the resulting mixture was stirred for 10 min at r.t. The mixture was extracted with DCM (3 x 20 mL). The combined organic layers were dried (filtered through phase separator) and concentrated. The residue was purified by flash column chromatography (®puriFlash $SiO_2$ column, 100:0 to 0:100 DCM/[90:5:0.5 DCM/MeOH/$NH_4OH$]) to afford the desired product.

***1.34. Intermediate 50: methyl 2-fluoropent-4-ynoate***

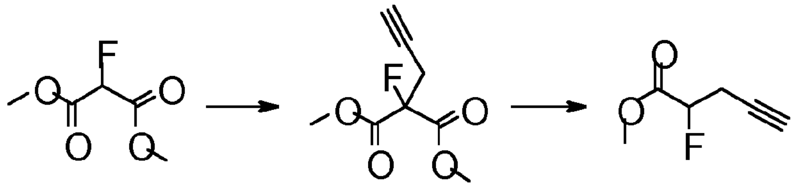

*Step i:* A solution of dimethyl 2-fluoromalonate (10.0 g, 63.3 mmol, 1.0 eq.) in THF (70 mL) was placed in a round bottom flask and cooled in an ice bath, under nitrogen flow. Sodium hydride (60% dispersion in mineral oil) (3.8 g, 95 mmol, 1.5 eq.) was added portion wise and the stirring at low temperature was continued for 10 min. The reaction mixture was then stirred at r.t. for 30 min and again cooled in an ice bath. 3-bromoprop-1-yne, 9.2 M in toluene (10 mL, 92 mmol, 1.5 eq.) was added dropwise over a 5 min period. The stirring at low temperature was continued for 5 min and then for 4 h at r.t. The volatiles were evaporated under reduced pressure. The residue was diluted with water and extracted twice with EtOAc. The combined organic phases were dried over sodium sulphate, filtered and evaporated under reduced pressure.

*Step ii:* Lithium chloride (3.97 g, 93.6 mmol, 3.0 eq.) was added to a solution of dimethyl 2-fluoro-2-prop-2-ynyl-propanedioate (5.88 g, 31.3 mmol, 1.0 eq.) in DMSO/$H_2O$ (40 / 1.5 mL). The vial was sealed and the reaction mixture was heated at 110 °C for 1 h. The residue was diluted with a mixture of water (300 mL) / saturated aq. solution of NaCl (200 mL) and extracted twice with EtOAc (2 x 500 mL). The combined organic phases were washed with a saturated aq. solution of NaCl. The organic phase was dried over sodium sulphate, filtered and evaporated under reduced pressure. The crude sample was purified by flash column chromatography eluting with EtOAc / n-heptane to afford the desired product.

***1.35. Intermediate 51: 3-fluoro-2-methyl-hex-5-yn-2-ol***

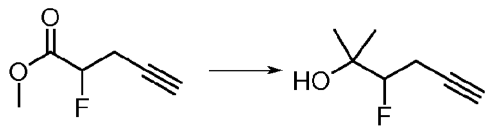

Methyl 2-fluoropent-4-ynoate (200 mg, 1.5 mmol, 1.0 eq.) was dissolved in THF (3 mL) at r.t. under nitrogen atmosphere. The solution was cooled in an ice bath and methylmagnesium bromide, 3.0 M in EtzO (1.5 mL, 4.5 mmol, 2.9 eq.) was added dropwise. After 5 min, the ice bath was removed and the solution was stirred at r.t. for 2 h. The reaction mixture was carefully quenched with a saturated aq. solution of $NH_4Cl$ until a clear solution was obtained. The solution was diluted with EtOAc and the organic phase was separated. The aq. phase was again extracted with DCM (2 x 50 mL). The combined organic phases were dried over sodium sulfate, filtered and evaporated under reduced pressure to afford the desired product.

***1.36. Intermediate 52: tert-butyl-(2-fluoro-1,1-dimethyl-pent-4ynoxy)-dimethyl-silane***

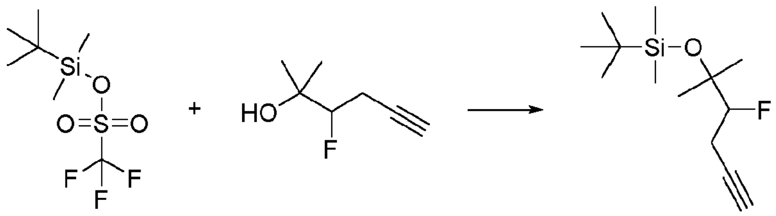

[tert-butyl(dimethyl)silyl] trifluoromethanesulfonate (13.5 g, 51.1 mmol, 2.5 eq.) was added dropwise to an ice cooled solution of 3-fluoro-2-methyl-hex-5-yn-2-ol (2.65 g, 20.4 mmol, 1.0 eq.) and pyridine (10 mL, 124 mmol, 6.0 eq.) in DCM (110 mL). After 10 min, the ice bath was removed and the solution was stirred at r.t for 20 h. The solution was washed with aq. HCl (2N) and then with a saturated aq. solution of $NaHCO_3$. The organic phase was dried over sodium sulfate, filtered and evaporated under reduced pressure. The crude sample was purified by flash column chromatography eluting with n-heptane / DCM from 100:0 to 94:6 to afford the desired product.

***1.37. Intermediate 53: tert-butyl N-[2-[5-[tert-butyl(dimethyl)silyl]oxy-4-fluoro-5-methyl-hex-1-ynyl]-5-methoxy-4-pyridyl]carbamate***

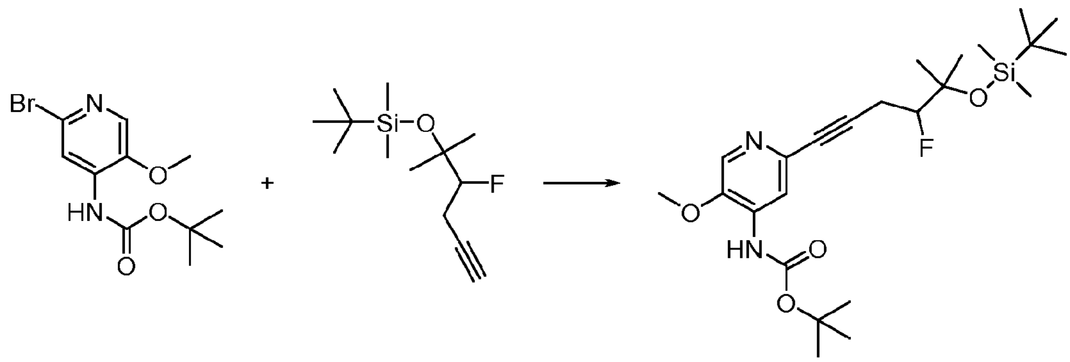

In a sealed tube, tert-butyl N-(2-bromo-5-methoxy-4-pyridyl)carbamate (1.7 g, 5.6 mmol, 1.0 eq.), tert-butyl-(2-fluoro-1,1-dimethyl-pent-4-ynoxy)-dimethyl-silane (1.4 g, 5.7 mmol, 1.0 eq.), Bis(triphenylphosphine)palladium (II) dichloride (400 mg, 0.6 mmol, 0.1 eq.) and copper (I) iodide (230 mg, 1.1 mmol, 0.2 eq.) were suspended in DMF (35 mL). Nitrogen was bubbled through the reaction mixture for 5 min and TEA (11 mL, 78.9 mmol, 14.0 eq.) was added. The reaction mixture was heated at 100 °C for 3 h and then cooled down to r.t. The reaction mixture was poured onto ice / water (400 mL) and stirred for 10 min. The aq. phase was extracted twice with EtOAc (2 x 200 mL). The combined organic phases were washed with a saturated aq. solution of NaCl. The organic phase was dried over sodium sulfate,

filtered and evaporated under reduced pressure. The crude sample was purified by flash column chromatography eluting with n-heptane / DCM from 100:0 to 0: 100 to afford the desired product.

### *1.38. Intermediate 54: tert-butyl N-[2-[3-[tert-butyl(dimethyl)silyl]oxy-2-fluoro-3-methyl-butyl]-6-methoxy-pyrazolo[1,5-a]pyridin-5-y]carbamate*

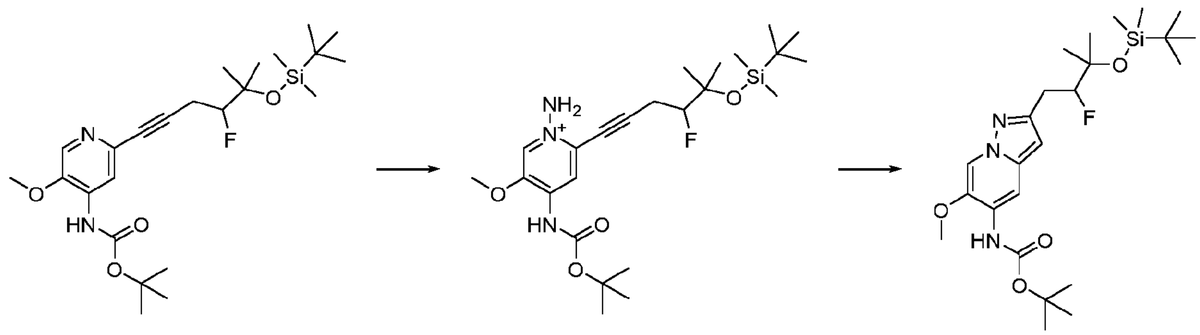

O-Diphenylphosphinylhydroxylamine (1.68 g, 7.1 mmol, 2.0 eq.) was added to a solution of tert-butyl N-[2-[5-[tert-butyl(dimethyl)silyl]oxy-4-fluoro-5-methyl-hex-1-ynyl]-5-methoxy-4-pyridyl] carbamate (1.67 g, 3.5 mmol, 1.0 eq.) in THF (60 mL) / water (30 mL). The reaction mixture was stirred at r.t. for 20 h.

*Step ii:* AcOH (120 mL) was added to the previous solution and then heated at 80 °C for 4 h. The reaction mixture was cooled down to r.t. and the volatiles were evaporated at 40 °C under reduced pressure. The crude sample was purified by flash column chromatography eluting with n-heptane/EtOAc from 95:5 to 60:40 to afford the desired product.

### *1.39. Intermediate 56: 2-bromo-5-(trideuteriomethoxy)pyridine*

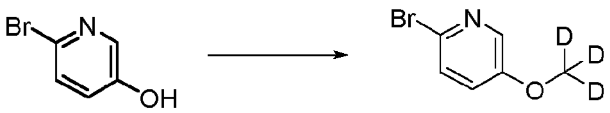

To a mixture of 6-bromopyridin-3-ol (390 g, 2.4 mol, 1.0 eq.) and cesium carbonate (1107 g, 3.6 mol, 1.5 eq.) in DMF (2000 mL) at r.t. was added iodomethane-$d_3$ (155 mL, 2.52 mol, 1.08 eq.) dropwise over 15 min. Temperature was kept at 40 °C and the mixture was stirred for 1 h. The mixture was cooled to r.t. and added to a mixture of water (6000 mL) and EtzO (2000 mL). The aq. layer was extracted with EtzO (2000 mL and 1500 mL). Gathered organic extracts were washed with brine (2 x 1500 mL). The organic layer was concentrated to dryness under reduced pressure to give the desired product.

### *1.40. Intermediate 57: 2-bromo-1-oxido-5-(trideuteriomethoxy)pyridin-1-ium*

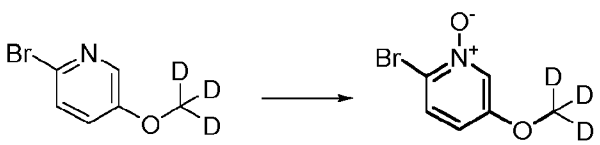

To a solution of 2-bromo-5-(trideuteriomethoxy)pyridine (98.0 %, 406 g, 2083 mmol, 1.0 eq.) in 1,2-dichloroethane (4 L) in a 5 L reactor was added 3-chlorobenzenecarboperoxoic acid (77.0 %, 702 g, 3131 mmol, 1.5 eq.) in one portion (endothermic, temperature dropping to 10 °C). The mixture was warmed-up to 80 °C and stirred for 2 h. The mixture was cooled to 35 °C, quenched by addition of diethylamine (345 mL, 3332 mmol, 1.5 eq.) and concentrated to

dryness under reduced pressure. To the resulting crude was added water (2 L) and DCM (3 L). By using diethylamine pH was adjusted to 10.6. The mixture was stirred for 5 min and the layers separated. The aq. layer was extracted with DCM (1 L). To the aq. layer was added solid NaCl (600 g) and upon dissolution extracted with DCM (1 L). Gathered organic extracts were washed with 2N aq. NaOH (400 mL) and concentrated to dryness to give the desired product.

***1.41. Intermediate 58: 2-bromo-5-methoxy-4-nitropyridine***

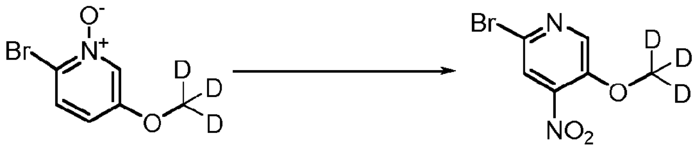

The experiment was done in parallel in a 2 liter three-necked round-bottom flasks equipped with a thermometer. The outlet of the flask was connected to an empty trap bottle and then to a 2 M aq. NaOH solution. To the flask was added 2-bromo-5-(trideuteriomethoxy)pyridine-1-oxide (95.0 %, 149 g, 683 mmol, 1.0 eq.) and then concentrated sulfuric acid, 96 % (456 mL, 8204 mmol, 12.0 eq.). The suspension was stirred until a solution was obtained. The solution was warmed-up to 90 °C and into it was added nitric acid, fuming, 90% (149 mL, 3213 mmol, 4.7 eq.) dropwise over 3 h while keeping the temperature between 110-120 °C. Upon completion, temperature was left to be lowered to 60 °C and the mixture was added dropwise, during 15 min, onto vigorously stirred cold water (4 L). The formed suspension was left to stir at 10 °C for 30 min, then filtered and the cake was washed with water. The cake was kept on the funnel under suction for 1 h and then left to air-dry in an open plate. After air-drying over 72 h the product was obtained.

***1.42. Intermediate 59: 2-bromo-5-(trideuteriomethoxy)pyridin-4-amine***

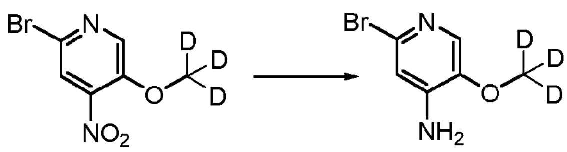

To a suspension of 2-bromo-4-nitro-5-(trideuteriomethoxy)pyridine (70.0 %, 230 g, 682 mmol, 1.0 eq.) and ammonium chloride (40.0 g, 748 mmol, 1.1 eq.) in a mixture of EtOH, 96 % (1000 mL) and water (350 mL) at r.t. was added iron, powder (400 g, 7163 mmol, 10.5 eq.) in one portion. The mixture was heated to 80 °C and stirred for 2 h. The mixture was cooled to r.t. and filtered through a Celite pad. The filtrate was concentrated to ca. 500 mL volume resulting in a suspension. The suspension was filtered through a sinter funnel. The cake was washed with water (2 x 400 mL) and left on the funnel under suction for 1 h. The Celite pad was washed with 3 x 400 mL of DCM and the washings were concentrated to dryness. The powder and DCM extracts were gathered and purified by column chromatography, gradient elution 0-5% MeOH/DCM to afford the desired product.

***1.43. Intermediate 60: tert-butyl N-[2-bromo-5-(trideuteriomethoxy)-4-pyridyl]carbamate***

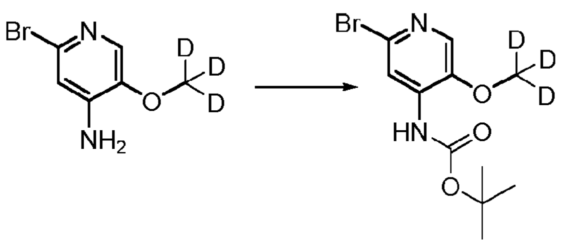

A solution of di-tert-butyl dicarbonate (74.0 g, 339 mmol, 13.0 eq.) and 4-dimethylaminopyridine (3.19 g, 26.1 mmol, 1.0 eq.) in DCM (1 L) in a 5 L reactor was cooled to 0 °C. Into it was added a solution of 2-bromo-5-(trideuteriomethoxy)pyridin-4-amine (96.0 %, 56.0 g, 261 mmol, 10.0 eq.) and TEA (106 g, 1044 mmol, 40.0 eq.) in DCM (1 L) dropwise over 1 h. The mixture was warmed to 21 °C and left to stir for 16 h. The reaction was quenched by addition of sat. aq. $NaHCO_3$ (2 L). The organic layer was separated and evaporated. The residue was dissolved in DCM and loaded on a pad of silica gel (15 cm height, 19 cm diameter). Gradient elution 0-2% MeOH/DCM over 15 L to give a mixture of desired product and starting material. The mixture was engaged again in the same reaction: a solution of di-tert-butyl dicarbonate (32.4 g, 148 mmol, 5.67 eq.) and 4-dimethylaminopyridine (1.39 g, 11.4 mmol, 0.43 eq.) in DCM (1 L) in a 5 L reactor was cooled to 0 °C. Into it was added a solution of 2-bromo-5-(trideuteriomethoxy)pyridin-4-amine (50% purity, 47 g, 114 mmol, 4.36 eq.) and TEA (46.2 g, 456 mmol, 17.4 eq.) in DCM (1 L) dropwise over 1 h. The mixture was warmed to 21 °C and left to stir overnight. The reaction was quenched by addition of sat. aq. $NaHCO_3$ (2 L). The organic layer was separated and evaporated to give the crude product, that was dissolved in DCM and loaded on a pad of silica gel (9 cm height, 13 cm diameter). Gradient elution 0-30% EtOAc/cyclohexane over 10 L to obtain the desired product.

### *1.44. Intermediate 61: tert-butyl N-[2-[5-[tert-butyl(dimethyl)silyl]oxy-5-methyl-hex-1-ynyl]-5-(trideuteriomethoxy)-4-pyridyl]carbamate*

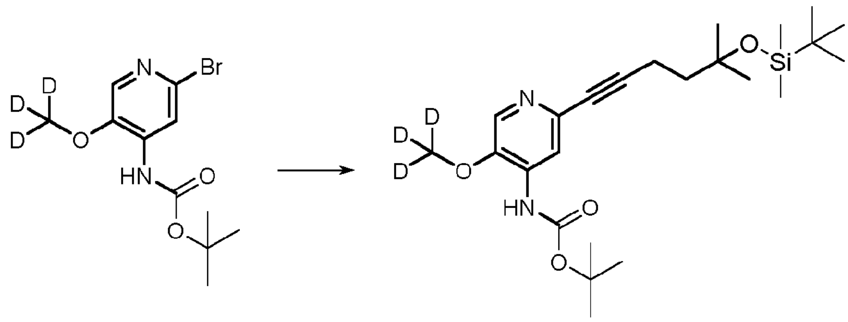

A mixture of tert-butyl N-[2-bromo-5-(trideuteriomethoxy)-4-pyridyl]carbamate (6.44 g, 21.0 mmol, 1.0 eq.), bis(triphenylphosphine)palladium(II) dichloride (1.48 g, 2.10 mmol, 0.1 eq.), copper (I) iodide (0.801 g, 4.21 mmol, 0.2 eq.) and TEA (41.0 mL, 294 mmol, 14.0 eq.) in DMF (100 mL) was purged with $N_2$ for 15 min. To the mixture was added a solution of tert-butyl-(1,1-dimethylpent-4-ynoxy)-dimethyl-silane (80.0 % purity, 7.14 g, 25.2 mmol, 1.2 eq.) in DMF (10 mL) and the purging continued for 10 min. The mixture was heated to 90 °C and the reaction was left to stir for 16 h. The mixture was cooled to r.t. and quenched by addition of saturated $NH_4Cl$ (300 mL) and EtOAc (100 mL). The aqueous layer was extracted with EtOAc (50 mL). The organic extracts were washed with saturated aq. $NH_4Cl$ (100 mL) and with brine (100 mL). The organic layer was concentrated and the residue was purified by flash column chromatography ($SiO_2$, 100:0 to 75:25 cyclohexane/EtOAc) to afford the desired product.

### *1.45. Intermediate 62: tert-butyl N-[2-[3-[tert-butyl(dimethyl)silyl]oxy-3-methyl-butyl]-6-(trideuteriomethoxy)pyrazolo[1,5-a]pyridin-5-yl]carbamate*

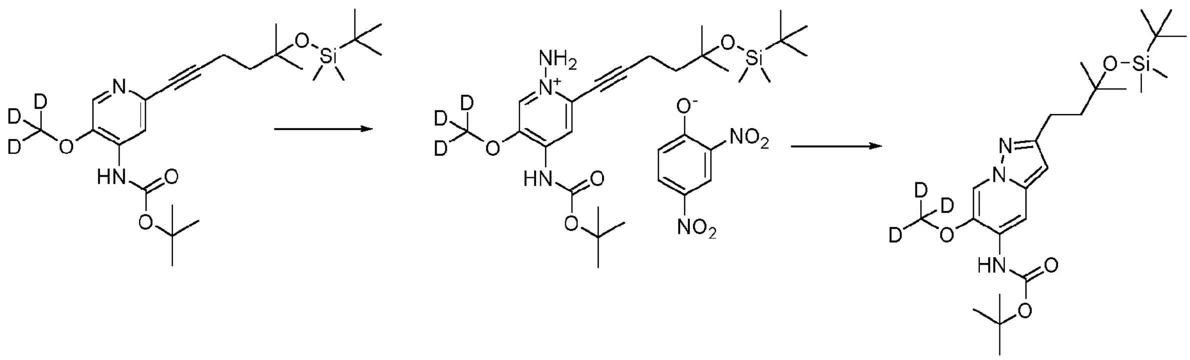

To a suspension of tert-butyl N-[2-[5-[tert-butyl(dimethyl)silyl]oxy-5-methyl-hex-1-ynyl]-5-(trideuteriomethoxy)-4-pyridyl]carbamate (3.46 g, 7.66 mmol, 1 eq.) in MeCN (30 mL) at r.t. O-(2,4-dinitrophenyl)hydroxylamine (3.07 g, 15.4 mmol, 2.0 eq.) was added in one portion. The mixture was heated to 50 °C and stirred for 24 h. The mixture was evaporated to dryness. DMF (50 mL) was added to the residue and the mixture was stirred at 80 °C for 16 h. Saturated aq. $NaHCO_3$ was added and the mixture was extracted with EtOAc. The combined organic layers were washed (saturated aq. $NH_4Cl$ and brine), dried ($Na_2SO_4$) and concentrated. The residue was purified by flash column chromatography ($SiO_2$, 100:0 to 80:20 EtOAc/cyclohexane) to afford the desired product.

### *1.46. Intermediate 64: 1,1,1-trideuterio-2-(trideuteriomethyl)hex-5-yn-2-ol*

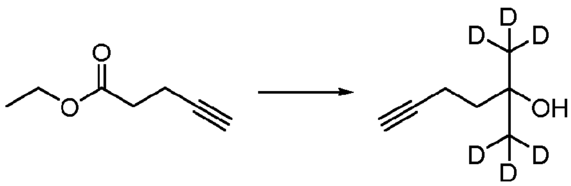

To a suspension of magnesium turnings (84.3 g, 3.47 mol, 3.5 eq.) in $Et_2O$ (1 L) in a 5 L reactor at r.t., a small spoon of iodine was added. The mixture was flushed with $N_2$. To the mixture, a solution of trideuterio(iodo)methane (430 g, 2.97 mol, 3.0 eq.) in $Et_2O$ (1 L) was added dropwise over 20 min (caution: exothermic). The mixture was stirred at reflux for 1 h and then cooled to -5 °C. A solution of ethyl pent-4-ynoate (125 g, 0.991 mol, 1.0 eq.) in $Et_2O$ (1 L) was added dropwise over 20 min. The mixture was warmed to 22 °C and stirred for 2 h. The reaction mixture was quenched by dropwise addition of saturated aq. $NH_4Cl$ (1 L) (caution: exothermic) and $H_2O$ (1 L). The layers were separated. The aq. layer was extracted with $Et_2O$ (1 L). The combined organic extracts were concentrated to afford the desired product.

### *1.47. Intermediate 65: 1,1-bis(trideuteriomethyl)pent-4-ynoxy-tert-butyl-dimethyl-silane*

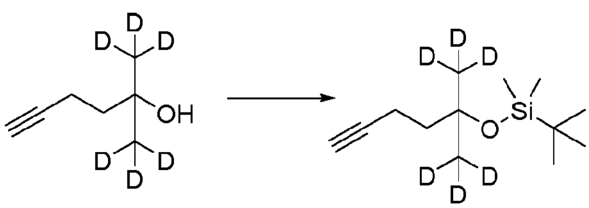

To a solution of 1,1,1-trideuterio-2-(trideuteriomethyl)hex-5-yn-2-ol (38.0 g, 289 mmol, 1.0 eq.) and pyridine (107 mL, 1.33 mol, 4.2 eq.) in DCM (500 mL) in a 2 L round bottom flask was cooled in an ice bath. [tert-butyl(dimethyl)silyl] trifluoromethanesulfonate (123 g , 465 mmol, 1.5 eq.) was added dropwise over 30 min. The reaction was warmed to ambient temperature and left to stir for 16 h. The mixture was concentrated under reduced pressure until DCM was removed. $Et_2O$ (1.5 L) was added to the residue and the resulting suspension was stirred for 5 min. The suspension was filtered and the cake was washed with $Et_2O$ (700 mL). The filtrate was concentrated to dryness under reduced

pressure to afford the desired product.

***1.48. Intermediate 67: tert-butyl N-[2-[3-[tert-butyl(dimethyl)silyl]oxy-4,4,4-trideuterio-3-(trideuteriomethyl)butyl]-6-methoxy-pyrazolo[1,5-a]pyridin-5-yl]carbamate***

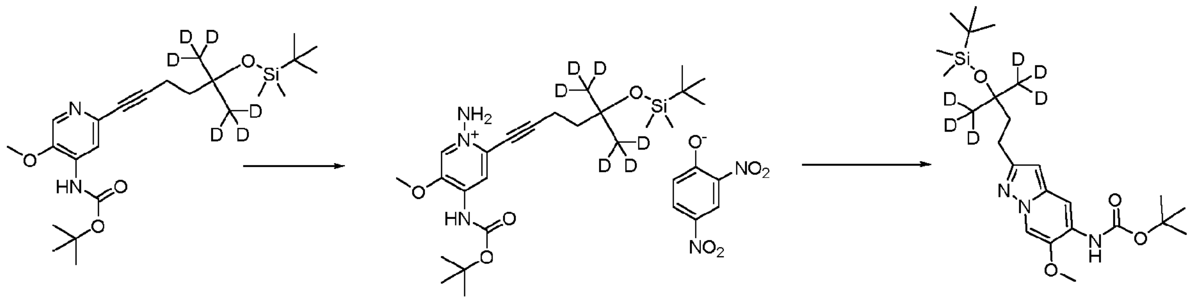

O-(2,4-dinitrophenyl)hydroxylamine, CAS# 17508-17-7 (5.08 g, 25.5 mmol, 2.0 eq.) was added in one portion to a suspension of tert-butyl N-[2-[5-[tert-butyl(dimethyl)silyl]oxy-6,6,6-trideuterio-5-(trideuteriomethyl)hex-1-ynyl]-5-methoxy-4-pyridyl]carbamate (5.80 g, 12.8 mmol, 1.0 eq.) in MeCN (60 mL) at r.t. The resulting mixture was heated to 50 °C and stirred overnight. The mixture was evaporated to dryness. DMF (100 mL) was added to the residue and the resulting mixture was stirred at 80 °C for 16 h. Saturated aq. $NaHCO_3$ (300 mL) and EtOAc (200 mL) were added. The mixture was filtered and the two layers composing the filtrate were separated. The aq. layer was further extracted with EtOAc (100 mL). The organic layers were combined, washed (saturated aq. $NH_4Cl$ and brine), dried ($Na_2SO_4$) and concentrated. The residue was purified by flash column chromatography ($SiO_2$, 100:0 to 80:20 EtOAc/cyclohexane) to afford the desired product.

***1.49. Intermediate 66: tert-butyl N-[2-[5-[tert-butyl(dimethyl)silyl]oxy-6,6,6-trideuterio-5-(trideuteriomethyl)hex-1-ynyl]-5-methoxy-4-pyridyl]carbamate***

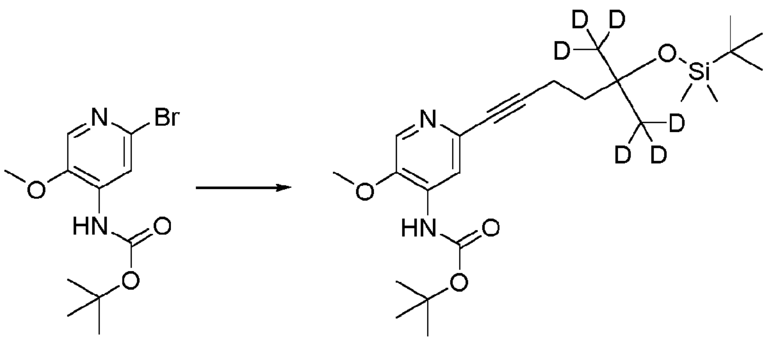

A mixture of tert-butyl N-(2-bromo-5-methoxy-4-pyridyl)carbamate, (89.0 g, 294 mmol, 1.0 eq.), bis(triphenylphosphine)palladium(II) dichloride (20.6 g, 29.4 mmol, 0.1 eq.), copper (I) iodide (11.2 g, 57.8 mmol, 0.2 eq.) and TEA (573 mL, 4.11 mol, 14.0 eq.) in DMF (1.5 L) was purged with $N_2$ for 15 min. A solution of 1,1-bis(trideuteriomethyl)pent-4-ynoxy-tert-butyl-dimethyl-silane (115 g, 323 mmol, 1.1 eq.) in DMF (500 mL) was added to the mixture and the purging continues for 10 min. The mixture was heated to 90 °C and the reaction was left to stir for 16 h. The mixture was cooled to r.t. and quenched by addition of saturated $NH_4Cl$ (3000 mL) and EtOAc (1500 mL). The aq. layer was extracted with EtOAc (700 mL). The organic layers were combined, washed (saturated aq. $NH_4Cl$, 1 L, and brine, 1 L) and concentrated. The residue was purified by flash column chromatography (SiOz, 100:0 to 70:30 cyclohexane/EtOAc) to afford the desired product.

***1.50. Intermediate 68: 4-(5-amino-6-methoxy-pyrazolo[1,5-a]pyridin-2-yl)-1,1,1-trideuterio-2-(trideuteriomethyl)butan-2-ol***

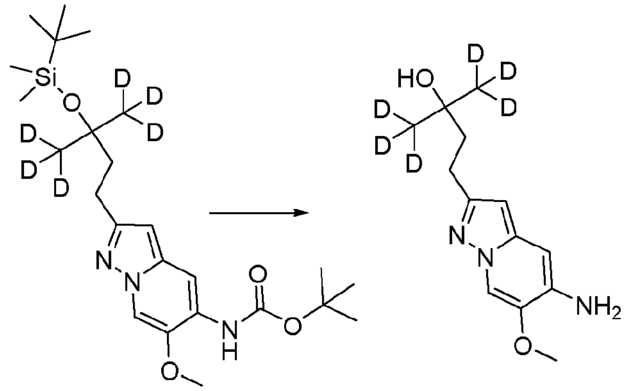

2 M aqueous HCl (15 mL) was added in one portion to a mixture of tert-butyl N-[2-[3-[tert-butyl(ditmethyl)silyl]oxy-4,4,4-trideuterio-3-(trideuteriomethyl)butyl]-6-methoxy-pyrazolo[1,5-a]pyridin-5-yl]carbamate (2.52 g, 5.2 mmol, 1.0 eq.) in MeOH (15 mL) and THF (15 mL). The resulting mixture was heated to 90 °C and stirred for 16 h. 6 M aq. HCl (6 mL) was added and the resulting mixture was stirred at reflux for an additional 4 h. The mixture was concentrated to remove MeOH and THF. DCM (10 mL) was added and the pH was adjusted to approximately 8 with 2 M aq. NaOH. The two layers were separated and concentrated. The residue was suspended in MeCN (20 mL). The mixture was stirred at reflux for 30 min. The mixture was cooled to r.t. and then with an ice bath. The mixture was stirred for 20 min and filtered. The solid so obtained was dried on the funnel under suction to afford the desired product.

**Table II. Intermediates towards Illustrative compounds of the invention**

| Int | Structure | Name | Name starting material | Mtd | MW (calc) | MW (obs) |
|---|---|---|---|---|---|---|
| 1 | 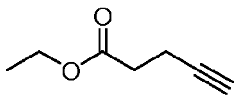 | ethyl pent-4-ynoate | pent-4-ynoic acid | NA | 126.2 | NA |
| 2 | 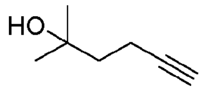 | 2-methylhex-5-yn-2-ol | ethyl pent-4-ynoate | NA | 112.2 | NA |
| 3 | 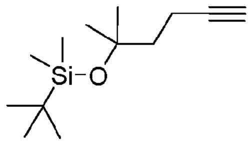 | tert-butyl-(1,1-dimethylpent-4-ynoxy)-dimethyl-silane | 2-methylhex-5-yn-2-ol | NA | 226.4 | NA |
| 4 | 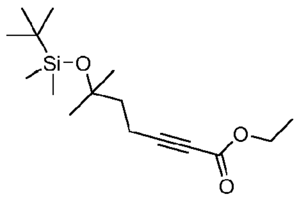 | ethyl 6-[tert-butyl(dimethyl)sil yl]oxy-6-methyl-hept-2-ynoate | tert-butyl-(1,1-dimethylpent-4-ynoxy)-dimethyl-silane | NA | 298.5 | NA |
| 5 | 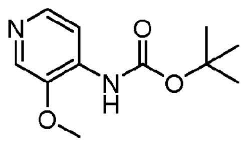 | tert-butyl N-(3-methoxy-4-pyridyl)carbamat e | 3-methoxypyridin-4-amine HCl salt | NA | 224.3 | 225.1 |

(continued)

| Int | Structure | Name | Name starting material | Mtd | MW (calc) | MW (obs) |
|---|---|---|---|---|---|---|
| 6 | 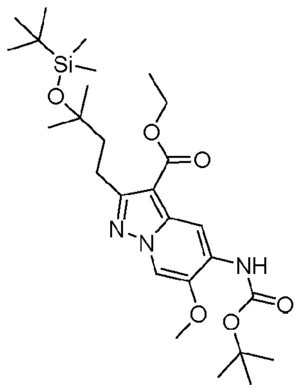 | ethyl 5-(tert-butoxycarbonyla mino)-2-[3-[tert-butyl(dimethyl)sil yl]oxy-3-methylbutyl]-6-methoxy-pyrazolo[1,5-a] pyridine-3-carboxylate | tert-butyl N-(3-methoxy-4-pyridyl)carbama te | NA | 535.8 | 536.2 |
| 7 | 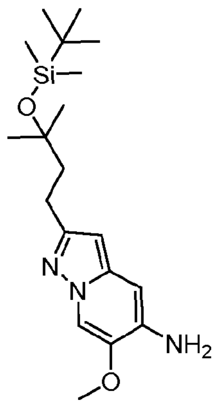 | 2-[3-[tert-butyl(dimethyl)sil yl]oxy-3-methylbutyl]-6-methoxy-pyrazolo[1,5-a] pyridin-5-amine | ethyl 5-(tert-butoxycarbonyla mino)-2-[3-[tert-butyl (dimethyl)s ilyl]oxy-3-methyl-butyl]-6-methoxy-pyrazolo[1,5-a]pyridine-3-carboxylate | NA | 363.6 | 364.2 |

(continued)

| Int | Structure | Name | Name starting material | Mtd | MW (calc) | MW (obs) |
|---|---|---|---|---|---|---|
| 8 | 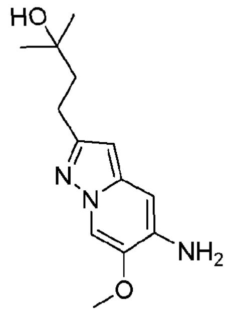 | 4-(5-amino-6-methoxy-pyrazolo[1,5-a] pyridin-2-yl)-2-methyl-butan-2-ol | 2-[3-[tert-butyl(dimethyl)s ilyl]oxy-3-methyl-butyl]-6-methoxy-pyrazolo[1,5-a]pyridin-5-amine or alternatively tert-butyl N-[2-[3-[tert-butyl(dimethyl)s ilyl] oxy-3-methyl-butyl]-6-methoxy-pyrazolo[1,5-a]pyridin-5-yl]carbamate | NA Or H | 249.3 | 250.1 |
| 9 | 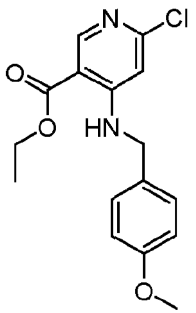 | ethyl 6-chloro-4-[(4-methoxyphenyl) methylamino]pyri dine-3-carboxylate | ethyl 4,6-dichloropyridine -3-carboxylate | NA | 320.8 | 321.1 |
| 10 | 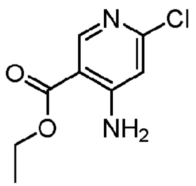 | ethyl 4-amino-6-chloro-pyridine-3-carboxylate | ethyl 6-chloro-4-[(4-methoxyphenyl) methylamino]py ridine-3-carboxylate | NA | 200.6 | 201. 0 |
| 11 | 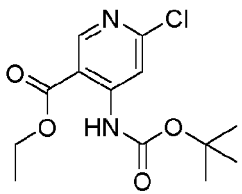 | ethyl 4-(tert-butoxycarbonyla mino)-6-chloropyridine-3-carboxylate | ethyl 4-amino-6-chloro-pyridine-3-carboxylate | NA | 300.7 | 301.1 |

(continued)

| Int | Structure | Name | Name starting material | Mtd | MW (calc) | MW (obs) |
|---|---|---|---|---|---|---|
| 12 | 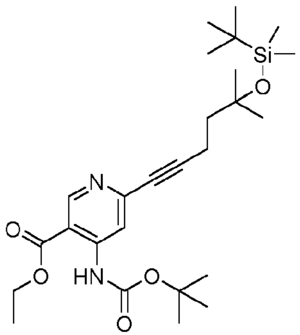 | ethyl 4-(tert-butoxycarbonyla mino)-6-[5-[tert-butyl(dimethyl)sil yl]oxy-5-methyl-hex-1-ynyl]pyridine-3-carboxylate | ethyl 4-(tert-butoxycarbonyla mino)-6-chloropyridine-3-carboxylate | NA | 490.7 | 491.2 |
| 13 | 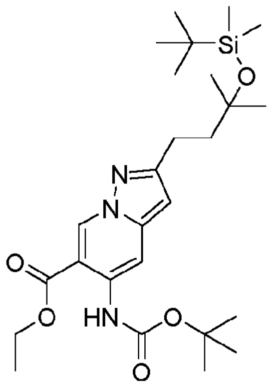 | ethyl 5-(tert-butoxycarbonyla mino)-2-[3-[tert-butyl(dimethyl)sil yl]oxy-3-methylbutyl]pyrazolo[1, 5-a]pyridine-6-carboxylate | ethyl 4-(tert-butoxycarbonyla mino)-6-[5-[tert-butyl (dimethyl)s ilyl]oxy-5-methyl-hex-1-ynyl]pyridine-3-carboxylate | NA | 505.7 | 506.2 |
| 14 | 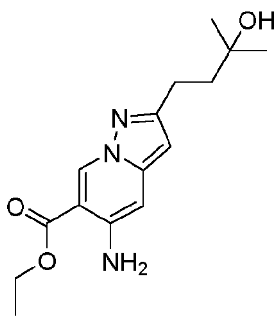 | ethyl 5-amino-2-(3-hydroxy-3-methylbutyl) pyrazolo[1, 5-a]pyridine-6-carboxylate | ethyl 5-(tert-butoxycarbonyla mino)-2-[3-[tert-butyl (dimethyl)s ilyl]oxy-3-methylbutyl]pyrazolo[1 ,5-a] pyridine-6-carboxylate | NA | 291.4 | 292.1 |

(continued)

| Int | Structure | Name | Name starting material | Mtd | MW (calc) | MW (obs) |
|---|---|---|---|---|---|---|
| 15 | 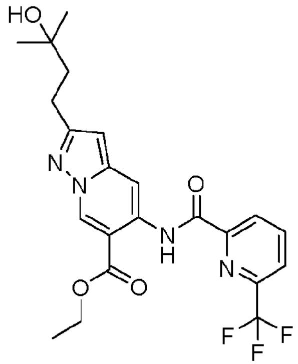 | ethyl 2-(3-hydroxy-3-methyl-butyl)-5-[[6-(trifluoromethyl)p yridine-2-carbonyl] amino]p yrazolo[1,5-a]pyridine-6-carboxylate | ethyl 5-amino-2-(3-hydroxy-3-methylbutyl)pyrazolo[1 , 5-a]pyridine-6-carboxylate | NA | 464.4 | 465.1 |
| 16 | 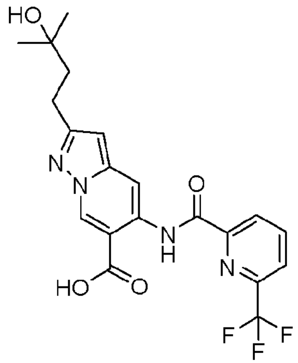 | 2-(3-hydroxy-3-methyl-butyl)-5-[[6-(trifluoromethyl)p yridine-2-carbonyl] amino]p yrazolo[1,5-a]pyridine-6-carboxylic acid | ethyl 2-(3-hydroxy-3-methyl-butyl)-5-[[6-(trifluoromethyl )pyridine-2-carbonyl]amino] pyrazolo[1,5-a]pyridine-6-carboxylate | NA | 436.4 | 437.1 |
| 17 | 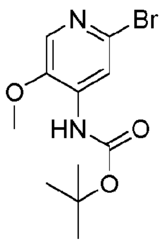 | tert-butyl N-(2-bromo-5-methoxy-4-pyridyl) carbamat e | 2-bromo-5-methoxy-pyridin-4-amine | NA | 303.2 | 305.1 |

(continued)

| Int | Structure | Name | Name starting material | Mtd | MW (calc) | MW (obs) |
|---|---|---|---|---|---|---|
| 18 | 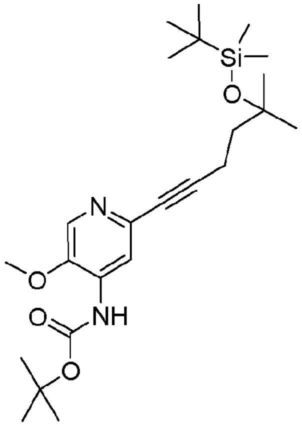 | N-[2-[5-[tert-butyl(dimethyl)sil yl]oxy-5-methyl-hex-1-ynyl]-5-methoxy-4-pyridyl] carbamat e | tert-butyl N-(2-bromo-5-methoxy-4-pyridyl)carbama te | Cl | 448.7 | 449.2 |
| 19 | 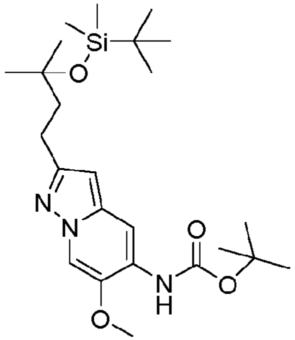 | tert-butyl N-[2-[3-[tert-butyl(dimethyl)sil yl] oxy-3-methylbutyl]-6-methoxy-pyrazolo [1,5-a]pyridin-5-yl]carbamate | N-[2-[5-[tert-butyl(dimethyl)s ilyl]oxy-5-methyl-hex-1-ynyl]-5-methoxy-4-pyridyl]carbama te | B | 463.7 | 464.5 |

(continued)

| Int | Structure | Name | Name starting material | Mtd | MW (calc) | MW (obs) |
|---|---|---|---|---|---|---|
| 20 | 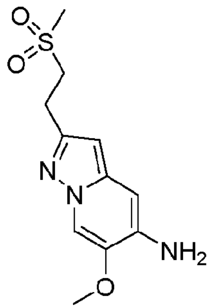 | 6-methoxy-2-(2-methylsulfonyleth yl) pyrazolo[1,5-a]pyridin-5-amine | tert-butyl N-[6-methoxy-2-(2-methylsulfonyle thyl) pyrazolo[1, 5-a]pyridin-5-yl]carbamate | I | 269.3 | 270.0 |
| 21 | 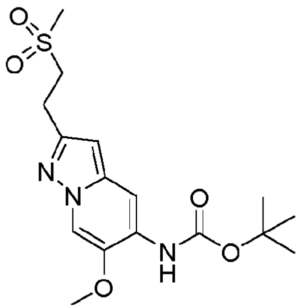 | tert-butyl N-[6-methoxy-2-(2-methylsulfonyleth yl)pyrazolo[1,5-a]pyridin-5-yl]carbamate | tert-butyl N-[5-methoxy-2-(4-methylsulfonylb ut-1-ynyl)-4-pyridyl]carbama te | NA | 369.4 | 370.4 |
| 22 | 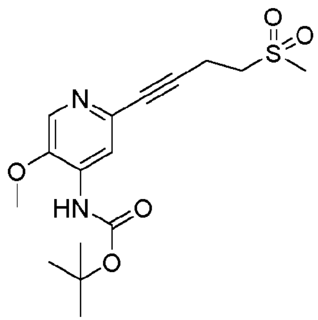 | tert-butyl N-[5-methoxy-2-(4-methylsulfonylbu t-1-ynyl)-4-pyridyl] carbamat e | tert-butyl N-(2-bromo-5-methoxy-4-pyridyl)carbama te | CI | 354.4 | 355.4 |

(continued)

| Int | Structure | Name | Name starting material | Mtd | MW (calc) | MW (obs) |
|---|---|---|---|---|---|---|
| 23 | 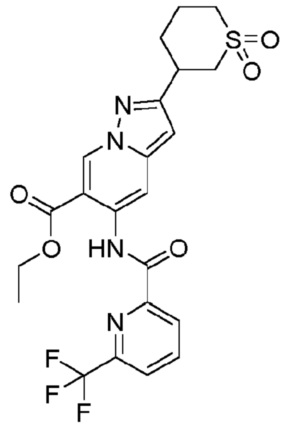 | ethyl 2-(1,1-dioxothian-3-yl)-5-[[6-(trifluoromethyl)p yridine-2-carbonyl] amino]p yrazolo[1,5-a]pyridine-6-carboxylate | ethyl 5-amino-2-(1,1-dioxothian-3-yl)pyrazolo[1,5-a] pyridine-6-carboxylate | J | 510.5 | 511.5 |
| 24 | 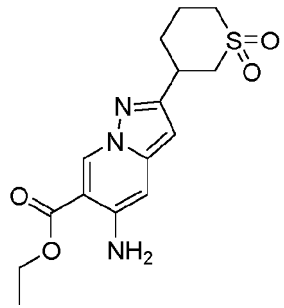 | ethyl 5-amino-2-(1,1-dioxothian-3-yl) pyrazolo[1,5-a]pyridine-6-carboxylate | ethyl 5-(tert-butoxycarbonyla mino)-2-(1,1-dioxothian-3-yl)pyrazolo[1,5-a]pyridine-6-carboxylate | I | 337.4 | 338.6 |

(continued)

| Int | Structure | Name | Name starting material | Mtd | MW (calc) | MW (obs) |
|---|---|---|---|---|---|---|
| 25 | 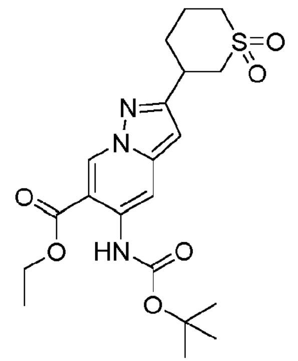 | ethyl 5-(tert-butoxycarbonyla mino)-2-(1,1-dioxothian-3-yl)pyrazolo[1,5-a]pyridine-6-carboxylate | ethyl 4-(tert-butoxycarbonyla mino)-6-[2-(1,1-dioxothian-3-yl)ethynyl]pyrid ine-3 - carboxylate | B | 437.5 | 438.6 |
| 26 | 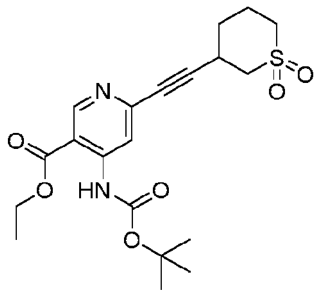 | ethyl 4-(tert-butoxycarbonyla mino)-6-[2-(1,1-dioxothian-3-yl)ethynyl]pyridi ne-3-carboxylate | ethyl 4-(tert-butoxycarbonyla mino)-6-chloropyridine-3-carboxylate | C2 | 422.5 | 423.6 |
| 27 | 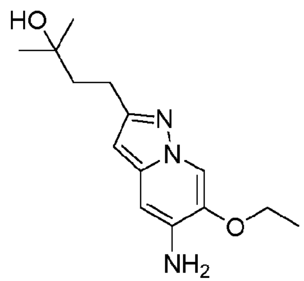 | 4-(5-amino-6-ethoxy-pyrazolo[1,5-a] pyridin-2-yl)-2-methyl-butan-2-ol | 2-[3-[tert-butyl(dimethyl)s ilyl]oxy-3-methyl-butyl]-6-ethoxy-pyrazolo[1,5-a]pyridin-5-amine | G | 263.3 | 264.6 |

(continued)

| Int | Structure | Name | Name starting material | Mtd | MW (calc) | MW (obs) |
|---|---|---|---|---|---|---|
| 28 | 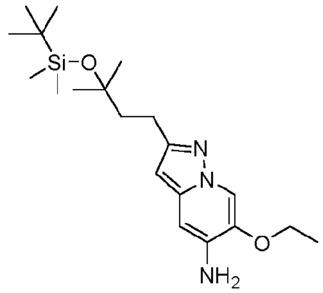 | 2-[3-[tert-butyl(dimethyl)sil yl]oxy-3-methyl-butyl] -6-ethoxy-pyrazolo[1,5-a]pyridin-5 -amine | ethyl 5-(tert-butoxycarbonyla mino)-2-[3-[tert-butyl (dimethyl)s ilyl]oxy-3-methyl-butyl]-6-ethoxy-pyrazolo [1,5-a]pyridine-3-carboxylate | F | 377.6 | 378.7 |
| 29 | 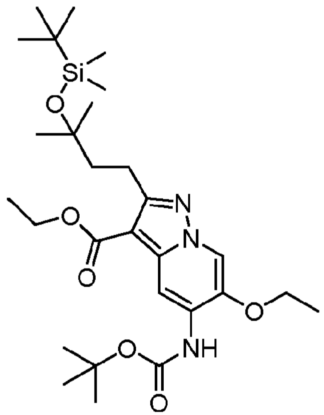 | ethyl 5-(tert-butoxycarbonyla mino)-2-[3-[tert-butyl(dimethyl)sil yl]oxy-3-methylbutyl] -6-ethoxy-pyrazolo[1,5-a] pyridine-3-carboxylate | tert-butyl N-(3-ethoxy-4-pyridyl)carbama te | E | 549.8 | 550.7 |
| 30 | 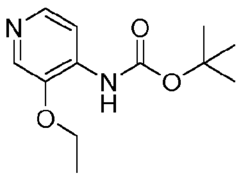 | tert-butyl N-(3-ethoxy-4-pyridyl)carbamat e | 3-ethoxypyridin-4-amine | D | 238.3 | 239.6 |

(continued)

| Int | Structure | Name | Name starting material | Mtd | MW (calc) | MW (obs) |
|---|---|---|---|---|---|---|
| 31 | 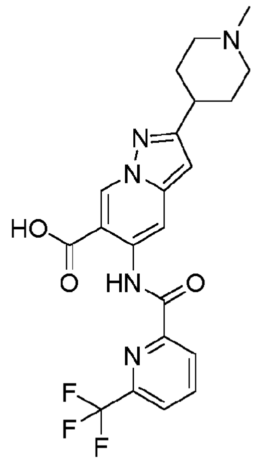 | 2-(1-methyl-4-piperidyl)-5-[[6-(trifluoromethyl)p yridine-2-carbonyl] amino]p yrazolo[1,5-a]pyridine-6-carboxylic acid | ethyl 2-(1-methyl-4-piperidyl)-5-[[6-(trifluoromethyl ) pyridine-2-carbonyl]amino] pyrazolo[1,5-a]pyridine-6-carboxylate | K | 447.4 | 448.5 |
| 32 | 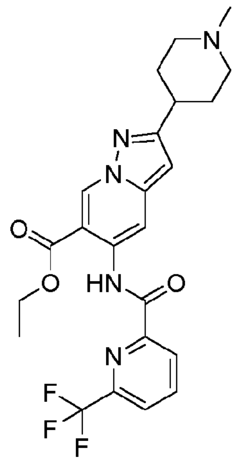 | ethyl 2-(1-methyl-4-piperidyl)-5-[[6-(trifluoromethyl)p yridine-2-carbonyl] amino]p yrazolo[1,5-a]pyridine-6-carboxylate | ethyl 5-amino-2-(1-methyl-4-piperidyl)pyrazo lo[1,5-a] pyridine-6-carboxylate | NA | 475.5 | 476.6 |

(continued)

| Int | Structure | Name | Name starting material | Mtd | MW (calc) | MW (obs) |
|---|---|---|---|---|---|---|
| 33 | 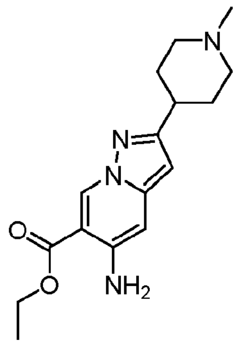 | ethyl 5-amino-2-(1-methyl-4-piperidyl) pyrazol o[1,5-a]pyridine-6-carboxylate | ethyl 5-amino-2-(4-piperidyl)pyrazo lo[1,5-a]pyridine-6-carboxylate | NA | 302.4 | 303.6 |
| 34 | 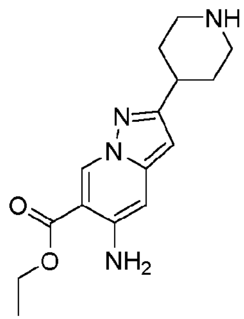 | ethyl 5-amino-2-(4-piperidyl)pyrazol o[1,5-a]pyridine-6-carboxylate | ethyl 5-(tert-butoxycarbonyla mino)-2-(1-tert-butoxycarbonyl-4-piperidyl)pyrazo lo[1,5-a]pyridine-6-carboxylate | NA | 288.3 | 289.6 |

(continued)

| Int | Structure | Name | Name starting material | Mtd | MW (calc) | MW (obs) |
|---|---|---|---|---|---|---|
| 35 | 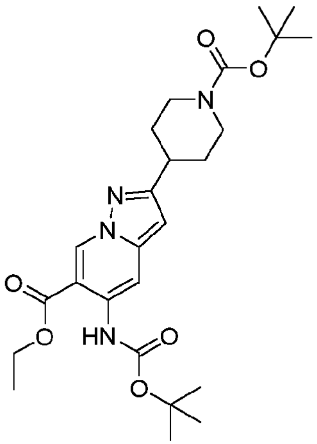 | ethyl 5-(tert-butoxycarbonyla mino)-2-(1-tert-butoxycarbonyl-4-piperidyl)pyrazol o [1,5-a]pyridine-6-carboxylate | ethyl 4-(tert-butoxycarbonyla mino)-6-[2-(1-tert-butoxycarbonyl-4-piperidyl)ethyny l]pyridine-3-carboxylate | B | 488.6 | 489.6 |
| 36 | 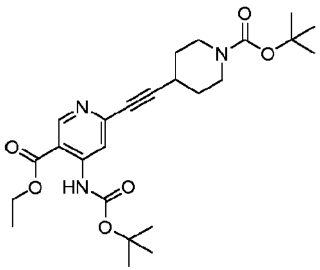 | ethyl 4-(tert-butoxycarbonyla mino)-6-[2-(1-tert-butoxycarbonyl-4-piperidyl)ethynyl] pyridine-3-carboxylate | ethyl 4-(tert-butoxycarbonyla mino)-6-chloropyridine-3-carboxylate | C2 | 473.6 | 474.1 |

(continued)

| Int | Structure | Name | Name starting material | Mtd | MW (calc) | MW (obs) |
|---|---|---|---|---|---|---|
| 37 | 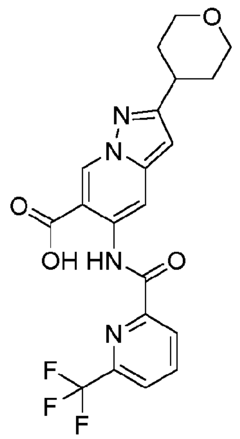 | 2-tetrahydropyran-4-yl-5-[[6-(trifluoromethyl)p yridine-2-carbonyl] amino]p yrazolo[1,5-a]pyridine-6-carboxylic acid | ethyl 2-tetrahydropyran-4-yl-5-[[6-(trifluoromethyl ) pyridine-2-carbonyl]amino] pyrazolo[1,5-a]pyridine-6-carboxylate | K | 434.4 | 435.5 |
| 38 | 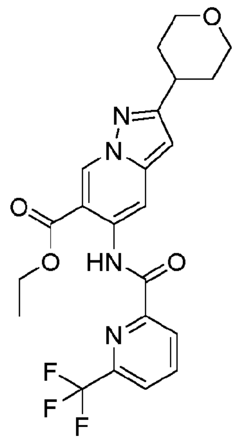 | ethyl 2-tetrahydropyran-4-yl-5-[[6-(trifluoromethyl)p yridine-2-carbonyl] amino]p yrazolo[1,5-a]pyridine-6-carboxylate | ethyl 5-amino-2-tetrahydropyran-4-yl-pyrazolo[1,5-a] pyridine-6-carboxylate | J | 462.4 | 463.5 |

(continued)

| Int | Structure | Name | Name starting material | Mtd | MW (calc) | MW (obs) |
|---|---|---|---|---|---|---|
| 39 | 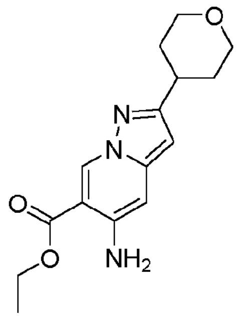 | ethyl 5-amino-2-tetrahydropyran-4-yl-pyrazolo[1,5-a]pyridine-6-carboxylate | ethyl 5-(tert-butoxycarbonyla mino)-2-tetrahydropyran-4-yl-pyrazolo[1,5-a]pyridine-6-carboxylate | I | 289.3 | 290.6 |
| 40 | 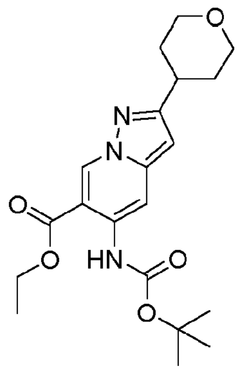 | ethyl 5-(tert-butoxycarbonyla mino)-2-tetrahydropyran-4-yl-pyrazolo[1,5-a] pyridine-6-carboxylate | ethyl 4-(tert-butoxycarbonyla mino)-6-(2-tetrahydropyran-4-ylethynyl)pyridi ne-3-carboxylate | B | 389.4 | 390.6 |
| 41 | 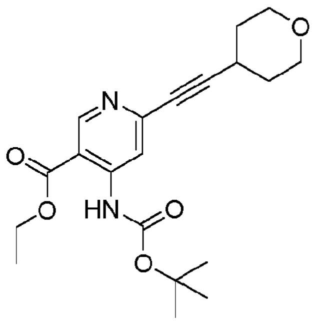 | ethyl 4-(tert-butoxycarbonyla mino)-6-(2-tetrahydropyran-4-ylethynyl)pyridin e-3-carboxylate | ethyl 4-(tert-butoxycarbonyla mino)-6-chloropyridine-3-carboxylate | C2 | 374.4 | 375.0 |

(continued)

| Int | Structure | Name | Name starting material | Mtd | MW (calc) | MW (obs) |
|---|---|---|---|---|---|---|
| 42 | 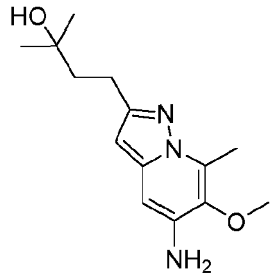 | 4-(5-amino-6-methoxy-7-methyl-pyrazolo [1,5-a]pyridin-2-yl)-2-methyl-butan-2-ol | 2-[3-[tert-butyl(dimethyl)s ilyl]oxy-3-methyl-butyl]-6-methoxy-7-methyl-pyrazolo[1,5-a]pyridin-5-amine | G | 263.3 | 264.6 |
| 43 | 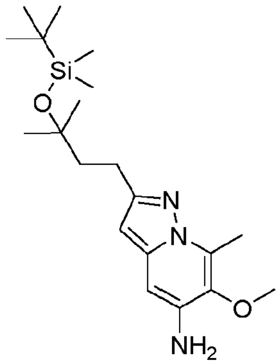 | 2-[3-[tert-butyl(dimethyl)sil yl]oxy-3-methylbutyl]-6-methoxy-7-methyl-pyrazolo [1,5-a]pyridin-5-amine | ethyl 5-(tert-butoxycarbonyla mino)-2-[3-[tert-butyl (dimethyl)s ilyl]oxy-3-methyl-butyl]-6-methoxy-7-methyl-pyrazolo[1,5-a]pyridine-3-carboxylate | F | 377.6 | 378.7 |

(continued)

| Int | Structure | Name | Name starting material | Mtd | MW (calc) | MW (obs) |
|---|---|---|---|---|---|---|
| 44 | 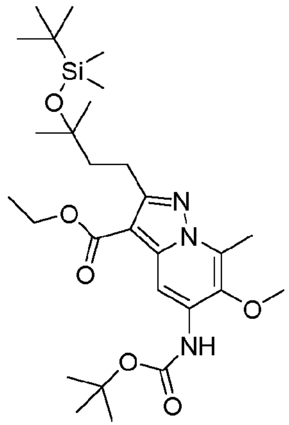 | ethyl 5-(tert-butoxycarbonyla mino)-2-[3-[tert-butyl(dimethyl)sil yl]oxy-3-methylbutyl]-6-methoxy-7-methyl-pyrazolo [1,5-a]pyridine-3-carboxylate | tert-butyl N-(3-methoxy-2-methyl-4-pyridyl)carbama te | E | 549.8 | 548.6 |
| 45 | 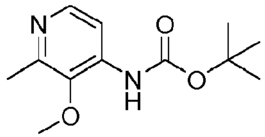 | tert-butyl N-(3-methoxy-2-methyl-4-pyridyl) carbamat e | 3-methoxy-2-methyl-pyridin-4-amine | D | 238.3 | 239.6 |
| 46 | 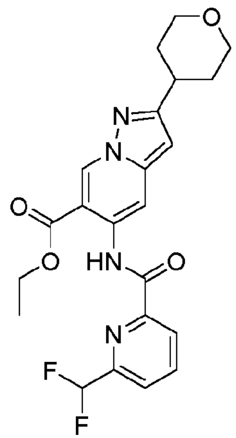 | ethyl 5-[[6-(difluoromethyl)p yridine-2-carbonyl]amino]-2-tetrahydropyran-4-yl-pyrazolo[1,5-a]pyridine-6-carboxylate | ethyl 5-amino-2-tetrahydropyran-4-yl-pyrazolo[1,5-a] pyridine-6-carboxylate | J | 444.4 | 445.6 |

(continued)

| Int | Structure | Name | Name starting material | Mtd | MW (calc) | MW (obs) |
|---|---|---|---|---|---|---|
| 47 | 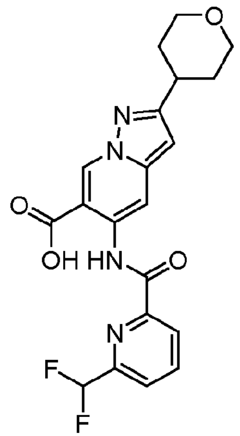 | 5-[[6-(difluoromethyl)p yridine-2-carbonyl] amino]-2-tetrahydropyran-4-yl-pyrazolo [1,5-a]pyridine-6-carboxylic acid | ethyl 5-[[6-(difluoromethyl) pyridine-2-carbonyl]amino] -2-tetrahydropyran-4-yl-pyrazolo[1,5-a]pyridine-6-carboxylate | K | 416.4 | 417.5 |
| 48 | 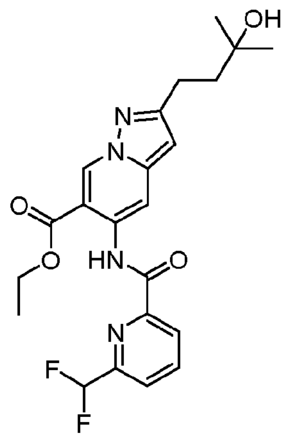 | ethyl 5-[[6-(difluoromethyl)p yridine-2-carbonyl]amino]-2-(3-hydroxy-3-methylbutyl)pyrazolo[1, 5-a]pyridine-6-carboxylate | ethyl 5-amino-2-(3-hydroxy-3-methylbutyl)pyrazolo[1 , 5-a]pyridine-6-carboxylate | NA | 446.4 | 447.6 |

(continued)

| Int | Structure | Name | Name starting material | Mtd | MW (calc) | MW (obs) |
|---|---|---|---|---|---|---|
| 49 | 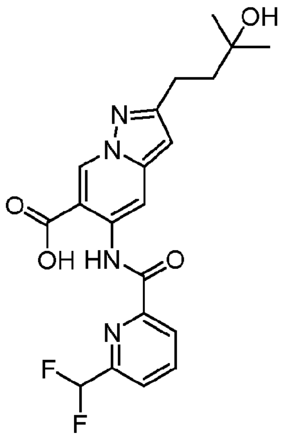 | 5-[[6-(difluoromethyl)p yridine-2-carbonyl] amino]-2-(3-hydroxy-3-methylbutyl) pyrazolo[1, 5-a]pyridine-6-carboxylic acid | ethyl 5-[[6-(difluoromethyl) pyridine-2-carbonyl]amino] -2-(3-hydroxy-3-methylbutyl)pyrazolo[1 ,5-a]pyridine-6-carboxylate | K | 418.4 | 419.5 |
| 50 | 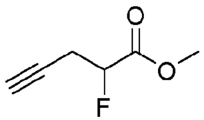 | methyl 2-fluoropent-4-ynoate | dimethyl 2-fluoropropanedi oate | NA | 130.1 | NA |
| 51 | 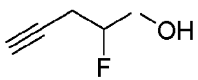 | 3-fluoro-2-methyl-hex-5-yn-2-ol | methyl 2-fluoropent-4-ynoate | NA | 130.2 | NA |
| 52 | 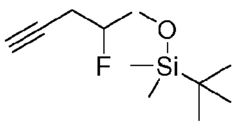 | tert-butyl-(2-fluoro-1,1-dimethyl-pent-4-ynoxy)-dimethyl-silane | 3-fluoro-2-methyl-hex-5-yn-2-ol | NA | 244.4 | NA |

(continued)

| Int | Structure | Name | Name starting material | Mtd | MW (calc) | MW (obs) |
|---|---|---|---|---|---|---|
| 53 | 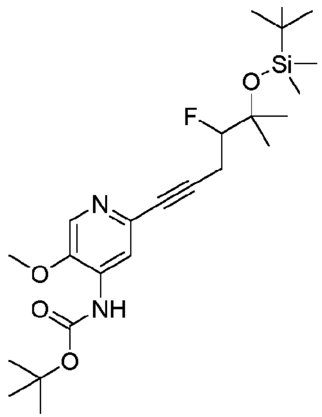 | tert-butyl N-[2-[5-[tert-butyl(dimethyl)sil yl] oxy-4-fluoro-5-methyl-hex-1-ynyl]-5-methoxy-4-pyridyl]carbamat e | tert-butyl N-(2-bromo-5-methoxy-4-pyridyl)carbama te | NA | 466.7 | 467.2 |
| 54 | 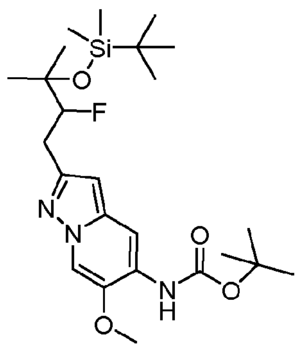 | tert-butyl N-[2-[3-[tert-butyl(dimethyl)sil yl] oxy-2-fluoro-3-methyl-butyl]-6-methoxy-pyrazolo[1,5-a]pyridin-5-yl]carbamate | tert-butyl N-[2-[5-[tert-butyl(dimethyl)s ilyl]oxy-4-fluoro-5-methyl-hex-1-ynyl]-5-methoxy-4-pyridyl]carbama te | NA | 481.7 | 482.1 |

(continued)

| Int | Structure | Name | Name starting material | Mtd | MW (calc) | MW (obs) |
|---|---|---|---|---|---|---|
| 55 | 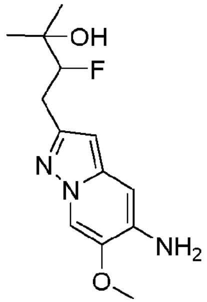 | 4-(5-amino-6-methoxy-pyrazolo[1,5-a] pyridin-2-yl)-3-fluoro-2-methyl-butan-2-ol | tert-butyl N-[2-[3-[tert-butyl(dimethyl)s ilyl]oxy-2-fluoro-3-methylbutyl]-6-methoxy-pyrazolo[1,5-a]pyridin-5-yl] carbamate | H | 267.3 | 268.1 |
| 56 | 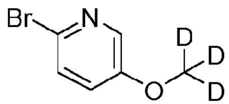 | 2-bromo-5-(trideuteriometho xy)pyridine | 6-bromopyridin-3-ol | NA | 191.0 | 191.0 193.1 |
| 57 | 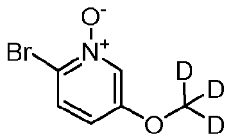 | 2-bromo-1-oxido-5-(trideuteriometho xy) pyridin-1-ium | 2-bromo-5-(trideuteriometh oxy)pyridine | NA | 207.0 | 207.0 209.0 |
| 58 | 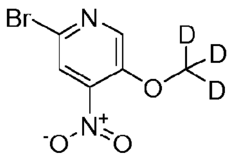 | 2-bromo-5-methoxy-4-nitropyridine | 2-bromo-1-oxido-5-(trideuteriometh oxy)pyridin-1-ium | NA | 236.0 | 233.9 235.9 |
| 59 | 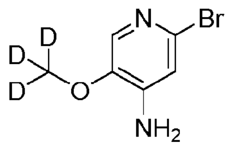 | 2-bromo-5-(trideuteriometho xy)pyridin-4-amine | 2-bromo-5-methoxy-4-nitropyridine | NA | 206.1 | 206.1 208.1 |

(continued)

| Int | Structure | Name | Name starting material | Mtd | MW (calc) | MW (obs) |
|---|---|---|---|---|---|---|
| 60 | 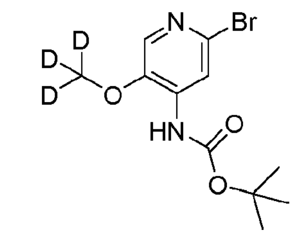 | tert-butyl N-[2-bromo-5-(trideuteriomethoxy)-4-pyridyl]carbamate | 2-bromo-5-(trideuteriomethoxy)pyridin-4-amine | NA | 306.2 | 306.1 308.1 |
| 61 | 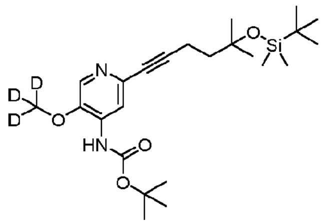 | tert-butyl N-[2-[5-[tert-butyl(dimethyl)silyl]oxy-5-methyl-hex-1-ynyl]-5-(trideuteriomethoxy)-4-pyridyl]carbamate | tert-butyl N-[2-bromo-5-(trideuteriomethoxy)-4-pyridyl]carbamate | NA | 451.7 | 452.4 |
| 62 | 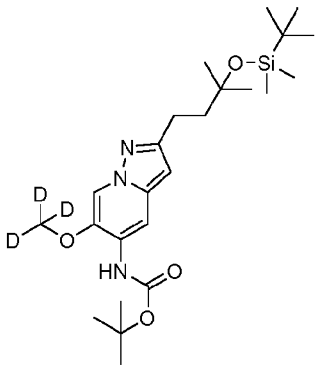 | tert-butyl N-[2-[3-[tert-butyl(dimethyl)silyl]oxy-3-methylbutyl]-6-(trideuteriomethoxy)pyrazolo[1,5-a]pyridin-5-yl]carbamate | tert-butyl N-[2-[5-[tert-butyl(dimethyl)silyl]oxy-5-methyl-hex-1-ynyl]-5-(trideuteriomethoxy)-4-pyridyl]carbamate | NA | 466.7 | 467.4 |

(continued)

| Int | Structure | Name | Name starting material | Mtd | MW (calc) | MW (obs) |
|---|---|---|---|---|---|---|
| 63 | 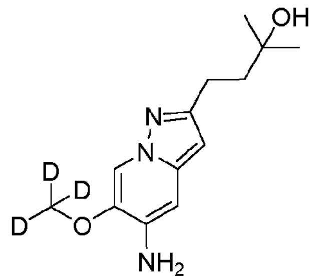 | 4-[5-amino-6-(trideuteriometho xy)pyrazolo [1,5-a]pyridin-2-yl]-2-methyl-butan-2-ol | tert-butyl N-[2-[3-[tert-butyl(dimethyl)s ilyl]oxy-3-methyl-butyl]-6-(trideuteriometh oxy)pyrazolo[1, 5-a] pyridin-5-yl]carbamate | H | 252.3 | 253.2 |
| 64 | 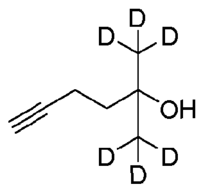 | 1,1,1-trideuterio-2-(trideuteriomethyl )hex-5-yn-2-ol | ethyl pent-4-ynoate | NA | 118.2 | NA |
| 65 | 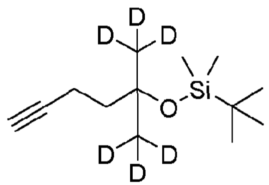 | 1,1-bis(trideuteriomet hyl)pent-4-ynoxy-tert-butyldimethyl-silane | 1,1,1-trideuterio-2-(trideuteriometh yl)hex-5-yn-2-ol | NA | 232.5 | NA |
| 66 | 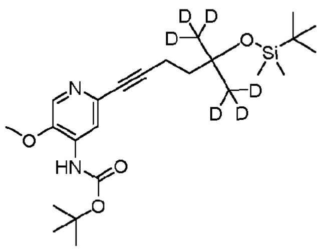 | tert-butyl N-[2-[5-[tert-butyl(dimethyl)sil yl] oxy-6,6,6-trideuterio-5-(trideuteriomethyl ) hex-1-ynyl]-5-methoxy-4-pyridyl]carbamat e | tert-butyl N-(2-bromo-5-methoxy-4-pyridyl)carbama te | NA | 454.7 | 455.4 |

(continued)

| Int | Structure | Name | Name starting material | Mtd | MW (calc) | MW (obs) |
|---|---|---|---|---|---|---|
| 67 | 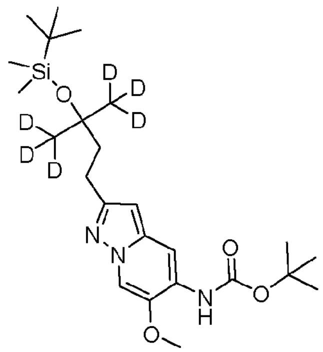 | tert-butyl N-[2-[3-[tert-butyl(dimethyl)sil yl] oxy-4,4,4-trideuterio-3-(trideuteriomethyl ) butyl]-6-methoxy-pyrazolo[1,5-a]pyridin-5-yl]carbamate | tert-butyl N-[2-[5-[tert-butyl(dimethyl)s ilyl]oxy-6,6,6-trideuterio-5-(trideuteriometh yl)hex-1-ynyl]-5-methoxy-4-pyridyl]carbama te | NA | 469.7 | 470.4 |
| 68 | 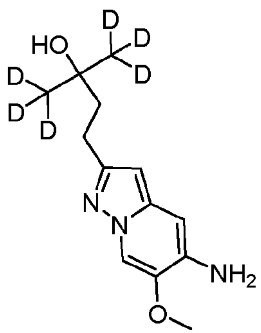 | 4-(5-amino-6-methoxy-pyrazolo[1,5-a] pyridin-2-yl)-1,1,1-trideuterio-2-(trideuteriomethyl )butan-2-ol | tert-butyl N-[2-[3-[tert-butyl(dimethyl)s ilyl]oxy-4,4,4-trideuterio-3-(trideuteriometh yl)butyl]-6-methoxy-pyrazolo[1,5-a]pyridin-5-yl]carbamate | NA | 255.3 | 256.3 |

**Example 2. Preparation of the Illustrative compounds of the invention**

***2.1. General method A: synthesis of amides***

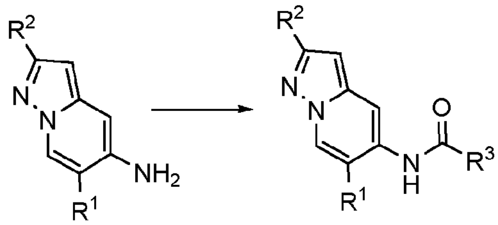

A mixture of amine (1.0 eq.), HATU, CAS#148893-10-1 (1.2 eq.), carboxylic acid (1.2 eq.) and DIPEA (2.0 eq.) in DCM is stirred at r.t. for 16 to 72 h. The reaction mixture undergoes an aq. work up. The two phases are separated and the organic layer is dried and concentrated. The residue is purified by flash column chromatography or by preparative HPLC.

*Illustrative example of method A, synthesis of compound 1: 1-cyclopropyl-N-[2-(3-hydroxy-3-methylbutyl)-6-methoxypyrazolo[1,5-a]pyridin-5-yl]-2-oxopyridine-3-carboxamide*

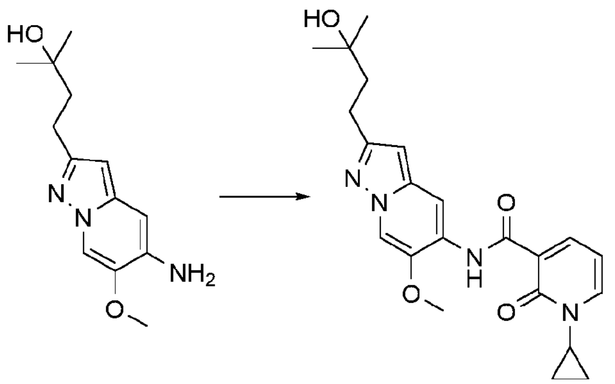

A mixture of 4-(5-amino-6-methoxy-pyrazolo[1,5-a]pyridin-2-yl)-2-methyl-butan-2-ol (105 mg, 0.42 mmol, 1.0 eq.), 1-cyclopropyl-2-oxo-pyridine-3-carboxylic acid (91 mg, 0.51 mmol, 1.2 eq.), HATU, CAS#148893-10-1 (192 mg, 0.51 mmol, 1.2 eq.) and DIPEA (0.15 mL, 0.84 mmol, 2.0 eq.) in DCM (16 mL) was stirred at r.t. for 16 h. Saturated aq. $NaHCO_3$ (15 mL) was added to the mixture and the resulting mixture was stirred at r.t. for 10 min. The two phases were separated and the aq. layer was extracted (DCM). The combined organic layers were dried (filtered through hydrophobic frit) and concentrated. The residue was purified by flash column chromatography ($SiO_2$, 100:0 to 0:100 DCM/ ternary mixture constituted by 90:5:0.5 DCM/MeOH/$NH_4OH$) to afford the desired product.

*Alternative synthesis of compound 1: 1-cyclopropyl-N-[2-(3-hydroxy-3-methylbutyl)-6-methoxypyrazolo [1,5-a]pyridin-5-yl]-2-oxopyridine-3-carboxamide*

1-cyclopropyl-2-oxo-1,2-dihydropyridine-3-carboxylic acid (4.63 g, 25.8 mmol, 1.2 eq.), HATU, CAS#148893-10-1 (9.83 g, 25.8 mmol, 1.23 eq.) and DIPEA (7.5 mL, 43.1 mmol, 2.0 eq.) were added to a mixture of 4-(5-amino-6-methoxy-pyrazolo[1,5-a]pyridin-2-yl)-2-methyl-butan-2-ol (5.37 g, 21.5 mmol, 1.0 eq.) in DCM (600 mL). The mixture was stirred at r.t. for 20 h. The reaction was quenched with saturated aq. $NaHCO_3$ (300 mL) and the resulting mixture was stirred for 10 min. The two layers were separated and the aqueous layer was extracted (2 x 150 mL of DCM). The organic layers were combined, dried (filtered through phase separator) and concentrated. The residue was purified by flash column chromatography ($SiO_2$, 100:0 to 0:100 DCM/ ternary mixture constituted by 90:5:0.5 DCM/MeOH/$NH_4OH$). Product obtained from less pure fractions undergoes a second flash column chromatography ($SiO_2$, 100:0

to 0:100 DCM/ ternary mixture constituted by 90:5:0.5 DCM/MeOH/$NH_4OH$). All fractions containing product with purity >95% were combined and concentrated. The residue so obtained was recrystallized from hot acetonitrile to afford the desired product.

*Alternative synthesis of compound 1: 1-cyclopropyl-N-[2-(3-hydroxy-3-methylbutyl)-6-methoxypyrazolo [1,5-a]pyridin-5-yl]-2-oxopyridine-3-carboxamide*

HATU, CAS#148893-10-1 (23.0 g, 59 mmol, 1.1 eq.) was added by portions to a mixture of 4-(5-amino-6-methoxy-pyrazolo[1,5-a]pyridin-2-yl)-2-methyl-butan-2-ol (14 g, 56 mmol, 1.0 eq.) and 1-cyclopropyl-2-oxo-1,2-dihydropyridine-3-carboxylic acid (11 g, 59 mmol, 1.0 eq.) in DCM (220 mL) in a round bottom flask under $N_2$ at 0 °C. DIPEA (15 g, 20 mL, 110 mmol, 2.0 eq.) was added and the reaction mixture was stirred for 5 min at 0 °C then the reaction mixture was allowed to warm to r.t. and stirred for 1 h. The reaction mixture was quenched with a saturated $NaHCO_3$ solution (250 mL) and diluted with 200 mL of DCM. The resulting mixture was stirred for 1 h. The insoluble present was filtered off. The solid was washed with 200 mL of water and dried under reduced pressure. The organic and aq. phases contained in the filtrate were separated after decantation. The aq. layer was extracted with DCM (1 × 200 mL and 1 × 100 mL). The combined organic layers were washed with $H_2O$ (1 × 400mL) and brine (1 × 400mL), filtered on phase separator and concentrated. The residue was taken up in 200 mL of EtOAc and 200 mL of a saturated $NH_4Cl$ solution. The resulting mixture was stirred for 15 min. The insoluble present was filtered off, washed with 2 x 200 mL of water and dried under reduced pressure. The solid materials so obtained were combined and taken up in MeCN. The resulting mixture was heated to 95 to 100 °C. The insoluble materials were filtered off and the filtrate was cooled to 0 °C. A precipitate was formed, filtered off and dried under vacuum to afford the desired product.

***2.2. Synthesis of compound 4: 2-(3-hydroxy-3-methylbutyl)-5-[[6-(trifluoromethyl)pyridine-2-carbonyl]amino]pyrazolo[1,5-a]pyridine-6-carboxamide***

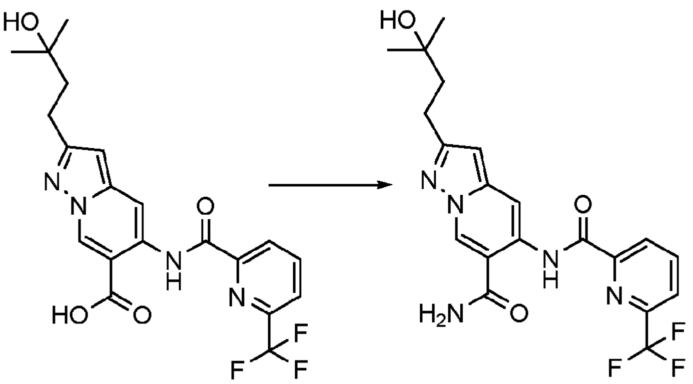

A mixture of 2-(3-hydroxy-3-methyl-butyl)-5-[[6-(trifluoromethyl)pyridine-2-carbonyl]amino] pyrazolo[1,5-a]pyridine-6-carboxylic acid (148 mg, 0.34 mmol, 1.0 eq.) and HATU, CAS#148893-10-1 (155 mg, 0.41 mmol, 1.2 eq.) in DCM (2 mL) was stirred at r.t. for 10 min. DIPEA (0.18 mL, 0.68 mmol, 2.0 eq.) and $NH_4Cl$ (54 mg, 1.0 mmol, 3.0 eq.) were added and the resulting mixture was stirred at r.t. for 2 h. Aq. ammonia (1 mL) was added and the resulting mixture was stirred for 1.5 h. The mixture was diluted (DCM), washed (saturated aq. $NH_4Cl$, saturated $NaHCO_3$, brine), dried ($Na_2SO_4$) and concentrated. The residue was purified by flash column chromatography ($SiO_2$, 100:0 to 0:100 DCM/ ternary mixture constituted by 90:4:1 DCM/MeOH/$NH_4OH$). The material so obtained was further purified in the following way: the material was taken up in a mixture composed by 10:1 DCM/DMF (11 mL) and saturated $NaHCO_3$ (15 mL). The two phases were separated and a precipitate was formed in the aq. layer to afford the desired product.

*2.3. Synthesis of compound 5: 2-(3-hydroxy-3-methylbutyl)-N-methyl-5-[[6-(trifluoromethyl) pyridine-2-carbonyl]amino]pyrazolo[1,5-a]pyridine-6-carboxamide*

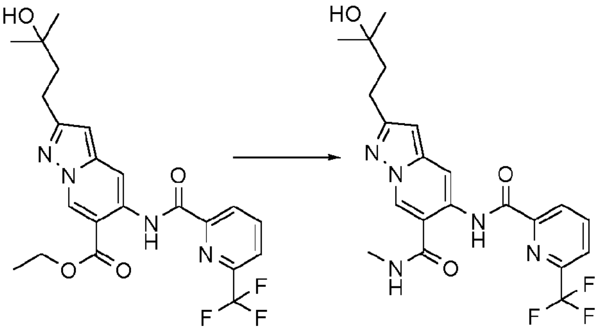

A mixture of ethyl 2-(3-hydroxy-3-methyl-butyl)-5-[[6-(trifluoromethyl)pyridine-2-carbonyl] amino]pyrazolo[1,5-a]pyridine-6-carboxylate (50 mg, 0.11 mmol, 1.0 eq.) in methylamine (33 wt.% solution in absolute EtOH, 1.0 mL) was stirred at 60 °C for 16 h. The mixture was concentrated and the residue was purified by flash column chromatography ($SiO_2$, 100:0 to 20:80 DCM/ ternary mixture constituted by 90:4:1 DCM/MeOH/$NH_4OH$) to afford the desired product.

*2.4. General method L: synthesis of amides by reaction of esters with methylamine*

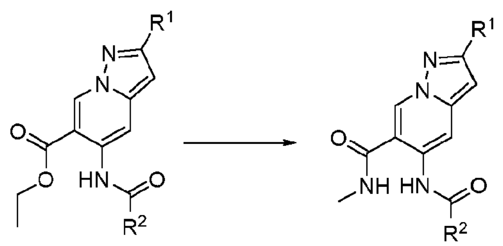

A mixture of ester derivative (1.0 eq.) in 33% wt methylamine in absolute EtOH (0.06 to 0.12 M) is stirred at 80 °C for 4-5 h. In case of uncomplete conversion, additional 33% wt methylamine in absolute EtOH may be added (1/3 of initial amount) and the mixture may be stirred at 60 °C for a further 16 to 72 h. The mixture is concentrated and the residue is purified by flash column chromatography to afford the desired product.

*Illustrative example of method L: synthesis of compound 19: 5-[[6-(difluoromethyl)pyridine-2-c*$\alpha$*rbonyl]*$\alpha$*mino]-2-(3-hydroxy-3-methyl-butyl)-N-methyl-pyrazolo[1,5-*$\alpha$*]pyridine-6-c*$\alpha$*rbox*$\alpha$*mide*

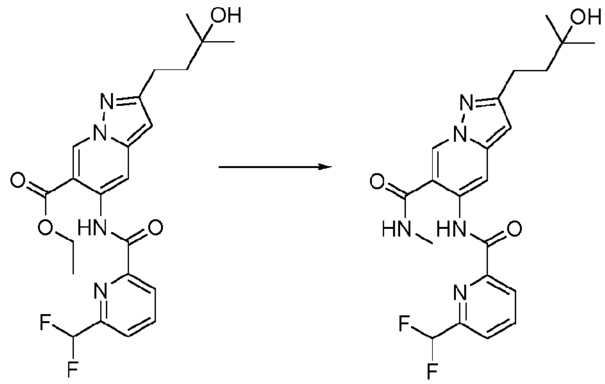

A mixture of ethyl 5-[[6-(difluoromethyl)pyridine-2-carbonyl]amino]-2-(3-hydroxy-3-methylbutyl)pyrazolo[1,5-a]pyridine-6-carboxylate (100 mg, 0.22 mmol, 1.0 eq.) in 33% wt methylamine in absolute EtOH (3 mL) for 4 h. The mixture was concentrated and the residue was purified by flash column chromatography ($SiO_2$, 100:0 to 20:80 DCM / ternary mixture constituted by 90:4:1 DCM/MeOH/$NH_4OH$) to afford the desired product.

*2.5. Synthesis of compound 12: N-methyl-2-(1-methyl-4-piperidyl)-5-[[6-(trifluoromethyl)pyridine-2-carbonyl]amino]pyrazolo[1,5-a]pyridine-6-carboxamide*

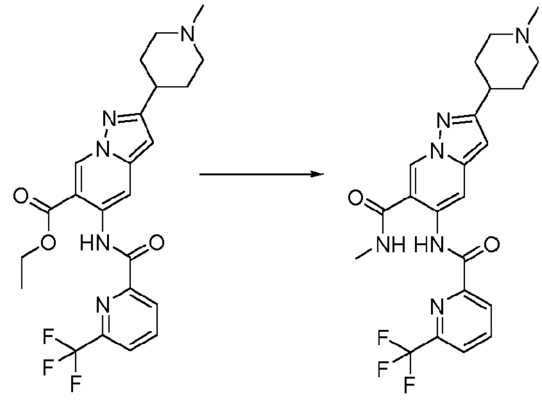

A mixture of ethyl ethyl 2-(1-methyl-4-piperidyl)-5-[[6-(trifluoromethyl)pyridine-2-carbonyl] amino]pyrazolo[1,5-a]pyridine-6-carboxylate (90 mg, 0.189 mmol, 1.0 eq.) in methylamine (33 w t.% solution in absolute EtOH, 3.0 mL) was stirred at 60 °C for 16 h. The mixture was concentrated and the residue was purified by flash column chromatography ($SiO_2$, 95:5 to 0:100 DCM / ternary mixture constituted by 90:9:0.5 DCM/MeOH/ $NH_4OH$) to afford the desired product.

*2.6. General method M: synthesis of primary amides by reaction of carboxylic acids with ammonium chloride*

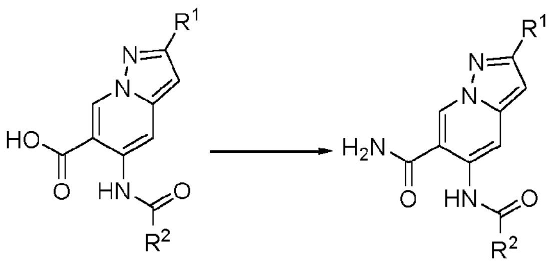

A mixture of HATU, CAS#148893-10-1 (1.2 eq.) and the carboxylic acid derivative (1.0 eq.) in DCM is stirred at r.t. for 10 min. DIPEA (2.0 eq.) and $NH_4Cl$ (3.0 eq.) are added. The mixture is stirred at r.t. for 2 h. To increase solubility, DMF may be added and the mixture is stirred for a further 3 to 4 h. Aq. ammonia is added and the mixture is stirred at r.t. for 16 h. The reaction undergoes aq. work up and the crude obtained after work up is purified by flash column chromatography to afford the desired product.

*Illustrative example of method M, synthesis of compound 11: 2-tetrahydropyran-4-yl-5-[[6-(trifluoromethyl)pyridine-2-carbonyl]amino]pyrazolo[1,5-a]pyridine-6-carboxamide*

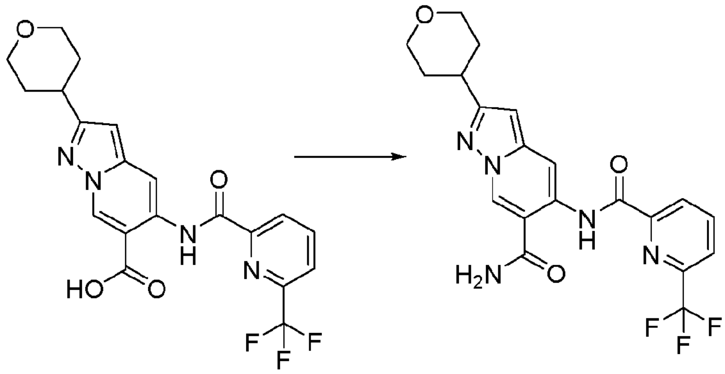

A mixture of 2-tetrahydropyran-4-yl-5-[[6-(trifluoromethyl)pyridine-2-carbonyl]amino] pyrazolo [1,5-a]pyridine-6-carboxylic acid (93 mg, 0.21 mmol, 1.0 eq.) and HATU, CAS#148893-10-1 (98 mg, 0.26 mmol, 1.2 eq.) in DCM (2 mL) was stirred at r.t. for 10 min. DIPEA (0.074 mL, 0.43 mmol, 2.0 eq.) and $NH_4Cl$ (34 mg, 0.64 mmol, 3.0 eq.) were added and the resulting mixture was stirred at r.t. for 2 h. DMF (2 mL) was added and the resulting mixture was stirred for 4 h. Aq. ammonia (2 mL) was added and the resulting mixture was stirred at r.t. for 16 h. The mixture was diluted (EtOAc), washed (saturated aqueous $NH_4Cl$, saturated $NaHCO_3$ and brine), dried ($Na_2SO_4$) and concentrated. The residue was purified by flash column chromatography ($SiO_2$, 100:0 to 0:100 DCM/ ternary mixture constituted by 90:4:1 DCM/MeOH/ $NH_4OH$) to afford the desired product.

*2.7. Synthesis of compound 10: 2-(1-methyl-4-piperidyl)-5-[[6-(trifluoromethyl)pyridine-2-carbonyl]amino]pyrazolo[1,5-a]pyridine-6-carboxamide formic acid salt*

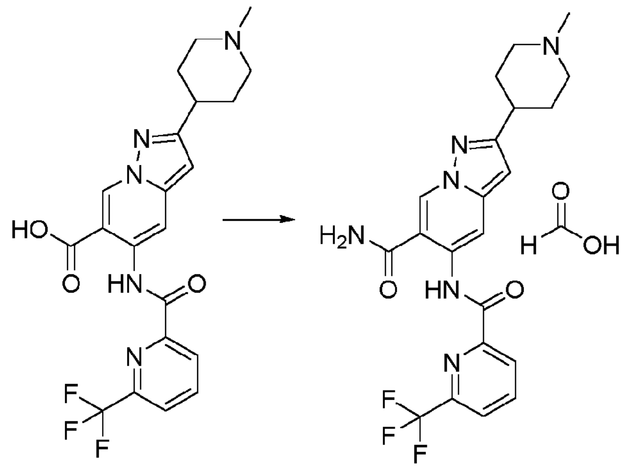

A mixture of 2-(1-methyl-4-piperidyl)-5-[[6-(trifluoromethyl)pyridine-2-carbonyl]amino] pyrazolo[1,5-a]pyridine-6-carboxylic acid (35 mg, 0.078 mmol, 1.0 eq.) and HATU, CAS#148893-10-1 (36 mg, 0.094 mmol, 1.2 eq.) in DMF (2 mL) was stirred at r.t. for 10 min. DIPEA (0.030 mL, 0.16 mmol, 2.0 eq.) and $NH_4Cl$ (13 mg, 0.24 mmol, 3.0 eq.) were added and the resulting mixture was stirred at r.t. for 3 h. DMF (3 mL) and DIPEA (2.0 eq.) were added and the mixture was stirred at 50 °C for 16 h. Additional HATU, CAS#148893-10-1 (0.3 eq.) was added and the mixture was stirred at 80 °C for 3 h. Aq. ammonia (2 mL) was added and the mixture was stirred for 16 h. The mixture was diluted (EtOAc), washed (saturated aqueous $NH_4Cl$, saturated $NaHCO_3$ and brine), dried ($Na_2SO_4$) and concentrated. The residue was purified by flash column chromatography ($SiO_2$, 100:0 to 20:80 DCM/ ternary mixture constituted by 90:15:1.5 DCM/MeOH/ $NH_4OH$). The material so obtained was further purified by preparative HPLC to afford the desired product as formic acid salt.

*2.8. Alternative synthesis of compound 4: 2-(3-hydroxy-3-methylbutyl)-5-[[6-(trifluoromethyl) pyridine-2-carbonyl]amino]pyrazolo[1,5-a]pyridine-6-carboxamide*

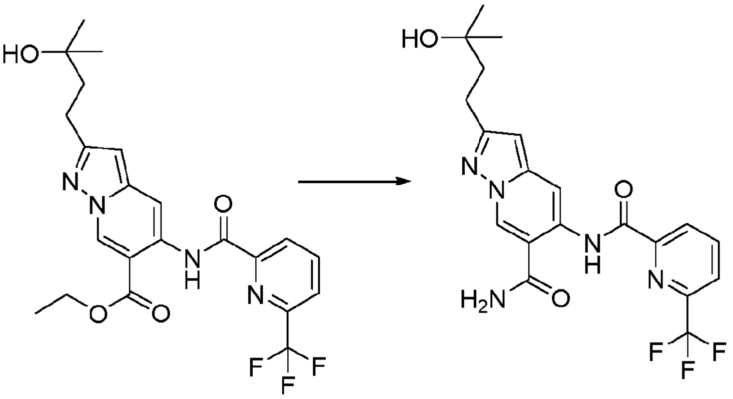

Ethyl 2-(3-hydroxy-3-methyl-butyl)-5-[[6-(trifluoromethyl)pyridine-2-carbonyl]amino]pyrazolo [1,5-a]pyridine-6-carboxylate (50 mg, 0.108 mmol, 1.0 eq.) was dissolved in 7 N $NH_3$ in MeOH (2 mL) and stirred at 60 °C for 16 h. Additional 7 N $NH_3$ in MeOH (1 mL) was added and the mixture was stirred at 50 °C for 4 h. The mixture was concentrated and the residue was purified by flash column chromatography ($SiO_2$, 100:0 to 0: 100 DCM/ ternary mixture constituted by 90:4:1 DCM/MeOH/ $NH_4OH$) to afford the desired product.

*2.9. Synthesis of compound 22: 1-cyclopropyl-N-[2-(2-fluoro-3-hydroxy-3-methyl-butyl)-6-methoxy-pyrazolo[1,5-ajpyridin-5-yl]2-oxo-pyridine-3-carboxamide*

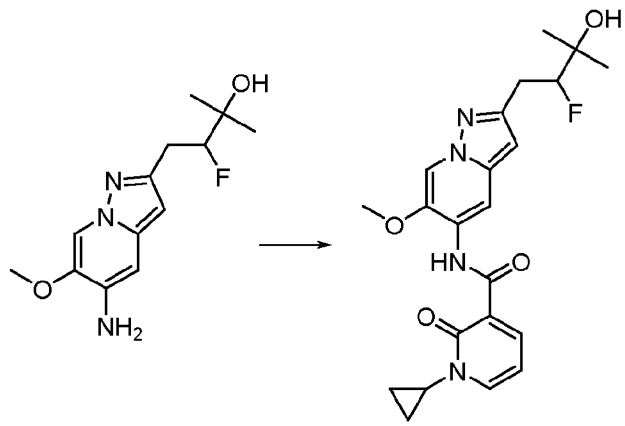

4-(5-amino-6-methoxy-pyrazolo[1,5-a]pyridin-2-yl)-3-fluoro-2-methyl-butan-2-ol (220 mg, 0.8 mmol, 1.0 eq.), 1-cyclopropyl-2-oxo-1,2-dihydropyridine-3-carboxylic acid (190 mg, 1.0 mmol, 1.25 eq.) were suspended in DCM (20 mL). HATU (415 mg, 1 mmol, 1.3 eq.) followed by DIPEA (430 μL, 2.47 mmol, 3.0 eq.) were added and the mixture was stirred at r.t. for 2 h. The solution was diluted with DCM and successively washed with a saturated aq. solution of $NH_4Cl$ and a saturated aq. solution of $NaHCO_3$. The organic phase was dried over sodium sulfate, filtered and concentrated under reduced pressure. The crude sample was purified by flash column chromatography eluting with DCM / MeOH from 100:0 to 94:6.

*2.10. Synthesis of compound 6: 1-cyclopropyl-N-[6-methoxy-2-(2-methylsulfonylethyl)pyrazolo[1,5-a]pyridin-5-yl]-2-oxo-pyridine-3-carboxamide*

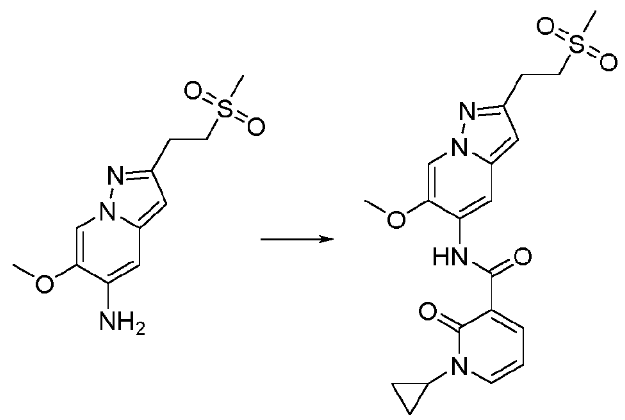

HATU, CAS#148893-10-1 (1.6 g, 4.1 mmol, 1.1 eq.) was added in portions to a mixture of 6-methoxy-2-(2-methylsulfonylethyl)pyrazolo[1,5-a]pyridin-5-amine (991 mg, 3.68 mmol, 1.0 eq.) and 1-cyclopropyl-2-oxo-1,2-dihydropyridine-3-carboxylic acid (750 mg, 4.06 mmol, 1.1 eq.) in DCM (100 mL) at 0°C. DIPEA (1.3 mL, 7.5 mmol, 2.0 eq.) was added and the reaction mixture was stirred at r.t. for 16 h. The reaction mixture was diluted with DCM (150 mL) and washed with a saturated aq. solution of $NaHCO_3$, followed by a saturated aq. solution of NaCl. The organic phase was dried over sodium sulfate, filtered and concentrated under reduced pressure. The crude residue was purified by flash column chromatography eluting with DCM / MeOH from 100:0 to 95:5 to afford the desired product.

*2.11. Synthesis of compounds 25 and 26: chiral separation of 1-cyclopropyl-N-[2-(2-fluoro-3-hydroxy-3-methyl-butyl)-6-methoxy-pyrazolo[1,5-a]pyridin-5-yl]-2-oxo-pyridine-3-carboxamide*

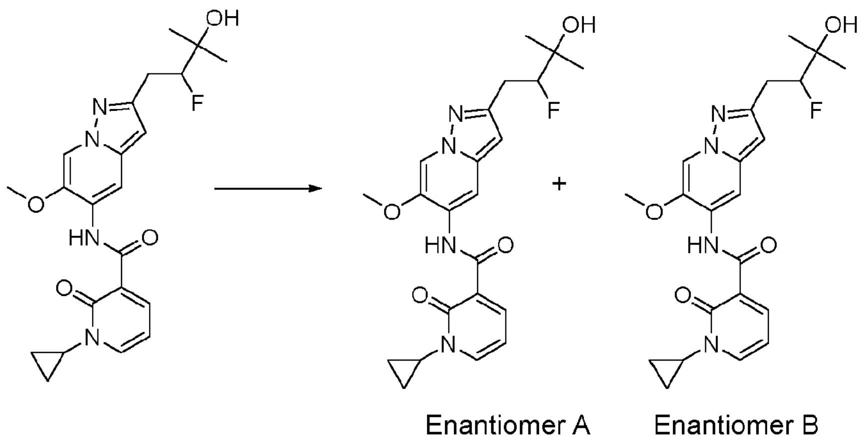

Enantiomer A Enantiomer B

129 mg of 1-cyclopropyl-N-[2-(2-fluoro-3-hydroxy-3-methyl-butyl)-6-methoxy-pyrazolo[1,5-a]pyridin-5-yl]-2-oxo-pyridine-3-carboxamide undergo a chiral separation (column: Chiralpak IA, 150 mm x 4.6 mm, 5 µm; column temperature: 40 °C; flow rate: 2 mL/min; eluent: 50:50 isopropanol/$CO_2$) to afford Enantiomer A (first eluting) and Enantiomer B (second eluting) of 1-cyclopropyl-N-[2-(2-fluoro-3-hydroxy-3-methyl-butyl)-6-methoxy-pyrazolo[1,5-a]pyridin-5-yl]-2-oxo-pyridine-3-carboxamide.

***2.12. Synthesis of compound 24: 1-cyclopropyl-N-[2-(3-hydroxy-3-methylbutyl)-6-(trideuteriomethoxy)pyrazolo[1,5-a]pyridin-5-yl]-2-oxopyridine-3-carboxamide***

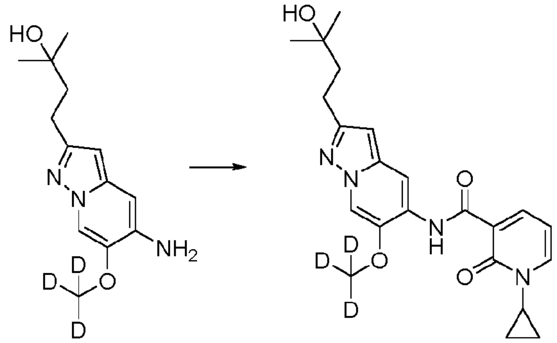

A mixture of 1-cyclopropyl-2-oxo-pyridine-3-carboxylic acid (0.441 g, 2.46 mmol, 0.83 eq.) and DIPEA (450 uL) in DCM (6 mL) was stirred at r.t. for 30 min. 4-[5-amino-6-(trideuteriomethoxy) pyrazolo[1,5-a]pyridin-2-yl]-2-methyl-butan-2-ol (518 mg, 2.05 mmol, 1.0 eq.), HATU, CAS#148893-10-1 (1.01 g, 2.67 mmol, 1.3 eq.), DIPEA (0.61mL, 3.58 mmol, 1.75 eq.) and DCM (6 mL) were added and the resulting mixture was stirred at r.t. for 18 h. The mixture was quenched by addition of saturated aq. $NaHCO_3$. The aq. layer was extracted with DCM. Combined organic layers were washed with saturated aq. $NH_4Cl$ and concentrated. The residue was purified by flash column chromatography (SiOz, 100:0 to 96:4 DCM/MeOH). The fractions containing the desired product were combined and concentrated. The residue was taken up in MeCN. The mixture was stirred at reflux for 30 min. The mixture was cooled to r.t. and then with an ice bath. The mixture was stirred for 15 min and filtered. The solid obtained was further washed with a small amount of MeCN to obtain the desired product.

***2.13. Synthesis of compound 23: 1-cyclopropyl-N-[6-methoxy-2-[4,4,4-trideuterio-3-hydroxy-3-(trideuteriomethyl)butyl]pyrazolo[1,5-a]pyridin-5-yl]-2-oxo-pyridine-3-carboxamide***

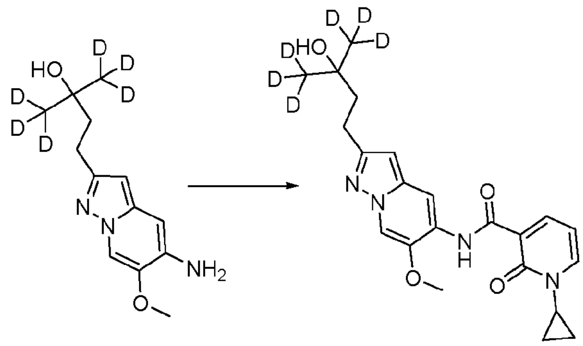

A mixture of 1-cyclopropyl-2-oxo-pyridine-3-carboxylic acid (1.27 g, 7.08 mmol, 1.3 eq.) and DIPEA (1.2 mL, 7.07 mmol, 1.3 eq.) in DCM (16 mL) was stirred at r.t. for 30 min. 4-(5-amino-6-methoxypyrazolo[1,5-a]pyridin-2-yl)-1,1,1-trideuterio-2-(trideuteriomethyl)butan-2-ol (1.42 g, 5.45 mmol, 1.0 eq.), HATU, CAS#148893-10-1 (2.69 g, 7.08 mmol, 1.3 eq.), DIPEA (1.6 mL, 9.4 mmol, 1.7 eq.) and DCM (16 mL) were added and the resulting mixture was stirred at r.t. overnight. The mixture was quenched by addition of saturated aq. $NaHCO_3$. The aq. layer was extracted with DCM. Combined organic layers were washed with saturated aq. $NH_4Cl$ and concentrated. The residue was purified by flash column chromatography ($SiO_2$, 100:0 to 90:10 DCM/MeOH). The fractions containing the desired product were combined and concentrated The residue ws taken up in MeCN. The mixture was stirred at reflux for 30 min. The mixture was cooled to r.t. and then with an ice bath. The mixture was stirred for 1 h and filtered. The solid obtained was further washed with a small amount of MeCN to obtain the desired product.

**Table III. Illustrative compounds of the invention**

| Cpd | Structure | Name | Name starting material | Mtd | MW (calc) | MW (obs) |
|---|---|---|---|---|---|---|
| 1 | 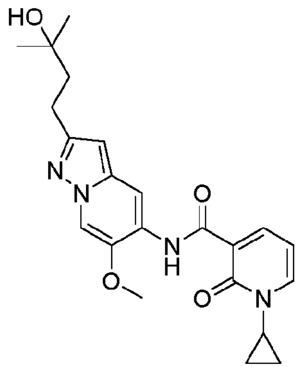 | 1-cyclopropyl-N-[2-(3- hydroxy-3-methylbutyl)-6-methoxypyrazolo[1,5-a]pyridin-5-yl]-2-oxopyridine-3-carboxamide | 4-(5-amino-6-methoxypyrazolo[1,5-a]pyridin-2-yl)-2-methyl-butan-2-ol | A | 410.5 | 411.1 |
| 2 | 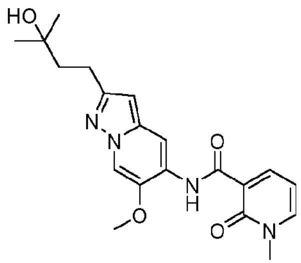 | N-[2-(3-hydroxy-3-methylbutyl)-6-methoxypyrazolo [1,5-a]pyridin-5-yl]-1-methyl-2-oxopyridine-3-carboxamide | 4-(5-amino-6-methoxypyrazolo[1,5-a]pyridin-2-yl)-2-methyl-butan-2-ol | A | 384.4 | 385.1 |
| 3 | 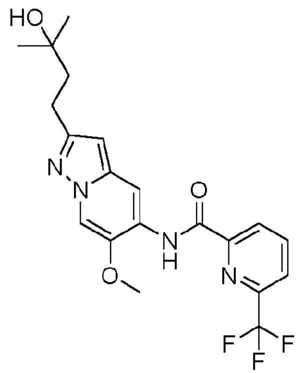 | N-[2-(3-hydroxy-3-methylbutyl)-6-methoxypyrazolo [1,5-a]pyridin-5-yl]-6-(trifluoromethyl)pyridin e-2-carboxamide | 4-(5-amino-6-methoxypyrazolo[1,5-a]pyridin-2-yl)-2-methyl-butan-2-ol | A | 422.4 | 423.1 |

(continued)

| Cpd | Structure | Name | Name starting material | Mtd | MW (calc) | MW (obs) |
|---|---|---|---|---|---|---|
| 4 | 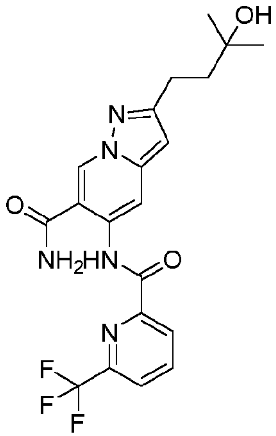 | 2-(3-hydroxy-3-methylbutyl)-5-[[6-(trifluoromethyl) pyridin e-2-carbonyl]amino]pyrazol o[1,5-a]pyridine-6-carboxamide | ethyl 2-(3-hydroxy-3-methyl-butyl)-5-[[6-(trifluoromethyl)pyr idine-2-carbonyl] amino]pyr azolo[1,5-a]pyridine-6-carboxylate | NA | 435.4 | 436.1 |
| 5 | 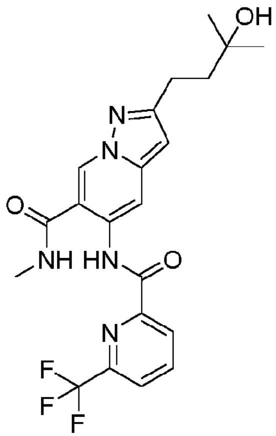 | 2-(3-hydroxy-3-methylbutyl)-N-methyl-5-[[6-(trifluoromethyl)pyridin e-2-carbonyl]amino] pyrazol o[1,5-a]pyridine-6-carboxamide | ethyl 2-(3-hydroxy-3-methyl-butyl)-5-[[6-(trifluoromethyl)pyr idine-2-carbonyl] amino]pyr azolo[1,5-a]pyridine-6-carboxylate | NA | 449.4 | 450.1 |

(continued)

| Cpd | Structure | Name | Name starting material | Mtd | MW (calc) | MW (obs) |
|---|---|---|---|---|---|---|
| 6 | 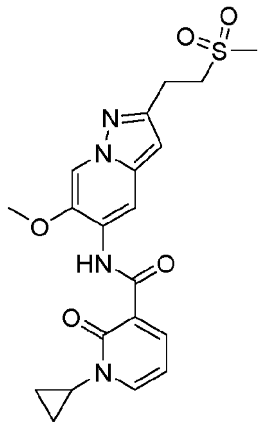 | 1-cyclopropyl-N-[6-methoxy-2-(2-methylsulfonylethyl)pyr azolo[1,5-a]pyridin-5-yl]-2-oxo-pyridine-3-carboxamide | 6-methoxy-2-(2-methylsulfonylethyl ) pyrazolo[1,5-a]pyridin-5-amine | NA | 430.5 | 431.1 |
| 7 | 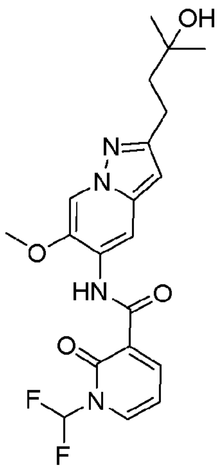 | 1-(difluoromethyl)-N-[2-(3-hydroxy-3-methylbutyl)-6-methoxypyrazolo[1,5-a]pyridin-5-yl]-2-oxo-pyridine-3- carboxamide | 4-(5-amino-6-methoxypyrazolo[1,5-a]pyridin-2-yl)-2-methyl-butan-2-ol | A | 420.4 | 421.6 |

(continued)

| Cpd | Structure | Name | Name starting material | Mtd | MW (calc) | MW (obs) |
|---|---|---|---|---|---|---|
| 8 | 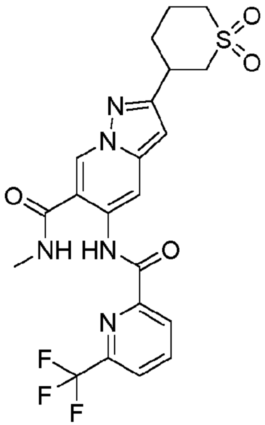 | 2-(1,1-dioxothian-3-yl)-N-methyl-5-[[6-(trifluoromethyl)pyridin e-2-carbonyl]amino] pyrazol o[1,5-a]pyridine-6-carboxamide | ethyl 2-(1,1-dioxothian-3-yl)-5-[[6-(trifluoromethyl)pyr idine-2-carbonyl] amino]pyr azolo[1,5-a]pyridine-6-carboxylate | L | 495.5 | 496.5 |
| 9 | 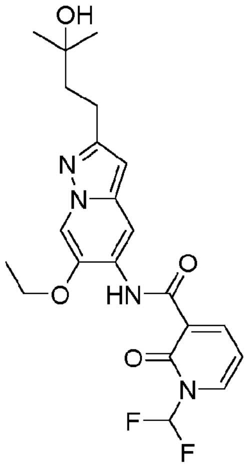 | 1-(difluoromethyl)-N-[6-ethoxy-2-(3-hydroxy-3-methylbutyl)pyrazolo[1,5-a]pyridin-5-yl]-2-oxo-pyridine-3-carboxamide | 4-(5-amino-6-ethoxy-pyrazolo[1,5-a]pyridin-2-yl)-2-methyl-butan-2-ol | A | 434.4 | 435.6 |

(continued)

| Cpd | Structure | Name | Name starting material | Mtd | MW (calc) | MW (obs) |
|---|---|---|---|---|---|---|
| 10 | 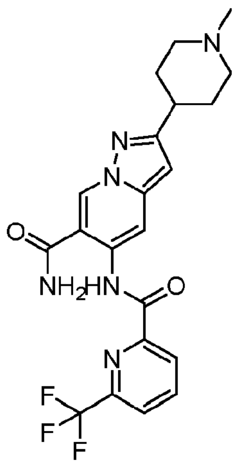 | 2-(1-methyl-4-piperidyl)-5-[[6-(trifluoromethyl) pyridin e-2-carbonyl]amino]pyrazol o[1,5-a]pyridine-6-carboxamide formic acid salt | 2-(1-methyl-4-piperidyl)-5-[[6-(trifluoromethyl)pyr idine-2-carbonyl] amino]pyr azolo[1,5-a]pyridine-6-carboxylic acid | NA | 446.4 | 447.6 |
| 11 | 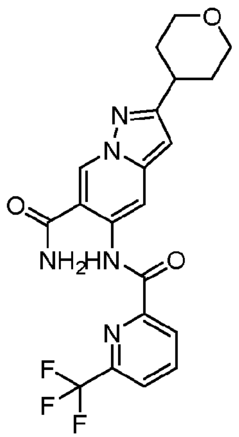 | 2-tetrahydropyran-4-yl-5-[[6-(trifluoromethyl)pyridin e-2-carbonyl]amino]pyrazol o[1,5-a]pyridine-6-carboxamide | 2-tetrahydropyran-4-yl-5-[[6-(trifluoromethyl) pyr idine-2-carbonyl]amino]pyr azolo[1,5-a] pyridine-6-carboxylic acid | M | 433.4 | 434.6 |

(continued)

| Cpd | Structure | Name | Name starting material | Mtd | MW (calc) | MW (obs) |
|---|---|---|---|---|---|---|
| 12 | 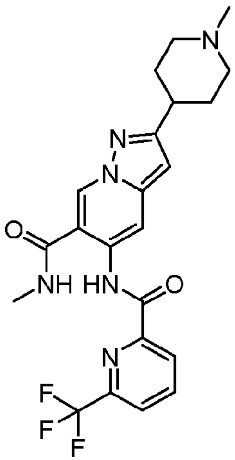 | N-methyl-2-(1-methyl-4-piperidyl)-5-[[6-(trifluoromethyl)pyridin e-2-carbonyl]amino] pyrazol o[1,5-a]pyridine-6-carboxamide | ethyl 2-(1-methyl-4-piperidyl)-5-[[6-(trifluoromethyl)pyr idine-2-carbonyl] amino]pyr azolo[1,5-a]pyridine-6-carboxylate | NA | 460.5 | 461.5 |
| 13 | 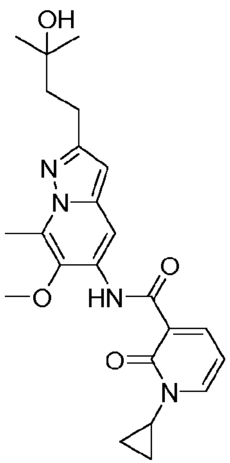 | 1-cyclopropyl-N-[2-(3-hydroxy-3-methylbutyl)-6-methoxy-7-methyl-pyrazolo[1,5-a]pyridin-5-yl]-2-oxo-pyridine-3-carboxamide | 4-(5-amino-6-methoxy-7-methyl-pyrazolo [1,5-a]pyridin-2-yl)-2-methyl-butan-2-ol | A | 424.5 | 425.7 |

(continued)

| Cpd | Structure | Name | Name starting material | Mtd | MW (calc) | MW (obs) |
|---|---|---|---|---|---|---|
| 14 | 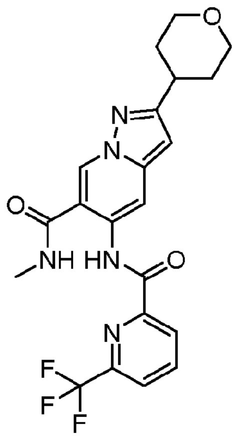 | N-methyl-2-tetrahydropyran-4-yl-5-[[6-(trifluoromethyl)pyridin e-2-carbonyl]amino] pyrazol o[1,5-a]pyridine-6-carboxamide | ethyl 2-tetrahydropyran-4-yl-5-[[6-(trifluoromethyl)pyr idine-2-carbonyl] amino]pyr azolo[1,5-a]pyridine-6-carboxylate | L | 447.4 | 448.6 |
| 15 | 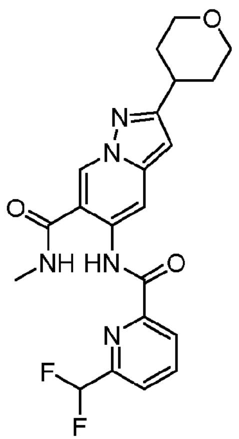 | 5-[[6-(difluoromethyl)pyridine -2-carbonyl]amino]-N-methyl-2-tetrahydropyran-4-yl-pyrazolo[1,5-a] pyridine-6-carboxamide | ethyl 5-[[6-(difluoromethyl)pyri dine-2-carbonyl]amino]-2-tetrahydropyran-4-yl-pyrazolo[1,5-a]pyridine-6-carboxylate | L | 429.4 | 430.6 |

(continued)

| Cpd | Structure | Name | Name starting material | Mtd | MW (calc) | MW (obs) |
|---|---|---|---|---|---|---|
| 16 | 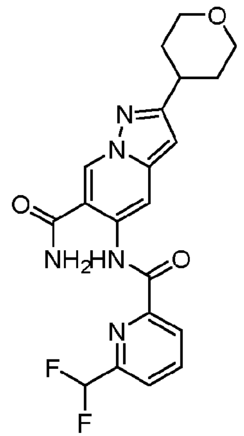 | 5-[[6-(difluoromethyl)pyridine -2-carbonyl]amino]-2-tetrahydropyran-4-yl-pyrazolo[1,5-a]pyridine-6-carboxamide | 5-[[6-(difluoromethyl)pyri dine-2-carbonyl] amino]-2-tetrahydropyran-4-yl-pyrazolo[1,5-a]pyridine-6-carboxylic acid | M | 415.4 | 416.6 |
| 17 | 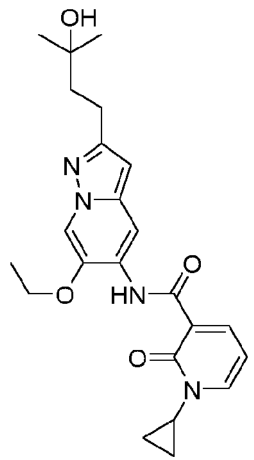 | 1-cyclopropyl-N-[6-ethoxy-2-(3-hydroxy-3-methylbutyl)pyrazolo[1,5-a]pyridin-5-yl]-2-oxo-pyridine-3-carboxamide | 4-(5-amino-6-ethoxy-pyrazolo[1,5-a]pyridin-2-yl)-2-methyl-butan-2-ol | A | 424.5 | 425.6 |

(continued)

| Cpd | Structure | Name | Name starting material | Mtd | MW (calc) | MW (obs) |
|---|---|---|---|---|---|---|
| 18 | 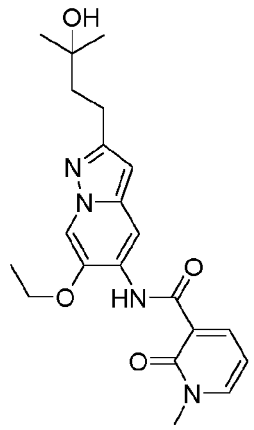 | N-[6-ethoxy-2-(3-hydroxy-3-methylbutyl)pyrazolo [1,5-a]pyridin-5-yl]-1-methyl-2-oxo-pyridine-3-carboxamide | 4-(5-amino-6-ethoxy-pyrazolo[1,5-a]pyridin-2-yl)-2-methyl-butan-2-ol | A | 398.5 | 399.6 |
| 19 | 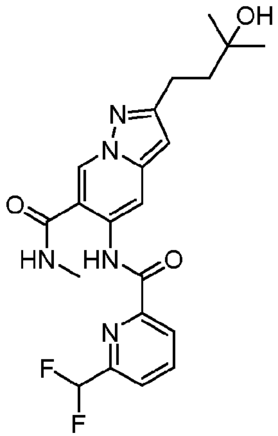 | 5-[[6-(difluoromethyl)pyridine -2-carbonyl] amino]-2-(3-hydroxy-3-methylbutyl)-N-methyl-pyrazolo[1,5-a]pyridine-6-carboxamide | ethyl 5-[[6-(difluoromethyl)pyri dine-2-carbonyl]amino]-2-(3-hydroxy-3-methylbutyl) pyrazolo[1,5-a]pyridine-6-carboxylate | L | 431.4 | 432.6 |

(continued)

| Cpd | Structure | Name | Name starting material | Mtd | MW (calc) | MW (obs) |
|---|---|---|---|---|---|---|
| 20 | 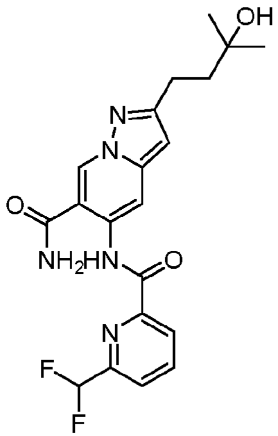 | 5-[[6-(difluoromethyl)pyridine -2-carbonyl]amino]-2-(3-hydroxy-3-methylbutyl)pyrazolo[1,5-a]pyridine-6-carboxamide | 5-[[6-(difluoromethyl)pyri dine-2-carbonyl] amino]-2-(3-hydroxy-3-methylbutyl)pyrazolo [1,5-a]pyridine-6-carboxylic acid | M | 417.4 | 418.6 |
| 21 | 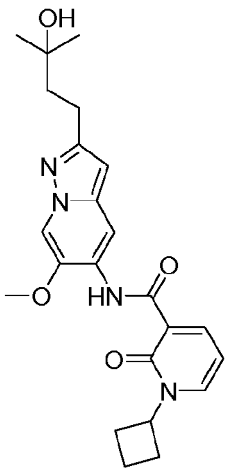 | 1-cyclobutyl-N-[2-(3-hydroxy-3-methylbutyl)-6-methoxypyrazolo[1,5-a]pyridin-5-yl]-2-oxo-pyridine-3-carboxamide | 4-(5-amino-6-methoxypyrazolo[1,5-a]pyridin-2-yl)-2-methyl-butan-2-ol | A | 424.5 | 425.6 |

(continued)

| Cpd | Structure | Name | Name starting material | Mtd | MW (calc) | MW (obs) |
|---|---|---|---|---|---|---|
| 22 | 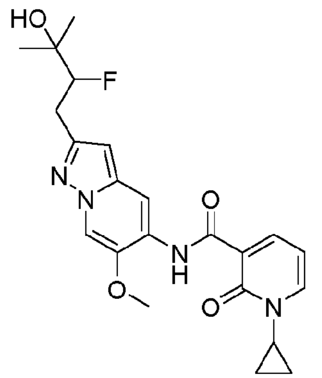 | 1-cyclopropyl-N-[2-(2-fluoro-3-hydroxy-3-methyl-butyl)-6-methoxy-pyrazolo[1,5-a]pyridin-5-yl]-2-oxo-pyridine-3-carboxamide | 4-(5-amino-6-methoxypyrazolo[1,5-a]pyridin-2-yl)-3-fluoro-2-methyl-butan-2-ol | NA | 428.5 | 429.4 |
| 23 | 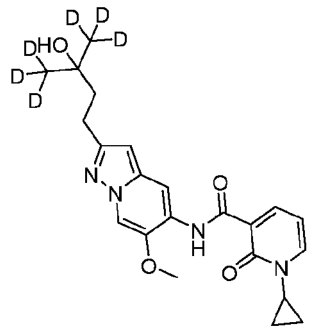 | 1-cyclopropyl-N-[6-methoxy-2-[4,4,4-trideuterio-3-hydroxy-3-(trideuteriomethyl)butyl] pyrazolo[1,5-a] pyridin-5-yl]-2-oxo-pyridine-3-carboxamide | 4-(5-amino-6-methoxypyrazolo[1,5-a]pyridin-2-yl)-1,1,1-trideuterio-2-(trideuteriomethyl)b utan-2-ol | NA | 416.5 | 417.3 |

(continued)

| Cpd | Structure | Name | Name starting material | Mtd | MW (calc) | MW (obs) |
|---|---|---|---|---|---|---|
| 24 | 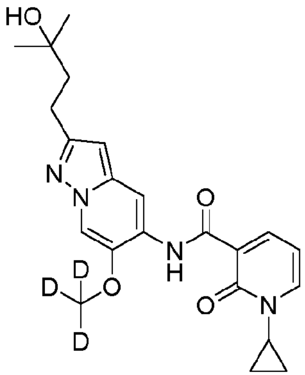 | 1-cyclopropyl-N-[2-(3-hydroxy-3-methylbutyl)-6-(trideuteriomethoxy)pyr azolo[1,5-a]pyridin-5-yl]-2-oxopyridine-3-carboxamide | 4-[5-amino-6-(trideuteriomethoxy )pyrazolo [1,5-a]pyridin-2-yl]-2-methyl-butan-2-ol | NA | 413.5 | 414.3 |
| 25 | 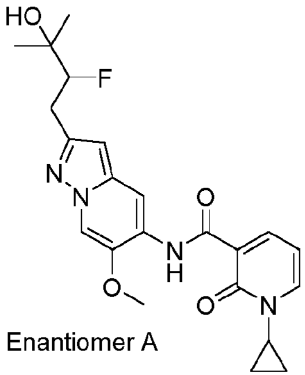 Enantiomer A | 1-cyclopropyl-N-[2-(2-fluoro-3-hydroxy-3-methyl-butyl)-6-methoxy-pyrazolo[1,5-a]pyridin-5-yl]-2-oxo-pyridine-3-carboxamide Enantiomer A | 1-cyclopropyl-N-[2-(2-fluoro-3-hydroxy-3-methylbutyl)-6-methoxypyrazolo[1,5-a] pyridin-5-yl]-2-oxo-pyridine-3-carboxamide | NA | 428.5 | 429.1 |

(continued)

| Cpd | Structure | Name | Name starting material | Mtd | MW (calc) | MW (obs) |
|---|---|---|---|---|---|---|
| 26 | 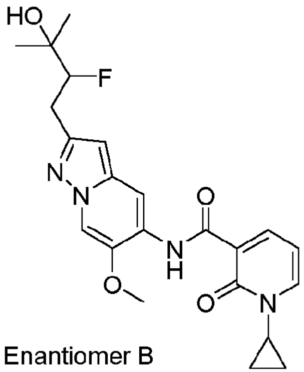 Enantiomer B | 1-cyclopropyl-N-[2-(2-fluoro-3-hydroxy-3-methyl-butyl)-6-methoxy-pyrazolo[1,5-a]pyridin-5-yl]-2-oxo-pyridine-3-carboxamide Enantiomer B | 1-cyclopropyl-N-[2-(2-fluoro-3-hydroxy-3-methylbutyl)-6-methoxypyrazolo[1,5-a] pyridin-5-yl]-2-oxo-pyridine-3-carboxamide | NA | 428.5 | 429.1 |

Table IV. NMR data of illustrative compounds of the invention

| Cpd. | NMR |
|---|---|
| 1 | $^1$H NMR (300 MHz, DMSO-$d_6$) δ 12.51 (s, 1H), 8.57 (s, 1H), 8.45 (m, 1H), 8.38 (s, 1H), 8.03 (m, 1H), 6.56 (m, 1H), 6.25 (s, 1H), 4.26 (s, 1H), 3.94 (s, 3H), 3.59 - 3.48 (m, 1H), 2.78 - 2.66 (m, 2H), 1.82 - 1.70 (m, 2H), 1.15 (s, 6H), 1.09 (m, 2H), 0.98 (m, 2H). |
| 2 | $^1$H NMR (300 MHz, DMSO-$d_6$) δ 12.54 (s, 1H), 8.56 (s, 1H), 8.46 (m, 1H), 8.37 (d, 1H), 8.17 (m, 1H), 6.60 (m, 1H), 6.24 (d, 1H), 4.26 (s, 1H), 3.93 (s, 3H), 3.63 (s, 3H), 2.78 - 2.66 (m, 2H), 1.82 - 1.70 (m, 2H), 1.15 (s, 6H). |
| 3 | $^1$H NMR (400 MHz, DMSO-$d_6$) δ 10.42 (s, 1H), 8.50 - 8.38 (m, 4H), 8.25 (m, 1H), 6.33 (s, 1H), 4.27 (s, 1H), 3.97 (s, 3H), 2.78 - 2.69 (m, 2H), 1.81 - 1.72 (m, 2H), 1.15 (s, 6H). |
| 4 | $^1$H NMR (300 MHz, DMSO-$d_6$) δ 13.45 (s, 1H), 9.22 (s, 1H), 8.83 (s, 1H), 8.49 - 8.40 (m, 1H), 8.38 (t, 1H), 8.33 (s, 1H), 8.19 (m, 1H), 7.97 (s, 1H), 6.46 (s, 1H), 4.31 (s, 1H), 2.85 - 2.74 (m, 2H), 1.85 - 1.73 (m, 2H), 1.16 (s, 6H). |
| 5 | $^1$H NMR (300 MHz, DMSO-$d_6$) δ 12.97 (s, 1H), 9.05 (s, 1H), 8.88 - 8.79 (m, 1H), 8.76 (s, 1H), 8.44 (m, 1H), 8.38 (t, 1H), 820 (m, 1H), 6.47 (s, 1H), 4.31 (s, 1H), 2.87 - 2.74 (m, 5H), 1.85 - 1.73 (m, 2H), 1.16 (s, 6H). |
| 6 | $^1$H NMR (400 MHz, DMSO-$d_6$) δ 12.54 (s, 1H), 8.60 (s, 1H), 8.48 - 8.40 (m, 2H), 8.03 (m, 1H), 6.56 (m, 1H), 6.40 (s, 1H), 3.96 (s, 3H), 3.59 - 3.46 (m, 3H), 3.18 - 3.09 (m, 2H), 3.01 (s, 3H), 1.16 - 0.93 (m, 4H). |
| 7 | $^1$H NMR (500 MHz, DMSO-$d_6$) δ 11.86 (s, 1H), 8.59 (m, 1H), 8.54 (s, 1H), 8.40 (s, 1H), 8.26 (m, 1H), 8.05 (t, 1H), 6.79 (t, 1H), 6.27 (s, 1H), 4.25 (s, 1H), 3.93 (s, 3H), 2.75 - 2.68 (m, 2H), 1.79 - 1.72 (m, 2H), 1.14 (s, 5H). |
| 8 | $^1$H NMR (300 MHz, DMSO-$d_6$) δ 12.96 (s, 1H), 9.09 (s, 1H), 8.90 - 8.81 (m, 1H), 8.79 (s, 1H), 8.47 - 8.31 (m, 2H), 8.19 (m, 1H), 6.60 (s, 1H), 3.49 - 3.36 (m, 2H), 3.24 - 3.05 (m, 2H), 2.81 (d, 3H), 2.13 (t, 2H), 1.97 (q, 1H), 1.74 (d, 1H). |
| 9 | $^1$H NMR (300 MHz, DMSO-$d_6$) δ 11.93 (s, 1H), 8.58 (m, 1H), 8.52 (s, 1H), 8.38 (s, 1H), 8.29 - 8.16 (m, 1H), 7.90 (t, 1H), 6.77 (t, 1H), 6.25 (s, 1H), 4.25 (s, 1H), 4.15 (q, 2H), 2.76 - 2.64 (m, 2H), 1.80 - 1.68 (m, 2H), 1.45 (t, 3H), 1.13 (s, 6H). |
| 10 | $^1$H NMR (500 MHz, DMSO-$d_6$) δ 13.45 (s, 1H), 9.26 (s, 1H), 8.84 (s, 1H), 8.44 (d, 1H), 8.38 (t, 1H), 8.31 (s, 1H), 8.22 (s, 1H), 8.19 (m, 1H), 7.96 (s, 1H), 6 52 (s, 1H), 2.87 (d, 2H), 2.74 (m, 1H), 2.22 (s, 3H), 2.07 (m, 2H), 1.99 - 1.93 (m, 2H), 1.75 (m, 2H). |
| 11 | $^1$H NMR (500 MHz, DMSO-$d_6$) δ 13.45 (s, 1H), 9.26 (s, 1H), 8.85 (s, 1H), 8.44 (d, 1H), 8.38 (t, 1H), 8.31 (s, 1H), 8.19 (d, 1H), 7.97 (s, 1H), 6.54 (s, 1H), 3.97 - 3.90 (m, 2H), 3.48 (m, 2H), 3.11 - 2.99 (m, 1H), 1.95 - 1.88 (m, 2H), 1.75 (m, 2H). |
| 12 | $^1$H NMR (500 MHz, DMSO-$d_6$) δ 12.97 (s, 1H), 9.09 (s, 1H), 8.81 (d, 1H), 8.77 (s, 1H), 8.43 (d, 1H), 8 38 (t, 1H), 8.20 (d, 1H), 6.51 (s, 1H), 2.86 - 2.80 (m, 5H), 2.72 (m, 1H), 2.19 (s, 3H), 2.06 - 1.90 (m, 4H), 1.73 (m, 2H). |
| 13 | $^1$H NMR (500 MHz, DMSO-$d_6$) δ 12.61 (s, 1H), 8.50 - 8.43 (m, 2H), 8.03 (m, 1H), 6.57 (t, 1H), 6.34 (s, 1H), 4.28 (s, 1H), 3.84 (s, 3H), 3.56 (m, 1H), 2.79 - 2.72 (m, 2H), 2.62 (s, 3H), 1.80 - 1.73 (m, 2H), 1.15 (s, 6H), 1.14 - 1.05 (m, 2H), 1.01 - 0.94 (m, 2H). |
| 14 | $^1$H NMR (300 MHz, DMSO-$d_6$) δ 12.97 (s, 1H), 9.09 (s, 1H), 8.79 (d, 2H), 8.47 - 8.31 (m, 2H), 8.19 (m, 1H), 6.53 (s, 1H), 3.92 (m, 2H), 3.47 (m, 2H), 3.04 (m, 1H), 2.81 (d, 3H), 1.90 (m, 2H), 1.83 - 1.64 (m, 2H). |
| 15 | $^1$H NMR (300 MHz, DMSO-$d_6$) δ 12.93 (s, 1H), 9.09 (s, 1H), 8.95 - 8.64 (m, 2H), 8.37 - 8.22 (m, 2H), 7.99 (m, 1H), 7.01 (t, 1H), 6.52 (s, 1H), 3.98 - 3.86 (m, 2H), 3.47 (m, 2H), 3.04 (m, 1H), 2.83 (d, 3H), 1.95 - 1.84 (m, 2H), 1.84 - 1.64 (m, 2H). |

(continued)

| Cpd. | NMR |
|---|---|
| 16 | $^1$H NMR (300 MHz, DMSO-$d_6$) δ 13.38 (s, 1H), 9.24 (s, 1H), 8.83 (s, 1H), 8.43 - 8.18 (m, 3H), 7.97 (m, 1H), 7.92 (s, 1H), 6.97 (t, 1H), 6.52 (s, 1H), 3.98 - 3.86 (m, 2H), 3.47 (m, 2H), 3.04 (m, 1H), 1.96 - 1.85 (m, 2H), 1.84 - 1.64 (m, 2H). |
| 17 | $^1$H NMR (300 MHz, DMSO-$d_6$) δ 12.58 (s, 1H), 8.53 (s, 1H), 8.42 (m, 1H), 8.36 (s, 1H), 8.00 (m, 1H), 6.53 (t, 1H), 6.23 (s, 1H), 4.24 (s, 1H), 4.15 (q, 2H), 3.53 (m, 1H), 2.76 - 2.64 (m, 2H), 1.80 - 1.68 (m, 2H), 1.47 (t, 3H), 1.13 (s, 6H), 1.11 - 0.91 (m, 4H). |
| 18 | $^1$H NMR (300 MHz, DMSO-$d_6$) δ 12.58 (s, 1H), 8.54 (s, 1H), 8.44 (m, 1H), 8.35 (s, 1H), 8.15 (m, 1H), 6.57 (m, 1H), 6.22 (s, 1H), 4.24 (s, 1H), 4.14 (q, 2H), 3.61 (s, 3H), 2.76 - 2.64 (m, 2H), 1.80 - 1.68 (m, 2H), 1.46 (t, 3H), 1.13 (s, 6H). |
| 19 | $^1$H NMR (500 MHz, DMSO-$d_6$) δ 12.92 (s, 1H), 9.04 (s, 1H), 8.82 (s, 1H), 8.75 (s, 1H), 8.35 - 8.26 (m, 2H), 7.99 (d, 1H), 7.02 (t, 1H), 6.45 (s, 1H), 4.30 (s, 1H), 2.84 (d, 3H), 2.82 - 2.75 (m, 2H), 1.82 - 1.75 (m, 2H), 1.15 (s, 6H). |
| 20 | $^1$H NMR (300 MHz, DMSO-$d_6$) δ 13.37 (s, 1H), 9.19 (s, 1H), 8.80 (s, 1H), 8.36 - 8.22 (m, 3H), 7.97 (m, 1H), 7.91 (s, 1H), 6.98 (t, 1H), 6.44 (s, 1H), 4.29 (s, 1H), 2.84 - 2.72 (m, 2H), 1.84 - 1.72 (m, 2H), 1.14 (s, 6H). |
| 21 | $^1$H NMR (500 MHz, DMSO-$d_6$) δ 12.50 (s, 1H), 8.55 (s, 1H), 8.45 (m, 1H), 8.36 (s, 1H), 8.24 (m, 1H), 6.65 (t, 1H), 6.24 (s, 1H), 5.19 - 5.09 (m, 1H), 4.25 (s, 1H), 3.92 (s, 3H), 2.75 - 2.68 (m, 2H), 2.47 - 2.37 (m, 2H), 2.36 - 2.24 (m, 2H), 1.90 - 1.72 (m, 4H), 1.14 (s, 6H). |
| 22 | $^1$H NMR (400 MHz, Chloroform-d) δ 12.40 (s, 1H), 8.71 (s, 1H), 8.61 (m, 1H), 8.02 (s,1H), 7.63 (m, 1H), 6.47 (t, 1H), 6.32 (s, 1H), 4.74 (m, 1H), 4.01 (s, 3H), 3.52 (m, 1H), 3.27 - 3.10 (m, 2H), 2.41 (s, 1H), 1.56 - 1.42 (m, 2H), 1.37 - 120 (m, 6H), 1.02 - 0.93 (m, 2H). |
| 23 | $^1$H NMR (300 MHz, DMSO-$d_6$) δ 12.50 (s, 1H), 8.55 (s, 1H), 8.43 (m, 1H), 8.37 (s, 1H), 8.02 (m, 1H), 6.60 - 6.49 (m, 1H), 6.23 (s, 1H), 4.22 (s, 1H), 3.93 (s, 3H), 3.59 - 3.46 (m, 1H), 2.76 - 2.64 (m, 2H), 1.80 - 1.68 (m, 2H), 1.15 - 0.90 (m, 4H). |
| 24 | $^1$H NMR (500 MHz, DMSO-$d_6$) δ 12.51 (s, 1H), 8.56 (s, 1H), 8.44 (m, 1H), 8.37 (s, 1H), 8.03 (m, 1H), 6.55 (t, 1H), 6.24 (s, 1H), 4.25 (s, 1H), 3.58 - 3.49 (m, 1H), 2.75 - 2.68 (m, 2H), 1.82 - 1.66 (m, 2H), 1.14 (s, 6H), 1.11 - 1.02 (m, 2H), 1.00 - 0.90 (m, 2H). |
| 25 | $^1$H NMR (400 MHz, Chloroform-d) δ 12.31 (s, 1H), 8.62 (s, 1H), 8.52 (m, 1H), 7.93 (s, 1H), 7.53 (m, 1H), 6.38 (t, 1H), 6.23 (s, 1H), 4.69 - 4.48 (m, 1H), 3.92 (s, 3H), 3.42 (m, 1H), 3.17 - 2.99 (m, 2H), 1.25 (m, 6H), 1.23 - 1.10 (m, 3H), 0.93 - 0.85 (m, 2H). |
| 26 | $^1$H NMR (400 MHz, Chloroform-d) δ 12.31 (s, 1H), 8.62 (s, 1H), 8.52 (m, 1H), 7.93 (s, 1H), 7.54 (m, 1H), 6.38 (t, 1H), 6.23 (s, 1H), 4.68 - 4.47 (m, 1H), 3.92 (s, 3H), 3.43 (m, 1H), 3.17 - 3.06 (m, 1H), 3.04 (d, 1H), 1.25 (m, 6H), 1.22 - 1.15 (m, 3H), 0.93 - 0.83 (m, 2H). |

## BIOLOGICAL EXAMPLES

**Example 3. In vitro assays**

***3.1. Phosphorylation $IC_{50}$ determination for human IRAK-4***

***3.1.1. Assay principle***

The phosphorylation of the substrate RIP140 (SEQ ID1) by IRAK4 at Km ATP was detected with the ADP-Glo Kinase Assay (Promega, Cat# V9103), a luminescent kinase assay which measures the ADP formed from a kinase reaction. (Zegzouti et al., 2009) In a second step the kinase reaction is terminated and all the remaining ATP was depleted. In a final step the ADP was converted into ATP and this newly synthesized ATP was measured by using a luciferase/luciferin reaction with a luminescent reader. The luminescent signal positively correlated with kinase activity,

in particular kinase inhibition giving a decrease of the luminescent signal.

### *3.1.2. Material*

For the semi-automated assay, the positive control (100% inhibition) was prepared by diluting 10 mM staurosporine stock mixture (20 μL) in water (3.8 mL) and DMSO (180 μL), thus resulting in a 10 μM staurosporine solution at 1% DMSO (final concentration after further dilution in the kinase reaction).

For the automated assay, no pre-dilution of the 10 mM staurosporine stock was made. 10 mM staurosporine was spotted directly in the assay plate using acoustic dispensing and complemented with DMSO to result in a similar final concentration as mentioned above.

For the semi-automated assay, the negative control (0% inhibition) was prepared by mixing water (3.8 mL) and DMSO (200 μL) resulting in a final concentration of 1% DMSO after further dilution in the kinase reaction. For the automated assay no pre-dilution of DMSO was done. 100% DMSO was spotted directly on the assay plate.

The assay buffer solution was prepared at a concentration corresponding to 5 fold the final (most diluted) assay concentration by mixing a solution of 125 mM TRIS pH 7.5 + 0.05% Triton X-100 + 2.5 mM EGTA (5.27 mL) with 1M $MgCl_2$ (72 μL), 1M DTT (57.6 μL), and 200 mM $MnCl_2$ (360 μL).

The enzyme-substrate mixture (aqueous buffer solution of 25 μM RIP140 and 0.125 ng/ μL IRAK4) was prepared at a concentration corresponding to 2.5 fold the final (most diluted) assay concentration of the semi-automated method and 3 fold the final (most diluted) assay concentration of the automated method. For example, for the semi-automated method, the enzyme-substrate mixture was prepared by mixing water (3999 μL), assay buffer solution (1380 μL), 1 mM RIP140 (138 μL - SEQ ID1), and 200 ng/mL IRAK 4 (3.45 μL Cama Biosciences, 09 145).

For semi-automated method, the ATP mixture was prepared at a concentration corresponding to 2.5 fold the final (most diluted) assay concentration of the semi-automated method and 1.5 fold the final (most diluted) assay concentration of the automated method. For example, for the semi-automated method, the ATP mixture was prepared by mixing water (4126 μL), assay buffer solution (1380 μL), and 10 mM ATP (13.80 μL).

### *3.1.3. Method*

The assay was performed either in a semi-automated or fully automated manner. The assay volume and the incubation time of the ADP detection were different accordingly the method used

#### *3.1.3.1. Semi-automated assay*

The compounds were prepared as a serial dilution of 10 point dose responses with 1/5 dilution steps in 100% DMSO starting from 2 mM highest concentration, diluted 1/20 in water. 1 μL was transferred dry to the assay plates.

On each assay plate 32 wells of each control (positive & negative) were added, followed by 2 μL enzyme-substrate mixture.

The reaction was started by adding 2 μL diluted ATP (final concentration Km ATP) on the assay plates. Plates were centrifuged for a few seconds at 1000 rpm followed by an incubation at r.t. for 120 min.

The reactions were stopped and the unconsumed ATP was depleted by adding 5 μL ADP-glo Reagent (Promega, Cat# V9103) to the reaction. The plates were quickly centrifuged at 1000 rpm and incubated at r.t. for 40 min corresponding to full ATP depletion.

The ADP was converted back to ATP and luciferase and luciferin were introduced to detect ATP by adding 10 μL kinase detection reagent (Promega, Cat# V9103) to the reaction. The plates were centrifuged for a few seconds at 1000 rpm and incubated at r.t. for a further 30 min.

Luminescent read out was performed on an Envision luminescent reader (Perkin Elmer).

#### *3.1.3.2. Automated assay*

For the automated assay, the compounds were prepared as a serial dilution of 10 point dose responses with 1/5 dilution steps in 100% DMSO starting from 2 mM highest concentration.

Subsequently, the compounds were transferred and/or diluted in DMSO into the assay plates reaching a final volume of 30 nL and controls are added.

For serial dilutions: 10 point serial dilution, 1/5 dilution steps, final highest concentration 20 μM in 1% DMSO

On each assay plate 32 wells of each control (positive & negative) were added.

Plates were moved to the HighRes platform and the following steps were executed:

a) 1 μL diluted enzyme/substrate mixture was dispensed in each well,

b) The reaction was started by adding 2 μL diluted ATP (final concentration Km ATP) on the assay plates,
c) Plates were centrifuged for 10 seconds at 300 g, sealed and followed by incubation at r.t. for 120 min.
d) The reactions were stopped and the unconsumed ATP was depleted by adding 3 μL ADP-Glo reagent.

The plates were sealed and incubated at r.t. for 40 min.

e) The ADP was converted to ATP and luciferase/luciferin was introduced to detect ATP by adding 6 μL kinase detection reagent to the reaction. The plates were sealed and incubated at r.t. for 60 min.
f) Luminescent read out was performed on a Perkin Envision luminescent reader.

### *3.1.4. Data Analysis*

From the raw data generated following the read-out performed on the luminescent reader, the percentage inhibition (PIN) were calculated using the following Formula:

$$PIN = \frac{(\text{RLUn} - \text{RLUtest compound})}{\text{RLUn} - \text{RLUp}} \times 100$$

Wherein RLU = Relative Chemiluminescent Light Units (background subtracted) and p and n subscripts refered to each plate based average of positive and negative controls, respectively.
PIN values were plotted in concentration-response and $IC_{50}$ values were derived applying 4-parameter nonlinear regression (sigmoidal) curve fitting.

**Table V. *In vitro* human IRAK-4 ADP-Glo $IC_{50}$ of the compounds of the invention**

| Semi quantitative score | $IC_{50}$ range |
|---|---|
| **** | 0.1 -5 nM |
| *** | >5-10 nM |
| ** | >10-50 nM |
| * | >50 nM |
| ND | Not determined |

| Cpd. | IRAK4 $IC_{50}$ |
|---|---|
| 1 | **** |
| 2 | **** |
| 3 | **** |
| 4 | **** |
| 5 | **** |
| 6 | *** |
| 7 | *** |
| 8 | **** |
| 9 | *** |
| 10 | ND |
| 11 | **** |
| 12 | **** |
| 13 | ** |
| 14 | *** |
| 15 | **** |

(continued)

| Cpd. | IRAK4 $IC_{50}$ |
|---|---|
| 16 | **** |
| 17 | **** |
| 18 | **** |
| 19 | **** |
| 20 | **** |
| 21 | **** |
| 22 | **** |
| 23 | **** |
| 24 | **** |
| 25 | **** |
| 26 | ** |

### *3.2. Kinase selectivity profiling (broad panel)*

The purpose of this assay is to determine the activity and selectivity of a compound of the invention on a selected range of human kinases which may result in undesirable side-effects when inhibited (Dy and Adjei, 2013; Force and Kolaja, 2011).

Inhibition of human kinases is determined in radiometric kinase assays at Eurofins Cerep SA (Le Bois L'Evêque, BP 30001, F- 86600 Celle-Lévescault).

To determine its $IC_{50}$, a compound is tested at 10 doses starting from 10 μM (highest concentration), with 3-fold serial dilutions. $IC_{50}$ values are derived by fitting dose-response curves of % Remaining Enzyme Activity (relative to DMSO controls).

### *3.3. NFκB-reporter assay*

#### *3.3.1. Assay principle*

Interleukin-1 Receptor Associated Kinase (IRAK4) activity has been shown to play a crucial role downstream of LPS and IL-1(3 triggering activating NFκB-dependent signaling, whereas IRAK4 is shown not to be required for TNFα mediated responses (Davidson et al., 2006; Jain et al., 2014).

This assay was used to evaluate the IRAK4 selectivity and potency of the compounds of the invention upon IRAK4 dependent (LPS and IL-1β) and independent triggering (TNFα), in a THP1-Lucia NFκB reporter assay.

#### *3.3.2. Assay protocol*

THP-1-Lucia NFκB cells (Invivogen - 5, rue Jean Rodier 31400 Toulouse France - cat# thp 1-nfkb) were cultivated as recommended by the supplier using split cycles each cycle comprising a succession of thawing/expansion/seeding. The data reported were generated using cells having between 3 to 9 cycles. On the day of the experiment THP-1-Lucia NFκB cells were counted and seeded at a density of 1,000,000 cells/mL in culture medium (RPMI 1640 (Gibco, Cat#52400-025) + 10% FBS (Sigma, Cat# F7524-500ML)+ 1% P/S (Gibco, Cat#15140-122)) by pipetting 54 μL/well in a 384 well plate. Thereafter, 6 μL of a 10x trigger solution was added to all wells, at final concentrations of 2.5, 10 and 3 ng/mL for respectively LPS, TNFα and IL-1β, except for 'no trigger wells' where 6 μL culture medium only was added.

After trigger addition, compounds were added with a digital compound dispenser, in a 8-points concentration range, using 3-fold dilution steps and normalizing the final DMSO concentration to 0.2% DMSO in all wells.

After 24 h incubation at 37 °C, 5% COz, 40 μL of supernatant was collected per well and transferred to a new 384 well plate that was then stored at -20 °C until further use.

For the readout, supernatant samples were thawed on ice and 5 μL sample was transferred from each well to a new 384-well plate. 20 μL of Quanti-Luc solution (Quanti-Luc powder (Invivogen, Cat#rep-qlc1) dissolved in 25 mL

sterile water as indicated by the manufacturer) was added to each well after which luminescence was immediately measured on an Envision instrument.

To measure the inhibition of LPS/TNFα/IL-1β-induced NFxB-reporter activity, percentage inhibition (PIN) values were calculated for all concentrations tested, compared to controls.

Unstimulated samples (no trigger/vehicle (0.2% DMSO) were used as negative control (100% inhibition). As a positive control (0% inhibition), the stimulated samples (trigger/vehicle)) were used.

$$\mathrm{PIN} = \frac{RLU_p - RLU_{Test\ compound}}{RLU_p - RLU_n} * 100$$

wherein RLU= Relative Light Unit (background substracted) and p&n respectively refer to the average of the positive and negative controls.

PIN values were plotted in concentration-response and $EC_{50}$ values were derived using GraphPad Prism Software, applying 4 parameter non-linear regression (sigmoidal) curve fitting. The analysis was performed with the following constrains: Top must be less than 120 and Hill Slope equal to 1. $EC_{50}$ values are only calculated for compounds reaching at least 40% PIN.

For example, when tested in this assay Compound 1 inhibited LPS driven NFκB-reporter activity with an average $pEC_{50}$ value of 7.0 (±0.1) and inhibited IL-1β driven NFκB-reporter activity with an average $pEC_{50}$ value of 7.0 (±0.1), whereas no activity was observed on TNFα mediated NFκB-reporter activity, illustrating the IRAK4 selectivity of compound 1.

**Example 4. ADME assays**

***4.1. Kinetic solubility***

Starting from a 10 mM stock solution of test compound in DMSO, a second concentration in DMSO of 3 mM is prepared. Both DMSO concentrations are diluted in 0.1 M phosphate buffer pH 7.4, by adding 200 μL of buffer to 2 μL of Compound solution. The final compound concentrations are 100 & 30 μM with a final DMSO concentration of 1%. Measurements are done in duplicate.

As a positive control for precipitation, Pyrene is added to the corner points of each 96 well plate and serves as a reference point for calibration of Z-axis on the microscope. As a negative control, DMSO is added to the 12 wells on columns between positive control wells.

The assay plates are sealed and incubated for 1 h at 37 °C while shaking at 230 rpm.

The plates are, then, scanned under a white light microscope using a Nikon microscope, yielding individual pictures (20x) of the precipitate per concentration.

The precipitate is analyzed visually:

- If a precipitate is observed at 100 μM and at 30 μM, the data generated will be : < 30 μM
- If a precipitate is observed at 100 μM but not at 30 μM, the data generated will be : > 30 μM
- If no precipitate is observed (neither at 30 or at 100 μM), the data generated will be : > 100 μM

Solubility values measured according to this protocol are reported in μM and in μg/mL.

***4.2. Thermodynamic solubility***

Poor solubility, in particular poor thermodynamic solubility can limit the absorption of compounds from the gastrointestinal tract which in turn may reduce oral bioavailability.

Thermodynamic solubility investigates the solubility of a compound as a saturated solution in equilibrium, by opposition to kinetic solubility, which measures the solubility of a metastable solution where supersaturation may occur and provide over estimation of the actual solubility of the compound.(Klein, 2010)

***4.2.1. Thermodynamic solubility - Protocol 1***

In a 8 mL glass vial, 1-2 mg of dry matter of compound are added and stirred with the suitable buffers (Fed State Simulated Intestine Fluid, FeSSIF, or Fasted State Simulated Intestine Fluid, FaSSIF, or Fasted State Simulated Gastric Fluid, FaSSGF, or phosphate buffer pH 7.4) for 24 h at room temperature (for the buffer pH 7.4) or 37 °C (for the GI fluids). The concentration of the mixture is 1 mg/mL.

A volume of 500 μL are sampled, centrifuged for 10 min at 10 000 rpm and filtered. The samples are diluted in duplicates in DMSO (F100 and F10). Then, a final dilution (F100) in 80/20 $H_2O$/MeCN containing the internal standard (warfarin) is used for LCMS-MS analysis.

A standard curve is made starting from a 200,000 ng/mL stock in DMSO, freshly prepared from dry matter. Then, successive concentrations at 15,000, 10,000, 2,500, 1,000, 200 and 75 ng/mL in DMSO are prepared by using the Tecan robot.

Two quality control samples are made: one of 10,000 ng/mL and one of 500 ng/mL in DMSO, also starting from the DMSO working stock solution at 200,000 ng/mL.

The standard curve and quality controls are diluted a F100 in 80/20 $H_2O$/MeCN (with internal standard) and analyzed on LC/MS-MS (API4000 or API5500).

The samples are analyzed on LC-MS with a flow rate of 0.6 mL/min. The mobile phase A is 0.1% formic acid in water and the mobile phase B is 0.1% formic acid in MeCN. The sample is run under positive or negative ion spray on Pursuit C18 - 5 μm (2.0 × 2 0mm) column, from Agilent.

The peak areas of the standard curve are plotted in a graph and a linear or polynomial of the second order equation is used to calculate the unknown concentrations of the test compound.

#### *4.2.2. Thermodynamic solubility - Protocol 2*

In a 8 mL glass vial, 1-2 mg of dry matter of compound were added and stirred with the suitable buffers (Fed State Simulated Intestine Fluid, FeSSIF, or Fasted State Simulated Intestine Fluid, FaSSIF, or Fasted State Simulated Gastric Fluid, FaSSGF, or phosphate buffer pH 7.4) for 24 h at r.t. (for the buffer pH 7.4) or 37 °C (for the GI fluids). The concentration of the mixture was 1 mg/mL.

A volume of 1000 μL were sampled, centrifuged for 10 min at 10 000 rpm and and 500μL of the supernatant were filtered on captiva plate. The samples were diluted in duplicates in DMSO (F100 and F10). Then, a final dilution (F100) in 80/20 $H_2O$/MeCN containing the internal standard (warfarin) was used for LCMS-MS analysis.

A standard curve was made starting from a 40,000 ng/mL stock in DMSO, freshly prepared from dry matter. Then, successive concentrations at 15,000, 11,000, 6,000, 2,500, 1,000, 375, 150 and 75 ng/mL in DMSO were prepared.

Three quality control samples were made: one of 10,000, 1,500 and 200 ng/mL in DMSO, also starting from the DMSO working stock solution at 40,000 ng/mL.

The standard curve and quality controls were diluted a F100 in 80/20 $H_2O$/MeCN (with internal standard) and analyzed on LC/MS-MS (API4000 or API5500).

The samples were analyzed on LC-MS with a flow rate of 0.6 mL/min. The mobile phase A was 0.1% formic acid in water and the mobile phase B was 0.1% formic acid in 90% MeCN and 10% of $H_2O$. The sample was run under positive or negative ion spray on Pursuit C18 - 5 μm (2.0 x 20 mm) column, from Agilent.

The ratio analyte/internal standard peaks areas of the standard curve were plotted in a graph and a linear or polynomial of the second order equation was used to calculate the unknown concentrations of the test compound.

Solubility values were reported in μg/mL.

**Table VI. Thermodynamic solubility of illustrative compounds of the invention**

| Cpd# | Tsol FaSSIF (μg/mL) | Tsol FaSSGF (μg/mL) | Tsol FeSSIF (μg/mL) |
|---|---|---|---|
| 1 | 9.47, 39.7, 32.2, 28.8, 113 | 251, 158, 91.8, 193 | 115, 48.4, 126, 44.1, 83.4 |
| 2 | 10.2 | ND | ND |
| 3 | 0.801 | 106 | 27.6 |
| 4 | 0.75 | <0.75 | 6.01 |
| 5 | 2.26 | 1.96 | 14.2 |
| 6 | 24.1, 4.06 | 15.9, 3.62 | 45.5, 10.8 |
| 7 | 28 | >1000 | 93.9 |
| 8 | <0.75 | <0.75 | <0.75 |
| 9 | 3.96 | 3.82 | ND |
| 12 | 183 | >1000 | ND |
| 13 | 1.77 | 8.45 | ND |
| 14 | <0.75 | <0.75 | ND |

(continued)

| Cpd# | Tsol FaSSIF (μg/mL) | Tsol FaSSGF (μg/mL) | Tsol FeSSIF (μg/mL) |
|---|---|---|---|
| 15 | <0.75 | <0.75 | ND |
| 16 | 1.29 | ND | ND |
| 17 | 24.6 | 75.7 | 81 |
| 18 | 20.6 | 68.9 | 84.1 |
| 19 | 12.5 | 8.26 | 70.6 |
| 20 | 3.09 | 1.62 | ND |
| 21 | 16.4 | ND | ND |
| 22 | 14 | 50.6 | 75.9 |
| 23 | 16 | 104 | 93 |
| 24 | 28.5 | 200 | 105 |
| 25 | 20.2 | 389 | 59.6 |
| 26 | 34.5 | 818 | 52.7 |

***4.3. Plasma Protein Binding PPB (Equilibrium Dialysis)***

Prior to the start of the experiment, dialysis membranes (membrane strips, MW cut-off 12-14kDa, HTDialysis, Cat.No.# 1101) are soaked in deionized water for 60 min, transferred and left overnight in 20% EtOH.

The day of experiment, a 10 mM stock solution of the compound in DMSO is diluted with a factor 10 in DMSO. This solution is further diluted in freshly thawed human, rat, mouse or dog plasma (BioReclamation INC) with a final concentration of 5 μM and final DMSO concentration of 0.5%.

From this solution, an aliquot of 50 μL was taken and matrix matched with an equivalent volume of PBS for the recovery plate. After that 6 volumes of STOP solution was added to the recovery plate. For these recovery plates, no incubation is done.

Equilibrium Dialysis Device (96-well, model HTD96b, HTDialysis, Cat.No.#1006) is assembled according to manufacturer's instructions. Immediately after assembly, a volume of 100 μL of plasma (spiked with compound) is placed on one side of the well and another 100 μL of blank PBS buffer are added to the other side, respectively. Each compound is tested in duplicate.

Acebutolol and Nicardipine are used as low and very high binding controls, except for the mouse, Caffeine is used as low binder instead Acebutolol. If the PPB values for these controls are not in the range determined by the historical data, the assay is not validated.

The plate is incubated for 4 h at 37 °C while shaking at 230 rpm.

Thereafter, an aliquot of 50 μL is taken from each side of the well and matrix matched (mix of equal volumes of spiked plasma with blank PBS buffer and samples from buffer compartment with blank plasma).

Matrix matched samples are further mixed with 64 volumes of STOP solution (acetonitrile with warfarin as internal standard). After brief mixing and centrifugation (at 2400 rpm for 15 min, at +4 °C), the supernatant is filtered and transferred into new 96-well plates for analysis on LC-MS/MS (systems API4000 orAPI5500).

The samples are analyzed on LC/MS-MS with a flow rate of 0.6 mL/min. The mobile phase A is 0.1% formic acid in water and the mobile phase B is 0.1% formic acid in MeCN. The sample is run under positive or negative ion spray on Pursuit C18 - 5 μm (2.0 x 20 mm) column, from Agilent. The solvent gradient has a total run time of 1.2 min with a gradient profile as followed:

| Time (min) | % B |
|---|---|
| 0.0 | 5 |
| 0.2 | 100 |
| 0.8 | 100 |
| 0.9 | 5 |

(continued)

| Time (min) | % B |
|---|---|
| 1.2 | 5 |

The percentage bound in plasma (PPB) is determined using the following equation:

$$PPB = \frac{(Cplasma - Cbuffer)}{Cplasma} * 100$$

Cplasma = Peak area of the compound in the plasma / Peak area of the IS in the plasma
Cbuffer = Peak area of the compound in the buffer / Peak area of the IS in the buffer
"Concentration" is the ratio between compound and internal standard peak areas.

The recovery is a control, it allows to be sure that the compound has not a non-specific binding to the plates or it is not stable in the plasma in these conditions.

$$\%\ recovery = \frac{(PBS + Plasma) * 100}{Recov}$$

With:

PBS = (ratio of the peak area of the cpd / peak area of IS) in the PBS compartment after 4h
Plasma = (ratio of the peak area of the cpd / peak area of IS) in the Plasma compartment after 4h
Recov = Recovery = ratio of the peak area of the cpd in the well recovery / peak area of the IS in the well recovery at T0

The solubility of the compound in the final test concentration in PBS is checked by microscope to indicate whether precipitation is observed or not. If a precipitate is observed, no data of PPB is generated.

***4.4. Liver microsomal stability***

A 10 mM stock solution of compound in DMSO is diluted three-fold in DMSO. This pre-diluted compound solution is then diluted to 2 μM in a 100 mM phosphate buffer (pH 7.4) and pre-warmed at 37 °C. This compound dilution is mixed F2 with microsomal/cofactor mix at 37 °C under shaking at 300 rpm.

Final reaction conditions are: 100 μL incubation volume, 1 μM of test compound (n=2), 0.2% DMSO, 0.5 mg/mL microsomes (Xeno-Tech), 0.6 U/mL Glucose-6-phosphate-dehydrogenase (G6PDH, Roche, 10127671001), 3.3 mM $mgCl_2$ (Sigma, M2670), 3.3 mM glucose-6-phosphate (Sigma, G-7879) and 1.3 mM NADP+ (Sigma, N-0505).

After 30 min of incubation at 300 rpm and 37 °C, the reaction was stopped with 600 μL of STOP solution (Acetonitrile with Diclofenac as internal standard). For the zero time point, 600 μL of STOP solution were added to the compound dilution before the microsome mix was added.

The samples of both time points were centrifuged, filtered and the supernatant analyzed by LC-MS/MS.

The samples are analyzed on LC/MS-MS with a flow rate of 0.6 mL/min. The mobile phase A is 0.1% formic acid in $H_2O$ and the mobile phase B is 0.1% formic acid in 90% MeCN and 10% $H_2O$ . The sample is run under positive or negative ion spray on Pursuit C18 - 5μm (2.0 x 20 mm) column, from Agilent. The solvent gradient has a total run time of 2.2 min with a gradient profile as followed:

| Time (min) | % B |
|---|---|
| 0.0 | 5 |
| 0.6 | 5 |
| 1 | 100 |
| 1.9 | 100 |
| 2.0 | 5 |

(continued)

| Time (min) | % B |
|---|---|
| 2.2 | 5 |

The instrument responses (peak areas/IS peak area) were referenced to the zero time-point samples (considered as 100%) in order to determine the percentage of compound remaining.

Verapamil (1 μM) and Warfarin (1 μM) were used as reference compounds, as unstable and stable compounds respectively. If the microsomal stability values for these controls are not in the range determined by the historical data, the assay is not validated.

The data on microsomal stability are expressed as a percentage of the total amount of compound remaining after 30 min incubation.

The solubility of the compound in the final test concentration in 100 mM buffer pH 7.4 is checked by microscope to indicate whether precipitation is observed or not. If a precipitate is observed, no data of microsomal stability is generated.

*4.5. Metabolic stability in S9 subcellular fraction*

The aim of this assay is to assess compound metabolism by aldehyde oxidase by determination of their *in vitro* metabolic stability in S9 subcellular fraction.

A 10 mM stock solution of compound in DMSO is first diluted in DMSO (40 fold) to obtain 250 μM concentration. This compound solution is further diluted with water (5 fold) to obtain a 50 μM compound working solution (to obtain compound final concentration of 1 μM). Hydralazine (selective inhibitor of aldehyde oxidase) is prepared in water at 5 mM (to obtain final concentration of 100 μM). Incubation mixtures are prepared by adding 10 μL of liver S9 suspension (human, rat, mouse, monkey, BD Gentest™, 20 mg/mL) to 86 μL of 50 mM potassium phosphate buffer, pH 7.4 at 37 °C (final concentration of 2 mg protein/mL). 2 μL of 5 mM hydralazine is added for incubations with the addition of selective inhibitor or 2 μL of water, for incubations without inhibitor. After 5 min pre-warming, the reaction is initiated by the addition of 2 μL of 50 μM test compound to the incubation mixture.

After 0, 3, 6, 12, 18 and 30 min of incubation, the reaction (50 μL) is terminated with 150 μL of MeCN:MeOH (2:1) with 1% AcOH mixture containing 10 ng/mL of warfarin as analytical internal standard. Samples are mixed, centrifuged and the supernatant analyzed by LC-MS/MS.

The samples are analyzed on LC/MS-MS with a flow rate of 0.7 mL/min. The mobile phase A is 0.1% formic acid in water and the mobile phase B is 0.1% formic acid in 90% acetonitrile and 10% Water.

Phtalazine is included as positive control.

The instrument responses (peak area ratios of compound and internal standard) are referenced to the zero time point samples (considered as 100%) in order to determine the percentage of compound remaining. Plots of the % of compound remaining are used to determine the half-life and intrinsic clearance in the S9 incubations using the GraphPad Prism® software. The following formula is used to calculate in vitro intrinsic clearance ( μL/min/mg):

$$CL_{int}\ (\mu L/min/mg) = 0.693/t_{1/2}\ (min) * (mL\ of\ incubation/mg\ protein) * 1000$$

Test compounds can be classified as substrates of aldehyde oxidase if clearance by S9 is inhibited by hydralazine. Species specific clearance of test compound may also indicate metabolism by aldehyde oxidase.

*4.6. Metabolic stability in hepatocytes*

The aim of this assay is to determine the metabolic stability of the compound in hepatocytes (cryopreserved) of different species. Low hepatocyte stability may result in the formation of unwanted metabolites, high clearance, and therefore is not desirable.

The decrease in parent was assessed by measuring the percentage remaining by LC-MS/MS analysis.

A 10 mM stock solution of test compound in DMSO was first diluted in DMSO to 3 mM, and then in modified Krebs-Henseleit buffer (Sigma, K3753) to 5 μM. This compound dilution was added to a suspension of pooled cryopreserved hepatocytes (BioreclamationIVT) at 37 °C under gentle shaking.

Final reaction conditions were: 1 μM of test compound, 0.03% DMSO, 0.5 million viable hepatocytes/mL, and 75 μL incubation volume.

Testosterone (1 μM) and 7-hydroxycoumarin (1 μM) were used, respectively as phase I and phase II metabolic reaction controls.

After 0, 10, 20, 45, 90, 120 and 180 min of incubation, the reaction was terminated with 225 μL of MeCN:MeOH (2:1) containing 100 ng/mL of diclofenac as analytical internal standard Samples were mixed, centrifuged and the supernatant analyzed by LC-MS/MS.

The instrument responses (ratios of test compound and internal standard peak areas) were referenced to the zero time point samples (considered as 100%) in order to determine the percentage of compound remaining.

Plots of percentage compound remaining were used to determine the intrinsic clearance in the hepatocyte incubations using the following equations:

$$Cl_{Int} = \frac{Ln(2) * Incubation\ volume}{T\,{}^{1}/_{2} * cell\ number\ per\ incubation} * 1000$$

Scaled $Cl_{int}$ [L/h/kg] = $Cl_{int}$ [ μL/min/$10^6$cells] * # ($10^6$) cells/g liver * 60 * (1/$10^6$)

Scaled $Cl_{int}$ unbound [L/h/kg] = $Cl_{int}$ [L/h/kg] / Fu,inc

where: fu,inc equals hepatocyte binding, derived from microsomal binding (fu,mic) by following equation:

$$\text{fu, inc} = \frac{1}{1 + 10^{\frac{\log\left(\frac{1-fu,mic}{fu,mic}\right)-0.06}{1.52}}}$$

**Table VII. Hepatocyte stability of illustrative compounds of the invention**

| Cpd# | Mouse Hepatocyte stability (Perc. remaining at 90 min) | Human Hepatocyte stability (Perc. remaining at 90 min) |
|---|---|---|
| 1 | 99.45 | 96.6 |
| 2 | 94.1 | 100.45 |
| 3 | 10.17 | 22.8 |
| 5 | 76.25 | 94.5 |
| 7 | 108.25 | ND |
| 8 | 75.15 | 107 |
| 9 | 83.65 | 85.6 |
| 12 | 97.05 | ND |
| 13 | 87.8 | 98.45 |
| 16 | 51.75 | 75.95 |
| 17 | 93 | 87.25 |
| 18 | 93 | 90.8 |
| 20 | 80.9 | 81.65 |
| 21 | 49.45 | 92.15 |
| 22 | ND | 100.05 |
| 23 | ND | 101.45 |
| 24 | ND | 105 |
| 25 | ND | 97.2 |
| 26 | ND | 97.8 |

### *4.7. hERG Channel test*

The aim of this assay is to determine the *in-vitro* effects of a test compound on hERG current ($I_{Kr}$) expressed

in Human Embryonic Kidney (HEK) cells (evaluation of the blocking profile of test substance on the $I_{Kr}$-like potassium current mediated by hERG channel stably transfected in a human cell line), which is linked to cardiac safety.

The test substance is dissolved in pure dimethylsulfoxide (DMSO) by cold stirring to give a stock solution concentrated 333-fold as compared with the highest concentration to be tested. This stock solution is used to prepare the other stock solutions in DMSO. Each stock solution is used to prepare the solutions containing the final concentrations tested by dilution in extracellular solution (0.1, 1, 10 and 100 μM). Final concentration of DMSO should not not exceed 0.3%.

All formulations are prepared in glass containers.

If a slight opalescence persists at the highest concentration, a 50 μM (instead of 100 μM) concentration is tested.

DMSO diluted in extracellular solution (at the different concentrations used in the final test substance solutions) is used as vehicle.

The extracellular solution is constituted as follows (mM): K-gluconate: 4 mM / Na-gluconate: 145 mM / Mg-gluconate: 2 mM / Ca-gluconate: 3.5 mM / HEPES: 5 mM / glucose: 5 mM / mannitol: 20 mM. The pH is adjusted with NaOH to 7.40 ± 0.05.

Human embryonic kidney (HEK293) cells are stably transfected with the hERG clone (Creacell) and are maintained at 37 °C in a 5% $CO_2$ / 95% air incubator. Cells used for the study are transferred to an experimental chamber of approximately 2 mL which is maintained at a temperature of 35 ± 0.5 °C by a thermoelectric device (Harvard Apparatus: Type TC-344B) and mounted on the platform of an inverted microscope (Olympus: Type IX-51 or Leica DMI3000 B). Cells are continuously superfused with Tyrode's solution constituted as follows (mM): NaCl: 145 / KCl: 4 / HEPES: 5 / glucose: 5 / $CaCl_2$: 1 / $MgCl_2$: 1.

Ionic currents from hERG-transfected cells are measured using the whole cell configuration of the patch clamp technique. Glass pipettes are pulled from borosilicate glass by a vertical puller (Sutter Instruments: Type P30). Pipette tip resistance is approximately 1.5 to 3.5 MΩ when filled with internal solution constituted as follows (mM): K-gluconate: 145/ Mg-gluconate: 1/ EGTA: 2/ HEPES:5/ $K_2ATP$: 2.

The pipettes are connected to the input stage of a patch-clamp amplifier (Axon Instruments: Multiclamp 700B-1). Stimulation, data recording and analysis are performed using specialized Axon Instruments software (pClamp 9.2.0. orpClamp 10.3.0.2).

After rupture of the cell membrane (entering whole-cell mode), cells are stimulated every 10 seconds using the following protocol: 500 ms pulse to +10 mV from a holding potential of -80 mV followed by a 500 ms pulse to -40 mV during which tail current is measured.

Once the current under control conditions is stable, recordings are taken before (control) and after addition of the test substance. The effect of the test substance on tail current is monitored continuously until steady-state is reached. The peak tail current amplitude is averaged for 3 stimuli.

The following parameters are measured:

- Cell capacitance (pF).
- Peak tail current amplitude (pA).

Peak tail current measurements are normalized using the cell capacitance as an index of cell surface.

The cells will be considered as valid if cell capacitance < 80 pF, access resistance < 20 MΩ and holding current > -200 pA.

Results are expressed as absolute values and as percentage change from control (percentage of tail current inhibition).

The test substance is studied at 4 ascending concentrations on 3 hERG-transfected cells.

The concentration of test substance inducing 50% of inhibition ($IC_{50}$) of tail current is determined, if possible, from each individual concentration-response curve. The equation is of the following form:

$$y = Min + \frac{Max - Min}{1 + \left(\frac{X}{X50}\right)^{-P}}$$

### *4.8. CYP inhibition*

The aim of this assay is to determine the inhibitory potential of a test compound. A major concern for drug-drug-interaction is cytochrome P450 inhibition. Reversible CYP inhibition was determined in human liver microsomes using specific probe substrates for human cytochrome P450 isoenzymes CYP1A2, 2C9, 2C19, 2D6 and 3A4.

A 5 mM stock solution of test compound is prepared in methanol. This stock is further serially diluted 1:3 in

methanol and then added to mixture containing 50 mM potassium phosphate buffer pH7.4, human liver microsomes (BD Gentest) and probe substrate. After pre-warming 5 min at 37 °C, the reaction is started by adding cofactor mix (7.65 mg/mL glucose-6-phosphate, 1.7 mg/mL NADP, 6 U/mL of glucose-6-phosphate dehydrogenase), resulting in seven final concentrations of test compound in the range 0.137 - 100 μM (2% MeOH).

Assay conditions for CYP inhibition in human liver microsomes:

| Cytochrome P450 Isoform | Microsomes (mg/mL) | Probe substrate | Probe metabolite | Incubation (min) | Positive Control | Negative Control |
|---|---|---|---|---|---|---|
| 1A2 | 0.1 | Phenacetin (35 μM) | Acetamino phen | 10 | Furafylline | Sulphaphe nazole |
| 2C9 | 0.1 | Diclofenac (10 μM) | 4'-OH-diclofenac | 5 | Sulphaphena zole | Furafyl line |
| 2C19 | 0.25 | S-(+)-Mepheny toin (30 μM) | 4'-OH-mephenytoin | 15 | Ticlopidine | Phenacetin |
| 2D6 | 0.1 | Bufuralol (10 μM) | OH-bufuralol | 10 | Quinidine | Sulphaphe nazole |
| 3A4 | 0.1 | Midazolam (3 μM) | 1'-OH-midazolam | 5 | Ketoconazol e | Sulphaphe nazole |
| 3A4 | 0.25 | Testosterone (100 μM) | 6β-OH-testosterone | 15 | Ketoconazol e | Sulphaphe nazole |

Final concentrations of cofactor mix components are as follows: 1.56 mg/mL glucose-6-phosphate, 0.34 mg/mL NADP, 1.2 U/mL of glucose-6-phosphate dehydrogenase.

After incubation at 37 °C, the reaction (aliquot of 50 μL) is terminated with 150 μL MeCN:MeOH (2:1) solution with internal standard (warfarin for 2C9, diclofenac for all other tested isoforms). Samples are centrifuged and the supernatant fractions analyzed by LC-MS/MS.

The instrument responses (ratio of tst compound and internal standard peak areas) are referenced to those for solvent controls (assumed as 100%) in order to determine the percentage reduction in probe metabolism. Percent of control activity vs concentration plots are generated and fitted using GraphPad Prism software to generate $IC_{50}$.

### *4.9. MDCKII MDR1 permeability*

MDCKII-MDR1 cells are Madin-Darby canine kidney epithelial cells, overexpressing the human multi-drug resistance (MDR1) gene, coding for P-glycoprotein (P-gp). Cells are obtained from the Netherlands Cancer Institute and used after a 3-4 day culture in 24-well Millicell® cell culture insert plates (Millipore, PSRP010R5). A bi-directional MDCKII-MDR1 permeability assay is performed as described below.

The transport across membrane is tested in the presence and absence of Elacridar, specific P-gp inhibitor. Such experimental set-up enabled the determination of passive permeability (with Elacridar) and influence of P-gp on test compound transport (without Elacridar).

MDCKII-MDR1 cells ($3 \times 10^5$ cells/mL; $1.2 \times 10^5$ cells/well) are seeded on 24-well Millicell cell culture insert plates (Millipore, PSRP010R5) in plating medium consisting of DMEM + 1% Ala-Gln + 1% Antibiotic/Antimycotic + 1% non-essential amino acids + 10% FBS. Cells are left in $CO_2$ incubator for 3-4 days. The medium is changed 24 h after seeding. On the day of permeability experiment, cells that are tested in the presence of Elacridar, specific P-gp inhibitor, are first pre-incubated for 45 min with Dulbecco's phosphate buffer saline (D-PBS, pH7.4), containing 1% DMSO and Elacridar at final concentration of 2 μM.

Test and reference compounds (Amprenavir and Diclofenac) are prepared in Dulbecco's phosphate buffer saline (D-PBS, pH 7.4; Sigma, D8662) with or without Elacridar (final concentration: 2 μM) and added to either the apical (400 μL) or basolateral (800 μL) chambers of the Millicell cell culture plates assembly at a final concentration of 10 μM (0.5 μM in case of Amprenavir) with a final DMSO concentration of 1%.

The reference compound Amprenavir has a high passive permeability but is substrate of the Pgp, and the Diclofenac is highly permeable and is not substrate of the Pgp.

100 μM Lucifer yellow (Sigma, L0259) are added to all donor buffer solutions, in order to assess integrity of the cell monolayers by monitoring Lucifer yellow permeation. Lucifer yellow is a fluorescent marker for the paracellular transport pathway and is used as internal control to verify tight junction integrity of every cell monolayer during the assay.

After a 1 h incubation at 37 °C while shaking on an orbital shaker at 150 rpm, aliquots are taken from both apical and basal chambers and added to 3 volumes of MeCN:$H_2O$ solution (2:1) containing analytical internal standard (10 ng/mL warfarin) in a 96 well plate. Samples are also taken at the beginning of the experiment from donor solutions to obtain initial (Co) concentration.
Concentration of compound in the samples is measured by high performance liquid-chromatography/mass spectroscopy (LC-MS/MS).
Lucifer yellow is measured with a Thermo Scientific Fluoroskan Ascent FL (excitation wavelength: 485 nm, measurement wavelength: 530 nm) in a 96 well plate containing 150 μL of liquid from all receiver wells (basolateral or apical side).

### *4.10. Whole blood assays*

#### *4.10.1. Ex vivo human IFNα and TNFα release inhibition (human whole blood assay)*

The aim of the assay is to evaluate the activity of compounds of the invention on the activated TLR/IRAK-4 pathway in an ex vivo human whole blood setting. Toll-like receptors (TLRs) are pattern recognition receptors that recognize a wide variety of microbial molecules, called pathogen-associated molecular patterns (PAMPs). Human TLR7 and TLR8 recognize imidazoquinoline compounds (e.g., CL097 - CAS n# 1026249-18-2) and single stranded RNAs as their natural ligands. Activation of TLRs leads to the production of several cytokines (e.g., IFNα, TNFα, IL-8, IL-6) by the TLR agonist-treated cells, whereas IRAK4 leads to the production of IFNα. Cytokine release is used as readout in this assay and represents a measure for the level of inhibition of the TLR/ IRAK-4 pathway by the tested compound. It should be noted that in the context of the complete organism, other sources for these cytokines exist that are not dependent on the TLR/IRAK-4 pathway, such as e.g., macrophages (upon activation of the Fcy receptor (Yan et al., 2012)) or T cells (upon activation of the T cell receptor (Brehm et al., 2005)).

##### *4.10.1.1. Experimental design*

Blood was collected from healthy volunteers into lithium heparin tubes by venipuncture, then gently inverted several times to prevent clotting and incubated for at least 15 min at 37 °C on a rocking mixer shaker. Then, 100 μL of blood was dispensed into polypropylene 96-well microplate and pre-incubated in duplicate with 0.3% DMSO or test compound at different concentrations (from 30 to 0.01 μM, 3-fold dilutions to get 0.3% DMSO at the final) for 15 min at 37 °C. After this pre-incubation, blood was triggered with CL097 (2 μg/mL from 1 mg/mL solution in water; InvivoGen, tlrl-c97) for 3 h 30 min at 37 °C. Microtubes were centrifuged at 5000 × g for 10 min at 4 °C and approximately 40 μL of plasma were collected into a polystyrene 96-well plate. Plasma could be analyzed freshly, within 30 min after triggering, or frozen at -80 °C. Finally, the quantification of TNFα and IFNα were performed in the plasma using AlphaLISA Kit for TNFα and IFNα according to the manufacturer's instructions and read on the Ensight (PerkinElmer).

##### *4.10.1.2. Data analysis*

A standard curve was created by plotting on log-log the mean absorbance on the y-axis against the concentration on the x-axis and a 4-parameters logistic regression (4PL) was made through the points. For each blood sample, the IFNα concentrations of the samples were determined from the fit.
Data were then expressed as a percentage of inhibition (PIN) for each replicate using the formula:

$$PIN_{sample1} = \frac{(\text{mean IFN}\alpha \text{ with CL097} - \text{IFN}\alpha_{sample1})*100}{(\text{mean IFN}\alpha \text{ with CL097} - \text{mean IFN}\alpha \text{ with vehicle})}$$

With mean IFNα with CL097 = mean IFNα concentration of replicate samples triggered with CL097; IFNα $_{sample1}$= IFNα concentration of sample 1; mean IFNα with vehicle = mean IFNα concentration of replicate samples treated with vehicle.
For each blood sample, the TNFα concentrations of the samples were determined from the fit. Data were then expressed as a percentage of inhibition (PIN) for each replicate using the formula:

$$PIN_{sample1} = \frac{(\text{mean TNF}\alpha \text{ with CL097} - \text{TNF}\alpha_{sample1})*100}{(\text{mean TNF}\alpha \text{ with CL097} - \text{mean TNF}\alpha \text{ with vehicle})}$$

With mean TNFα with CL097 = mean TNFα concentration of replicate samples triggered with CL097; TNFα $_{sample1}$=

TNFα concentration of sample 1; mean TNFα with vehicle = mean TNFα concentration of replicate samples treated with vehicle.

Curve fitting for $pIC_{50}$ determination were generated using mean PIN ± sem. Graphs and $pIC_{50}$ calculations were derived using Prism 5.03 software (GraphPad).

*4.10.1.3. Results*

| Semi quantitative score | $IC_{50}$ range |
|---|---|
| **** | 0.1 -500 nM |
| *** | >500-1000 nM |
| ** | > 1000 - 5000 nM |
| * | >5000 nM |
| ND | Not determined |

| Cpd. | CL097 triggered IFNα release in human whole blood $IC_{50}$ | CL097 triggered TNFα release in human whole blood $IC_{50}$ |
|---|---|---|
| 1 | **** | **** |
| 2 | *** | **** |
| 3 | ** | ** |
| 4 | ** | **** |
| 5 | ** | ** |
| 6 | *** | **** |
| 7 | ** | ** |
| 9 | ** | *** |
| 12 | ** | ** |
| 15 | * | * |
| 17 | ** | *** |
| 18 | ** | *** |
| 19 | *** | ND |
| 20 | ** | ND |
| 21 | ** | ** |
| 22 | *** | **** |
| 23 | *** | **** |
| 24 | **** | **** |
| 25 | *** | *** |
| 26 | ** | ** |

***4.10.2. Ex vivo mouse TNFα release inhibition (mouse whole blood assay)***

The objective of the assay is to assess the activity of compounds of the invention on the activated TLR / IRAK-4 pathway in an ex vivo mouse whole blood setting. Toll-like receptors (TLRs) are pattern recognition receptors that recognize a wide variety of microbial molecules, called pathogen-associated molecular patterns (PAMPs). While human TLR7 and TLR8 both recognize imidazoquinoline compounds (e.g., CL097) and single stranded RNAs as their natural ligands, rodent TLR8 needs additional factors such as oligodeoxynucleotides (e.g., poly(dT)) for activation.

*4.10.2.1. Experimental design*

Balb/cJ female mice (7-8 weeks old) are obtained from Janvier Labs (France).

Blood, obtained by exsanguinations, is collected (around 1 mouse for 5 data points) into lithium heparinate tubes and then incubated for at least 15 min at 37 °C on a rocking mixer shaker. The blood from all the mice is mixed into a 50 mL polypropylene tube. Then, 100 μL of blood are dispensed into 2 mL-microtubes and pre-incubated with DMSO 0.3 % or tested compound at different concentrations (from 10 to 0.01 μM, 3 fold dilutions made in DMSO) for 15 min at 37 °C. After this incubation, blood is triggered with CL097 (2 μg/mL) and poly(dT) (0.2 μM) or vehicle (distilled water) for 3 5 h at 37 °C. Microtubes are centrifuged at 5000 g for 10 min at 4 °C and around 30 μL of plasma are collected into a polystyrene 96-well plate. Plasma can be analyzed freshly or frozen at -80 °C. Finally, the quantification of TNFα is performed with 2.5 μL of undiluted plasma (in duplicate) and using the mouse TNF-α alphaLISA kit according to the manufacturer's instructions. The reading (optical density=OD) is performed on the Ensight (PerkinElmer).

*4.10.2.2. Data analysis*

A standard curve is created by plotting on log-log the mean absorbance on the y-axis against the concentration on the x-axis and a 4 parameter logistic regression is made through the points.

For each blood sample, the TNFα concentrations of the samples are determined from the fit. Data are then expressed as a percentage of inhibition (PIN) for each replicate using the formula:

$$PIN_{sample1} = \frac{(\text{mean TNF}\alpha \text{ with CL097} - \text{TNF}\alpha_{sample1})*100}{(\text{mean TNF}\alpha \text{ with CL097} - \text{mean TNF}\alpha \text{ with vehicle})}$$

With mean TNFα with CL097= mean TNFα concentration of replicate samples triggered with CL097 /poly(dT); $TNF\alpha_{sample1}$= TNFα concentration of sample 1; mean TNFα with vehicle = mean TNFα concentration of replicate samples treated with Vehicle.

Curve fitting are generated using mean PIN ± sem.

Graphs and $IC_{50}$ calculations are performed with the Prism 5.03 software (GraphPad). Data are presented as mean of $IC_{50}$ (nM) obtained with 24 independent experiments.

**Example 5. In vivo assays**

***5.1. CIA model***

***5.1.1. Materials***

Completed Freund's adjuvant (CFA) and incomplete Freund's adjuvant (IFA) were purchased from Difco. Bovine collagen type II (CII), lipopolysaccharide (LPS), and Enbrel was obtained from Chondrex (Isle d'Abeau, France); Sigma (P4252, L'Isle d'Abeau, France), Whyett (25mg injectable syringe, France) Acros Organics (Palo Alto, CA), respectively. All other reagents used were of reagent grade and all solvents were of analytical grade.

***5.1.2. Animals***

DBA1/J mice (male, 7-8 weeks old) were obtained from Charles River Laboratories (France). Mice were kept on a 12 h light/dark cycle (07h00 - 19h00). Temperature was maintained at 22 °C, and food and water were provided *ad libitum.*

***5.1.3. Collagen induced arthritis (CIA)***

One day before the experiment, CII solution (2 mg/mL) was prepared with 0.05 M AcOH and stored at 4 °C. Just before the immunization, equal volumes of adjuvant (IFA) and CII were mixed by a homogenizer in a pre-cooled glass bottle in an ice water bath. Extra adjuvant and prolonged homogenization may be required if an emulsion was not formed. 0.2 mL of the emulsion was injected intradermally at the base of the tail of each mice on day 1, a second booster intradermal injection (CII solution at 2 mg/mL in CFA 0.1 mL saline) was performed on day 9. This immunization method was modified from published methods (Jou et al., 2005; Sims et al., 2004).

### *5.1.4. Study design*

The therapeutic effects of the compounds were tested in the mouse CIA model. Mice were randomly divided into equal groups and each group contained 10 mice. All mice were immunized on day 1 and boosted on day 21. The negative control group was treated with vehicle (MC 0.5%) and the positive control group with Enbrel (10 mg/kg, 3x week, s.c.). A compound of interest was typically tested at 3 doses *per os* (p.o.). At day 32, randomization between groups was performed with respect with clinical score and animals were therapeutically treated regarding their group until day 47. Body weight and clinical score, were recorded twice a week.

### *5.1.5. Clinical assessment of arthritis*

Arthritis is scored according to the method of (Khachigian, 2006; Lin et al., 2007; Nishida et al., 2004). The swelling of each of the four paws is ranked with the arthritic score as follows: 0-no symptoms; 1-mild, but definite redness and swelling of one type of joint such as the ankle or wrist, or apparent redness and swelling limited to individual digits, regardless of the number of affected digits; 2-moderate redness and swelling of two or more types of joints; 3-severe redness and swelling of the entire paw including digits; 4-maximally inflamed limb with involvement of multiple joints (maximum cumulative clinical arthritis score 16 per animal) (Nishida et al., 2004).

#### *5.1.5.1. Change in body weight* (%) *after onset of arthritis*

Clinically, body weight loss is associated with arthritis (Argilés and López-Soriano, 1998; Rail and Roubenoff, 2004; Shelton et al., 2005; Walsmith et al., 2004). Hence, changes in body weight after onset of arthritis can be used as a non-specific endpoint to evaluate the effect of therapeutics in the rat model. The change in body weight (%) after onset of arthritis was calculated as follows:

$$\text{Mice: } \frac{\text{Body Weigh t}_{(\text{week6})} - \text{Body Weigh t}_{(\text{week5})}}{\text{Body Weigh t}_{(\text{week5})}} \times 100\%$$

#### *5.1.5.2. Radiology*

X-ray photos were taken of the hind paws of each individual animal. A random blind identity number was assigned to each of the photos, and the severity of bone erosion was ranked by two independent scorers with the radiological Larsen's score system as follows: 0- normal with intact bony outlines and normal joint space; 1- slight abnormality with any one or two of the exterior metatarsal bones showing slight bone erosion; 2-definite early abnormality with any three to five of the exterior metatarsal bones showing bone erosion; 3-medium destructive abnormality with all the exterior metatarsal bones as well as any one or two of the interior metatarsal bones showing definite bone erosions; 4-severe destructive abnormality with all the metatarsal bones showing definite bone erosion and at least one of the inner metatarsal joints completely eroded leaving some bony joint outlines partly preserved; 5-mutilating abnormality without bony outlines. This scoring system is a modification from (Bush et al., 2002; Jou et al., 2005; Salvemini et al., 2001; Sims et al., 2004).

#### *5.1.5.3. Results*

For each readout, mean and sem are calculated. A difference statistically significant between intact or treated groups and in disease vehicle group is evaluated with Prism software using a one-way ANOVA (for treatment groups) followed by a Dunnett's multiple comparisons post-hoc test. *: $p < 0.05$; **: $p < 0.01$; ***: $p < 0.001$ versus disease Vehicle group.

When tested at 3, 10 and 30 mg/kg b.i.d. following the above protocol, compound 1, showed a statistically significant effect on both on clinical score and bone erosion Larsen Score.

#### *5.1.5.4. Steady State PK*

At day 7, blood samples were collected at the retro-orbital sinus with lithium heparin as anticoagulant at the following time points: predose, 1, 3 and 6 hs. Whole blood samples were centrifuged and the resulting plasma samples were stored at -20 °C pending analysis. Plasma concentrations of each test compound were determined by an LC-MS/MS method in which the mass spectrometer was operated in positive electrospray mode.

***5.2. Murine model of psoriatic-like epidermal hyperplasia induced by topical applications of imiquimod, a TLR7/8 agonist.***

***5.2.1. Materials***

Aldara® 5% imiquimod cream is obtained from MEDA.
Anti-mouse IL-12/IL-23 p40 FG purified antibody (C17.8) is obtained from Affymetrix eBioscience (cat no. 16-7123-85).

***5.2.2. Animals***

Balb/cJ mice (female, 18-20 g body weight) are obtained from Janvier Labs (France). Mice are kept on a 12 h light/dark cycle (07:00 - 19:00). Temperature is maintained at 22 ± 2 °C, food and water are provided *ad libitum.*

***5.2.3. Study design***

The design of the study is adapted from Van der Fits L. *et al.* (van der Fits et al., 2009).
On the first day, the mice are shaved around the two ears under light anaesthesia with isoflurane.
30 mg of commercially available imiquimod cream (Aldara 5% cream) are applied on both internal and external surfaces of each ear for 4 consecutive days, translating in a daily dose of 1.5 mg of the active compound. Control animals received the same quantity of vaseline.
From day 1 to day 5, mice are dosed with test compound, 10 or 30 mg/kg, *p.o.*, *b.i.d.* in methyl cellulose 0.5%, before application of imiquimod (on day 5, the mice are dosed only once, 2 h before euthanasia).
In a positive reference group, the animals receive two intraperitoneal injections of anti-mouse IL-12/IL-23 p40 antibody, 10 mg/kg, on day 1 and 3 days before day 1.

***5.2.4. Assessment of disease***

The thickness of both ears is measured daily with a thickness gage (Mitutoyo, Absolute Digimatic, 547-321). Body weight is assessed at initiation of the experiment and at sacrifice. At day 5, 2 h after the last dosing, the mice are sacrificed. The pinnae of the ear are cut, excluding cartilage. The pinnae are weighed and then immersed in a vial containing 1 mL of RNA*later*® solution to assess gene expression or in formalin for histology.
There are 14 mice per group. The results are expressed as mean ± SEM and statistical analysis is performed using one-way ANOVA followed by Dunnett's post-hoc test versus imiquimod-vehicle group.

***5.2.5. Histology***

After sacrifice, ears are collected and fixed in 3.7% formaldehyde before embedding in paraffin. 2 μm thick sections are cut and stained with haematoxylin and eosin. Ear epidermis thickness is measured by image analysis (SisNcom software) with 6 images per ear captured at 20x magnification. Data are expressed as mean ± SEM and statistical analysis is performed using one-way ANOVA followed by Dunnett's post-hoc test versus imiquimod-vehicle group.

***5.2.6. Gene expression analysis***

Ears are removed from the RNA*later*® solution and put in Trizol® after disruption with 1.4 mm ceramic beads in a Precellys device. Total RNA is then purified using NucleoSpin® RNA kit. cDNA is prepared and quantitative PCR is performed with gene-specific primers from Qiagen using SYBR Green technology in a ViiA7 real-time PCR system (Applied Biosystems). Expression levels of each gene (IL17A, IL1B, IL22, LCN2, S100A8 and S100A9) are calculated relative to the cyclophilin A housekeeping gene expression level. Data are expressed as mean ± SEM of the relative quantity ($RQ= 2^{-\Delta C_T}$, where $\Delta C_T = C_T$ sample - $C_T$ cyclophilin A). The statistical test used is ANOVA analysis of variance with Dunnett's post-hoc test versus imiquimod-vehicle group.

***5.3. Murine model of psoriatic-like epidermal hyperplasia induced by intradermal injections of IL-23***

***5.3.1. Materials***

Mouse recombinant IL-23, carrier free (14-8231, CF) is provided by e-Bioscience.

*5.3.2. Animals*

Balb/c mice (female, 18-20 g body weight) are obtained from CERJ (France). Mice are kept on a 12 h light/dark cycle (07:00 - 19:00). Temperature is maintained at 22 °C, food and water are provided *ad libitum.*

*5.3.3. Study design*

The design of the study is adapted from Rizzo HL. *et al.* (Rizzo et al., 2011).
On the first day (D1), the mice are shaved around the two ears.
For 4 consecutive days (D1 to D4), the mice receive a daily intradermal dose of mouse recombinant IL-23 (1 μg/20 μL in PBS/0.1% BSA) in the right pinna ear and 20 μL of PBS/0.1% BSA in the left pinna ear under anesthesia induced by inhalation of isoflurane.
From D1 to D5, mice are dosed with test-compound (10, 30, or 100 mg/kg, *p.o., q.d.* in methylcellulose 0.5%) or with vehicle, 1 h prior IL-23 injection.

*5.3.4. Assessment of disease*

The thickness of both ears is measured daily with an automatic caliper. Body weight is assessed at initiation and at sacrifice. On fifth day, 2 h after the last dosing, the mice are sacrificed. The pinnae of the ear are cut, excluding cartilage. The pinnae are weighed and then, placed in a vial containing 1 mL of RNA*later*® solution or in formaldehyde.
At D4, blood samples are also collected from the retro-orbital sinus for PK profiling just before dosing (T0) and 1 h, 3 h, 6 h post-dosing.
There are 8 mice per group. The results are expressed as mean ± SEM and statistical analysis is performed using one-way ANOVA followed by Dunnett's post-hoc test versus IL-23 vehicle groups.

*5.3.5. Histology*

After sacrifice, ears are collected and fixed in 3.7% formaldehyde before embedding in paraffin. 2 μm thick sections are done and stained with hematoxylin and eosin. Ear epidermis thickness is measured by image analysis (Sis'Ncom software) with 6 images per ear captured at magnification x20. Data are expressed as mean ± SEM and statistical analysis is performed using one-way ANOVA followed by Dunnett's post-hoc test versus IL-23 vehicle groups.

*5.3.6. Gene expression analysis*

Half ears are removed from RNA*later*® solution and put in Trizol® after disruption with 1.4 mm ceramic beads in a Precellys device. Total RNA is then purified using NucleoSpin® RNA kit. cDNA is prepared and quantitative PCR is performed with gene-specific primers from Qiagen using SYBR Green technology in a ViiA7 real-time PCR system (Applied Biosystems). Expression levels of each gene (IL17A, IL1B, IL22, LCN2, S100A8 and S100A9) are calculated relative to the cyclophilin A housekeeping gene expression level. Data are expressed as mean ± SEM of the relative quantity ($RQ = 2^{-\Delta C_T}$, where $\Delta C_T = C_T$ sample - $C_T$ cyclophilin A). The statistical test used is ANOVA analysis of variance with Dunnett's post-hoc test versus the IL-23 vehicle group.

*5.4. PK/PD model: TNFα release induced by CL097, a specific TLR7/8 agonist*

The aim of this assay is to determine the relationship between the inhibition of an IRAK-4 dependent event *in vivo* upon administration of a compound of the invention and the circulating concentration levels of this compound.

*5.4.1. Materials*

CL097 (cat no. tlrl-c97) and poly(dT) (cat no. tlrl-pt17) are obtained from InvivoGen.
AlphaLISA® mouse TNFα kits are obtained from Perkin-Elmer (cat no. AL505C).

*5.4.2. Animals*

*DBA/1J* mice (male, 18-20 g body weight) are obtained from Janvier Labs (France). Mice are kept on a 12 h light/dark cycle (07:00 - 19:00). Temperature is maintained at 22 ± 2 °C, food and water are provided *ad libitum.*

### *5.4.3. Study design*

The mice receive an oral dose of test-compound. A group of intact animals which does not receive any dosing is used as the t = 0 time point.

Two blood samples obtained by intra-cardiac sampling (under isoflurane anesthesia) are collected into lithium heparinate tubes at 30 min, 1 h, 3 h, 8 h or 24 h post-dosing. One is used for pharmacokinetics (PK) analysis and the second for pharmacodynamic (PD) marker quantification.

### *5.4.4. Quantification of compound levels in plasma*

Whole blood samples are centrifuged at 5000 rpm for 10 min and the resulting plasma samples are stored at -20 °C pending analysis. Plasma concentrations of each test compound are determined by an LC-MS/MS method.

### *5.4.5. Determination of pharmacokinetic parameters*

Pharmacokinetic parameters are calculated using WinNonlin® (Pharsight®, United States).

### *5.4.6. Quantification of PD marker*

Each blood sample is stimulated with CL097 and poly(dT) for 2 h at 37 °C. Then, plasma is collected and analyzed for TNFα by AlphaLISA according to the manufacturer's instructions.

There are 6 mice per group. The results are expressed as TNFα concentration (pg/mL), or as percentage of inhibition (PIN) relative to the t = 0 time point. The data are presented as mean ± SEM and statistical analysis is performed using one-way ANOVA followed by Dunnett's post-hoc test versus vehicle group of the corresponding time point.

## *5.5. Murine prophylactic model of atopic dermatitis induced by topical application of MC903*

### *5.5.1. Materials*

Methylcellulose 0.5% was obtained from VWR (cat no. AX021233). MC903 (calcipotriol) was obtained from Tocris Bioscience (cat no. 2700/50). ProSense® 680 was obtained from PerkinElmer (cat no. NEV10003). RNA*later*® was obtained from Ambion (cat no. AM7021). Imalgene® 1000 (Merial) and Rompun® 2% (Bayer) were obtained from Centravet (cat no. IMA004-6827812 and ROM001-6835444).

### *5.5.2. Animals*

BALB/cN mice (female, 18-20 g body weight) or CD1/Swiss mice (female, 24-26 g body weight) were obtained from Janvier Labs (France). Mice were kept on a 12 h light/dark cycle (07:00 - 19:00). Temperature was maintained at 22 ± 2 °C, food and water were provided *ad libitum.*

### *5.5.3. Study design*

The design of the study was adapted from Li M. et al. (Li et al., 2006).

On the first day (D1), the mice were anesthetized with an intraperitoneal injection of Imalgene and Rompun (7.5% / 2.5%; 0.1 mL/10 g) and shaved around the two ears.

As of D1, either 20 µL EtOH or 2 nmol of MC903 (in 20 µL EtOH) were topically applied on both ears of mice for five consecutive days.

From D1 to D8, the mice were dosed with test compound (15 or 30 mg/kg, *p.o.*, *b.i.d.* in methylcellulose 0.5%) or dexamethasone (5 mg/kg, *p.o.*, *q.d.* in methylcellulose 0.5%), or with vehicle.

### *5.5.4. Quantification of compound levels in plasma*

Plasma concentrations of each test compound were determined by an LC-MS/MS method in which the mass spectrometer was operated in positive or negative electrospray mode.

### *5.5.5. Determination of pharmacokinetic parameters*

Pharmacokinetic parameters were calculated using Phoenix® WinNonlin® (Pharsight®, United States).

### *5.5.6. Assessment of disease*

The thickness of both ears was measured (after anaesthesia induced by isoflurane inhalation) at initiation of the study, every other day and at sacrifice using a thickness gage (Mitutoyo, Absolute Digimatic, 547-321).

Body weight was assessed at initiation of the study, every other day and at sacrifice.

On D4, mice from all groups receive ProSense® 680 probe (0.8 nmol/10 g, IP). On D5, the mice were anesthetized with an intraperitoneal injection of Imalgene and Rompun (7.5% / 2.5%; 0.1 mL/10 g). Granulocyte infiltration was measured using in vivo molecular imaging (Bruker In-Vivo Xtreme imaging system, excitation wavelength: 630 nm, emission wavelength: 700 nm, acquisition time: 5 seconds).

On D8, 2 h after the last dosing, mice were sacrificed and total blood was collected on EDTA-coated tubes and plasma was frozen for further measurements (including circulating compound). A sample of blood was also collected in heparin-coated tubes.

The pinnae of the ears were collected and weighed. One ear was cut longitudinally into 2 halves. One half was fixed in formaldehyde buffer 4% for histology; the other one was immersed in RNA*later*® to assess gene expression.

There were 8 mice per group. The results were expressed as mean $\pm$ SEM and statistical analysis was performed using one-way ANOVA followed by Dunnett's post-hoc test versus MC903 vehicle groups for ear thickness and weight, versus EtOH vehicle group for body weight.

#### *5.5.6.1. Results*

For each readout, mean and sem were calculated. A difference statistically significant between intact or treated groups and in disease vehicle group was evaluated with Prism software using a one-way ANOVA (for treatment groups) followed by a Dunnett's multiple comparisons post-hoc test. *: $p < 0.05$; **: $p < 0.01$; ***: $p < 0.001$ versus disease Vehicle group.

When tested at 10 mg/kg b.i.d.following the above protocol, compound 1, showed a statistically significant effect on ear thickening at D8, granulocyte and helper T cells infiltrate.

### *5.5.7. Histology*

After sacrifice, half ears are collected and fixed in 3.7% formaldehyde before embedding in paraffin. 4 $\mu$m thick sections are immunostained by immunohistochemistry with specific cell marker antibody: CD3 for T cells and EPX for eosinophils. The immunostained cell areas from a whole section per mouse are measured by image analysis (CaloPix software, TRIBVN Healthcare). Data are expressed as mean $\pm$ SEM and statistical analysis is performed using one-way ANOVA followed by Dunnett's post-hoc test versus MC903 vehicle groups.

### *5.5.8. Gene expression analysis*

Ears are removed from RNA*later*® solution and placed in Trizol® after disruption with 1.4 mm ceramic beads in a Berlin Instruments Precellys® homogenizer. Total RNA is then extracted using a phenol/chloroform protocol and purified with a QIAcube using an RNeasy® 96 QIAcube® HT Kit (Qiagen, cat no. 74171). cDNA is prepared and quantitative PCR performed with gene-specific primers from Qiagen using SYBR Green technology in a ViiA 7 real-time PCR system (Applied Biosystems). Expression levels of each gene (IL4, IL5, IL13, TSLP, IL33, ST2, IL25, IL31, IFN$\gamma$, IL6, IL10, LCN2, S100A8 and S100A9) are calculated relative to the HPRT, GAPDH and $\beta$-actin housekeeping gene expression levels. Data are expressed as mean $\pm$ SEM of the relative quantity (RQ = $2^{-\Delta C_T}$, where $\Delta C_T = C_T$ sample - average ($C_T$ HPRT, $C_T$ GAPDH, $C_T$ $\beta$-actin). The statistical test used is ANOVA analysis of variance with Dunnett's post-hoc test versus the EtOH/MC903 vehicle group.

## *5.6. Murine therapeutic model of atopic dermatitis induced by topical application of MC903*

### *5.6.1. Materials*

Methylcellulose 0.5% is obtained from VWR (cat no. AX021233). MC903 (calcipotriol) is obtained from Tocris Bioscience (cat no. 2700/50). ProSense® 680 is obtained from PerkinElmer (cat no. NEV10003). RNA*later*® is obtained from Ambion (cat no. AM7021). Imalgene® 1000 (Merial) and Rompun® 2% (Bayer) are obtained from Centravet (cat no. IMA004-6827812 and ROM001-6835444).

### *5.6.2. Animals*

BALB/cN mice (female, 18-20 g body weight) or CD1/Swiss mice (female, 24-26 g body weight) are obtained from Janvier Labs (France). Mice are kept on a 12 h light/dark cycle (07:00 - 19:00). Temperature is maintained at 22 ± 2 °C, food and water are provided *ad libitum.*

### *5.6.3. Study design*

The design of the study is adapted from Li M. et al. (Li et al., 2006).

On the first day (D1), the mice are anesthetized with an intraperitoneal injection of Imalgene and Rompun (7.5% / 2.5%; 0.1 mL/10 g) and shaved around the two ears.

As of D1, either 20 μL EtOH or 2 nmol of MC903 (in 20 μL EtOH) are topically applied on both ears of mice up to D9, D11 or D15 (except during the weekend).

From D5, the mice are dosed with test compound (15 or 30 mg/kg, *p.o., b.i.d.* in methylcellulose 0.5%) or dexamethasone (5 mg/kg, *p.o.*, *q.d.* in methylcellulose 0.5%), or with vehicle, until D10, D12, or D16.

### *5.6.4. Quantification of compound levels in plasma*

Plasma concentrations of each test compound are determined by an LC-MS/MS method in which the mass spectrometer is operated in positive or negative electrospray mode.

### *5.6.5. Determination of pharmacokinetic parameters*

Pharmacokinetic parameters are calculated using Phoenix® WinNonlin® (Pharsight®, United States).

### *5.6.6. Assessment of disease*

The thickness of both ears is measured (after anaesthesia induced by isoflurane inhalation), prior to application of MC903, at initiation of the study, three times a week and at sacrifice using a thickness gage (Mitutoyo, Absolute Digimatic, 547-321).

Body weight is assessed at initiation of the study, three times a week and at sacrifice.

On D8, D10 or D11, mice from all groups receive ProSense® 680 probe (0.8 nmol/10 g, IP). On the next day (D9, D11 or D12), the mice are anesthetized with an intraperitoneal injection of Imalgene and Rompun (7.5% / 2.5%; 0.1 mL/10 g). Granulocyte infiltration is then measured using in vivo molecular imaging (Bruker In-Vivo Xtreme imaging system, excitation wavelength: 630 nm, emission wavelength: 700 nm, acquisition time: 5 seconds).

On D10, D12, or D16, 2 h after the last dosing, the mice are sacrificed; total blood is collected on EDTA-coated tubes and plasma is frozen for further measurements (including circulating compound).

The pinnae of the ears are collected. One ear is cut longitudinally into 2 halves. One half is fixed in formaldehyde buffer 4% for histology; the other one is immersed in RNA*later*® to assess gene expression.

There are 8 mice per group. The results are expressed as mean ± SEM and statistical analysis is performed using one-way ANOVA followed by Dunnett's post-hoc test versus MC903 vehicle groups for ear thickness and weight, versus EtOH vehicle group for body weight.

### *5.6.7. Histology*

After sacrifice, half ears are collected and fixed in 3.7% formaldehyde before embedding in paraffin. 4 μm thick sections are immunostained by immunohistochemistry with anti-CD3 antibody. The immunostained cell areas from a whole section per mouse are measured by image analysis (CaloPix software, TRIBVN Healthcare). Data are expressed as mean ± SEM and statistical analysis is performed using one-way ANOVA followed by Dunnett's post-hoc test versus MC903 vehicle groups.

### *5.6.8. Gene expression analysis*

Ears are removed from *RNAlater*® solution and placed in Trizol® after disruption with 1.4 mm ceramic beads in a Bertin Instruments Precellys® homogenizer. Total RNA is then extracted using a phenol/chloroform protocol and purified with a QIAcube using an RNeasy® 96 QIAcube® HT Kit (Qiagen, cat no. 74171). cDNA is prepared and quantitative PCR performed with gene-specific primers from Qiagen using SYBR Green technology in a ViiA 7 real-time PCR system (Applied Biosystems). Expression levels of each gene of interest (GOI = IL4, IL5, IL13, TSLP, IL33, ST2, IL25, IL31,

IFN$\gamma$, IL6, IL10, LCN2, S100A8 and S100A9) are calculated relative to the HPRT, GAPDH and $\beta$-actin housekeeping gene expression levels.

All qPCR data are expressed as mean ± SEM of the normalized relative quantity (NRQ = 2^($\Delta$Cq GOI)/Geomean (2^($\Delta$Cq HPRT), 2^($\Delta$Cq GAPDH), 2^($\Delta$Cq $\beta$-actin)) where $\Delta$Cq = Cq average - Cq sample. The statistical test used is ANOVA analysis of variance with Dunnett's post-hoc test versus the EtOH/MC903 vehicle group.

### *5.7. Murine model of systemic lupus erythematosus induced by epicutaneous applications of imiquimod*

#### *5.7.1. Materials*

Aldara® 5% imiquimod cream is obtained from MEDA.

Mouse anti-double-stranded DNA antibodies ELISA kits are obtained from Alpha Diagnostic International (cat no. 5120). Mouse urinary albumin ELISA kits are obtained from Abcam (cat no. ab108792). Urine creatinine assay kits are obtained from Abnova (cat no. KA4344).

#### *5.7.2. Animals*

BALB/cJ mice (female, 18-20 g body weight) are obtained from Janvier Labs (France). Mice are kept on a 12 h light/dark cycle (07:00 - 19:00). Temperature is maintained at 22 ± 2 °C, food and water are provided *ad libitum.*

#### *5.7.3. Study design*

The design of the study is adapted from Yokogawa M. et al. (Yokogawa et al., 2014).

On the first day (D1), the mice are shaved around the right ears.

The mice receive an epicutaneous application of 1.25 mg of imiquimod 3 times per week on the right pinna ear for 12 consecutive weeks (D1 to D86). The control group receives the same quantity of vaseline.

From D1 to D86, mice are dosed with test compound (30 mg/kg, p.o., q.d. in methylcellulose 0.5%) or with vehicle (10 mL/kg).

#### *5.7.4. Assessment of disease*

The thickness of the ears is measured once a week with an automatic gage (Mitutoyo, Absolute Digimatic, 547-321).

Body weight is assessed at initiation and once a week until sacrifice. At necropsy, the spleen weight is also measured. The mice are sacrificed 2 h after the last dosing.

At different time points (e.g., on days D28, D56 and D84), the mice are individually placed in a metabolic cage to perform urinalysis and assess proteinuria (albumin to creatinine ratio).

Serums are collected at different time points (e.g., on D28, D56 and D86) to assess anti-double stranded-DNA IgG levels.

At D13, blood samples are also collected from the retro-orbital sinus for PK profiling just before dosing (T0) and 1 h, 3 h, 6 h post-dosing.

There are 8-19 mice per group. The results are expressed as mean ± SEM and statistical analysis is performed using one-way ANOVA followed by Dunnett's post-hoc test versus imiquimod vehicle groups.

#### *5.7.5. Quantification of compound levels in plasma*

Plasma concentrations of each test compound are determined by an LC-MS/MS method in which the mass spectrometer is operated in positive or negative electrospray mode.

#### *5.7.6. Determination of pharmacokinetic parameters*

Pharmacokinetic parameters are calculated using Phoenix® WinNonlin® (Pharsight®, United States).

#### *5.7.7. Histology*

After sacrifice, left kidneys are collected and cut longitudinally into 2 parts. One part is fixed in 3.7% formaldehyde before embedding in paraffin. 4 $\mu$m thick sections are made and stained with Period acid-Schiff (PAS) or immunostained with CD3 (T cells), CD20 (B cells) and F4/80 (macrophages).

*5.7.7.1. Histopathology*

In each glomerulus, 4 different readouts including mesangioproliferation, endocapillary proliferation, mesangial matrix expansion and segmental sclerosis are graded on a scale of 0 to 2 and then summed. For each kidney, about 50 glomeruli are scored and then averaged giving one glomerular lesion score (Yokogawa et al., 2014). Data are expressed as mean ± SEM and statistical analysis is performed using the Kruskal-Wallis test followed by Dunn's post-hoc test versus imiquimod vehicle group.

*5.7.7.2. Cellular quantifications*

For each cell type, immunohistochemical analysis is performed using image analysis (CaloPix software, TRIBVN Healthcare) on the whole tissue section at a magnification of x20. Data are expressed as mean ± SEM and statistical analysis is performed using one-way ANOVA followed by Dunnett's post-hoc test versus imiquimod vehicle group.

*5.7.7.3. Gene expression analysis*

At sacrifice, the second part of the left kidneys is placed in tubes containing 1.4 mm ceramic beads and disrupted in 1% DTT RLT lysis buffer (Qiagen, cat no. 79216) with a Bertin Instruments Precellys® homogenizer. Total RNA is then purified with a QIAcube using an RNeasy® 96 QIAcube® HT Kit (Qiagen, cat no. 74171). cDNA is prepared and quantitative PCR performed with gene-specific primers from Qiagen using SYBR Green technology in a ViiA 7 real-time PCR system (Applied Biosystems). Expression levels of each gene of interest (GOI = CD3, CD68, CD20, OAS1, Mx1, IFIT1, CXCL11 and Usp18) are calculated relative to the cyclophilin, GAPDH and β-actin housekeeping gene expression levels.

At sacrifice, one-third of the spleen is placed into tubes containing 1.4 mm ceramic beads and disrupted in Trizol® with a Bertin Instruments Precellys® homogenizer. Total RNA is extracted using a phenol/chloroform process and then purified with a QIAcube using an RNeasy® 96 QIAcube® HT Kit (Qiagen, cat no. 74171). cDNA is prepared and quantitative PCR performed with gene-specific primers from Qiagen using SYBR Green technology in a ViiA 7 real-time PCR system (Applied Biosystems). Expression levels of each gene of interest (GOI = CD20, IRF7, OAS1, Mx1, IFIT1, CXCL11, Usp18, BCL6, CXCL13, $CXCR^5$, MAF, ICOSL, PDCD1, SH2D1a) are calculated relative to the cyclophilin, GAPDH and β-actin housekeeping gene expression levels.

All qPCR data are expressed as mean ± SEM of the normalized relative quantity (NRQ = 2^(ΔCq GOI)/Geomean (2^(ΔCq cyclophilin), 2^(ΔCq GAPDH), 2^(ΔCq β-actin)) where ΔCq= Cq average - Cq sample. The statistical test used is ANOVA analysis of variance with Dunnett's post-hoc test versus imiquimod vehicle group.

***5.8. Systemic lupus erythematosus (SLE) model in MRL/MpJ-Faslpr/Jmice***

The purpose of this study is to evaluate the activity of test compounds of the invention in the treatment of systemic lupus erythematosus (SLE).

***5.8.1. Materials***

The test compounds are stored as dry matters in the dark and formulated weekly as suspensions using magnetic stirring in the vehicle solution (aqueous methyl cellulose 5%). The resulting suspensions are kept under magnetic stirring protected from light.

***5.8.2. Animals***

MRL/MpJ mice (female, 20-week old) and MRL/MpJ-Faslpr/J mice (female, 8-week old) are obtained from the Jackson laboratory (USA). The mice are 28 weeks old at the time of first treatment.

***5.8.3. Study design***

At 27 weeks old (study day 0), the mice with developing disease are randomized and animal body weight into each group.

Treatment is initiated after randomization when the animals are 28 weeks old and continued until the animals are sacrificed at 39 weeks of age.

The animals are observed daily for significant clinical signs, morbidity and mortality.

The activity of test compounds of the invention is evaluated based on weight, proteinuria levels, tissue weights

at necropsy (kidney, spleen, and lymph nodes); anti-dsDNA Ab, Igs, cytokine/chemokine and gene expression levels; and histopathology and immunohistochemistry.

The study is carried out on the following groups (15 mice/group):

| Group (n=15) | Treatment | Dose Level (mg/kg) | Dose Route | Regimen | Dose Vol (mL/kg)3 | Dose Conc (mg/mL) |
|---|---|---|---|---|---|---|
| 1 | Non-Diseased Vehicle Control | N/A | PO | BID* | 5 | N/A |
| 2 | Diseased Vehicle Control | N/A | PO | BID* | 5 | N/A |
| 3 | DEX Positive Control | 1 | PO | QD* | 10 | 0.1 |
| 4 | Test compound | 10 | PO | BID* | 5 | 2.0 |
| *BID dosing to occur at approximately 10-12 h intervals - QD dosing at approximately 24 h intervals The test compound doses to be administered are calculated daily in mg/kg based on the latest body weight of the animal | | | | | | |

### *5.8.4. End points*

Proteinuria score is recorded for all animals once a week starting on week 28 until week 39, from fresh urine samples using colorimetric Albustix test strips (Siemens cat# 2872A).

The resulting score is obtained matching the color to the code scale within 1 to 2 min from sampling, giving the following endpoints:

Capture urine on an Albustix test strip and determine score by matching to color code on bottle between 1 and 2 min later.

| | |
|---|---|
| 0 = | none |
| 1 = | 1 to 30 mg/dL |
| 2 = | 3 1 to 99 mg/dL |
| 3 = | 100 to 299 mg/dL |
| 4 = | 300 to 1999 mg/dL |
| 5 = | > 2000 mg/dL |

Body weight is recorded once a week for all animals from week 28 to week 39.

Blood is collected under anesthesia on week 27, 33 and 38 for all animals for blood ds DNAAb and Igs.

Blood is collected for PK analysis in the test compound treated animal group on week 29 at the following time points; pre-dose 0 h, 0.25 h, 1h and 6 h.

anti-dsDNA Ab, Igs, cytokine/chemokine and gene expression levels; and histopathology and immunohistochemistry analysis

tissue weights at necropsy (kidney, spleen, and lymph nodes); anti-dsDNA Ab (ELISA (Alpha Diagnostics Cat. # 5120), Igs (Luminex BBP, EMD Millipore Cat. # Mouse MGAMMAG-300K), cytokine/chemokine (ELISA) and gene expression levels; and histopathology and immunohistochemistry.

### *5.8.5. Statistical analysis*

Based on individual animal raw data, the means for each group are determined and percent change from disease controls is calculated. Treatment groups are compared to disease controls using a one-way analysis of variance (1-way ANOVA) with a Dunnett's post-hoc analysis for measured (parametric) data or a Kruskal-Wallis test with a Dunn's post-hoc analysis for scored (non-parametric) data.

Data is reported as 1) all animals including those that died interim and 2) only animals that survived to study termination (surviving animals). Statistical analysis is performed using Prism 6.0d software (GraphPad).

Significance for all tests is set at $p < 0.05$, and p values are rounded to the third decimal place. Percent inhibition is calculated using the following formula:

$$percentage\ change = \frac{\text{mean [treated]} - \text{mean [disease control]}}{0 - \text{mean [disease control]}} * 100$$

### *5.9. Murine model of psoriatic arthritis induced by overexpression of IL-23*

#### *5.9.1. Materials*

Mouse IL-23 enhanced episomal expression vector (EEV) is obtained from System Biosciences (cat no. EEV651A-1). Ringers solution tablets are obtained from Sigma-Aldrich (cat no. 96724-100TAB). Mouse IL-23 Quantikine ELISA Kits are obtained from R&D Systems (cat no. M2300). ProSense® 680 and OsteoSense® 750EX are obtained from PerkinElmer (cat no. NEV10003 and NEV10053EX). RNA*later*® is obtained from Ambion (cat no. AM7021). Imalgene® 1000 (Merial) and Rompun® 2% (Bayer) are obtained from Centravet (cat no. IMA004-6827812 and ROM001-6835444).

#### *5.9.2. Animals*

B10.RIII mice (male, 8-week old) are obtained from Charles River (France). Mice are kept on a 12 h light/dark cycle (07:00 - 19:00). Temperature is maintained at 22 ± 2°C, food and water are provided *ad libitum.*

#### *5.9.3. Study design*

The design of the study is adapted from Sherlock JP. et al..

On the first day (D1), the mice undergo a hydrodynamic injection of Ringer or IL-23 EEV in Ringer into the tail vein (3 μg/2.1 mL, IV injected over a period of 4-6 sec).

As of D5, twice a week, the mice are scored for clinical symptoms until the end of the experiment.

On D5, blood is collected by puncture in the submandibular vein to assess the serum IL-23 concentration.

On D9, mice from all groups receive ProSense® 680 probe (0.8 nmol/10 g, IP). On D10, the mice are anesthetized with an intraperitoneal injection of Imalgene and Rompun (7.5% / 2.5%; 0.1 mL/10 g). Granulocyte infiltration is then measured using in vivo molecular imaging (Bruker In-Vivo Xtreme imaging system, excitation wavelength: 630 nm, emission wavelength: 700 nm, acquisition time: 5 seconds).

On D11, randomization is performed according to ProSense® 680 molecular imaging and scoring.

As of D12, mice are dosed with test compound (30 mg/kg, *p.o.*, *b.i.d.* in methylcellulose 0.5%) or with vehicle.

On D19, blood is sampled at time T0, T1h, T3h and T6h after last dosing. Plasma is separated and kept at 20 °C until bioanalysis.

On D36, mice from all groups are sacrificed 2 h after last administration of compound. The following is collected:

Heels around enthesis (without skin) of the left hindlimb are immediately snap frozen in Precellys tubes. Fingers are collected in tubes containing RNA*later*®. The right hindlimb is immediately fixed in formaldehyde buffer 4% for histology evaluation. X-ray measurement is performed 48 h after fixation.

One ear is collected in tube containing RNA*later*® for transcript analysis.

Total blood is collected in a serum blood tube and mixed by gentle inversion 8-10 times. After clotting, blood samples are centrifuged 10 min at 1800 × g. After centrifugation, serum is stored at -80 °C.

Part of the colon (1 cm distal colon) is immediately snap frozen in Precellys tube for transcript analysis. Another part (1 cm distal colon) is immediately fixed in formaldehyde buffer 4% for further histology analysis.

#### *5.9.4. Assessment of disease*

Body weight is assessed at initiation of the study, then twice a week and at sacrifice.

Twice weekly, clinical signs of inflammation are scored: 0 for normal paw; 1 if swelling of one digit; 2 if swelling of two or more digits; 3 if swelling of the entire paw. The scores of all limbs are summed up to produce a global score.

On D23, mice from all groups receive ProSense® 680 probe (0.8 nmol/10 g, IP). On D24, the mice are anesthetized with an intraperitoneal injection of Imalgene and Rompun (7.5% / 2.5%; 0.1 mL/10 g). Granulocyte infiltration is then measured using in vivo molecular imaging (Bruker In-Vivo Xtreme imaging system, excitation wavelength: 630 nm, emission wavelength: 700 nm, acquisition time: 5 seconds).

On D32, mice from all groups receive ProSense® 680 probe (0.8 nmol/10 g, IP) and OsteoSense® 750EX probe (0.8 nmol/10 g, IP). On D33, the mice are anesthetized with an intraperitoneal injection of Imalgene and Rompun (7.5% / 2.5%; 0.1 mL/10 g). Granulocyte infiltration and bone remodelling are measured using in vivo molecular imaging (Bruker In-Vivo Xtreme imaging system; excitation wavelength: 630 nm, emission wavelength: 700 nm, acquisition time: 5

seconds for ProSense® 680 probe; excitation wavelength: 720 nm, emission wavelength: 790 nm, acquisition time: 5 seconds for OsteoSense® 750EX probe).

There are 10 mice per group. The results are expressed as mean ± SEM and statistical analysis is performed using one-way ANOVA followed by Dunnett's post-hoc test versus diseased vehicle group for scoring and imaging analysis, versus sham vehicle group for body weight.

### *5.10. Murine sclerodermatous chronic graft-versus host disease (cGvHD)*

#### *5.10.1. General overview*

In this cGvHD model, fibrosis is induced in BALB/c (H-2d) mice by allogeneic transplantation of bone marrow cells and splenocytes from B10.D2 (H-2d) donor mice (minor HLA mismatch). The recipient mice develop inflammation-driven dermal and pulmonary fibrosis resembling patients with rapidly progressive diffuse cutaneous systemic sclerosis. (Zerr et al., 2012)

The treatment is provided only after the onset of first clinical symptoms of sclerodermatous cGvHD.

#### *5.10.2. Study groups*

The following groups with each eight mice were used in this study

- *Syngeneically transplanted, placebo-treated control group:*

  Syngeneic bone marrow and splenocyte transplantation (BALB/c (H-$2^d$) → BALB/c (H-$2^d$)).
  Application of the solvent methyl cellulose 0.5% from day 21 to day 56 post transplantation.

- *Vehicle-treated fibrosis group:*

  Allogeneic bone marrow and splenocyte transplantation (B 10.D2 (H-2d) → BALB/c (H-2d)).
  Application of the solvent methyl cellulose 0.5% from day 21 to day 56 post transplantation

- *Control group to assess pretreatment levels of fibrosis induced by allogeneic transplantation:*
  Allogeneic bone marrow and splenocyte transplantation (B10.D2 (H-$2^d$) → BALB/c (H-$2^d$)). Sacrifice at day 21, before treatment was initiated in the other groups.
- *Treatment group:*

  Allogeneic bone marrow and splenocyte transplantation (B10.D2 (H- $2^d$) → BALB/c (H-$2^d$)).
  Application of a test compound of the invention at 10 mg/kg po bid in 0.5% Methylcellulose from day 21 to day 56 post transplantation.

- *Positive control group:*

  Allogeneic bone marrow and splenocyte transplantation (B10.D2 (H-$2^d$) → BALB/c (H-$2^d$)).
  Application of 50 mg/kg qd Nintedanib from day 21 to day 56 post transplantation.

#### *5.10.3. Steady state PK:*

On D20, for the group receiving test compounds, blood was collected from the tail vein from 2 animals per timepoint, at the following timepoints, pre-dose, 1, 3 and 6 h with anticoagulant Li- Heparin.

The blood samples were kept on ice and centrifuged at approx. 3500 × g, for 10 min at +4 °C, within 1 h after blood sampling; plasma was transferred in labelled polypropylene tubes stored at -20 °C.

#### *5.10.4. Sampling and analysis*

Animals are sacrificed 2 h (Tmax + 1 h) post-last dose, and samples for skin (3 mm punch biopsies), lung, spleen and blood were collected for histology and gene expression analysis

#### *5.10.5. Main readouts*

The anti-fibrotic effects on skin were analysed by determination of dermal thickness, quantification of lesional collagen and staining for myofibroblasts.

In case of positive effects on skin fibrosis, effects on pulmonary fibrosis were analysed by Ashcroft scoring, by hydroxyproline content and by quantification of the collagen covered area using SirCol staining.

#### *5.10.5.1. Results*

Based on individual animal raw data, the means for each group were determined and percent change from disease controls was calculated. Treatment groups were compared to disease controls using a one-way analysis of variance (1-way ANOVA) with a Dunnett's post-hoc analysis for measured (parametric) data or a Kruskal-Wallis test with a Dunn's post-hoc analysis for scored (non-parametric) data.

When tested at 10 mg/kg b.i.d. following the above protocol, compound 1, showed a statistically significant effect on skin (Hydroxyproline) and Lung fibrosis (Ashcroft, SirCol).

### *5.11. MIA rat Pain Model*

#### *5.11.1. Principle of the assay*

The mono-iodoacetate (MIA) model has become a standard model established by Van der Kraan (van der Kraan et al., 1989) for modelling joint disruption in OA in rat.

Intra-articular injection of MIA in rodents reproduces OA-like lesions and functional impairment that can be analyzed and quantified. MIA is an inhibitor of glyceraldehyde-3-phosphatase, disrupting cellular glycolysis and eventually resulting in cell death (van der Kraan et al., 1989).

The MIA pain mouse model described by Pitcher et al. (Pitcher et al. 2016) is used to evaluate the effect of a test compound against tactile allodynia by intra-articular injection of MIA causes chondrocyte cell death, leading to cartilage degeneration and subsequent subchondral bone alterations such as appearance of bone osteophytes (Janusz et al., 2001).

MIA is the one most often used, particularly to test the efficacy of pharmacologic agents to treat pain, as this model generates a reproducible, robust, and rapid pain-like phenotype that can be graded by altering MIA dosage.

#### *5.11.2. Animals*

Male Sprague Dawley (250-300 g) were housed at 22 ± 1 °C and in light-controlled environment (lights on from 7 am to 8 pm) with *ad libitum* access to food and water.

#### *5.11.3. Protocol*

Inflammatory hypersensitivity to pain was induced by an injection of Monosodium Iodoacetate (MIA) 25ul 80mg/ml solution, into the left knee joint. Animals developped inflammatory response within 3 days post MIA.

The test compounds were administered to the treatment groups on a both in day basis, starting from day 3 (D3), and continued until the end-point day on D28.

On day 3, weight-bearing measurements were taken and animals ranked and randomized to treatment groups.

On the test days, pre-dose allodynia was evaluated prior to dosing, and the effects of the administered compounds assessed 2 hours post dosing.

The test compounds were administered to the treatment groups on daily basis, according to treatment group, (some were dosed days 3-7 post MIA, and some were dosed days 24-28 post MIA) and 2 hours post dosing weight-bearing measurements were taken.

The negative control group (n=6) was daily dosed *per os* with the vehicle (methylcellulose, MC 0.5%), the positive control group (n=10) was daily dosed *per os* with Celecoxib (50 mg/kg), and the test compound group (n=10) was daily dosed *per os,* with 0.5% MC/Solutol for days 3-7 post MIA (Inflammatory Phase).

The negative control group (n=6) was daily dosed *per os* with the vehicle (methylcellulose, MC 0.5%), the positive control group (n=10) was dosed daily *per os* with Pregabalin (30 mg/kg), and the test compound group (n=10) was daily dosed *per os.* with 0.5% MC/Solutol for days 24-28 post MIA (Neuropathic Phase).

*5.11.4. Steady state PPK*

Steady state PK blood sampling wasperformed on the 2 groups treated, at sacrifice time post last treatment, the following time points for all groups: T0 (pre dosing, n=2), T0.25h (n=3), T2h (n=3) and T6h (n=2)
Blood was sampled in Li-heparin tubes on ice and then centrifuged at +4°C and resulting plasma was frozen at -20°C pending bioanalysis.

*5.11.5. Tactile allodynia - weight-bearing deficit tests*

Weight-bearing deficit tests test approaches were used to assess the congenital (referred to as baseline) tactile allodynia levels of the animals. In order to avoid false sensitization in the test results, the mice were subjected to a sufficient habituation period and two handling procedures prior to all baseline tests. In addition, the baseline weight-bearing deficit took place at maximum of 2 days prior to the surgery.
In each test, the hind paw ipsilateral to the injury (left) was tested for each mouse

*5.11.1. Data analysis*

For each readout, mean and sem are calculated. A difference statistically significant between intact or treated groups and in MIA vehicle group is evaluated with Prism software using a Two-way ANOVA (for treatment groups) followed by a Dunnett's multiple comparisons post-hoc test. *: $p < 0.05$; **: $p < 0.01$; ***: $p < 0.001$ versus MIA Vehicle group.

*5.11.2. Results*

Compound 1 at 5 mg/kg daily *per os* significantly reversed the inflammatory response from days 5-7 post dosing on weight bearing.
Compound 1 at 30 mg/kg daily *per os* also significantly reversed the inflammatory response from days 3-7 post dosing on weight bearing.
Compound 1 at 5 mg/kg and 30 mg/kg daily *per os* significantly reversed the inflammatory response from days 26-28 post dosing on weight bearing.

**FINAL REMARKS**

It should be understood that factors such as the differential cell penetration capacity of the various compounds can contribute to discrepancies between the activity of the compounds in the *in vitro* biochemical and cellular assays.
At least some of the chemical names of compound of the invention as given and set forth in this application, may have been generated on an automated basis by use of a commercially available chemical naming software program, and have not been independently verified. Representative programs performing this function include the Lexichem naming tool sold by Open Eye Software, Inc. and the Autonom Software tool sold by MDL, Inc. In the instance where the indicated chemical name and the depicted structure differ, the depicted structure will control.

**REFERENCES**

Argilés, J.M., and Lopez-Soriano, F.J. (1998). Catabolic proinflammatory cytokines. Curr. Opin. Clin. Nutr. Metab. Care 1, 245-251.

Bain, J., McLauchlan, H., Elliott, M., and Cohen, P. (2003). The specificities of protein kinase inhibitors: an update. Biochem. J. 371, 199-204.

Brehm, M.A., Daniels, K.A., and Welsh, R.M. (2005). Rapid Production of TNF-α following TCR Engagement of Naive CD8 T Cells. J. Immunol. 175, 5043-5049.

Broekman, F., Giovannetti, E., and Peters, G.J. (2011). Tyrosine kinase inhibitors: Multi-targeted or single-targeted? World J. Clin. Oncol. 2, 80-93.

Bundgaard, H. (1985). Design of prodrugs (Elsevier).

Bush, K.A., Farmer, K.M., Walker, J.S., and Kirkham, B.W. (2002). Reduction of joint inflammation and bone erosion in rat adjuvant arthritis by treatment with interleukin-17 receptor IgG1 Fc fusion protein. Arthritis Rheum. 46, 802-805.

Carmi, Y., Dotan, S., Rider, P., Kaplanov, I., White, M.R., Baron, R., Abutbul, S., Huszar, M., Dinarello, C.A., Apte, R.N., et al. (2013). The Role of IL-1β in the Early Tumor Cell-Induced Angiogenic Response. J. Immunol. 190, 3500-3509.

Chiang, E.Y., Yu, X., and Grogan, J.L. (2011). Immune Complex-Mediated Cell Activation from Systemic Lupus Erythematosus and Rheumatoid Arthritis Patients Elaborate Different Requirements for IRAK1/4 Kinase Activity across Human Cell Types. J. Immunol. 186, 1279-1288.

Cohen, P. (2009). Targeting protein kinases for the development of anti-inflammatory drugs. Curr. Opin. Cell Biol. 21, 317-324.

Davidson, D.J., Currie, A.J., Bowdish, D.M.E., Brown, K.L., Rosenberger, C.M., Ma, R.C., Bylund, J., Campsall, P.A., Puel, A., Picard, C., et al. (2006). IRAK-4 mutation (Q293X): rapid detection, and characterisation of defective post-transcriptional TLR/IL-1R responses in human myeloid and non-myeloid cells. J. Immunol. Baltim. Md 1950 177, 8202-8211.

Dinarello, C.A., Simon, A., and van der Meer, J.W.M. (2012). Treating inflammation by blocking interleukin-1 in abroad spectrum of diseases. Nat. Rev. Drug Discov. 11, 633-652.

Dy, G.K., and Adjei, A.A. (2013). Understanding, recognizing, and managing toxicities of targeted anticancer therapies. CA. Cancer J. Clin. 63, 249-279.

Fabian, M.A., Biggs, W.H., Treiber, D.K., Atteridge, C.E., Azimioara, M.D., Benedetti, M.G., Carter, T.A., Ciceri, P., Edeen, P.T., Floyd, M., et al. (2005). A small molecule-kinase interaction map for clinical kinase inhibitors. Nat. Biotechnol. 23, 329-336.

van der Fits, L., Mourits, S., Voerman, J.S.A., Kant, M., Boon, L., Laman, J.D., Cornelissen, F., Mus, A.-M., Florencia, E., Prens, E.P., et al. (2009). Imiquimod-Induced Psoriasis-Like Skin Inflammation in Mice Is Mediated via the IL-23/IL-17 Axis. J. Immunol. 182, 5836-5845.

Force, T., and Kolaja, K.L. (2011). Cardiotoxicity of kinase inhibitors: the prediction and translation of preclinical models to clinical outcomes. Nat. Rev. Drug Discov. 10, 111-126.

Genovese, M.C., Cohen, S., Moreland, L., Lium, D., Robbins, S., Newmark, R., Bekker, P., and for the 20000223 Study Group (2004). Combination therapy with etanercept and anakinra in the treatment of patients with rheumatoid arthritis who have been treated unsuccessfully with methotrexate. Arthritis Rheum. 50, 1412-1419.

Jain, A., Kaczanowska, S., and Davila, E. (2014). IL-1 Receptor-Associated Kinase Signaling and Its Role in Inflammation, Cancer Progression, and Therapy Resistance. Front. Immunol. 5.

Janusz, M.J., Hookfin, E.B., Heitmeyer, S.A., Woessner, J.F., Freemont, A.J., Hoyland, J.A., Brown, K.K., Hsieh, L.C., Almstead, N.G., De, B., et al. (2001). Moderation of iodoacetate-induced experimental osteoarthritis in rats by matrix metalloproteinase inhibitors. Osteoarthritis Cartilage 9, 751-760.

Jou, I.-M., Shiau, A.-L., Chen, S.-Y., Wang, C.-R., Shieh, D.-B., Tsai, C.-S., and Wu, C.-L. (2005). Thrombospondin 1 as an effective gene therapeutic strategy in collagen-induced arthritis. Arthritis Rheum. 52, 339-344.

Khachigian, L.M. (2006). Collagen antibody-induced arthritis. Nat. Protoc. 1, 2512-2516.

Klein, S. (2010). The Use of Biorelevant Dissolution Media to Forecast the In Vivo Performance of a Drug. AAPS J. 72, 397-406.

Koziczak-Holbro, M., Littlewood-Evans, A., Pöllinger, B., Kovarik, J., Dawson, J., Zenke, G., Burkhart, C., Müller, M., and Gram, H. (2009). The critical role of kinase activity of interleukin-1 receptor-associated kinase 4 in animal models of joint inflammation. Arthritis Rheum. 60, 1661-1671.

van der Kraan, P.M., Vitters, E.L., van de Putte, L.B., and van den Berg, W.B. (1989). Development of osteoarthritic lesions in mice by "metabolic" and "mechanical" alterations in the knee joints. Am. J. Pathol. 135, 1001-1014.

Kroeger, K.M., Sullivan, B.M., and Locksley, R.M. (2009). IL-18 and IL-33 elicit Th2 cytokines from basophils via a MyD88- and p38α-dependent pathway. J. Leukoc. Biol. 86, 769-778.

Li, D., Guabiraba, R., Besnard, A.-G., Komai-Koma, M., Jabir, M.S., Zhang, L., Graham, G.J., Kurowska-Stolarska, M., Liew, F.Y., McSharry, C., et al. (2014). IL-33 promotes ST2-dependent lung fibrosis by the induction of alternatively activated macrophages and innate lymphoid cells in mice. J. Allergy Clin. Immunol. 134, 1422-1432.e11.

Li, M., Hener, P., Zhang, Z., Kato, S., Metzger, D., and Chambon, P. (2006). Topical vitamin D3 and low-calcemic analogs induce thymic stromal lymphopoietin in mouse keratinocytes and trigger an atopic dermatitis. Proc. Natl. Acad. Sci. 103, 11736-11741.

Li, S., Strelow, A., Fontana, E.J., and Wesche, H. (2002). IRAK-4: A novel member of the IRAK family with the properties of an IRAK-kinase. Proc. Natl. Acad. Sci. U. S. A. 99, 5567-5572.

Li, Z., Younger, K., Gartenhaus, R., Joseph, A.M., Hu, F., Baer, M.R., Brown, P., and Davila, E. (2015). Inhibition of IRAK1/4 sensitizes T cell acute lymphoblastic leukemia to chemotherapies. J. Clin. Invest. 125, 1081-1097.

Lin, H.-S., Hu, C.-Y., Chan, H.-Y., Liew, Y.-Y., Huang, H.-P., Lepescheux, L., Bastianelli, E., Baron, R., Rawadi, G., and Clément-Lacroix, P. (2007). Anti-rheumatic activities of histone deacetylase (HDAC) inhibitors in vivo in collagen-induced arthritis in rodents. Br. J. Pharmacol. 150, 862-872.

McHedlidze, T., Waldner, M., Zopf, S., Walker, J., Rankin, A.L., Schuchmann, M., Voehringer, D., McKenzie, A.N.J., Neurath, M.F., Pflanz, S., et al. (2013). Interleukin-33-dependent innate lymphoid cells mediate hepatic fibrosis. Immunity 39, 357-371.

Nabe, T. (2014). Interleukin (IL)-33: New Therapeutic Target for Atopic Diseases. J. Pharmacol. Sci. 126, 85-91.

Ngo, V.N., Young, R.M., Schmitz, R., Jhavar, S., Xiao, W., Lim, K.-H., Kohlhammer, H., Xu, W., Yang, Y., Zhao, H., et al. (2011). Oncogenically active MYD88 mutations in human lymphoma. Nature 470, 115-119.

Nishida, K., Komiyama, T., Miyazawa, S., Shen, Z.-N., Furumatsu, T., Doi, H., Yoshida, A., Yamana, J., Yamamura, M., Ninomiya, Y., et al. (2004). Histone deacetylase inhibitor suppression of autoantibody-mediated arthritis in mice via regulation of p16INK4a and p21WAF1/Cip1 expression. Arthritis Rheum. 50, 3365-3376.

Rail, L.C., and Roubenoff, R. (2004). Rheumatoid cachexia: metabolic abnormalities, mechanisms and interventions. Rheumatology 43, 1219-1223.

Rankin, A.L., Mumm, J.B., Murphy, E., Turner, S., Yu, N., McClanahan, T.K., Bourne, P.A., Pierce, R.H., Kastelein, R., and Pflanz, S. (2010). IL-33 Induces IL-13-Dependent Cutaneous Fibrosis. J. Immunol. 184, 1526-1535.

Rhyasen, G.W., and Starczynowski, D.T. (2015). IRAK signalling in cancer. Br. J. Cancer 112, 232-237.

Ringwood, L., and Li, L. (2008). The involvement of the interleukin-1 Receptor-Associated Kinases (IRAKs) in cellular signaling networks controlling inflammation. Cytokine 42, 1-7.

Rizzo, H.L., Kagami, S., Phillips, K.G., Kurtz, S.E., Jacques, S.L., and Blauvelt, A. (2011). IL-23-Mediated Psoriasis-Like Epidermal Hyperplasia Is Dependent on IL-17A. J. Immunol. 186, 1495-1502.

Salimi, M., Barlow, J.L., Saunders, S.P., Xue, L., Gutowska-Owsiak, D., Wang, X., Huang, L.-C., Johnson, D., Scanlon, S.T., McKenzie, A.N.J., et al. (2013). A role for IL-25 and IL-33-driven type-2 innate lymphoid cells in atopic dermatitis. J. Exp. Med. 210, 2939-2950.

Salvemini, D., Mazzon, E., Dugo, L., Serraino, I., De Sarro, A., Caputi, A.P., and Cuzzocrea, S. (2001). Amelioration of joint disease in a rat model of collagen-induced arthritis by M40403, a superoxide dismutase mimetic. Arthritis Rheum. 44, 2909-2921.

Seltzer, Z., Dubner, R., and Shir, Y. (1990). A novel behavioral model of neuropathic pain disorders produced in rats by partial sciatic nerve injury. Pain 43, 205-218.

Shelton, D.L., Zeller, J., Ho, W.-H., Pons, J., and Rosenthal, A. (2005). Nerve growth factor mediates hyperalgesia and cachexia in auto-immune arthritis. Pain 116, 8-16.

Sims, N.A., Green, J.R., Glatt, M., Schlict, S., Martin, T.J., Gillespie, M.T., and Romas, E. (2004). Targeting osteoclasts with zoledronic acid prevents bone destruction in collagen-induced arthritis. Arthritis Rheum. 50, 2338-2346.

Staschke, K.A., Dong, S., Saha, J., Zhao, J., Brooks, N.A., Hepbum, D.L., Xia, J., Gulen, M.F., Kang, Z., Altuntas, C.Z., et al. (2009). IRAK4 Kinase Activity Is Required for Th17 Differentiation and Th17-Mediated Disease. J. Immunol. 183, 568-577.

Sundberg, T.B., Xavier, R.J., Schreiber, S.L., and Shamji, A.F. (2014). Small-molecule control of cytokine function: new opportunities for treating immune disorders. Curr. Opin. Chem. Biol. 23, 23-30.

Treon, S.P., Xu, L., Yang, G., Zhou, Y., Liu, X., Cao, Y., Sheehy, P., Manning, R.J., Patterson, C.J., Tripsas, C., et al. (2012). MYD88 L265P Somatic Mutation in Waldenström's Macroglobulinemia. N. Engl. J. Med. 367, 826-833.

Vidal-Vanaclocha, F., Fantuzzi, G., Mendoza, L., Fuentes, A M., Anasagasti, M.J., Martin, J., Carrascal, T., Walsh, P., Reznikov, L.L., Kim, S.-H., et al. (2000). IL-18 regulates IL-1β-dependent hepatic melanoma metastasis via vascular cell adhesion molecule-1. Proc. Natl. Acad. Sci. 97, 734-739.

Walsmith, J., Abad, L., Kehayias, J., and Roubenoff, R. (2004). Tumor necrosis factor-alpha production is associated with less body cell mass in women with rheumatoid arthritis. J. Rheumatol. 31, 23-29.

Wang, Z., Wesche, H., Stevens, T., Walker, N., and Yeh, W.-C. (2009). IRAK-4 Inhibitors for Inflammation. Curr. Top. Med. Chem. 9, 724-737.

Yan, C., Zhu, M., Staiger, J., Johnson, P.F., and Gao, H. (2012). C5a-regulated CCAAT/Enhancer-binding Proteins β and δ Are Essential in Fcγ Receptor-mediated Inflammatory Cytokine and Chemokine Production in Macrophages. J. Biol. Chem. 287, 3217-3230.

Yokogawa, M., Takaishi, M., Nakajima, K., Kamijima, R., Fujimoto, C., Kataoka, S., Terada, Y., and Sano, S. (2014). Epicutaneous Application of Toll-like Receptor 7 Agonists Leads to Systemic Autoimmunity in Wild-Type Mice: A New Model of Systemic Lupus Erythematosus. Arthritis Rheumatol. 66, 694-706.

Zegzouti, H., Zdanovskaia, M., Hsiao, K., and Goueli, S.A. (2009). ADP-Glo: A Bioluminescent and Homogeneous ADP Monitoring Assay for Kinases. ASSAY Drug Dev. Technol. 7, 560-572.

Zerr, P., Distler, A., Palumbo-Zerr, K., Tomcik, M., Vollath, S., Dees, C., Egberts, F., Tinazzi, I., Del Galdo, F., Distler, O., et al. (2012). Combined Inhibition of c-Abl and PDGF Receptors for Prevention and Treatment of Murine Sclerodermatous Chronic Graft-versus-Host Disease. Am. J. Pathol. 181, 1672-1680.

(1985). Remington's pharmaceutical sciences (Easton, Pa: Mack).

(2003). Public statement on the increased risk of serious infection and neutropenia in patients trested concurrently with Kineret (anakinra) and Enbrel (etanercept) (European Medicines Agency).

## Claims

**1.** A compound according to Formula I:

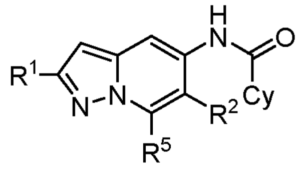

I

wherein

$R^1$ is

a) $C_{2-6}$ alkyl substituted with one or more independently selected -OH, -CN, $C_{1-4}$ alkoxy, halo, or $-S(=O)_2-C_{1-4}$ alkyl, or
b) 6 membered heterocycloalkyl comprising one or two independently selected S, N, or O atoms, which heterocycloalkyl is unsubstituted or substituted with one or more independently selected oxo, halo, or $C_{1-4}$ alkyl, which alkyl is unsubstituted or substituted with one or more halo;

$R^2$ is

a) $C_{1-4}$ alkoxy which alkoxy is unsubstituted or substituted with one or more independently selected halo or -OH,
b) $-O-C_{3-4}$ cycloalkyl, which cycloalkyl is unsubstituted or substituted with one or more independently selected halo or -OH, or
c) $-C(=O)NR^{3a}R^{3b}$;

Cy is 6 membered heteroaryl, comprising 1 or 2 N atoms, substituted with one or two independently selected $R^4$ substituents;
each $R^{3a}$ and $R^{3b}$ is independently selected from

a) H,
b) $C_{1-4}$ alkyl, which alkyl is unsubstituted or substituted with one or more independently selected halo, -OH, -CN, $C_{1-4}$ alkoxy, or $C_{3-7}$ cycloalkyl, which cycloalkyl is unsubstituted or substituted with one or more independently selected halo,
c) $C_{3-6}$ cycloalkyl which cycloalkyl is unsubstituted or substituted with one or more independently selected oxo, -OH, -CN, $C_{1-4}$ alkyl, $C_{1-4}$ alkoxy, or halo, or
d) 4-6 membered heterocycloalkyl comprising one or two independently selected N, S, or O atoms, which heterocycloalkyl is unsubstituted or substituted with one or more independently selected oxo, -OH, -CN, $C_{1-4}$ alkyl, $C_{1-4}$ alkoxy, or halo; or

$R^{3a}$ and $R^{3b}$ together with the N atom to which they are attached form a 4-6 membered monocyclic heterocycloalkyl;
each $R^4$ is independently

a) oxo,
b) -OH,
c) -CN,
d) halo,
e) $C_{1-4}$ alkyl unsubstituted or substituted with one or independently selected more halo, -OH, or -CN,
f) $C_{1-4}$ alkoxy unsubstituted or substituted with one or more independently selected halo, -OH, or - CN, or
g) $C_{3-7}$ cycloalkyl unsubstituted or substituted with one or more independently selected halo, -OH or -CN; and

$R^5$ is selected from H, halo, $-CH_3$ or $-CF_3$;
or a pharmaceutically acceptable salt thereof, or a solvate or the salt of the solvate thereof.

**2.** The compound or pharmaceutically acceptable salt thereof according to claim 1, wherein $R^1$ is $C_{2-6}$ alkyl substituted with one or more independently selected -OH, -CN, $-OCH_3$, F, Cl, or $-S(=O)_2CH_3$.

**3.** The compound or pharmaceutically acceptable salt thereof according to claim 1, wherein $R^1$ is tetrahydropyranyl, dioxanyl, morpholinyl, piperidinyl, piperazinyl, thiomorpholinyl, or 1,4-oxathianyl.

**4.** The compound or pharmaceutically acceptable salt thereof according to claim 1, wherein the compound is according to Formula IIa or IIb:

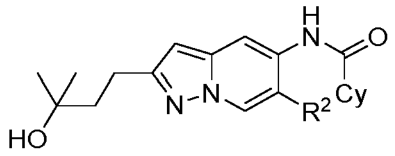

IIa,

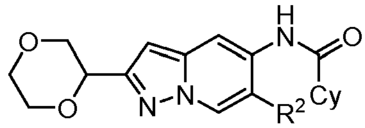

IIb

**5.** The compound or pharmaceutically acceptable salt thereof according to any one of claims 1-4, wherein $R^2$ is $-OCH_3$, or $-OCH_2CH_3$, each of which is unsubstituted or substituted with one or more independently selected halo or -OH.

**6.** The compound or pharmaceutically acceptable salt thereof according to any one of claims 1-4, wherein $R^2$ is $-C(=O)NR^{3a}R^{3b}$.

**7.** The compound or pharmaceutically acceptable salt thereof according to claim 6, wherein $R^{3a}$ is H, $-CH_3$, $-CH_2-CH_3$, or $-CH(CH_3)_2$.

**8.** The compound or pharmaceutically acceptable salt thereof according to claim 6 or 7, wherein $R^{3b}$ is H, $-CH_3$, $-CH_2-CH_3$, or $-CH(CH_3)_2$.

**9.** The compound or pharmaceutically acceptable salt thereof according to claim 1, wherein the compound is according to Formula IIIa, IIIb IIIc, IVa, IVb, or IVc:

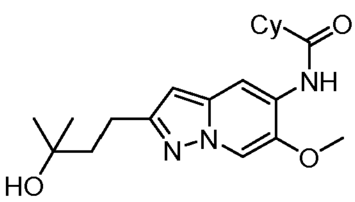

IIIa,

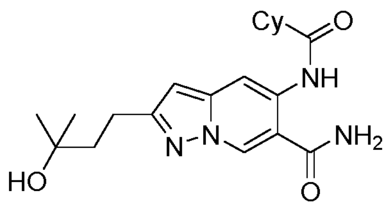

IIIb,

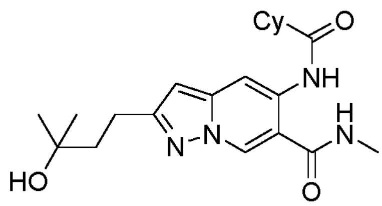

IIIc,

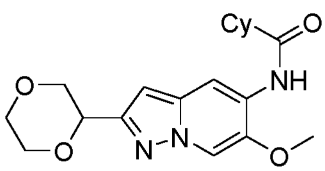

Iva,

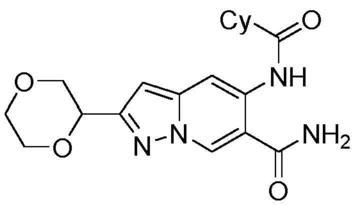

IVb,

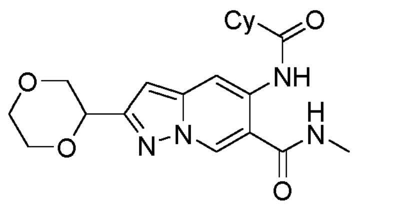

IVc.

**10.** The compound or pharmaceutically acceptable salt thereof according to any one of claims 1-9, wherein Cy is pyridinyl, or pyrazinyl, each of which is substituted with one or two independently selected $R^4$ substituents.

**11.** The compound or pharmaceutically acceptable salt thereof according to claim 10, wherein each $R^4$ is independently selected from oxo, -OH, -CN, F, Cl, $-CH_3$, $-CH_2-CH_3$, $-CH(CH_3)_2$, $-CF_3$, $-CHF_3$, $-CH_2CF_3$, $-CH_2CN$, $-CH_2OH$, $-CH_2CH_2-CN$, $-O-CH_2-CH_3$, cyclopropyl, cyclobutyl, cyclopropyl substituted with one or two independently selected F, or -CN, cyclobutyl substituted with one or two independently selected -F, -OH, or -CN.

**12.** A pharmaceutical composition comprising a compound according to any one of claims 1-11, and a pharmaceutically

acceptable carrier thereof.

13. The pharmaceutical composition according to claim 12, further comprising a further therapeutic agent.

14. A compound or pharmaceutically acceptable salt according to claims 1-11, or a pharmaceutical composition according to claim 12 or 13, for use in medicine.

15. A compound or pharmaceutically acceptable salt according to claims 1-11, or a pharmaceutical composition according to claim 12 or 13, for use in the prophylaxis and/or treatment of inflammatory diseases, autoimmune diseases, pain, fibrosis and/or proliferative diseases.

**Patentansprüche**

1. Verbindung gemäß Formel I:

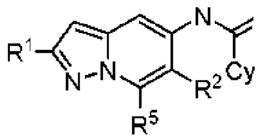

wobei

$R^1$

a) $C_{2-6}$-Alkyl, substituiert mit einem oder mehreren unabhängig voneinander ausgewählten -OH, -CN, $C_{1-4}$-Alkoxy, Halogen oder -S(=0)$_2$-$C_{1-4}$-Alkyl, oder
b) 6-gliedriges Heterocycloalkyl ist, umfassend ein oder zwei unabhängig voneinander ausgewählte S-, N- oder O-Atome, wobei das Heterocycloalkyl unsubstituiert oder substituiert ist mit einem oder mehreren unabhängig voneinander ausgewählten Oxo, Halogen oder $C_{1-4}$-Alkyl, wobei das Alkyl unsubstituiert oder substituiert ist mit einem oder mehreren Halogenen;

$R^2$

a) $C_{1-4}$-Alkoxy, wobei das Alkoxy unsubstituiert oder mit einem oder mehreren unabhängig voneinander ausgewählten Halogenen oder -OH substituiert ist,
b) -O-$C_{3-4}$-Cycloalkyl, wobei das Cycloalkyl unsubstituiert oder mit einem oder mehreren unabhängig voneinander ausgewählten Halogenen oder -OH substituiert ist, oder
c) -C(=0)$NR^{3a}R^{3b}$ ist;

Cy ein 6-gliedriges Heteroaryl ist, das 1 oder 2 N-Atome umfasst und mit einem oder zwei unabhängig voneinander ausgewählten $R^4$-Substituenten substituiert ist;
jedes $R^{3a}$ und $R^{3b}$ unabhängig ausgewählt ist aus

a) H,
b) C1-4-Alkyl, wobei das Alkyl unsubstituiert oder mit einem oder mehreren unabhängig ausgewählten Halogenen, -OH, -CN, $C_{1-4}$-Alkoxy oder $C_{3-7}$-Cycloalkyl substituiert ist, wobei das Cycloalkyl unsubstituiert oder mit einem oder mehreren unabhängig ausgewählten Halogenen substituiert ist,
c) $C_{3-6}$-Cycloalkyl, wobei das Cycloalkyl unsubstituiert oder mit einem oder mehreren unabhängig voneinander ausgewählten Halogenatomen, -OH, -CN, $C_{1-4}$-Alkyl, C1-4-Alkoxy oder Halogen substituiert ist, oder
d) 4-6-gliedriges Heterocycloalkyl, umfassend ein oder zwei unabhängig voneinander ausgewählte N-, S- oder O-Atome, wobei das Heterocycloalkyl unsubstituiert oder mit einem oder mehreren unabhängig voneinander ausgewählten Oxo-, -OH-, -CN-, $C_{1-4}$-Alkyl-, $C_{1-4}$-Alkoxy- oder Halogenatomen substituiert ist; oder

$R^{3a}$ und $R^{3b}$ zusammen mit dem N-Atom, an das sie gebunden sind, ein 4-6-gliedriges monocyclisches Heterocycloalkyl bilden;

jedes $R^4$ unabhängig

a. Oxo,
b. -OH,
c. -CN,
d. Halo,
e. $C_{1-4}$-Alkyl, unsubstituiert oder substituiert mit einem oder mehreren unabhängig voneinander ausgewählten Halogenen, -OH oder -CN,
f. $C_{1-4}$-Alkoxy, unsubstituiert oder substituiert mit einem oder mehreren unabhängig voneinander ausgewählten Halogenen, -OH oder -CN, oder
g. $C_{3-7}$-Cycloalkyl, unsubstituiert oder substituiert mit einem oder mehreren unabhängig voneinander ausgewählten Halogenatomen, -OH oder -CN ist; und

$R^5$ ausgewählt ist aus H, Halo, -CH3 oder -CF3;
oder ein pharmazeutisch annehmbares Salz davon, oder ein Solvat oder das Salz des Solvats davon.

**2.** Verbindung oder pharmazeutisch annehmbares Salz davon nach Anspruch 1, wobei $R^1$ $C_{2-6}$-Alkyl ist, das mit einem oder mehreren unabhängig voneinander ausgewählten -OH, -CN, $-OCH_3$, F, Cl oder $-S(=0)_2CH_3$ substituiert ist.

**3.** Verbindung oder pharmazeutisch annehmbares Salz davon nach Anspruch 1, wobei $R^1$ Tetrahydropyranyl, Dioxanyl, Morpholinyl, Piperidinyl, Piperazinyl, Thiomorpholinyl oder 1,4-Oxathianyl ist.

**4.** Verbindung oder pharmazeutisch annehmbares Salz davon nach Anspruch 1, wobei die Verbindung der Formel IIa oder IIb entspricht:

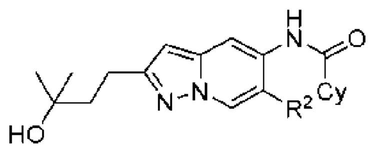
IIa,

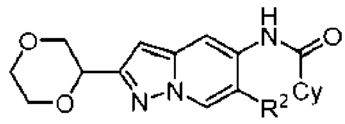
IIb

**5.** Verbindung oder pharmazeutisch verträgliches Salz davon nach einem der Ansprüche 1 bis 4, wobei $R^2$ $-OCH_3$ oder $-OCH_2CH_3$ ist, die jeweils unsubstituiert oder mit einem oder mehreren unabhängig voneinander ausgewählten Halogenen oder -OH substituiert sind.

**6.** Verbindung oder pharmazeutisch verträgliches Salz davon nach einem der Ansprüche 1-4, wobei $R^2$ $-C(=0)NR^{3a}R^{3b}$ ist.

**7.** Verbindung oder pharmazeutisch verträgliches Salz davon nach Anspruch 6, wobei $R^{3a}$ H, - $CH_3$, $-CH_2-CH_3$ oder $-CH(CH_3)_2$ ist.

**8.** Verbindung oder pharmazeutisch verträgliches Salz davon nach Anspruch 6 oder, wobei $R^{3b}$ H, $-CH_3$, $-CH_2-CH_3$ oder $-CH(CH_3)_2$ ist.

**9.** Verbindung oder pharmazeutisch annehmbares Salz davon nach Anspruch 1, wobei die Verbindung der Formel IIIa, IIIb, IIIe, IVa, IVb oder IYc entspricht:

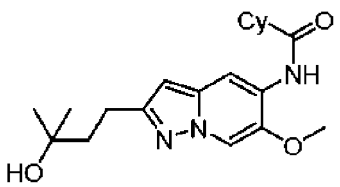
IIIa,

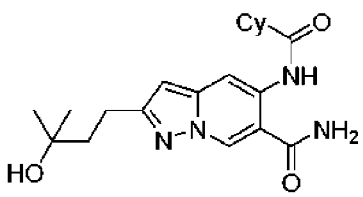
IIIb,

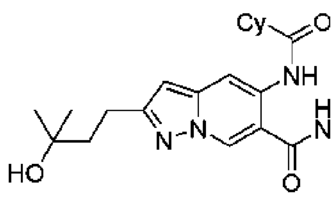
IIIc,

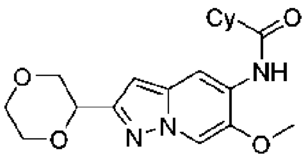

Iva,

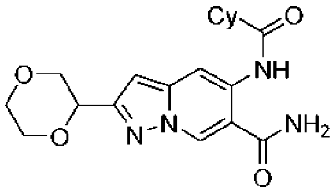

IVb,

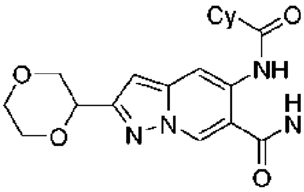

IVc.

**10.** Verbindung oder pharmazeutisch annehmbares Salz davon nach einem der Ansprüche 1-9, wobei Cy Pyridinyl oder Pyrazinyl ist, die jeweils mit einem oder zwei unabhängig voneinander ausgewählten $R^4$-Substituenten substituiert sind.

**11.** Verbindung oder pharmazeutisch annehmbares Salz davon nach Anspruch 10, wobei jedes $R^4$ unabhängig ausgewählt ist aus Oxo, -OH, -CN, F, Cl, $-CH_3$, $-CH_2-CH_3$, $-CH(CH_3)_2$, $-CF_3$, $-CHF_3$, $-CH_2CF_3$, $-CH_2CN$, $-CH_2OH$, $-CH_2CH_2-CN$, $-O-CH_2-CH_3$, Cyclopropyl, Cyclobutyl, Cyclopropyl substituiert mit einem oder zwei unabhängig ausgewählten F oder -CN, Cyclobutyl substituiert mit einem oder zwei unabhängig ausgewählten -F, -OH oder -CN.

**12.** Pharmazeutische Zusammensetzung, umfassend eine Verbindung nach einem der Ansprüche 1 bis 11 und einen pharmazeutisch annehmbaren Träger davon.

**13.** Pharmazeutische Zusammensetzung nach Anspruch 12, die ferner ein weiteres therapeutisches Mittel umfasst.

**14.** Verbindung oder pharmazeutisch annehmbares Salz gemäß den Ansprüchen 1-11 oder eine pharmazeutische Zusammensetzung gemäß Anspruch 12 oder 13 zur Verwendung in der Medizin.

**15.** Verbindung oder pharmazeutisch annehmbares Salz gemäß den Ansprüchen 1-11 oder eine pharmazeutische Zusammensetzung gemäß Anspruch 12 oder 13 zur Verwendung bei der Prophylaxe und/oder Behandlung von Entzündungskrankheiten, Autoimmunkrankheiten, Schmerzen, Fibrose und/oder proliferativen Krankheiten.

**Revendications**

**1.** Composé selon la formule I :

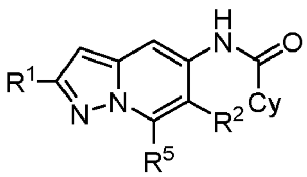

I

dans lequel

$R^1$ représente

a) un alkyle en $C_2$ à $C_6$ substitué par un ou plusieurs groupes -OH, -CN, alkoxy en $C_1$ à $C_4$, halogéno ou $-S(=O)_2$-alkyle en $C_1$ à $C_4$ choisis indépendamment, ou
b) un hétérocycloalkyle hexagonal comprenant un ou deux atomes de S, N ou O choisis indépendamment, lequel hétérocycloalkyle est non substitué ou substitué par un ou plusieurs groupes oxo, halogéno ou alkyle en $C_1$ à $C_4$ choisis indépendamment, lequel alkyle est non substitué ou substitué par un ou plusieurs groupes halogéno ;

$R^2$ représente

a) un alkoxy en $C_1$ à $C_4$, lequel alkoxy est non substitué ou substitué par un ou plusieurs groupes halogéno

ou -OH choisis indépendamment,
b) un -O-cycloalkyle en $C_3$ ou $C_4$, lequel cycloalkyle est non substitué ou substitué par un ou plusieurs groupes halogéno ou -OH choisis indépendamment, ou
c) $-C(=O)NR^{3a}R^{3b}$ ;

Cy représente un hétéroaryle hexagonal, comprenant 1 ou 2 atomes de N, substitué par un ou deux substituants $R^4$ choisis indépendamment ;
chaque $R^{3a}$ et $R^{3b}$ est choisi indépendamment parmi

a) H,
b) un alkyle en $C_1$ à $C_4$, lequel alkyle est non substitué ou substitué par un ou plusieurs groupes halogéno, -OH, -CN, alkoxy en $C_1$ à $C_4$ ou cycloalkyle en $C_3$ à $C_7$ choisis indépendamment, lequel cycloalkyle est non substitué ou substitué par un ou plusieurs groupes halogéno choisis indépendamment,
c) un cycloalkyle en $C_3$ à $C_6$, lequel cycloalkyle est non substitué ou substitué par un ou plusieurs groupes oxo, -OH, -CN, alkyle en $C_1$ à $C_4$, alkoxy en $C_1$ à $C_4$ ou halogéno choisis indépendamment, ou
d) un hétérocycloalkyle tétra- à hexagonal comprenant un ou deux atomes de N, S ou O choisis indépendamment, lequel hétérocycloalkyle est non substitué ou substitué par un ou plusieurs groupes choisis indépendamment parmi oxo, -OH, -CN, alkyle en $C_1$ à $C_4$, alkoxy en $C_1$ à $C_4$ ou halogéno ; ou

$R^{3a}$ et $R^{3b}$, conjointement avec l'atome de N auquel ils sont fixés, forment un hétérocycloalkyle monocyclique tétra- à hexagonal ;
chaque $R^4$ représente indépendamment

a) oxo,
b) -OH,
c) -CN,
d) halogéno,
e) un alkyle en $C_1$ à $C_4$ non substitué ou substitué par un ou plusieurs groupes halogéno, -OH ou -CN choisis indépendamment,
f) un alkoxy en $C_1$ à $C_4$ non substitué ou substitué par un ou plusieurs groupes halogéno, -OH ou -CN choisis indépendamment, ou
g) un cycloalkyle en $C_3$ à $C_7$ non substitué ou substitué par un ou plusieurs groupes halogéno, -OH ou -CN choisis indépendamment ; et

$R^5$ est choisi parmi H, halogéno, $-CH_3$ ou $-CF_3$ ;
ou un de ses sels pharmaceutiquement acceptables, ou un solvate ou le sel de son solvate.

**2.** Composé ou un de ses sels pharmaceutiquement acceptables selon la revendications 1, dans lequel $R^1$ représente un alkyle en $C_2$ à $C_6$ substitué par un ou plusieurs groupes -OH, -CN, $-OCH_3$, F, Cl ou $-S(=O)_2CH_3$ choisis indépendamment.

**3.** Composé ou un de ses sels pharmaceutiquement acceptables selon la revendications 1, dans lequel $R^1$ représente un groupe tétrahydropyranyle, dioxanyle, morpholinyle, pipéridinyle, pipérazinyle, thiomorpholinyle ou 1,4-oxathianyle.

**4.** Composé ou un de ses sels pharmaceutiquement acceptables selon la revendications 1, le composé répondant à la formule II or IIb :

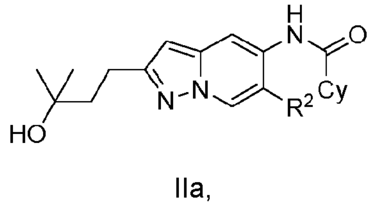

IIa,

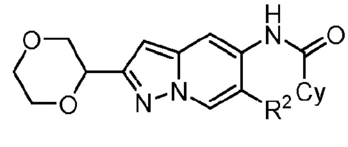

IIb

**5.** Composé ou un de ses sels pharmaceutiquement acceptables selon l'une quelconque des revendications 1 à 4, dans lequel $R^2$ représente $-OCH_3$ ou $-OCH_2CH_3$, chacun d'eux étant non substitué ou substitué par un ou plusieurs

groupes halogéno ou -OH choisis indépendamment.

6. Composé ou un de ses sels pharmaceutiquement acceptables selon l'une quelconque des revendications 1 à 4, dans lequel $R^2$ représente $-C(=O)NR^{3a}R^{3b}$.

7. Composé ou un de ses sels pharmaceutiquement acceptables selon la revendication 6, dans lequel $R^{3a}$ représente H, $-CH_3$, $-CH_2-CH_3$ ou $-CH(CH_3)_2$.

8. Composé ou un de ses sels pharmaceutiquement acceptables selon la revendication 6 ou 7, dans lequel $R^{3b}$ représente H, $-CH_3$, $-CH_2-CH_3$ ou $-CH(CH_3)_2$.

9. Composé ou un de ses sels pharmaceutiquement acceptables selon la revendication 1, le composé répondant à la formule IIIa, IIIb, IIIc, IVa, IVb, or IVc :

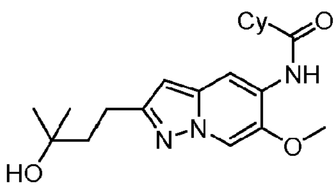

IIIa,

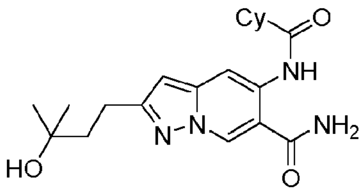

IIIb,

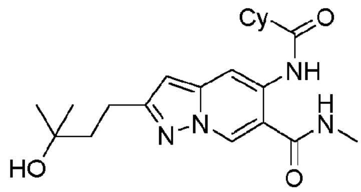

IIIc,

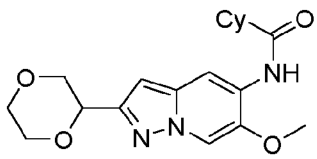

Iva,

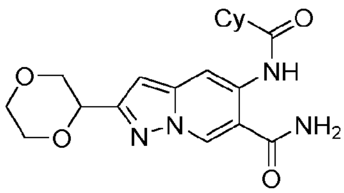

IVb,

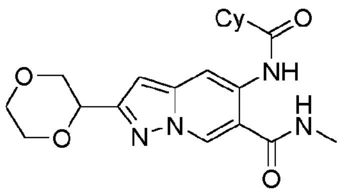

IVc.

10. Composé ou un de ses sels pharmaceutiquement acceptables selon l'une quelconque des revendications 1 à 9, dans lequel Cy représente un groupe pyridinyle ou pyrazinyle, chacun d'eux étant substitué par un ou deux substituants $R^4$ choisis indépendamment.

11. Composé ou un de ses sels pharmaceutiquement acceptables selon la revendication 10, dans lequel chaque $R^4$ est choisi indépendamment parmi les groupes oxo, -OH, -CN, F, Cl, $-CH_3$, $-CH_2-CH_3$, $-CH(CH_3)_2$, $-CF_3$, $-CHF_3$, $-CH_2CF_3$, $-CH_2CN$, $-CH_2OH$, $-CH_2CH_2-CN$, $-O-CH_2-CH_3$, cyclopropyle, cyclobutyle, cyclopropyle substitué par un ou deux F ou -CN choisis indépendamment, cyclobutyle substitué par un ou deux F, -OH ou -CN choisis indépendamment.

12. Composition pharmaceutique comprenant un composé selon l'une quelconque des revendications 1 à 11, et un de ses véhicules pharmaceutiquement acceptables.

13. Composition pharmaceutique selon la revendication 12, comprenant en outre un agent thérapeutique supplémentaire.

14. Composé ou un de ses sels pharmaceutiquement acceptables selon les revendications 1 à 11, ou composition pharmaceutique selon la revendication 12 ou 13, pour une utilisation en médecine.

15. Composé ou un de ses sels pharmaceutiquement acceptables selon les revendications 1 à 11, ou composition pharmaceutique selon la revendication 12 ou 13, pour une utilisation dans la prophylaxie et/ou le traitement de maladies inflammatoires, de maladies auto-immunes, de la douleur, de la fibrose et/ou de maladies prolifératives.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012129258 A
- WO 2018083085 A
- WO 2017009798 A


### Non-patent literature cited in the description


- Remington's Pharmaceutical Sciences. Mack Publishing Company, 1985
- *CHEMICAL ABSTRACTS,* 148893-10-1
- *CHEMICAL ABSTRACTS,* 1026249-18-2
- **ARGILÉS, J.M ; LOPEZ-SORIANO, F.J.** Catabolic proinflammatory cytokines. *Curr. Opin. Clin. Nutr. Metab. Care,* 1998, vol. 1, 245-251
- **BAIN, J ; MCLAUCHLAN, H. ; ELLIOTT, M ; COHEN, P.** The specificities of protein kinase inhibitors: an update. *Biochem. J.,* 2003, vol. 371, 199-204
- **BREHM, M.A ; DANIELS, K.A. ; WELSH, R.M.** Rapid Production of TNF-$\alpha$ following TCR Engagement of Naive CD8 T Cells. *J. Immunol.,* 2005, vol. 175, 5043-5049
- **BROEKMAN, F ; GIOVANNETTI, E. ; PETERS, G.J.** Tyrosine kinase inhibitors: Multi-targeted or single-targeted. *World J. Clin. Oncol.,* 2011, vol. 2, 80-93
- **BUNDGAARD, H.** Design of prodrugs. Elsevier, 1985
- **BUSH, K.A. ; FARMER, K.M ; WALKER, J.S ; KIRKHAM, B.W.** Reduction of joint inflammation and bone erosion in rat adjuvant arthritis by treatment with interleukin-17 receptor IgG1 Fc fusion protein. *Arthritis Rheum.,* 2002, vol. 46, 802-805
- **CARMI, Y ; DOTAN, S ; RIDER, P. ; KAPLANOV, I ; WHITE, M.R ; BARON, R. ; ABUTBUL, S ; HUSZAR, M ; DINARELLO, C.A ; APTE, R.N et al.** The Role of IL-1$\beta$ in the Early Tumor Cell-Induced Angiogenic Response. *J. Immunol.,* 2013, vol. 190, 3500-3509
- **CHIANG, E.Y. ; YU, X. ; GROGAN, J.L.** Immune Complex-Mediated Cell Activation from Systemic Lupus Erythematosus and Rheumatoid Arthritis Patients Elaborate Different Requirements for IRAK1/4 Kinase Activity across Human Cell Types. *J. Immunol.,* 2011, vol. 186, 1279-1288
- **COHEN, P.** Targeting protein kinases for the development of anti-inflammatory drugs. *Curr. Opin. Cell Biol.,* 2009, vol. 21, 317-324
- **DAVIDSON, D.J. ; CURRIE, A.J. ; BOWDISH, D.M.E. ; BROWN, K.L. ; ROSENBERGER, C.M. ; MA, R.C. ; BYLUND, J. ; CAMPSALL, P.A. ; PUEL, A. ; PICARD, C. et al.** IRAK-4 mutation (Q293X): rapid detection, and characterisation of defective post-transcriptional TLR/IL-1R responses in human myeloid and non-myeloid cells. *J. Immunol. Baltim. Md,* 1950, vol. 177, 8202-8211
- **DINARELLO, C.A. ; SIMON, A ; VAN DER MEER, J.W.M.** Treating inflammation by blocking interleukin-1 in abroad spectrum of diseases. *Nat. Rev. Drug Discov.,* 2012, vol. 11, 633-652
- **DY, G.K ; ADJEI, A.A.** Understanding, recognizing, and managing toxicities of targeted anticancer therapies. CA. *Cancer J. Clin.,* 2013, vol. 63, 249-279
- **FABIAN, M.A ; BIGGS, W.H. ; TREIBER, D.K ; ATTERIDGE, C.E. ; AZIMIOARA, M.D ; BENEDETTI, M.G ; CARTER, T.A. ; CICERI, P. ; EDEEN, P.T. ; FLOYD, M et al.** A small molecule-kinase interaction map for clinical kinase inhibitors. *Nat. Biotechnol.,* 2005, vol. 23, 329-336
- **VAN DER FITS, L. ; MOURITS, S. ; VOERMAN, J.S.A. ; KANT, M ; BOON, L. ; LAMAN, J.D. ; CORNELISSEN, F ; MUS, A.-M ; FLORENCIA, E. ; PRENS, E.P et al.** Imiquimod-Induced Psoriasis-Like Skin Inflammation in Mice Is Mediated via the IL-23/IL-17 Axis. *J. Immunol.,* 2009, vol. 182, 5836-5845
- **FORCE, T. ; KOLAJA, K.L.** Cardiotoxicity of kinase inhibitors: the prediction and translation of preclinical models to clinical outcomes. *Nat. Rev. Drug Discov.,* 2011, vol. 10, 111-126
- **GENOVESE, M.C. ; COHEN, S. ; MORELAND, L ; LIUM, D. ; ROBBINS, S. ; NEWMARK, R ; BEKKER, P.** Combination therapy with etanercept and anakinra in the treatment of patients with rheumatoid arthritis who have been treated unsuccessfully with methotrexate. *Arthritis Rheum.,* 2004, vol. 50, 1412-1419

- **JAIN, A. ; KACZANOWSKA, S ; DAVILA, E.** IL-1 Receptor-Associated Kinase Signaling and Its Role in Inflammation. *Cancer Progression, and Therapy Resistance. Front. Immunol.,* 2014, vol. 5
- **JANUSZ, M.J ; HOOKFIN, E.B. ; HEITMEYER, S.A. ; WOESSNER, J.F. ; FREEMONT, A.J. ; HOYLAND, J.A. ; BROWN, K.K ; HSIEH, L.C ; ALMSTEAD, N.G. ; DE, B. et al.** Moderation of iodoacetate-induced experimental osteoarthritis in rats by matrix metalloproteinase inhibitors. *Osteoarthritis Cartilage,* 2001, vol. 9, 751-760
- **JOU, I.-M ; SHIAU, A.-L ; CHEN, S.-Y. ; WANG, C.-R ; SHIEH, D.-B. ; TSAI, C.-S. ; WU, C.-L.** Thrombospondin 1 as an effective gene therapeutic strategy in collagen-induced arthritis. *Arthritis Rheum.,* 2005, vol. 52, 339-344
- **KHACHIGIAN, L.M.** Collagen antibody-induced arthritis. *Nat. Protoc.,* 2006, vol. 1, 2512-2516
- **KLEIN, S.** The Use of Biorelevant Dissolution Media to Forecast the In Vivo Performance of a Drug. *AAPS J.,* 2010, vol. 72, 397-406
- **KOZICZAK-HOLBRO, M ; LITTLEWOOD-EVANS, A ; PÖLLINGER, B. ; KOVARIK, J. ; DAWSON, J. ; ZENKE, G ; BURKHART, C. ; MÜLLER, M ; GRAM, H.** The critical role of kinase activity of interleukin-1 receptor-associated kinase 4 in animal models of joint inflammation. *Arthritis Rheum.,* 2009, vol. 60, 1661-1671
- **VAN DER KRAAN, P.M. ; VITTERS, E.L ; VAN DE PUTTE, L.B ; VAN DEN BERG, W.B.** Development of osteoarthritic lesions in mice by ''metabolic'' and ''mechanical'' alterations in the knee joints. *Am. J. Pathol.,* 1989, vol. 135, 1001-1014
- **KROEGER, K.M ; SULLIVAN, B.M ; LOCKSLEY, R.M.** IL-18 and IL-33 elicit Th2 cytokines from basophils via a MyD88- and p38$\alpha$-dependent pathway. *J. Leukoc. Biol.,* 2009, vol. 86, 769-778
- **LI, D ; GUABIRABA, R. ; BESNARD, A.-G. ; KOMAI-KOMA, M. ; JABIR, M.S ; ZHANG, L ; GRAHAM, G.J. ; KUROWSKA-STOLARSKA, M. ; LIEW, F.Y ; MCSHARRY, C. et al.** IL-33 promotes ST2-dependent lung fibrosis by the induction of alternatively activated macrophages and innate lymphoid cells in mice. *J. Allergy Clin. Immunol.,* 2014, vol. 134, 1422-1432
- **LI, M ; HENER, P ; ZHANG, Z. ; KATO, S. ; METZGER, D ; CHAMBON, P.** Topical vitamin D3 and low-calcemic analogs induce thymic stromal lymphopoietin in mouse keratinocytes and trigger an atopic dermatitis. *Proc. Natl. Acad. Sci.,* 2006, vol. 103, 11736-11741
- **LI, S. ; STRELOW, A. ; FONTANA, E.J. ; WESCHE, H.** IRAK-4: A novel member of the IRAK family with the properties of an IRAK-kinase. *Proc. Natl. Acad. Sci. U. S. A.,* 2002, vol. 99, 5567-5572
- **LI, Z ; YOUNGER, K. ; GARTENHAUS, R ; JOSEPH, A.M. ; HU, F. ; BAER, M.R ; BROWN, P ; DAVILA, E.** Inhibition of IRAK1/4 sensitizes T cell acute lymphoblastic leukemia to chemotherapies. *J. Clin. Invest,* 2015, vol. 125, 1081-1097
- **LIN, H.-S. ; HU, C.-Y. ; CHAN, H.-Y. ; LIEW, Y.-Y. ; HUANG, H.-P. ; LEPESCHEUX, L. ; BASTIANELLI, E. ; BARON, R. ; RAWADI, G ; CLÉMENT-LACROIX, P.** Anti-rheumatic activities of histone deacetylase (HDAC) inhibitors in vivo in collagen-induced arthritis in rodents. *Br. J. Pharmacol.,* 2007, vol. 150, 862-872
- **MCHEDLIDZE, T ; WALDNER, M. ; ZOPF, S ; WALKER, J. ; RANKIN, A.L. ; SCHUCHMANN, M. ; VOEHRINGER, D ; MCKENZIE, A.N.J ; NEURATH, M.F. ; PFLANZ, S. et al.** Interleukin-33-dependent innate lymphoid cells mediate hepatic fibrosis. *Immunity,* 2013, vol. 39, 357-371
- **NABE, T.** Interleukin (IL)-33: New Therapeutic Target for Atopic Diseases. *J. Pharmacol. Sci.,* 2014, vol. 126, 85-91
- **NGO, V.N. ; YOUNG, R.M. ; SCHMITZ, R. ; JHAVAR, S. ; XIAO, W ; LIM, K.-H ; KOHLHAMMER, H. ; XU, W ; YANG, Y. ; ZHAO, H et al.** Oncogenically active MYD88 mutations in human lymphoma. *Nature,* 2011, vol. 470, 115-119
- **NISHIDA, K. ; KOMIYAMA, T ; MIYAZAWA, S ; SHEN, Z.-N. ; FURUMATSU, T ; DOI, H. ; YOSHIDA, A ; YAMANA, J. ; YAMAMURA, M ; NINOMIYA, Y et al.** Histone deacetylase inhibitor suppression of autoantibody-mediated arthritis in mice via regulation of p16INK4a and p21WAF1/Cip1 expression. *Arthritis Rheum.,* 2004, vol. 50, 3365-3376
- **RAIL, L.C ; ROUBENOFF, R.** Rheumatoid cachexia: metabolic abnormalities, mechanisms and interventions. *Rheumatology,* 2004, vol. 43, 1219-1223
- **RANKIN, A.L. ; MUMM, J.B. ; MURPHY, E. ; TURNER, S. ; YU, N. ; MCCLANAHAN, T.K. ; BOURNE, P.A. ; PIERCE, R.H. ; KASTELEIN, R ; PFLANZ, S.** IL-33 Induces IL-13-Dependent Cutaneous Fibrosis. *J. Immunol.,* 2010, vol. 184, 1526-1535
- **RHYASEN, G.W ; STARCZYNOWSKI, D.T.** IRAK signalling in cancer. *Br. J. Cancer,* 2015, vol. 112, 232-237
- **RINGWOOD, L ; LI, L.** The involvement of the interleukin-1 Receptor-Associated Kinases (IRAKs) in cellular signaling networks controlling inflammation. *Cytokine,* 2008, vol. 42, 1-7
- **RIZZO, H.L. ; KAGAMI, S ; PHILLIPS, K.G. ; KURTZ, S.E. ; JACQUES, S.L ; BLAUVELT, A.** IL-23-Mediated Psoriasis-Like Epidermal Hyperplasia Is Dependent on IL-17A. *J. Immunol.,* 2011, vol. 186, 1495-1502

- **SALIMI, M. ; BARLOW, J.L. ; SAUNDERS, S.P. ; XUE, L. ; GUTOWSKA-OWSIAK, D. ; WANG, X ; HUANG, L.-C ; JOHNSON, D ; SCANLON, S.T. ; MCKENZIE, A.N.J. et al.** A role for IL-25 and IL-33-driven type-2 innate lymphoid cells in atopic dermatitis. *J. Exp. Med.,* 2013, vol. 210, 2939-2950
- **SALVEMINI, D ; MAZZON, E. ; DUGO, L. ; SERRAINO, I. ; DE SARRO, A. ; CAPUTI, A.P. ; CUZZOCREA, S.** Amelioration of joint disease in a rat model of collagen-induced arthritis by M40403, a superoxide dismutase mimetic. *Arthritis Rheum.,* 2001, vol. 44, 2909-2921
- **SELTZER, Z. ; DUBNER, R. ; SHIR, Y.** A novel behavioral model of neuropathic pain disorders produced in rats by partial sciatic nerve injury. *Pain,* 1990, vol. 43, 205-218
- **SHELTON, D.L ; ZELLER, J. ; HO, W.-H. ; PONS, J. ; ROSENTHAL, A.** Nerve growth factor mediates hyperalgesia and cachexia in auto-immune arthritis. *Pain,* 2005, vol. 116, 8-16
- **SIMS, N.A. ; GREEN, J.R. ; GLATT, M. ; SCHLICT, S. ; MARTIN, T.J. ; GILLESPIE, M.T. ; ROMAS, E.** Targeting osteoclasts with zoledronic acid prevents bone destruction in collagen-induced arthritis. *Arthritis Rheum.,* 2004, vol. 50, 2338-2346
- **STASCHKE, K.A. ; DONG, S ; SAHA, J. ; ZHAO, J. ; BROOKS, N.A. ; HEPBUM, D.L. ; XIA, J. ; GULEN, M.F ; KANG, Z ; ALTUNTAS, C.Z. et al.** IRAK4 Kinase Activity Is Required for Th17 Differentiation and Th17-Mediated Disease. *J. Immunol.,* 2009, vol. 183, 568-577
- **SUNDBERG, T.B ; XAVIER, R.J ; SCHREIBER, S.L. ; SHAMJI, A.F.** Small-molecule control of cytokine function: new opportunities for treating immune disorders. *Curr. Opin. Chem. Biol.,* 2014, vol. 23, 23-30
- **TREON, S.P ; XU, L. ; YANG, G. ; ZHOU, Y. ; LIU, X. ; CAO, Y. ; SHEEHY, P. ; MANNING, R.J ; PATTERSON, C.J. ; TRIPSAS, C. et al.** MYD88 L265P Somatic Mutation in Waldenström's Macroglobulinemia. *N. Engl. J. Med.,* 2012, vol. 367, 826-833
- **VIDAL-VANACLOCHA, F ; FANTUZZI, G ; MENDOZA, L ; FUENTES, A M ; ANASAGASTI, M.J. ; MARTIN, J. ; CARRASCAL, T. ; WALSH, P ; REZNIKOV, L.L ; KIM, S.-H et al.** IL-18 regulates IL-1β-dependent hepatic melanoma metastasis via vascular cell adhesion molecule-1. *Proc. Natl. Acad. Sci.,* 2000, vol. 97, 734-739
- **WALSMITH, J. ; ABAD, L ; KEHAYIAS, J. ; ROUBENOFF, R.** Tumor necrosis factor-alpha production is associated with less body cell mass in women with rheumatoid arthritis. *J. Rheumatol.,* 2004, vol. 31, 23-29
- **WANG, Z ; WESCHE, H ; STEVENS, T ; WALKER, N. ; YEH, W.-C.** IRAK-4 Inhibitors for Inflammation. *Curr. Top. Med. Chem.,* 2009, vol. 9, 724-737
- **YAN, C. ; ZHU, M. ; STAIGER, J. ; JOHNSON, P.F ; GAO, H.** C5a-regulated CCAAT/Enhancer-binding Proteins β and δ Are Essential in Fcγ Receptor-mediated Inflammatory Cytokine and Chemokine Production in Macrophages. *J. Biol. Chem.,* 2012, vol. 287, 3217-3230
- **YOKOGAWA, M ; TAKAISHI, M. ; NAKAJIMA, K. ; KAMIJIMA, R. ; FUJIMOTO, C ; KATAOKA, S ; TERADA, Y ; SANO, S.** Epicutaneous Application of Toll-like Receptor 7 Agonists Leads to Systemic Autoimmunity in Wild-Type Mice: A New Model of Systemic Lupus Erythematosus. *Arthritis Rheumatol.,* 2014, vol. 66, 694-706
- **ZEGZOUTI, H. ; ZDANOVSKAIA, M. ; HSIAO, K ; GOUELI, S.A.** ADP-Glo: A Bioluminescent and Homogeneous ADP Monitoring Assay for Kinases. *ASSAY Drug Dev. Technol.,* 2009, vol. 7, 560-572
- **ZERR, P. ; DISTLER, A. ; PALUMBO-ZERR, K. ; TOMCIK, M. ; VOLLATH, S ; DEES, C. ; EGBERTS, F ; TINAZZI, I ; DEL GALDO, F ; DISTLER, O. et al.** Combined Inhibition of c-Abl and PDGF Receptors for Prevention and Treatment of Murine Sclerodermatous Chronic Graft-versus-Host Disease. *Am. J. Pathol.,* 2012, vol. 181, 1672-1680
- *Remington's pharmaceutical sciences,* 1985